# EUROPEAN PATENT APPLICATION

(11) **EP 4 484 434 A1**
(43) Date of publication of application: **01.01.2025**
(21) Application number: 23759202.7
(22) Date of filing: 22.02.2023
(51) Int. Cl.: C07D 519/00, C07D 471/04, A61K 31/4035, A61K 31/407, A61K 31/4164, A61K 31/4188, A61K 31/4355, A61K 31/437, A61K 31/4985, A61K 31/5025, A61P 35/00, A61P 35/02

(54) **FUSED BICYCLIC COMPOUND CONTAINING PYRROLINONE**

(30) Priority: 23.02.2022 CN 202210166821; 18.10.2022 CN 202211272746; 14.02.2023 CN 202310115154
(71) Applicant: Chia Tai Tianqing Pharmaceutical Group Co., Ltd, Lianyungang, Jiangsu 222062 (CN)
(72) Inventor: ZHANG, Yinsheng, Lianyungang, Jiangsu 222062 (CN); GAO, Yong, Lianyungang, Jiangsu 222062 (CN); YIN, Yuan, Lianyungang, Jiangsu 222062 (CN); SHI, Wei, Lianyungang, Jiangsu 222062 (CN); WANG, Chengqi, Lianyungang, Jiangsu 222062 (CN)
(74) Representative: Engelhard, Markus
(86) International application number: PCT/CN2023/077638
(87) International publication number: WO 2023/160577

(57) **Abstract**

The present application relates to the field of pharmaceutical chemistry, relates to a fused bicyclic compound containing pyrrolinone, and in particular relates to a compound represented by formula (II), a stereoisomer or a pharmaceutically acceptable salt thereof, a preparation method therefor, a pharmaceutical composition containing the compound, and the use thereof in the treatment of diseases (such as cancer).

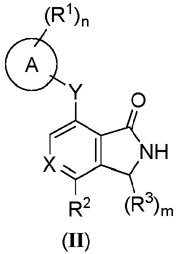

## Description

### CROSS-REFERENCE TO RELATED APPLICATIONS

The present application claims priority and benefits to the following Chinese patent applications filed with the China National Intellectual Property Administration, the contents of which are incorporated herein by reference in their entireties:
Chinese Patent Application No. 202210166821.7, filed on February 23, 2022;
Chinese Patent Application No. 202211272746.9, filed on October 18, 2022; and
Chinese Patent Application No. 202310115154.4, filed on February 14, 2023.

### TECHNICAL FIELD

The present application relates to a fused bicyclic compound containing pyrrolinone, a preparation method therefor, a pharmaceutical composition containing the compound, and use thereof for treating a disease, e.g., cancer.

### BACKGROUND

Hematopoietic progenitor kinase 1(HPK1), also known as mitogen-activated protein kinase 1 (MAP4K1), is a mammalian Ste20-related serine/threonine protein kinase, which is a microtubule-associated protein and is a member of the mitogen-activated protein kinase (MAP4K) family. The MAP4K family also includes five substypes: GCK/MAP4K2, GLK/MAP4K3, HGK/MAP4K4, KHS1/MAP4K5, and MINK1/MAP4K6. Unlike the other five MAP4K subtypes that are extensively expressed in histiocytes, HPK1 is only expressed in hematopoietic histiocytes and can be involved in regulating the signaling of the hematopoietic system including lymphocytes by mediating multiple cell signaling pathways (including MAPK signaling, antigen receptor signaling, and cytokine signaling, etc.).

Research shows that HPK1 functions mainly through the c-Jun N-terminal kinase (JNK) and extracellular regulated protein kinases (ERK) signaling pathways, inhibiting immune cell responses. In T cells, after T cell receptor (TCR) proteins are activated, HPK1 interacts with a large number of TCRs and is phosphorylated by tyrosine kinases Lck and Zap70, and the activated HPK1 further phosphorylates T cell receptor adaptor protein SLP-76 to establish a docking site for negative regulatory factor 14-3-3 and finally undermines the stability of the TCR signaling complex (lato-gads-SLP76) and blocks the transmission of downstream mitogen-activated protein (MAP) kinase signals, negatively regulating TCR signaling and further inhibiting T cell proliferation. In B cells, a similar negative feedback mechanism also exists. B cell receptor (BCR) signals are transmitted through HPK1-mediated phosphorylation and activated B cell linker protein (BLNK), so that downstream signal transmission is blocked and B cell proliferation is inhibited. In addition, HPK1 also has a negative feedback regulatory effect on NK (natural killer) cells and dendritic cells (DCs).

There have now been no HPK1-targeted drugs available on the market. There remains a need in the art to develop compounds with selective inhibitory activity, better pharmacodynamics, or better pharmacokinetics.

### SUMMARY

In one aspect, the present application relates to a compound of formula (II), a stereoisomer thereof, or a pharmaceutically acceptable salt thereof, 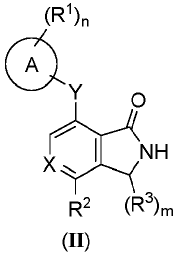 wherein,
X is selected from the group consisting of N and CH;
Y is selected from the group consisting of a bond, -O-, -S-, -NR^{a}-, -C(R^{a})₂-, -S(O)₂-, -S(O)₂NR^{a}-, -S(O)-, -S(O)NR^{a}-, -C(O)-, -C(O)O-, -C(O)NR^{a}-, -C(O)N(R^{a})O-, -OC(O)-, -OC(O)NR^{a}-, -N(R^{a})C(O)O-, -N(R^{a})C(O)-, and - N(R^{a})S(O)₂-;
ring A is selected from the group consisting of 3- to 10-membered heterocyclyl, C₅₋₁₀ aryl, and 5- to 10-membered heteroaryl;
R¹ are each independently selected from the group consisting of -NH₂, -NHR^{d}, -N(R^{d})₂, -OH, -OR^{d}, -CN, halogen, -COOR^{d}, -OCOR^{d}, -N(R^{d})C(O)(R^{d}), -CONH(R^{d}), -CON(R^{d})₂, -NHSO₂(R^{d}), -SO₂NH(R^{d}), -SO₂N(R^{d})₂, -PO(R^{d})₂, 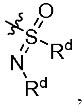 C₁₋₆ alkyl, C₅₋₁₀ aryl, 5- to 10-membered heteroaryl, and 3- to 10-membered heterocyclyl, wherein the C₁₋₆ alkyl, C₅₋₁₀ aryl, 5- to 10-membered heteroaryl, or 3- to 10-membered heterocyclyl is optionally substituted with one or more R^{b};
R^{d} are each independently selected from the group consisting of C₁₋₄ alkyl and C₃₋₇ cycloalkyl;
R² is selected from the group consisting of 5- to 6-membered monocyclic heteroaryl and a 9- to 14-membered saturated, partially saturated, or aromatic tricyclic ring, wherein the tricyclic ring contains 0-6 heteroatoms independently selected from the group consisting of N, O, and S, and R² is optionally substituted with one or more R^{c};
R³ are each independently selected from the group consisting of halogen, C₁₋₄ alkyl, and halogenated C₁₋₄ alkyl;
R^{a} are each independently selected from the group consisting of hydrogen and C₁₋₄ alkyl;
R^{b} are each independently selected from the group consisting of deuterium, -NH₂, -NH(C₁₋₄ alkyl), -N(C₁₋₄ alkyl)₂, -OH, -OC₁₋₄ alkyl, -CN, halogen, 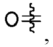 C₁₋₄ alkyl, and 3- to 6-membered heterocycloalkyl, wherein the -NH(C₁₋₄ alkyl), -N(C₁₋₄ alkyl)₂, -OC₁₋₄ alkyl, C₁₋₄ alkyl, and 3- to 6-membered heterocycloalkyl are optionally substituted with one or more deuterium, halogen, or 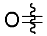 substituents;
R^{c} are each independently selected from the group consisting of -NH₂, -NH(C₁₋₄ alkyl), -N(C₁₋₄ alkyl)₂, -OH, -OC₁₋₄ alkyl, halogen, 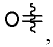 and C₁₋₆ alkyl optionally substituted with one or more deuterium;
n is selected from the group consisting of 1, 2, 3, and 4;
m is selected from the group consisting of 0, 1, and 2;
each X, Y, ring A, R¹, R^{d}, R², R³, R^{a}, R^{b}, or R^{c} is independently optionally substituted with one or more substituents.

In another aspect, the present application relates to a compound of formula (I), a stereoisomer thereof, or a pharmaceutically acceptable salt thereof, wherein 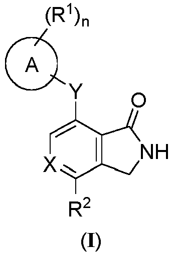 wherein,
X is selected from the group consisting of N and CH;
Y is selected from the group consisting of a bond, -O-, -S-, and -NR^{a}-;
ring A is selected from the group consisting of C₅₋₁₀ aryl and 5- to 10-membered heteroaryl;
R¹ are each independently selected from the group consisting of -NH₂, -NH(C₁₋₄ alkyl), -N(C₁₋₄ alkyl)₂, -OH, -OC₁₋₄ alkyl, -CN, halogen, C₁₋₆ alkyl, and 3- to 10-membered heterocycloalkyl, wherein the C₁₋₆ alkyl and 3- to 10-membered heterocycloalkyl are optionally substituted with one or more R^{b};
R² is selected from the group consisting of 5- to 6-membered monocyclic heteroaryl and a 9- to 14-membered saturated, partially saturated, aromatic tricyclic ring, wherein the tricyclic ring contains 0-6 heteroatoms independently selected from the group consisting of N, O, and S, and R² is optionally substituted with one or more R^{c};
R^{a} is selected from the group consisting of hydrogen and C₁₋₄ alkyl;
R^{b} are each independently selected from the group consisting of -NH₂, -NH(C₁₋₄ alkyl), -N(C₁₋₄ alkyl)₂, -OH, -OC₁₋₄ alkyl, -CN, halogen, 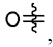 and 3- to 6-membered heterocycloalkyl, wherein the -NH(C₁₋₄ alkyl), -N(C₁₋₄ alkyl)₂, -OC₁₋₄ alkyl, and 3- to 6-membered heterocycloalkyl are optionally substituted with one or more deuterium, halogen, or 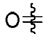 substituents;
R^{c} are each independently selected from the group consisting of -NH₂, -NH(C₁₋₄ alkyl), -N(C₁₋₄ alkyl)₂, -OH, -OC₁₋₄ alkyl, halogen, 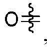 and C₁₋₆ alkyl optionally substituted with one or more deuterium;
n is selected from the group consisting of 1, 2, 3, and 4.

In some embodiments, the present application relates to a compound of formula (II), a stereoisomer thereof, or a pharmaceutically acceptable salt thereof, wherein,
X is selected from the group consisting of N and CH;
Y is selected from the group consisting of a bond, -O-, -S-, and -NR^{a}-;
ring A is selected from the group consisting of C₅₋₁₀ aryl and 5- to 10-membered heteroaryl;
R¹ are each independently selected from the group consisting of -NH₂, -NHR^{d}, -N(R^{d})₂, -OH, -OR^{d}, -CN, halogen, -COOR^{d}, -OCOR^{d}, -N(R^{d})C(O)(R^{d}), -CONH(R^{d}), -CON(R^{d})₂, -NHSO₂(R^{d}), -SO₂NH(R^{d}), -SO₂N(R^{d})₂, -PO(R^{d})₂, 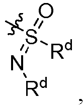 C₁₋₆ alkyl, and 3- to 10-membered heterocycloalkyl, wherein the C₁₋₆ alkyl and 3- to 10-membered heterocycloalkyl are optionally substituted with one or more R^{b};
R^{d} are each independently selected from the group consisting of C₁₋₄ alkyl and C₃₋₇ cycloalkyl;
R² is selected from the group consisting of 5- to 6-membered monocyclic heteroaryl and a 9- to 14-membered saturated, partially saturated, or aromatic tricyclic ring, wherein the tricyclic ring contains 0-6 heteroatoms independently selected from the group consisting of N, O, and S, and R² is optionally substituted with one or more R^{c};
R³ is selected from the group consisting of halogen, C₁₋₄ alkyl, and halogenated C₁₋₄ alkyl;
R^{a} is selected from the group consisting of hydrogen and C₁₋₄ alkyl;
R^{b} are each independently selected from the group consisting of deuterium, -NH₂, -NH(C₁₋₄ alkyl), -N(C₁₋₄ alkyl)₂, -OH, -OC₁₋₄ alkyl, -CN, halogen, 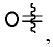 C₁₋₄ alkyl, and 3- to 6-membered heterocycloalkyl, wherein the -NH(C₁₋₄ alkyl), -N(C₁₋₄ alkyl)₂, -OC₁₋₄ alkyl, C₁₋₄ alkyl, and 3- to 6-membered heterocycloalkyl are optionally substituted with one or more deuterium, halogen, or 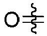 substituents;
R^{c} are each independently selected from the group consisting of -NH₂, -NH(C₁₋₄ alkyl), -N(C₁₋₄ alkyl)₂, -OH, -OC₁₋₄ alkyl, halogen, 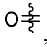 and C₁₋₆ alkyl optionally substituted with one or more deuterium;
n is selected from the group consisting of 1, 2, 3, and 4;
m is selected from the group consisting of 0, 1, and 2.

In some embodiments, Y is selected from the group consisting of a bond, -O-, -S-, and -NR^{a}-; ring A is selected from the group consisting of C₅₋₁₀ aryl and 5- to 10-membered heteroaryl; R¹ are each independently selected from the group consisting of -NH₂, -NHR^{d}, -N(R^{d})₂, -OH, -OR^{d}, -CN, halogen, -COOR^{d}, -OCOR^{d}, -N(R^{d})C(O)(R^{d}), - CONH(R^{d}), -CON(R^{d})₂, -NHSO₂(R^{d}), -SO₂NH(R^{d}), -SO₂N(R^{d})₂, -PO(R^{d})₂, 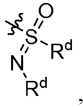 C₁₋₆ alkyl, and 3- to 10-membered heterocycloalkyl, wherein the C₁₋₆ alkyl and 3- to 10-membered heterocycloalkyl are optionally substituted with one or more R^{b}; R³ is selected from the group consisting of halogen, C₁₋₄ alkyl, and halogenated C₁₋₄ alkyl; R^{a} is selected from the group consisting of hydrogen and C₁₋₄ alkyl.

In some embodiments, the heteroaryl and heterocycloalkyl contain 1, 2, 3, or 4 heteroatoms selected from the group consisting of N, O, S, and P, with the remaining ring atoms selected from carbon. In some embodiments, the heteroaryl and heterocycloalkyl contain 1, 2, or 3 heteroatoms selected from the group consisting of N, O, and S, with the remaining ring atoms selected from carbon. In some embodiments, the heteroaryl and heterocycloalkyl contain 1, 2, or 3 heteroatoms selected from the group consisting of N and O, with the remaining ring atoms selected from carbon. In some embodiments, the heteroaryl and heterocycloalkyl contain 1 or 2 heteroatoms selected from the group consisting of N and O, with the remaining ring atoms selected from carbon.

In some embodiments, X is CH. In some embodiments, X is N.

In some embodiments, R^{a} is hydrogen.

In some embodiments, Y is selected from the group consisting of a bond, -O-, -S-, and -NR^{a}-.

In some embodiments, Y is selected from -NR^{a}-. In some embodiments, Y is -NH-.

In some embodiments, ring A is selected from the group consisting of C₅₋₁₀ aryl and 5- to 10-membered heteroaryl.

In some embodiments, ring A is selected from the group consisting of C₅₋₈ aryl and 5- to 8-membered heteroaryl.

In some embodiments, ring A is selected from the group consisting of C₅₋₆ aryl and 5- to 6-membered heteroaryl.

In some other embodiments, ring A is selected from the group consisting of 3- to 10-membered heterocycloalkyl, 5- to 8-membered heterocycloalkyl, and 5- to 6-membered heterocycloalkyl.

In some other embodiments, ring A is selected from the group consisting of phenyl or 5-to 6-membered heteroaryl containing 1 or 2 heteroatoms selected from the group consisting of N, O, and S.

In some embodiments, ring A is selected from the group consisting of phenyl or 6-membered heteroaryl containing 1 or 2 N atoms.

In some other embodiments, ring A is selected from the group consisting of phenyl, pyridinyl, pyrimidinyl, pyridazinyl, pyrazinyl, thiazolyl, imidazolyl, or oxazolyl.

In some embodiments, ring A is selected from the group consisting of phenyl, pyridinyl, pyrimidinyl, pyridazinyl, or pyrazinyl.

In some other embodiments, ring A is selected from the group consisting of phenyl, pyridinyl, or thiazolyl.

In some embodiments, ring A is selected from the group consisting of phenyl or pyridinyl.

In some embodiments, ring A is pyridinyl.

In some other embodiments, ring A is thiazolyl.

In some other embodiments, R¹ are each independently selected from the group consisting of -NH₂, -NH(R^{d}), - N(R^{d})₂, halogen, -N(R^{d})C(O)(R^{d}), -NHSO₂(R^{d}), -SO₂NH(R^{d}), PO(R^{d})₂, 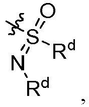 C₁₋₆ alkyl, 5- to 8-membered heteroaryl, and 3- to 10-membered heterocyclyl containing a heteroatom selected from the group consisting of N, O, S, and P, wherein the C₁₋₆ alkyl, 5- to 8-membered heteroaryl, or 3- to 10-membered heterocyclyl containing a heteroatom selected from the group consisting of N, O, S, and P is optionally substituted with one or more R^{b}. In some other embodiments, R¹ are each independently selected from the group consisting of -NH₂, -NH(R^{d}), - N(R^{d})₂, halogen, -N(R^{d})C(O)(R^{d}), -NHSO₂(R^{d}), -SO₂NH(R^{d}), PO(R^{d})₂, 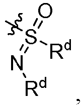 C₁₋₆ alkyl, 3- to 10-membered heterocycloalkyl, 5- to 6-membered heteroaryl, and 5- to 10-membered heterocycloalkenyl, wherein the C₁₋₆ alkyl, 3- to 10-membered heterocycloalkyl, 5- to 6-membered heteroaryl, or 5- to 10-membered heterocycloalkenyl is optionally substituted with one or more R^{b}.

In some embodiments, R¹ are each independently selected from the group consisting of -NH₂, -NHR^{d}, -N(R^{d})₂, - OH, -OR^{d}, -CN, halogen, -COOR^{d}, -OCOR^{d}, -N(R^{d})C(O)(R^{d}), -CONH(R^{d}), -CON(R^{d})₂, -NHSOz(R^{d}), -SO₂NH(R^{d}), -SO₂N(R^{d})₂, -PO(R^{d})₂, 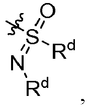 C₁₋₆ alkyl, and 3- to 10-membered heterocycloalkyl, wherein the C₁₋₆ alkyl and 3-to 10-membered heterocycloalkyl are optionally substituted with one or more R^{b}.

In some embodiments, R¹ are each independently selected from the group consisting of -NH₂, -NH(R^{d}), -N(R^{d})₂, halogen, -N(R^{d})C(O)(R^{d}), -NHSO₂(R^{d}), -SO₂NH(R^{d}), PO(R^{d})₂, 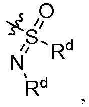 C₁₋₆ alkyl, and 3- to 10-membered heterocycloalkyl, wherein the C₁₋₆ alkyl and 3- to 10-membered heterocycloalkyl are optionally substituted with one or more R^{b}.

In some other embodiments, R¹ are each independently selected from the group consisting of -NH₂, -NH(R^{d}), - N(R^{d})₂, F, Cl, Br, -N(R^{d})C(O)(R^{d}), -NHSO₂(R^{d}), -SO₂NH(R^{d}), -PO(R^{d})₂, 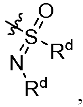 C₁₋₄ alkyl, 5- to 10-membered heterocycloalkyl, and 5- to 8-membered heterocycloalkenyl, wherein the C₁₋₄ alkyl, 5- to 10-membered heterocycloalkyl, or 5- to 8-membered heterocycloalkenyl is optionally substituted with one or more R^{b}.

In some embodiments, R¹ are each independently selected from the group consisting of -NH₂, -NH(R^{d}), -N(R^{d})₂, F, Cl, Br, -N(R^{d})C(O)(R^{d}), -NHSO₂(R^{d}), -SO₂NH(R^{d}), -PO(R^{d})₂, 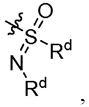 C₁₋₄ alkyl, and 5- to 10-membered heterocycloalkyl, wherein the C₁₋₄ alkyl and 5- to 10-membered heterocycloalkyl are optionally substituted with one or more R^{b}.

In some other embodiments, R¹ are each independently selected from the group consisting of -NH₂, -NH(R^{d}), - N(R^{d})₂, -N(R^{d})C(O)(R^{d}), -NHSO₂(R^{d}), -SO₂NH(R^{d}), -PO(R^{d})₂, 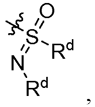 C₁₋₃ alkyl, 5- to 9-membered heterocycloalkyl, and 5- to 6-membered heterocycloalkenyl, wherein the C₁₋₃ alkyl, 5- to 9-membered heterocycloalkyl, or 5- to 6-membered heterocycloalkenyl is optionally substituted with one or more R^{b}.

In some embodiments, R¹ are each independently selected from the group consisting of -NH₂, -NH(R^{d}), -N(R^{d})₂, - N(R^{d})C(O)(R^{d}), -NHSO₂(R^{d}), -SO₂NH(R^{d}), -PO(R^{d})₂, 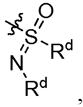 C₁₋₃ alkyl, and 5- to 9-membered heterocycloalkyl, wherein the C₁₋₃ alkyl and 5- to 9-membered heterocycloalkyl are optionally substituted with one or more R^{b}.

In some other embodiments, the heteroatom in the heterocyclyl, heterocycloalkyl, or heterocycloalkenyl of R¹ is selected from the group consisting of N, O, S, and P, or the group consisting of N, O, and S.

In some other embodiments, R¹ are each independently selected from the group consisting of -NH₂, -NHCH₃, - N(CH₃)₂, F, Cl, Br, methyl, ethyl, n-propyl, isopropyl, n-butyl, isobutyl, tert-butyl, tetrahydropyrrolyl, tetrahydropyranyl, tetrahydrofuranyl, tetrahydrothienyl, morpholinyl, piperidinyl, piperazinyl, tetrahydroimidazolyl, azoxybicycloheptyl, azoxyspirononanyl, azoxyspiroheptyl, -N(CH₃)C(O)CH₃, - NHSO₂(CH₃), -SO₂NH(CH₃), -PO(CH₃)₂, 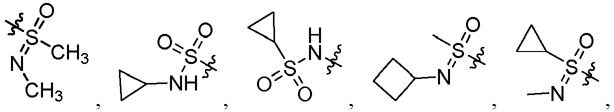 oxazolidinyl, phosphoxycyclohexyl, azoxycyclohexyl, phosphazacyclohexyl, dihydropyridinyl, furanyl, and thiazinyl, wherein the methyl, ethyl, n-propyl, isopropyl, n-butyl, isobutyl, tert-butyl, tetrahydropyrrolyl, tetrahydropyranyl, tetrahydrofuranyl, tetrahydrothienyl, morpholinyl, piperidinyl, piperazinyl, tetrahydroimidazolyl, azoxybicycloheptyl, azoxyspirononanyl, azoxyspiroheptyl, oxazolidinyl, phosphoxycyclohexyl, azoxycyclohexyl, phosphazacyclohexyl, dihydropyridinyl, furanyl, and thiazinyl are optionally substituted with one or more R^{b}.

In some embodiments, R¹ are each independently selected from the group consisting of -NH₂, -NHCH₃, -N(CH₃)₂, F, Cl, Br, methyl, ethyl, n-propyl, isopropyl, n-butyl, isobutyl, tert-butyl, tetrahydropyrrolyl, tetrahydropyranyl, tetrahydrofuranyl, tetrahydrothienyl, morpholinyl, piperidinyl, piperazinyl, tetrahydroimidazolyl, azoxybicycloheptyl, azoxyspirononanyl, azoxyspiroheptyl, -N(CH₃)C(O)CH₃, -NHSO₂(CH₃), -SO₂NH(CH₃), - PO(CH₃)₂, 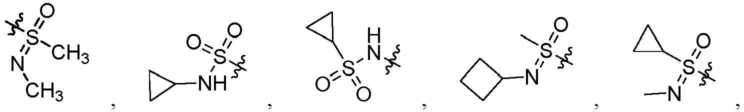 oxazolidinyl, phosphoxycyclohexyl, azoxycyclohexyl, and phosphazacyclohexyl, wherein the methyl, ethyl, n-propyl, isopropyl, n-butyl, isobutyl, tert-butyl, tetrahydropyrrolyl, tetrahydropyranyl, tetrahydrofuranyl, tetrahydrothienyl, morpholinyl, piperidinyl, piperazinyl, tetrahydroimidazolyl, azoxybicycloheptyl, azoxyspirononanyl, azoxyspiroheptyl, oxazolidinyl, phosphoxycyclohexyl, azoxycyclohexyl, or phosphazacyclohexyl is optionally substituted with one or more R^{b}.

In some embodiments, R¹ are each independently selected from the group consisting of -NH₂, F, methyl, ethyl, tetrahydropyrrolyl, tetrahydrofuranyl, tetrahydropyranyl, piperidinyl, morpholinyl, tetrahydroimidazolyl, - N(CH₃)C(O)CH₃, azoxybicycloheptyl, azoxyspirononanyl, azoxyspiroheptyl, phosphoxycyclohexyl, azoxycyclohexyl, phosphazacyclohexyl, oxazolidinyl, -NHSO₂(CH₃), -SO₂NH(CH₃), -PO(CH₃)₂, 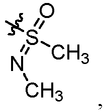 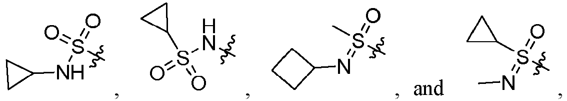 wherein the methyl, ethyl, tetrahydropyrrolyl, tetrahydrofuranyl, tetrahydropyranyl, piperidinyl, morpholinyl, tetrahydroimidazolyl, azoxybicycloheptyl, azoxyspirononanyl, azoxyspiroheptyl, phosphoxycyclohexyl, azoxycyclohexyl, phosphazacyclohexyl, or oxazolidinyl is optionally substituted with 1, 2, or 3 R^{b}.

In some other embodiments, R¹ are each independently selected from the group consisting of -NH₂, F, methyl, ethyl, 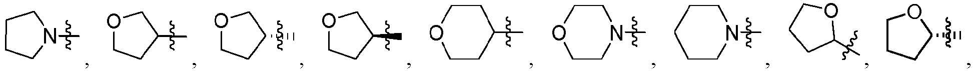 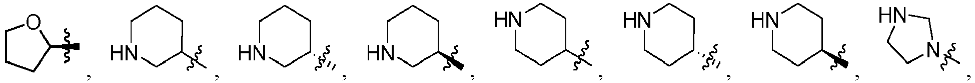 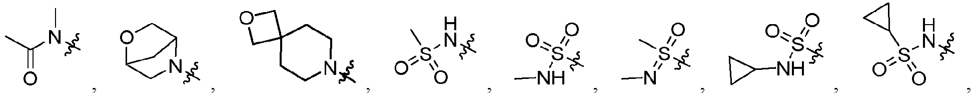 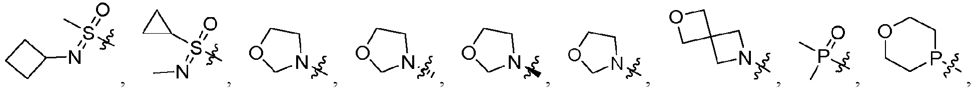 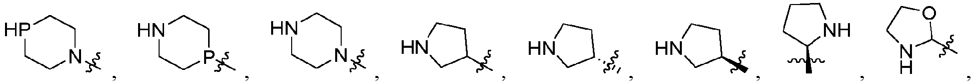 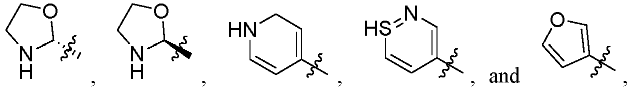 wherein the methyl, ethyl, 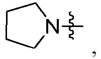 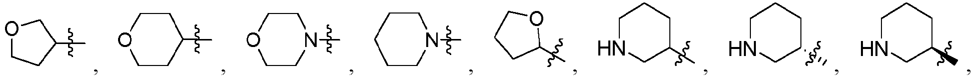 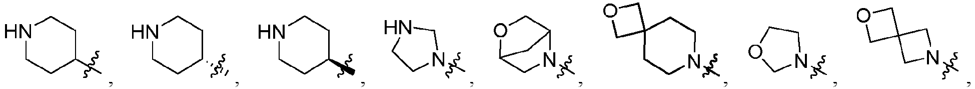 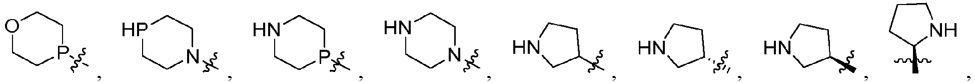 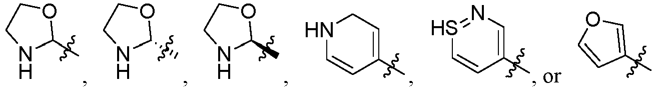 is optionally substituted with 1, 2, or 3 R^{b}.

In some embodiments, R¹ are each independently selected from the group consisting of -NH₂, F, methyl, ethyl, 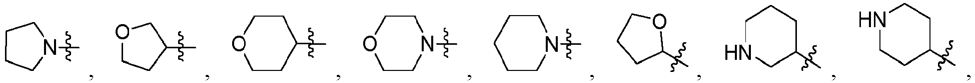 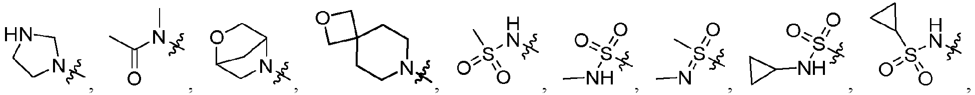 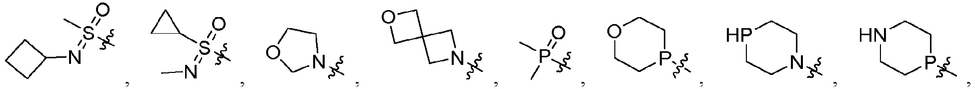 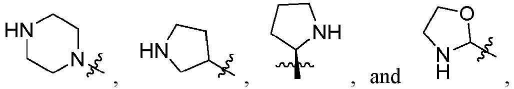 wherein the methyl, ethyl, 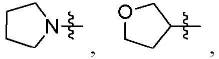 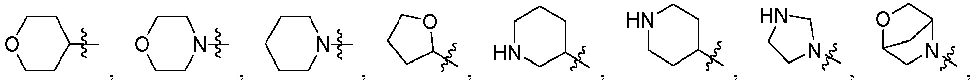 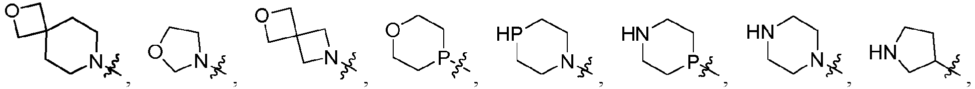 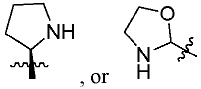 is optionally substituted with 1, 2, or 3 R^{b}.

In some embodiments, R¹ are each independently selected from the group consisting of -NH₂, -NH(C₁₋₄ alkyl), - N(C₁₋₄ alkyl)₂, halogen, C₁₋₆ alkyl, and 3- to 10-membered heterocycloalkyl, wherein the C₁₋₆ alkyl and 3- to 10-membered heterocycloalkyl are optionally substituted with one or more R^{b}.

In some embodiments, R¹ are each independently selected from the group consisting of -NH₂, -NH(C₁₋₂ alkyl), - N(C₁₋₂ alkyl)₂, halogen, C₁₋₄ alkyl, and 5- to 6-membered heterocycloalkyl, wherein the C₁₋₄ alkyl and 5- to 6-membered heterocycloalkyl are optionally substituted with one or more R^{b}.

In some embodiments, R¹ are each independently selected from the group consisting of -NH₂, -NHCH₃, -N(CH₃)₂, F, Cl, Br, methyl, ethyl, n-propyl, isopropyl, n-butyl, isobutyl, tert-butyl, tetrahydropyrrolyl, tetrahydrofuranyl, tetrahydrothienyl, tetrahydropyranyl, piperidinyl, piperazinyl, and morpholinyl, wherein the methyl, ethyl, n-propyl, isopropyl, n-butyl, isobutyl, tert-butyl, tetrahydropyrrolyl, tetrahydrofuranyl, tetrahydrothienyl, tetrahydropyranyl, piperidinyl, piperazinyl, or morpholinyl is optionally substituted with one or more R^{b}.

In some embodiments, R¹ are each independently selected from the group consisting of -NH₂, F, methyl, tetrahydropyrrolyl, tetrahydrofuranyl, tetrahydropyranyl, piperidinyl, and morpholinyl, wherein the methyl, tetrahydropyrrolyl, tetrahydrofuranyl, tetrahydropyranyl, piperidinyl, and morpholinyl are optionally substituted with one or more R^{b}.

In some embodiments, R¹ are each independently selected from the group consisting of -NH₂, F, methyl, tetrahydropyrrolyl, tetrahydrofuranyl, tetrahydropyranyl, piperidinyl, and morpholinyl, wherein the methyl, tetrahydropyrrolyl, tetrahydrofuranyl, tetrahydropyranyl, piperidinyl, and morpholinyl are optionally substituted with 1, 2, or 3 R^{b}.

In some embodiments, R¹ are each independently selected from the group consisting of -NH₂, F, methyl, 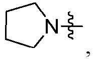 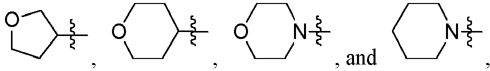 wherein the methyl, 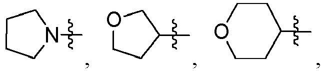 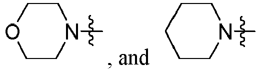 are optionally substituted with 1, 2, or 3 R^{b}.

In some embodiments, R^{d} are each independently selected from the group consisting of C₁₋₄ alkyl and C₃₋₆ cycloalkyl.

In some embodiments, R^{d} are each independently selected from the group consisting of C₁₋₂ alkyl and C₃₋₄ cycloalkyl.

In some embodiments, R^{d} are each independently selected from C₁₋₂ alkyl. In some embodiments, R^{d} are each independently selected from C₃₋₄ cycloalkyl.

In some embodiments, R^{b} are each independently selected from the group consisting of deuterium, -OH, C₁₋₄ alkyl, halogen, 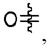 -N(C₁₋₄ alkyl)₂, and 3- to 6-membered heterocycloalkyl, wherein the -N(C₁₋₄ alkyl)₂ and 3- to 6-membered heterocycloalkyl are optionally substituted with one or more deuterium, halogen, or 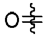 substituents.

In some embodiments, R^{b} are each independently selected from the group consisting of deuterium, -OH, C₁₋₃ alkyl, F, Cl, Br, 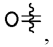 -N(C₁₋₄ alkyl)₂, and 5-membered heterocycloalkyl, wherein the -N(C₁₋₄ alkyl)₂ and 5-membered heterocycloalkyl are optionally substituted with one or more deuterium, F, Cl, Br, or 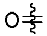 substituents.

In some embodiments, R^{b} are each independently selected from the group consisting of deuterium, -OH, CH₃, F, 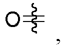 -N(CH₃)₂, -N(CH₃CH₂)₂, -N(CH₃CH₂CH₂)₂, -N(CH₃)CH₂CH₃, -N(CH₃)C(CH₃)₂, tetrahydropyrrolyl, tetrahydrofuranyl, and tetrahydrothienyl, wherein the -N(CH₃)₂, -N(CH₃CH₂)₂, -N(CH₃CH₂CH₂)₂, - N(CH₃)CH₂CH₃, -N(CH₃)C(CH₃)₂, tetrahydropyrrolyl, tetrahydrofuranyl, and tetrahydrothienyl are optionally substituted with one or more deuterium, F, Cl, Br, or 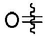 substituents.

In some embodiments, R^{b} are each independently selected from the group consisting of deuterium, -OH, CH₃, F, 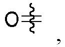 -N(CH₃)₂, -N(CH₃)CH₂CH₃, -N(CH₃)C(CH₃)₂, and tetrahydropyrrolyl, wherein the -N(CH₃)₂, tetrahydropyrrolyl, or -N(CH₃)CH₂CH₃ is optionally substituted with one or more deuterium, F, Cl, Br, or substituents.

In some embodiments, R^{b} are each independently selected from the group consisting of deuterium, -OH, CH₃, F, 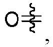 -N(CH₃)₂, -N(CH₃)CH₂CH₃, -N(CH₃)C(CH₃)₂, and tetrahydropyrrolyl, wherein the -N(CH₃)₂ is optionally substituted with 6 deuterium, and the tetrahydropyrrolyl or -N(CH₃)CH₂CH₃ is optionally substituted with 2 F or 1 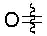 substituent.

In some embodiments, R^{b} are each independently selected from the group consisting of deuterium, -OH, CH₃, F, 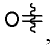 -N(CH₃)₂, -N(CD₃)₂,

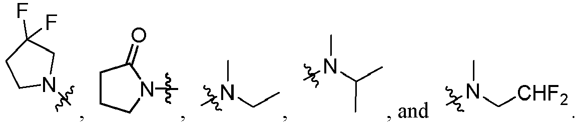

In some embodiments, R^{b} are each independently selected from the group consisting of -OH, halogen, 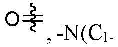 ₄ alkyl)₂, and 3- to 6-membered heterocycloalkyl, wherein the -N(C₁₋₄ alkyl)₂ and 3- to 6-membered heterocycloalkyl are optionally substituted with one or more deuterium, halogen, or 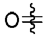 substituents.

In some embodiments, R^{b} are each independently selected from the group consisting of -OH, F, Cl, Br, 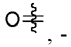 N(C₁₋₄ alkyl)₂, and 5-membered heterocycloalkyl, wherein the -N(C₁₋₄ alkyl)₂ and 5-membered heterocycloalkyl are optionally substituted with one or more deuterium, halogen, or 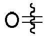 substituents.

In some embodiments, R^{b} are each independently selected from the group consisting of -OH, F, 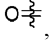 -N(CH₃)₂, - N(CH₃CH₂)₂, -N(CH₃CH₂CH₂)₂, tetrahydropyrrolyl, tetrahydrofuranyl, and tetrahydrothienyl, wherein the - N(CH₃)₂, -N(CH₃CH₂)₂, -N(CH₃CH₂CH₂)₂, tetrahydropyrrolyl, tetrahydrofuranyl, and tetrahydrothienyl are optionally substituted with one or more deuterium, halogen, or 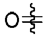 substituents.

In some embodiments, R^{b} are each independently selected from the group consisting of -OH, F, 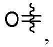 -N(CH₃)₂, and tetrahydropyrrolyl, wherein the -N(CH₃)₂ and tetrahydropyrrolyl are optionally substituted with one or more deuterium, halogen, or 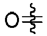 substituents.

In some embodiments, R^{b} are each independently selected from the group consisting of -OH, F, 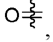 -N(CH₃)₂, and tetrahydropyrrolyl, wherein the -N(CH₃)₂ is optionally substituted with 3 deuterium, and the tetrahydropyrrolyl is optionally substituted with 2 F or 1

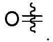

In some embodiments, R^{b} are each independently selected from the group consisting of -OH, F, 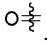 -N(CH₃)₂, - N(CD₃)₂,

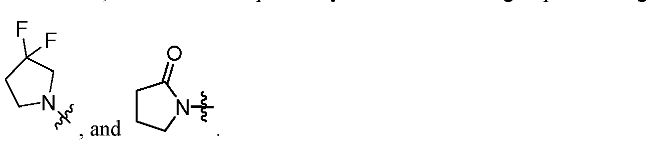

In some embodiments, R¹ are each independently selected from the group consisting of -NH₂, F,

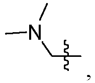

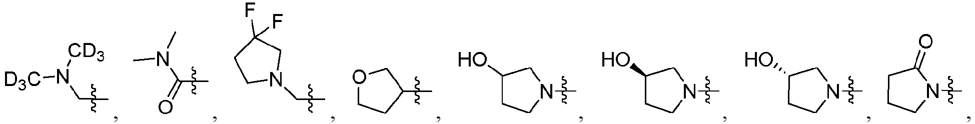

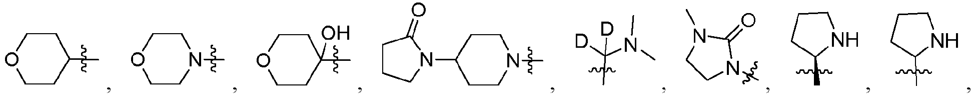

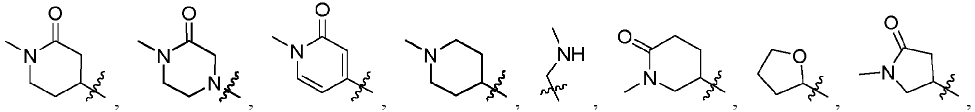

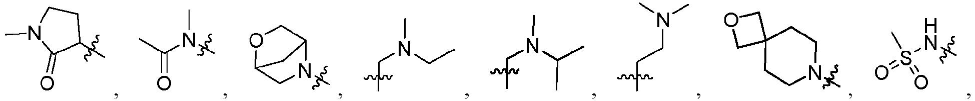

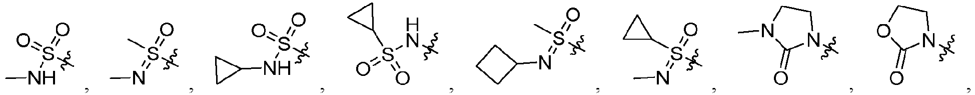

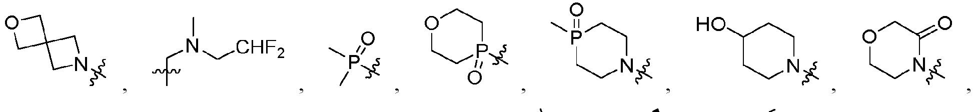

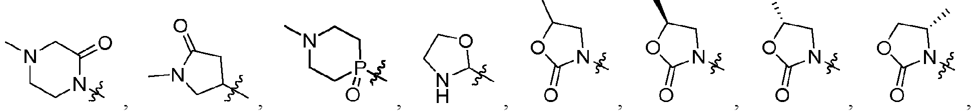

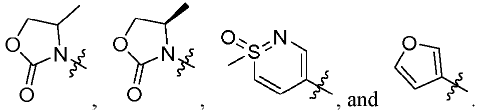

In some embodiments, R¹ are each independently selected from the group consisting of -NH₂, F,

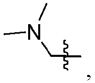

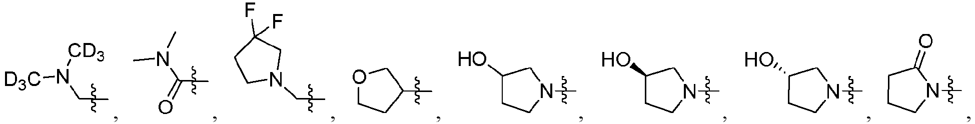

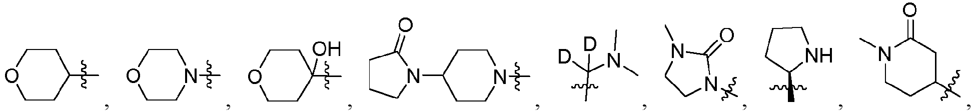

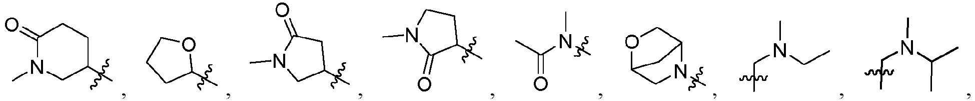

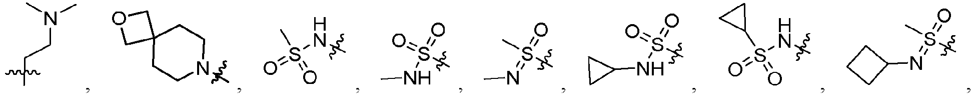

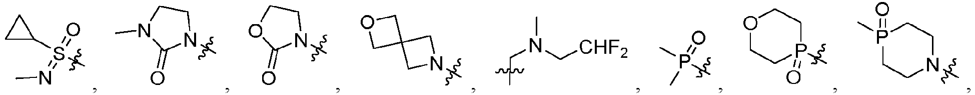

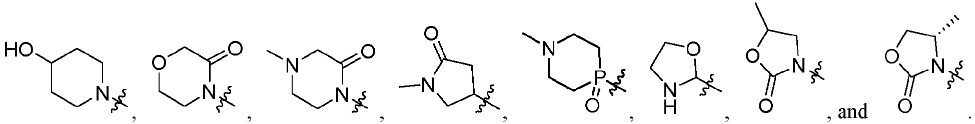

In some embodiments, R¹ are each independently selected from the group consisting of -NH₂, F,

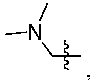

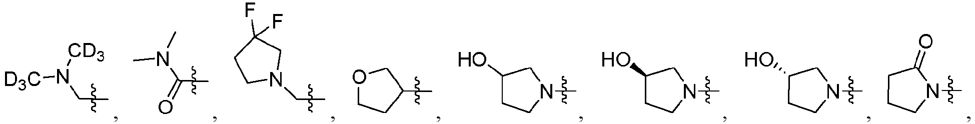

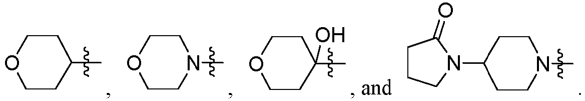

In some embodiments, n is selected from the group consisting of 1, 2, and 3.

In some embodiments, n is 2.

In some embodiments, m is selected from the group consisting of 0, 1, and 2.

In some embodiments, m is selected from the group consisting of 0 and 1.

In some embodiments, m is 0.

In some embodiments, R³ is selected from the group consisting of halogen, C₁₋₃ alkyl, and halogenated C₁₋₃ alkoxy.

In some embodiments, R³ is selected from the group consisting of F, Cl, Br, and C₁₋₃ alkyl.

In some embodiments, R³ is selected from the group consisting of F and methyl.

In some other embodiments, one, two, or three rings in the tricyclic ring are aromatic rings.

In some embodiments, one or two rings in the tricyclic ring are aromatic rings.

In some embodiments, the monocyclic ring in the tricyclic ring to which the 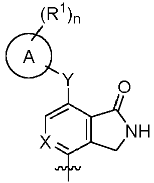 moiety of formula (I) is attached is an aromatic ring.

In some embodiments, the monocyclic ring in the tricyclic ring to which the 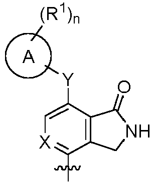 moiety of formula (**I**) or the 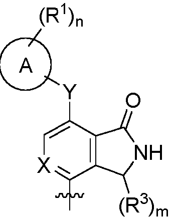 moiety of formula (II) is attached is an aromatic ring.

In some embodiments, the monocyclic ring in the tricyclic ring to which the 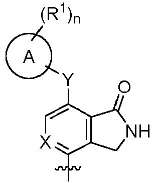 moiety of formula (I) or the 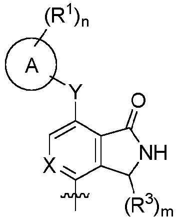 moiety of formula (II) is attached is an aromatic ring containing an N atom.

In some embodiments, any two adjacent rings in the tricyclic ring optionally form a fused, bridged, or spiro ring.

In some embodiments, the monocyclic ring in the tricyclic ring to which the 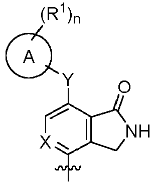 moiety of formula (I) is attached and the ring adjacent thereto form a fused ring.

In some embodiments, the monocyclic ring in the tricyclic ring to which the 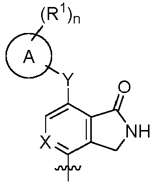 moiety of formula (I) or the 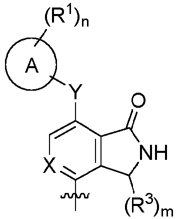 moiety of formula (II) is attached and the ring adjacent thereto form a fused ring.

In some embodiments, R² is selected from the group consisting of 6-membered monocyclic heteroaryl and a 9- to 14-membered saturated, partially saturated, or aromatic tricyclic ring, wherein the tricyclic ring contains 0-6 heteroatoms independently selected from the group consisting of N, O, and S, and the R² is optionally substituted with one or more R^{c}.

In some embodiments, R² is selected from a 9- to 14-membered saturated, partially saturated, or aromatic tricyclic ring, wherein the tricyclic ring contains 0-6 heteroatoms independently selected from the group consisting of N, O, and S, and the R² is optionally substituted with one or more R^{c}.

In some embodiments, R² is selected from the group consisting of 6-membered monocyclic heteroaryl containing 1 or 2 N atoms, and a 9- to 14-membered saturated, partially saturated, or aromatic tricyclic ring, wherein the tricyclic ring contains 1-6 heteroatoms independently selected from the group consisting of N, O, and S, and the R² is optionally substituted with one or more R^{c}.

In some embodiments, R² is selected from a 9- to 14-membered saturated, partially saturated, or aromatic tricyclic ring, wherein the tricyclic ring contains 1-6 heteroatoms independently selected from the group consisting of N, O, and S, and the R² is optionally substituted with one or more R^{c}.

In some other embodiments, the heteroatom in the tricyclic ring of R² is selected from the group consisting of N and O.

In some other embodiments, the heteroatom in the tricyclic ring of R² is N.

In some embodiments, R² is selected from the group consisting of 6-membered monocyclic heteroaryl containing 1 or 2 N atoms, and a 9- to 14-membered saturated, partially saturated, or aromatic tricyclic ring, wherein the tricyclic ring contains 1-6 heteroatoms independently selected from the group consisting of N and O, and the R² is optionally substituted with one or more R^{c}. In some embodiments, R² is selected from a 9- to 14-membered saturated, partially saturated, or aromatic tricyclic ring, wherein the tricyclic ring contains 1-6 heteroatoms independently selected from the group consisting of N and O, and the R² is optionally substituted with one or more R^{c}.

In some embodiments, R² is selected from the group consisting of 6-membered monocyclic heteroaryl containing 1 or 2 N atoms, and a 9- to 14-membered partially saturated tricyclic ring, wherein the tricyclic ring contains 1-6 heteroatoms independently selected from the group consisting of N and O, and the R² is optionally substituted with one or more R^{c}.

In some embodiments, R² is selected from a 9- to 14-membered partially saturated or aromatic tricyclic ring, wherein the tricyclic ring contains 1-6 heteroatoms independently selected from the group consisting of N and O, and the R² is optionally substituted with one or more R^{c}.

In some embodiments, R² is selected from the group consisting of 6-membered monocyclic heteroaryl containing 1 N atom, and a 9- to 14-membered partially saturated or aromatic tricyclic ring, wherein the tricyclic ring contains 1-6 heteroatoms independently selected from the group consisting of N and O, and the R² is optionally substituted with one or more R^{c}.

In some embodiments, R² is selected from the group consisting of 6-membered monocyclic heteroaryl containing 1 N atom, and a 9-, 11-, 12-, 13-, or 14-membered partially saturated tricyclic ring, wherein the tricyclic ring contains 1-6 heteroatoms independently selected from the group consisting of N and O, and the R² is optionally substituted with one or more R^{c}.

In some embodiments, R² is selected from a 9-, 10-, 11-, 12-, 13-, or 14-membered partially saturated or aromatic tricyclic ring, wherein the tricyclic ring contains 1-4 heteroatoms independently selected from the group consisting of N and O, and the R² is optionally substituted with one or more R^{c}.

In some embodiments, R² is selected from a 9-, 11-, 12-, 13-, or 14-membered partially saturated or aromatic tricyclic ring, wherein the tricyclic ring contains 1-4 heteroatoms independently selected from the group consisting of N and O, and the R² is optionally substituted with one or more R^{c}.

In some other embodiments, the heteroatom in the tricyclic ring of R² is selected from the group consisting of N, O, and S.

In some other embodiments, the heteroatom in the tricyclic ring of R² is selected from the group consisting of N and S.

In some other embodiments, R² is selected from the group consisting of 6-membered monocyclic heteroaryl containing 1 or 2 N atoms, and a 9- to 14-membered saturated, partially saturated, aromatic tricyclic ring, wherein the tricyclic ring contains 1-6 heteroatoms independently being S, and the R² is optionally substituted with one or more R^{c}.

In some other embodiments, R² is selected from the group consisting of 6-membered monocyclic heteroaryl containing 1 or 2 N atoms, and a 9- to 14-membered partially saturated tricyclic ring, wherein the tricyclic ring contains 1-6 heteroatoms independently being S, and the R² is optionally substituted with one or more R^{c}.

In some other embodiments, R² is selected from the group consisting of a 9- to 14-membered partially saturated or aromatic tricyclic ring, wherein the tricyclic ring contains 1-6 heteroatoms independently being S, and the R² is optionally substituted with one or more R^{c}.

In some other embodiments, R² is selected from the group consisting of 6-membered monocyclic heteroaryl containing 1 N atom, and a 9- to 14-membered partially saturated or aromatic tricyclic ring, wherein the tricyclic ring contains 1-6 heteroatoms independently being S, and the R² is optionally substituted with one or more R^{c}.

In some other embodiments, R² is selected from the group consisting of 6-membered monocyclic heteroaryl containing 1 N atom, and a 9-, 11-, 12-, 13-, or 14-membered partially saturated tricyclic ring, wherein the tricyclic ring contains 1-6 heteroatoms independently being S, and the R² is optionally substituted with one or more R^{c}.

In some other embodiments, R² is selected from the group consisting of a 9-, 10-, 11-, 12-, 13-, or 14-membered partially saturated or aromatic tricyclic ring, wherein the tricyclic ring contains 1-4 heteroatoms independently being S, and the R² is optionally substituted with one or more R^{c}.

In some other embodiments, R² is selected from the group consisting of 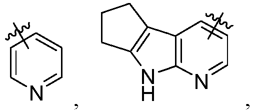 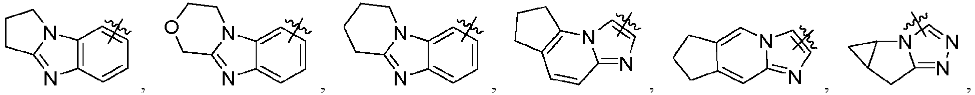 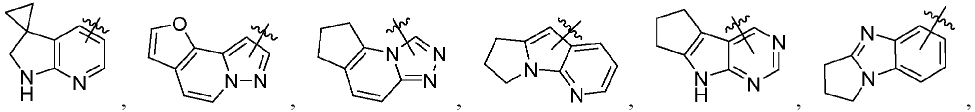 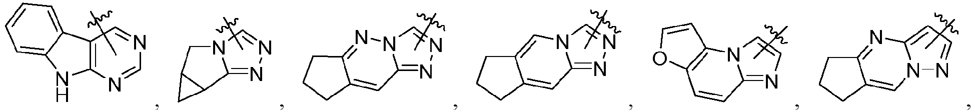 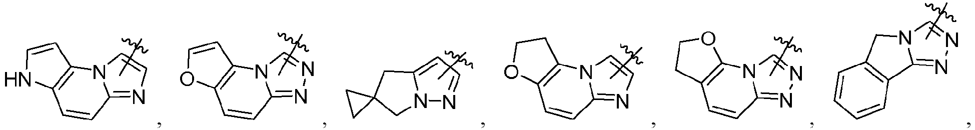 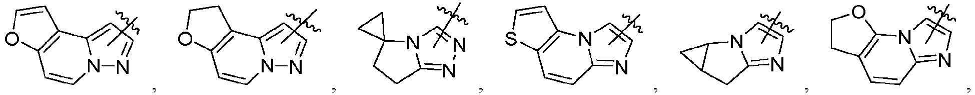 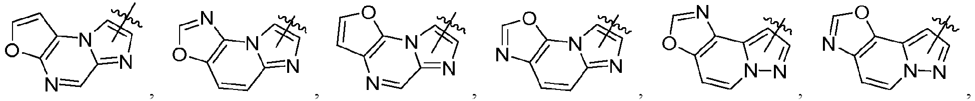 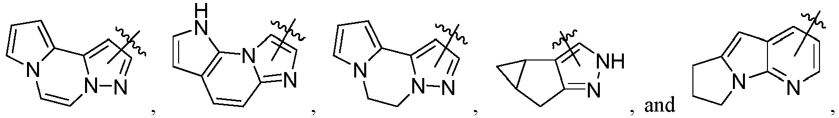 wherein the R² is optionally substituted with one or more R^{c}.

In some embodiments, R² is selected from the group consisting of 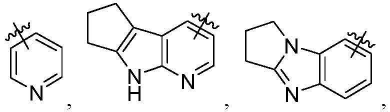 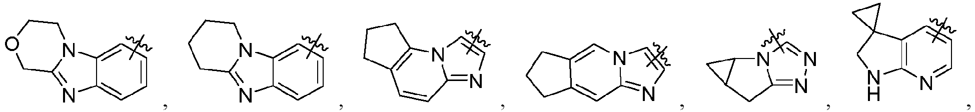 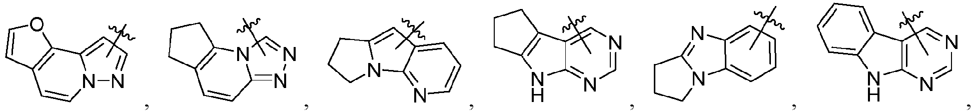 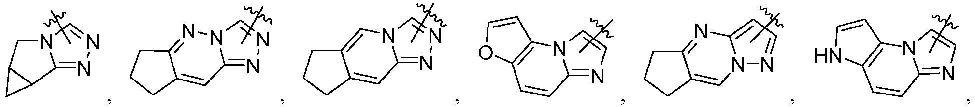 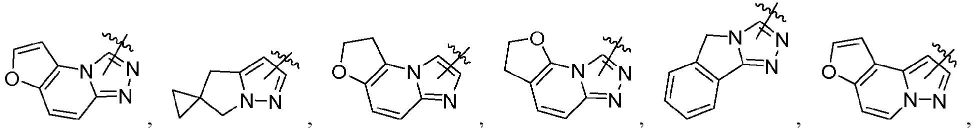 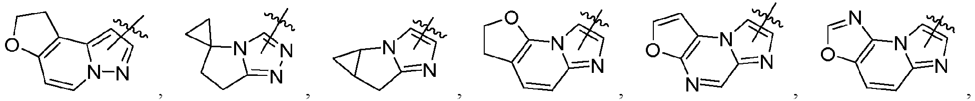 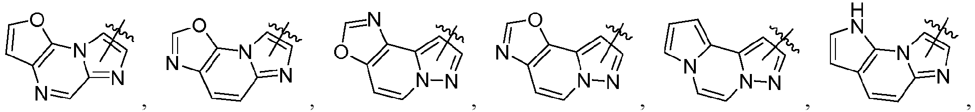 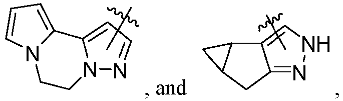 wherein the R² is optionally substituted with one or more R^{c}.

In some other embodiments, R² is selected from the group consisting of 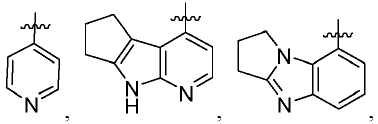 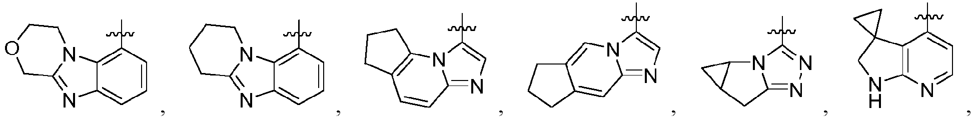 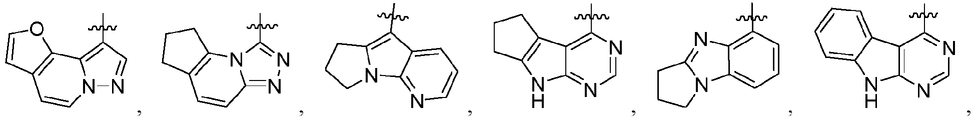 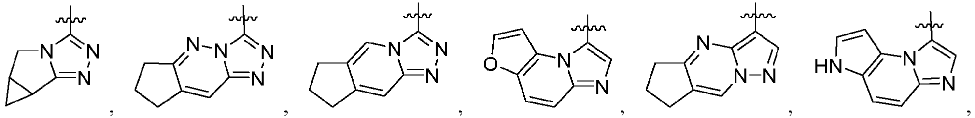 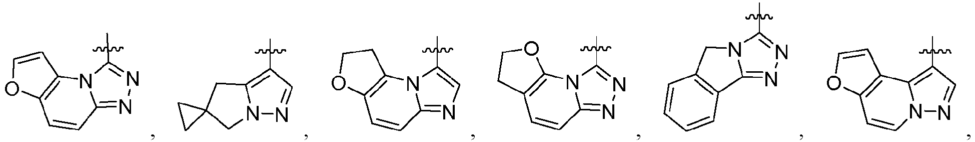 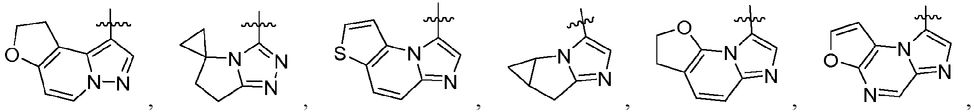 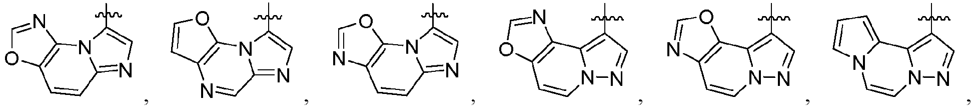 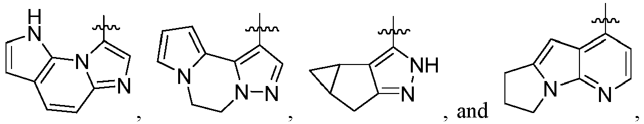 wherein the R² is optionally substituted with one or more R^{c}.

In some embodiments, R² is selected from the group consisting of 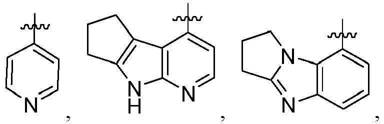 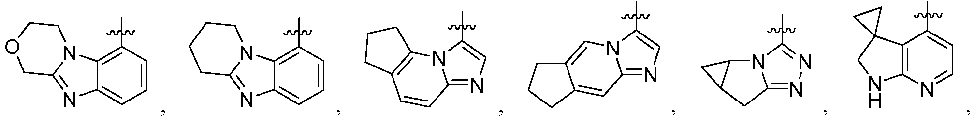 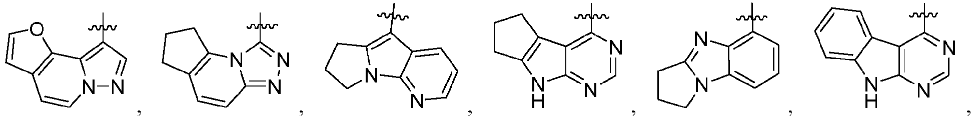 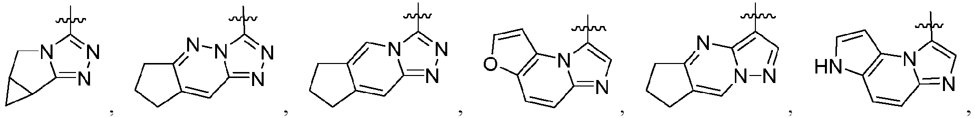 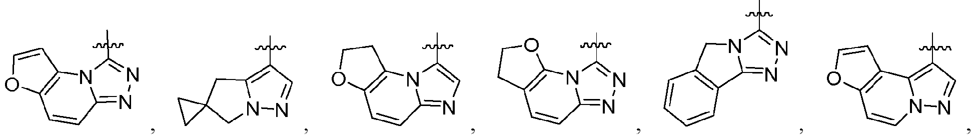 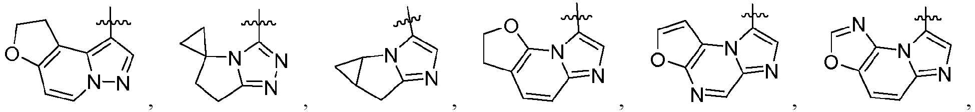 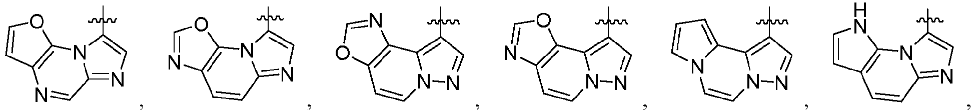 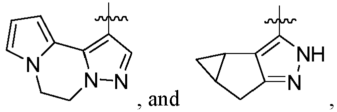 wherein the R² is optionally substituted with one or more R^{c}.

In some other embodiments, R² is selected from the group consisting of

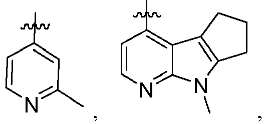

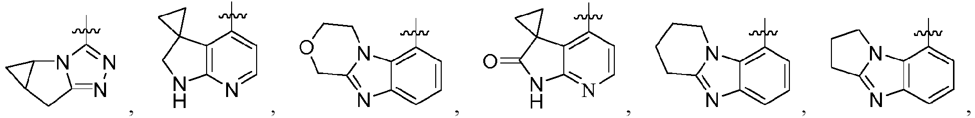

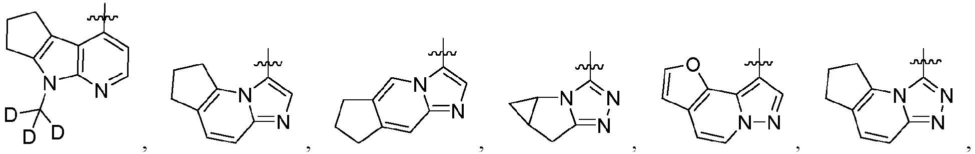

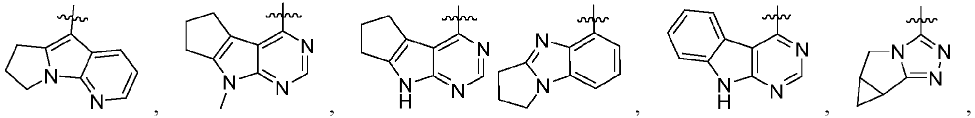

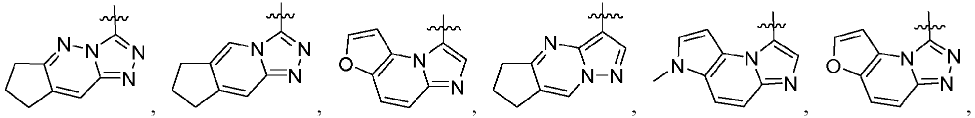

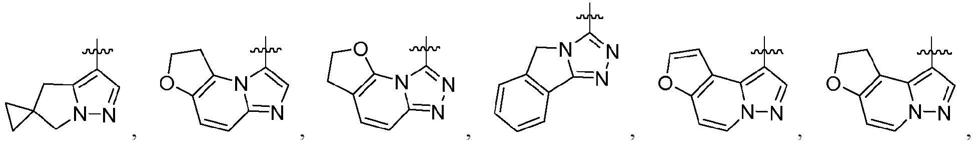

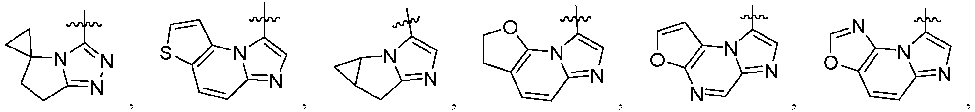

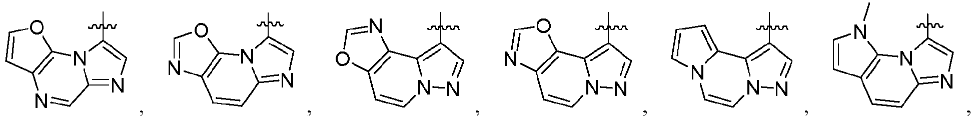

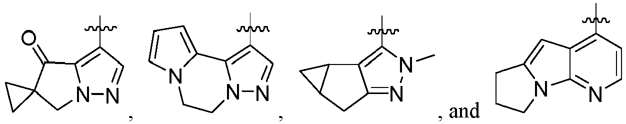

In some embodiments, R² is selected from the group consisting of 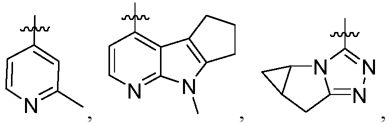 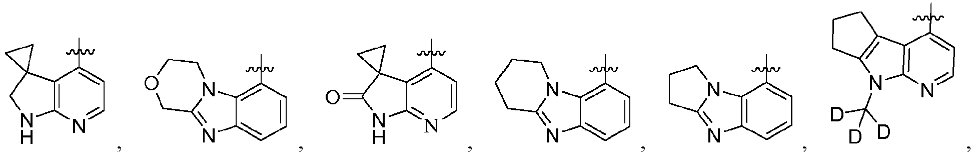 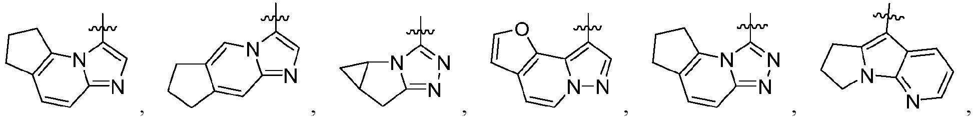 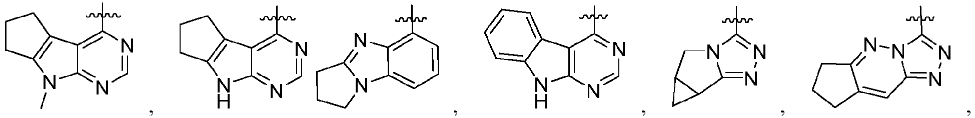 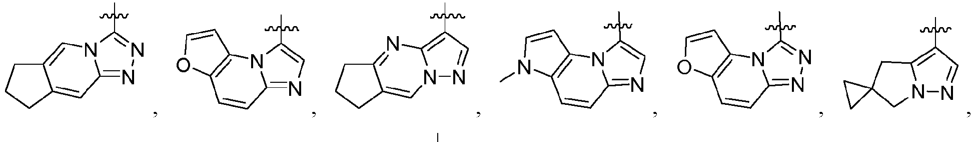 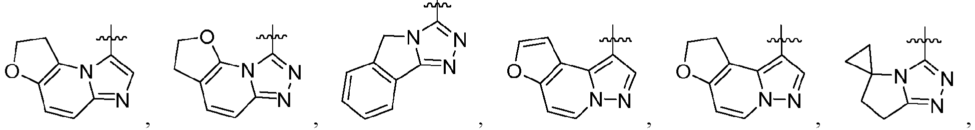 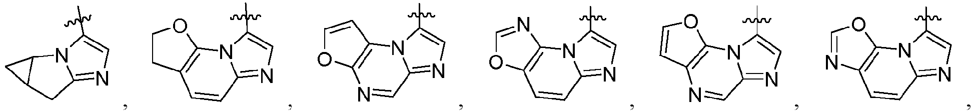 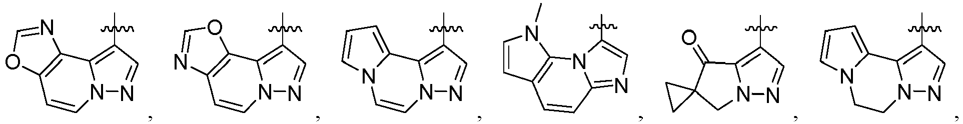 and

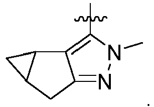

In some embodiments, R² is selected from the group consisting of 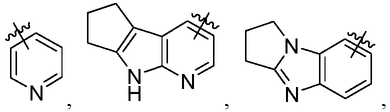 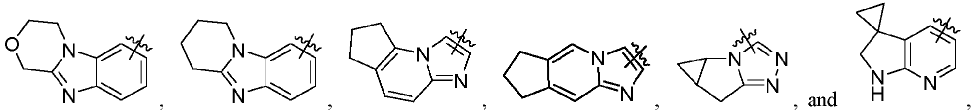 wherein the R² is optionally substituted with one or more R^{c}.

In some embodiments, R² is selected from the group consisting of 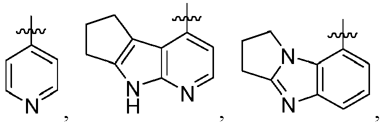 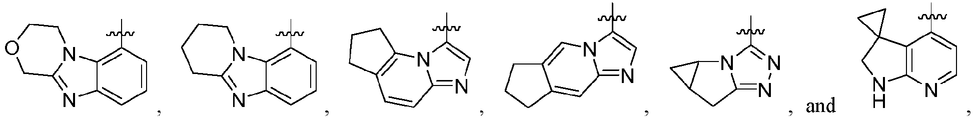 wherein the R² is optionally substituted with one or more R^{c}.

In some embodiments, R² is selected from the group consisting of 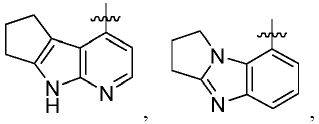 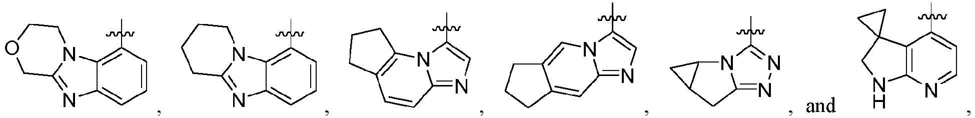 wherein the R² is optionally substituted with one or more R^{c}.

In some embodiments, R^{c} are each independently selected from the group consisting of 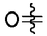 and C₁₋₆ alkyl optionally substituted with one or more deuterium.

In some embodiments, R^{c} are each independently selected from the group consisting of 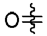 and C₁₋₄ alkyl optionally substituted with one or more deuterium.

In some embodiments, R^{c} are each independently selected from the group consisting of 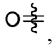 and methyl, ethyl, n-propyl, isopropyl, n-butyl, isobutyl, or tert-butyl optionally substituted with one or more deuterium.

In some embodiments, R^{c} are each independently selected from the group consisting of 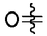 and methyl optionally substituted with one or more deuterium.

In some embodiments, R^{c} are each independently selected from the group consisting of 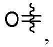 methyl, and -CD₃.

In some embodiments, R² is selected from the group consisting of

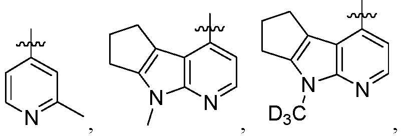

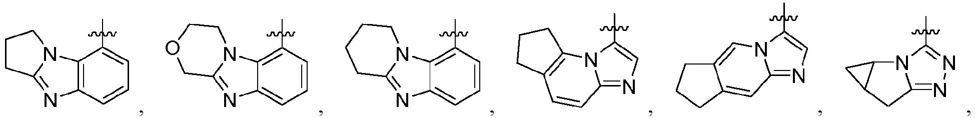

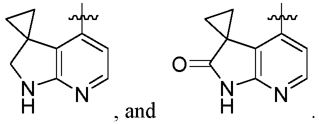

In some embodiments, the compound of formula (II), the stereoisomer thereof, or the pharmaceutically acceptable salt thereof, and the compound of formula (I), the stereoisomer thereof, or the pharmaceutically acceptable salt thereof of the present application are selected from the group consisting of the following compounds of formula (Ia), formula (Ib), formula (Ic), and formula (Id), stereoisomers thereof, or pharmaceutically acceptable salts thereof: 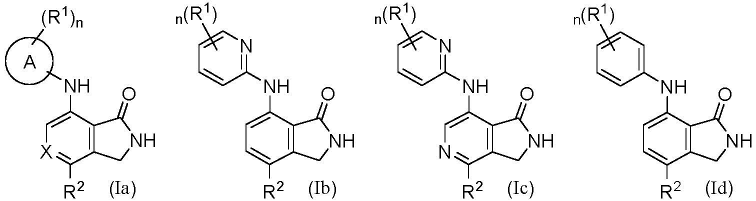 wherein, X, ring A, R¹, R², and n are as described herein.

In some embodiments, the present application encompasses the variables defined above and embodiments thereof, as well as any combination thereof.
[1] In another aspect, the present application provides a compound of formula (I), a stereoisomer thereof, or a pharmaceutically acceptable salt thereof, 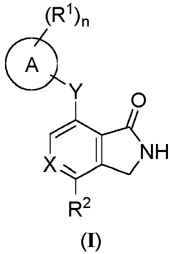 wherein,
   X is selected from the group consisting of N and CH;
   Y is selected from the group consisting of a bond, -O-, -S-, and -NR^{a}-;
   ring A is selected from the group consisting of C₅₋₁₀ aryl and 5- to 10-membered heteroaryl;
   R¹ are each independently selected from the group consisting of -NH₂, -NH(C₁₋₄ alkyl), -N(C₁₋₄ alkyl)₂, -OH, -OC₁₋₄ alkyl, -CN, halogen, C₁₋₆ alkyl, and 3- to 10-membered heterocycloalkyl, wherein the C₁₋₆ alkyl and 3- to 10-membered heterocycloalkyl are optionally substituted with one or more R^{b};
   R² is selected from the group consisting of 5- to 6-membered monocyclic heteroaryl and a 9- to 14-membered saturated, partially saturated, aromatic tricyclic ring, wherein the tricyclic ring contains 0-6 heteroatoms independently selected from the group consisting of N, O, and S, and R² is optionally substituted with one or more R^{c};
   R^{a} is selected from the group consisting of hydrogen and C₁₋₄ alkyl;
   R^{b} are each independently selected from the group consisting of -NH₂, -NH(C₁₋₄ alkyl), -N(C₁₋₄ alkyl)₂, -OH, -OC₁₋₄ alkyl, -CN, halogen, 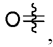 and 3- to 6-membered heterocycloalkyl, wherein the -NH(C₁₋₄ alkyl), -N(C₁₋₄ alkyl)₂, -OC₁₋₄ alkyl, and 3- to 6-membered heterocycloalkyl are optionally substituted with one or more deuterium, halogen, or 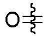 substituents;
   R^{c} are each independently selected from the group consisting of -NH₂, -NH(C₁₋₄ alkyl), -N(C₁₋₄ alkyl)₂, -OH, -OC₁₋₄ alkyl, halogen, 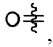 and C₁₋₆ alkyl optionally substituted with one or more deuterium;
   n is selected from the group consisting of 1, 2, 3, and 4.
[2] In some embodiments, provided is the compound of formula (I), the stereoisomer thereof, or the pharmaceutically acceptable salt thereof as described in the preceding paragraph [1], wherein X is CH; or, X is N.
[3] In some embodiments, provided is the compound of formula (I), the stereoisomer thereof, or the pharmaceutically acceptable salt thereof as described in the preceding paragraph [1] or [2], wherein R^{a} is hydrogen.
[4] In some embodiments, provided is the compound of formula (I), the stereoisomer thereof, or the pharmaceutically acceptable salt thereof as described in any of the preceding paragraphs [1] to [3], wherein Y is selected from -NR^{a}-; or, Y is -NH-.
[5] In some embodiments, provided is the compound of formula (I), the stereoisomer thereof, or the pharmaceutically acceptable salt thereof as described in any of the preceding paragraphs [1] to [4], wherein ring A is selected from the group consisting of C₅₋₈ aryl and 5- to 8-membered heteroaryl; or, ring A is selected from the group consisting of C₅₋₆ aryl and 5- to 6-membered heteroaryl; or, ring A is selected from the group consisting of phenyl or 6-membered heteroaryl containing 1 or 2 N atoms; or, ring A is selected from the group consisting of phenyl, pyridinyl, pyrimidinyl, pyridazinyl, or pyrazinyl; or, ring A is selected from the group consisting of phenyl or pyridinyl.
[6] In some embodiments, provided is the compound of formula (I), the stereoisomer thereof, or the pharmaceutically acceptable salt thereof as described in any of the preceding paragraphs [1] to [5], wherein R¹ are each independently selected from the group consisting of -NH₂, -NH(C₁₋₄ alkyl), -N(C₁₋₄ alkyl)₂, halogen, C₁₋₆ alkyl, and 3- to 10-membered heterocycloalkyl, wherein the C₁₋₆ alkyl and 3- to 10-membered heterocycloalkyl are optionally substituted with one or more R^{b}; or, R¹ are each independently selected from the group consisting of - NH₂, -NH(C₁₋₂ alkyl), -N(C₁₋₂ alkyl)₂, halogen, C₁₋₄ alkyl, and 5- to 6-membered heterocycloalkyl, wherein the C₁₋₄ alkyl and 5- to 6-membered heterocycloalkyl are optionally substituted with one or more R^{b}; or, R¹ are each independently selected from the group consisting of -NH₂, -NHCH₃, -N(CH₃)₂, F, Cl, Br, methyl, ethyl, n-propyl, isopropyl, n-butyl, isobutyl, tert-butyl, tetrahydropyrrolyl, tetrahydrofuranyl, tetrahydrothienyl, tetrahydropyranyl, piperidinyl, piperazinyl, and morpholinyl, wherein the methyl, ethyl, n-propyl, isopropyl, n-butyl, isobutyl, *tert-*butyl, tetrahydropyrrolyl, tetrahydrofuranyl, tetrahydrothienyl, tetrahydropyranyl, piperidinyl, piperazinyl, or morpholinyl is optionally substituted with one or more R^{b}; or, R¹ are each independently selected from the group consisting of -NH₂, F, methyl, tetrahydropyrrolyl, tetrahydrofuranyl, tetrahydropyranyl, piperidinyl, and morpholinyl, wherein the methyl, tetrahydropyrrolyl, tetrahydrofuranyl, tetrahydropyranyl, piperidinyl, and morpholinyl are optionally substituted with one or more R^{b}; or, R¹ are each independently selected from the group consisting of -NH₂, F, methyl, 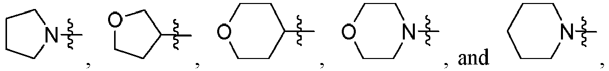 wherein the methyl, 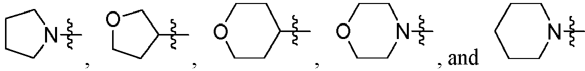 are optionally substituted with 1, 2, or 3 R^{b}; or, R¹ are each independently selected from the group consisting of -NH₂, F, 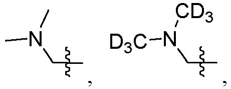 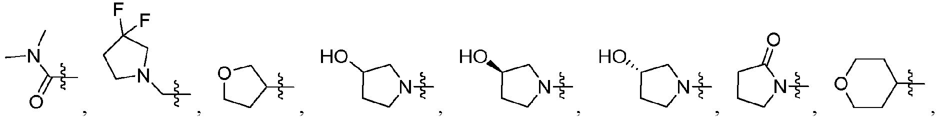 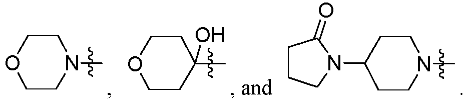
[7] In some embodiments, provided is the compound of formula (I), the stereoisomer thereof, or the pharmaceutically acceptable salt thereof as described in any of the preceding paragraphs [1] to [6], wherein n is selected from the group consisting of 1, 2, and 3; or, n is 2.
[8] In some embodiments, provided is the compound of formula (I), the stereoisomer thereof, or the pharmaceutically acceptable salt thereof as described in any of the preceding paragraphs [1] to [7], wherein R^{b} are each independently selected from the group consisting of -OH, halogen, 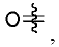 -N(C₁₋₄ alkyl)₂, and 3- to 6-membered heterocycloalkyl, wherein the -N(C₁₋₄ alkyl)₂ and 3- to 6-membered heterocycloalkyl are optionally substituted with one or more deuterium, halogen, or 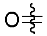 substituents; or, R^{b} are each independently selected from the group consisting of -OH, F, Cl, Br, 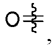 -N(C₁₋₄ alkyl)₂, and 5-membered heterocycloalkyl, wherein the -N(C₁₋₄ alkyl)₂ and 5-membered heterocycloalkyl are optionally substituted with one or more deuterium, halogen, or 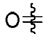 substituents; or, R^{b} are each independently selected from the group consisting of -OH, F, 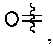 -N(CH₃)₂, - N(CH₃CH₂)₂, -N(CH₃CH₂CH₂)₂, tetrahydropyrrolyl, tetrahydrofuranyl, and tetrahydrothienyl, wherein the - N(CH₃)₂, -N(CH₃CH₂)₂, -N(CH₃CH₂CH₂)₂, tetrahydropyrrolyl, tetrahydrofuranyl, and tetrahydrothienyl are optionally substituted with one or more deuterium, halogen, or 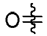 substituents; or, R^{b} are each independently selected from the group consisting of -OH, F, 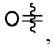 -N(CH₃)₂, and tetrahydropyrrolyl, wherein the -N(CH₃)₂ and tetrahydropyrrolyl are optionally substituted with one or more deuterium, halogen, or 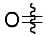 substituents; or, R^{b} are each independently selected from the group consisting of -OH, F, 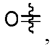 -N(CH₃)₂, -N(CD₃)₂, 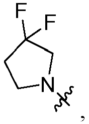 and 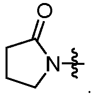
[9] In some embodiments, provided is the compound of formula (I), the stereoisomer thereof, or the pharmaceutically acceptable salt thereof as described in any of the preceding paragraphs [1] to [8], wherein one or two rings in the tricyclic ring are aromatic rings; or, the monocyclic ring in the tricyclic ring to which the 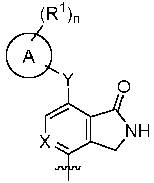 moiety of formula (I) is attached is an aromatic ring; or, any two adjacent rings in the tricyclic ring optionally form a fused, bridged, or spiro ring; or, the monocyclic ring in the tricyclic ring to which the 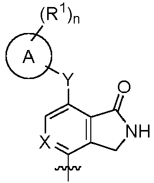 moiety of formula (I) is attached and the ring adjacent thereto form a fused ring.
[10] In some embodiments, provided is the compound of formula (I), the stereoisomer thereof or the pharmaceutically acceptable salt thereof as described in any of the preceding paragraphs [1] to [9], wherein R² is selected from the group consisting of 6-membered monocyclic heteroaryl and a 9- to 14-membered saturated, partially saturated, aromatic tricyclic ring, wherein the tricyclic ring contains 0-6 heteroatoms independently selected from the group consisting of N, O, and S, and the R² is optionally substituted with one or more R^{c}; or, R² is selected from the group consisting of 6-membered monocyclic heteroaryl containing 1 or 2 N atoms, and a 9- to 14-membered saturated, partially saturated, aromatic tricyclic ring, wherein the tricyclic ring contains 1-6 heteroatoms independently selected from the group consisting of N, O, and S, and the R² is optionally substituted with one or more R^{c}; or, R² is selected from the group consisting of 6-membered monocyclic heteroaryl containing 1 or 2 N atoms, and a 9- to 14-membered saturated, partially saturated, aromatic tricyclic ring, wherein the tricyclic ring contains 1-6 heteroatoms independently selected from the group consisting of N and O, and the R² is optionally substituted with one or more R^{c}; or, R² is selected from the group consisting of 6-membered monocyclic heteroaryl containing 1 or 2 N atoms, and a 9- to 14-membered partially saturated tricyclic ring, wherein the tricyclic ring contains 1-6 heteroatoms independently selected from the group consisting of N and O, and the R² is optionally substituted with one or more R^{c}; or, R² is selected from the group consisting of 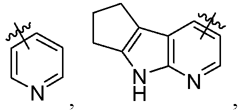 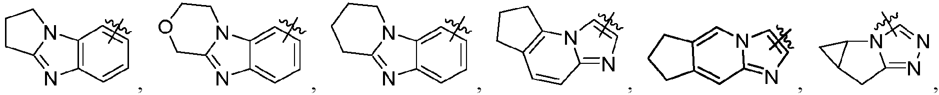 and 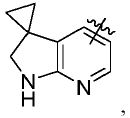 wherein the R² is optionally substituted with one or more R^{c}; or, R² is selected from the group consisting of 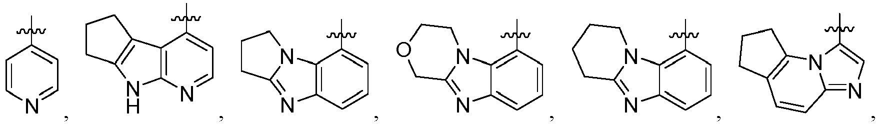 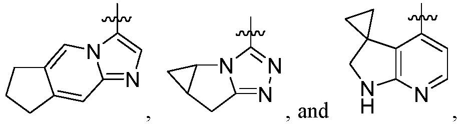 wherein the R² is optionally substituted with one or more R^{c}; or, R² is selected from the group consisting of 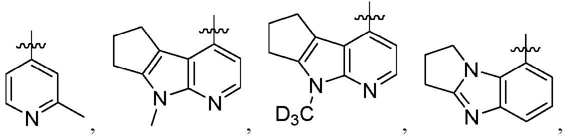 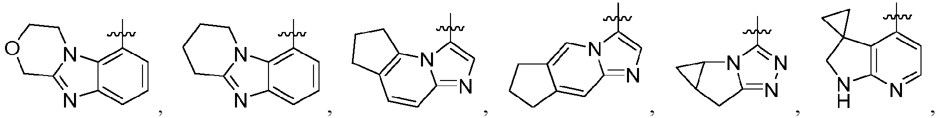 and 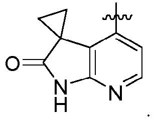
[11] In some embodiments, provided is the compound of formula (I), the stereoisomer thereof, or the pharmaceutically acceptable salt thereof as described in any of the preceding paragraphs [1] to [10], wherein R^{c} are each independently selected from the group consisting of 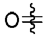 and C₁₋₆ alkyl optionally substituted with one or more deuterium; or, R^{c} are each independently selected from the group consisting of 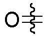 and C₁₋₄ alkyl optionally substituted with one or more deuterium; or, R^{c} are each independently selected from the group consisting of 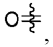 and methyl, ethyl, n-propyl, isopropyl, n-butyl, isobutyl, or tert-butyl optionally substituted with one or more deuterium; or, R^{c} are each independently selected from the group consisting of 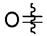 and methyl optionally substituted with one or more deuterium; or, R^{c} are each independently selected from the group consisting of 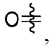 methyl, and -CD₃.
[12] In some embodiments, provided is the compound of formula (I), the stereoisomer thereof, or the pharmaceutically acceptable salt thereof as described in any of the preceding paragraphs [1] to [10], being selected from the group consisting of the following compounds of formula (Ia), formula (Ib), formula (Ic), and formula (Id), stereoisomers thereof, or pharmaceutically acceptable salts thereof: 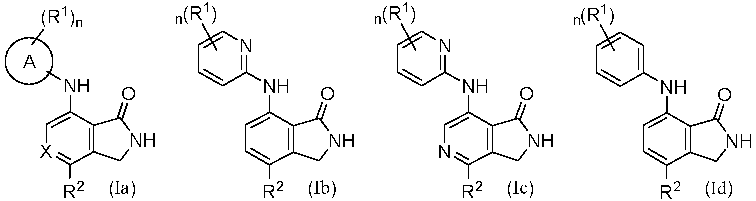 wherein, X, ring A, R¹, R², and n are as described in any of the preceding paragraphs [1] to [2] or paragraphs [5] to [11].

It should be understood that any embodiment of the compounds of the present application as described above, and any specific substituents set forth herein with respect to particular X, Y, ring A, R¹, R², and R³ substituents in the compounds of the present disclosure as described above, may be independently combined with other embodiments of the present invention and/or substituents of the compounds to form embodiments of the present invention not specifically set forth above. Further, when a range of substituents are disclosed in the detailed description and/or claims with respect to any particular X, Y, ring A, R¹, R², and R³ substituents, it should be understood that one or more substituents may be deleted from the range and the remaining range of substituents should also be considered an embodiment of the application.

In some embodiments, the heteroatom in the heterocycloalkenyl, heterocycloalkyl, heterocyclyl, or heteroaryl is selected from the group consisting of N, O, S, and P, and the number of heteroatoms is selected from the group consisting of 1, 2, 3, 4, and 5; or, the number of heteroatoms is selected from the group consisting of 1, 2, 3, and 4; or, the number of heteroatoms is selected from the group consisting of 1, 2, and 3; or, the number of heteroatoms is selected from the group consisting of 1 and 2.

In some embodiments, the compound of formula (II), the stereoisomer thereof, or the pharmaceutically acceptable salt thereof, and the compound of formula (I), the stereoisomer thereof, or the pharmaceutically acceptable salt thereof of the present application are selected from the group consisting of the following compounds, stereoisomers thereof, or pharmaceutically acceptable salts thereof:

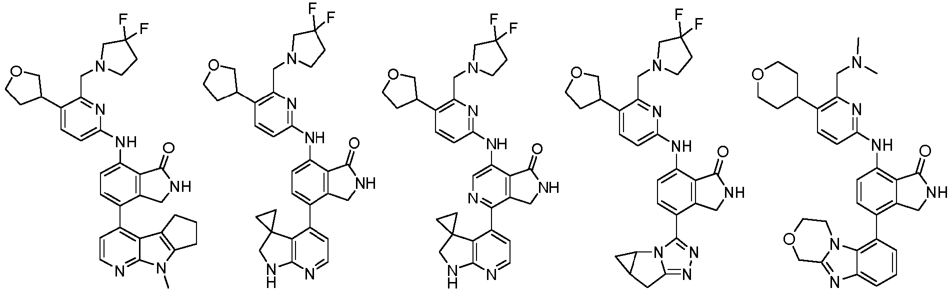

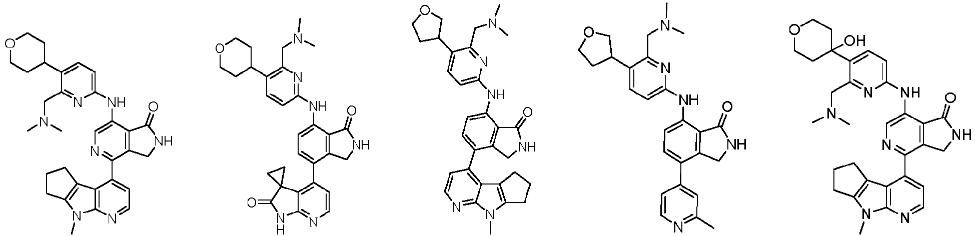

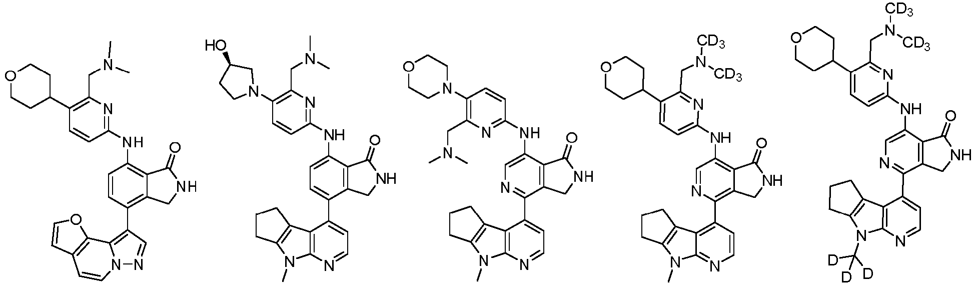

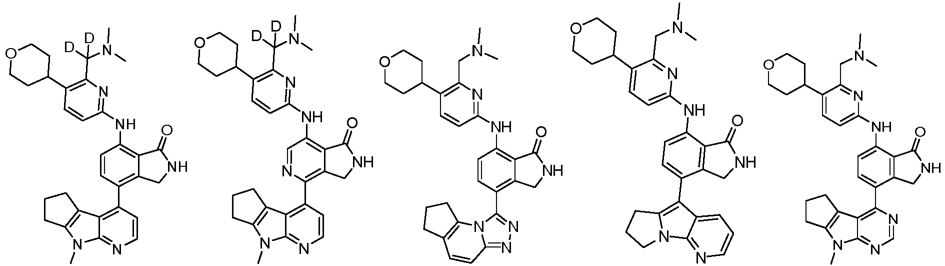

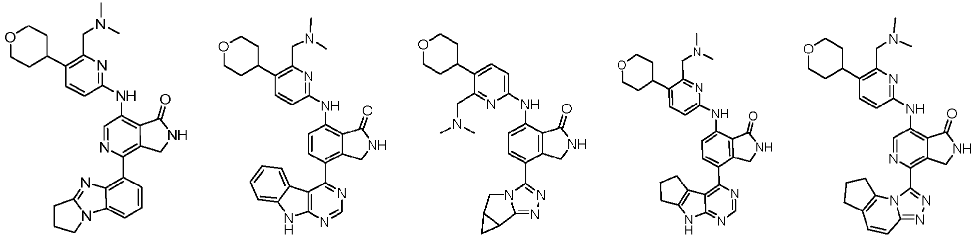

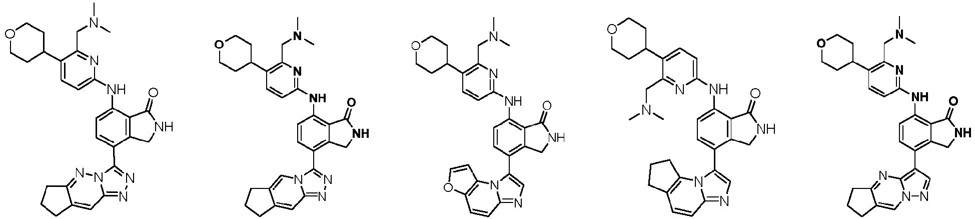

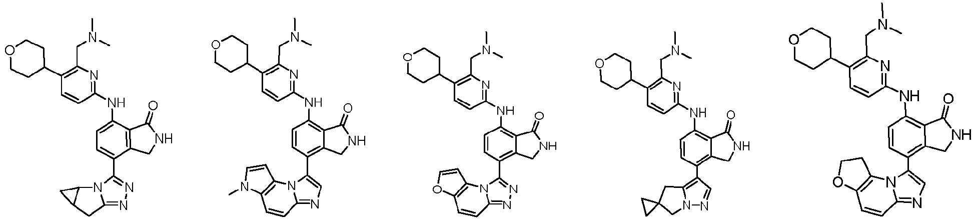

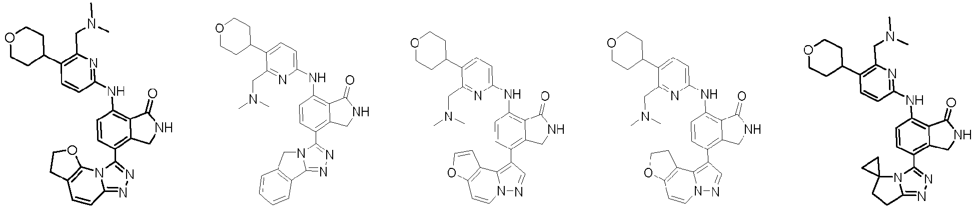

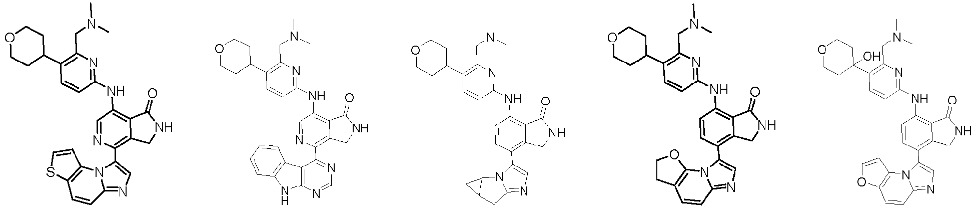

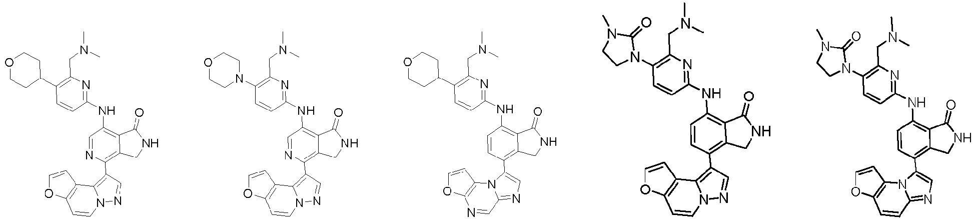

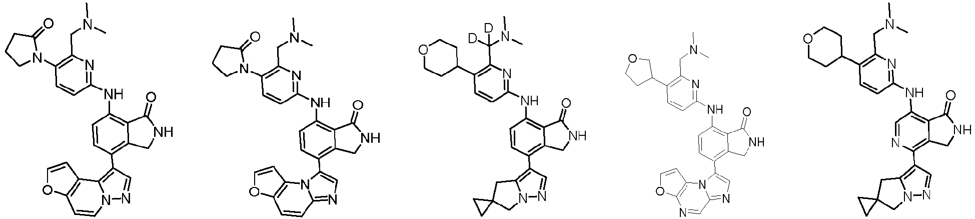

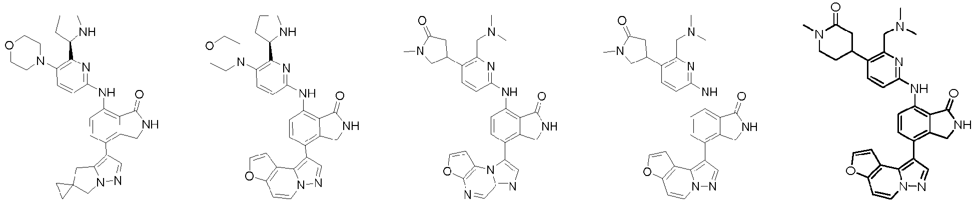

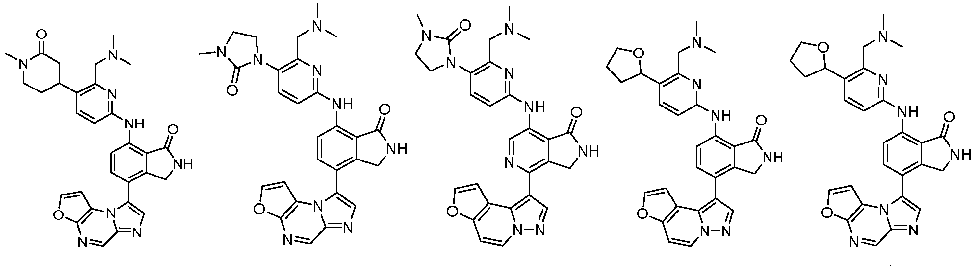

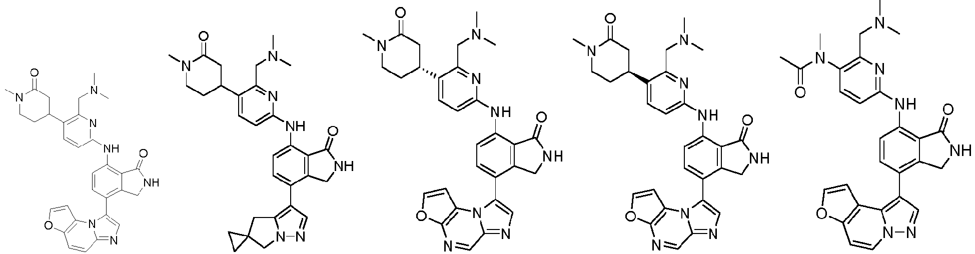

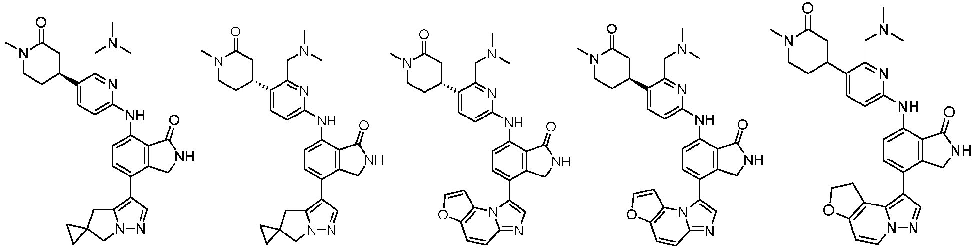

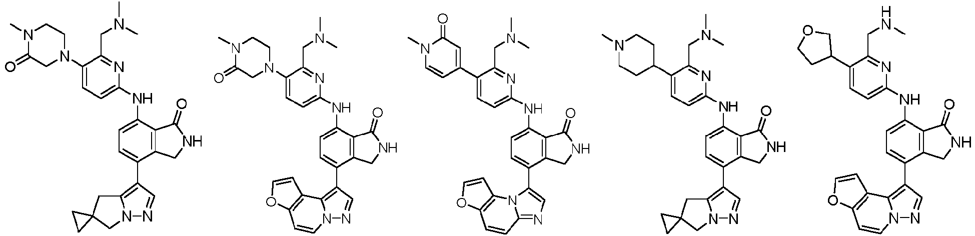

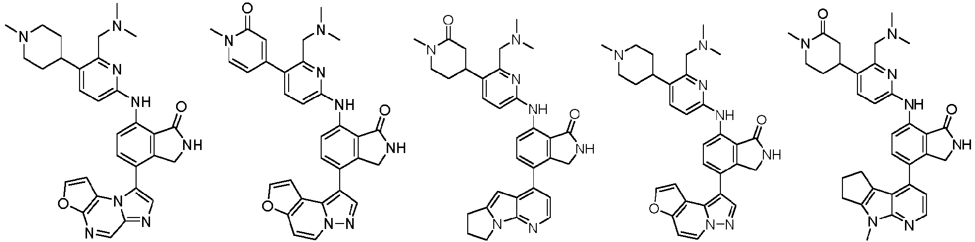

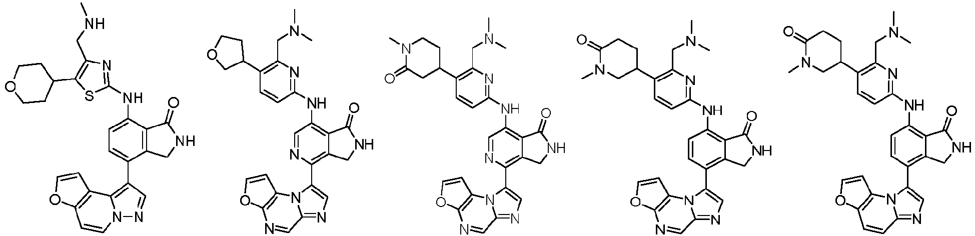

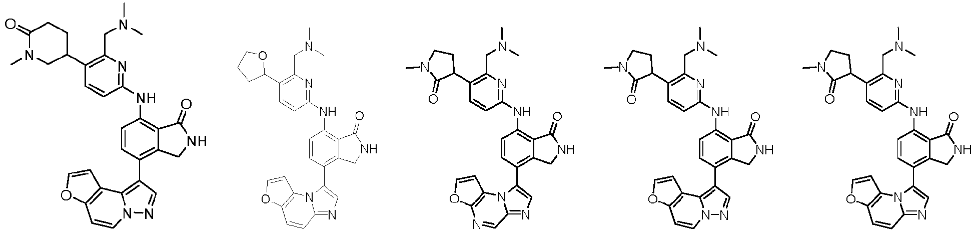

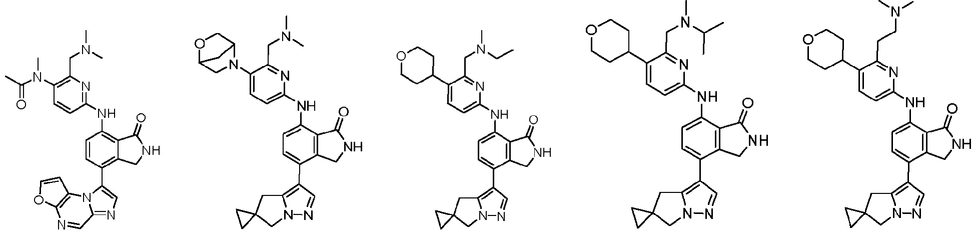

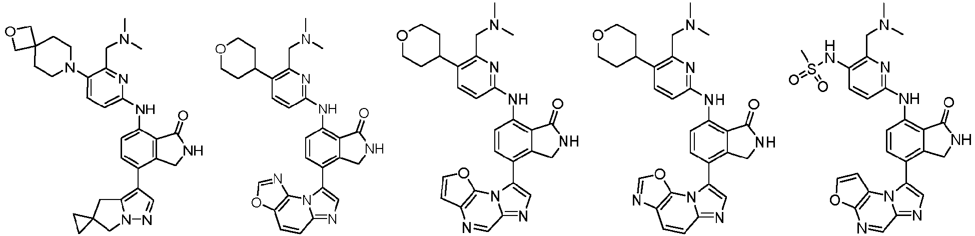

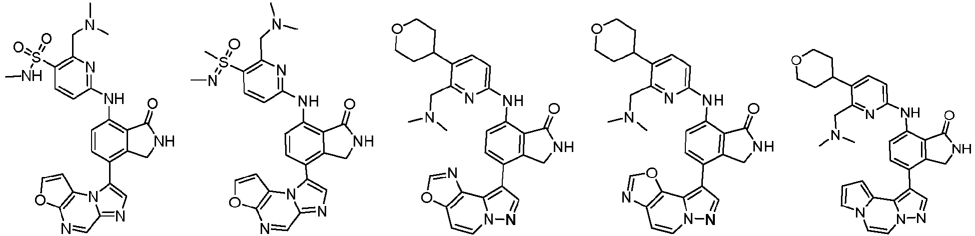

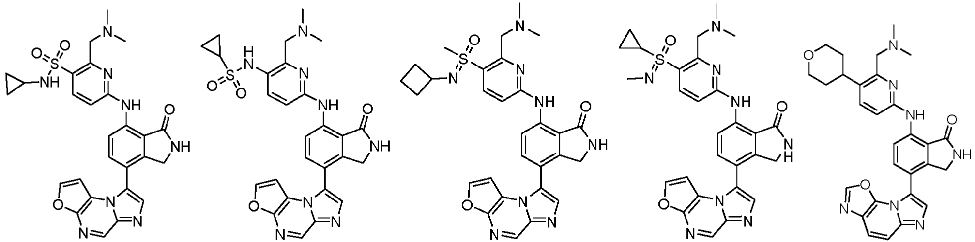

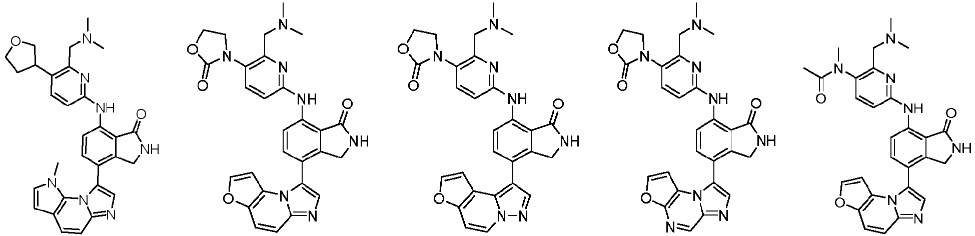

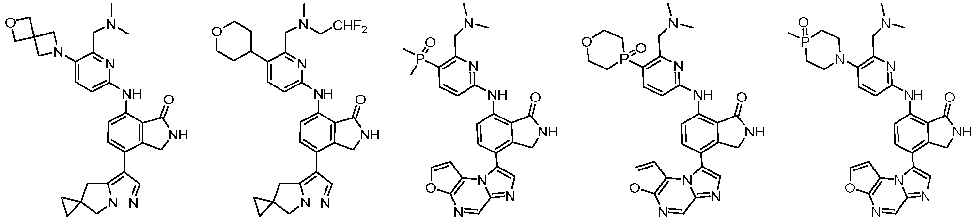

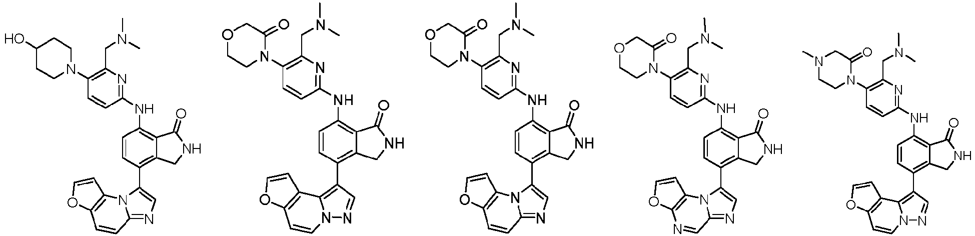

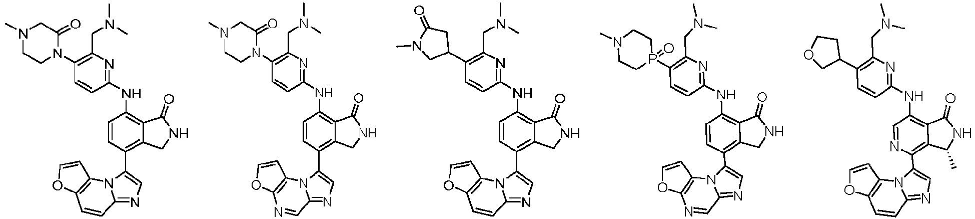

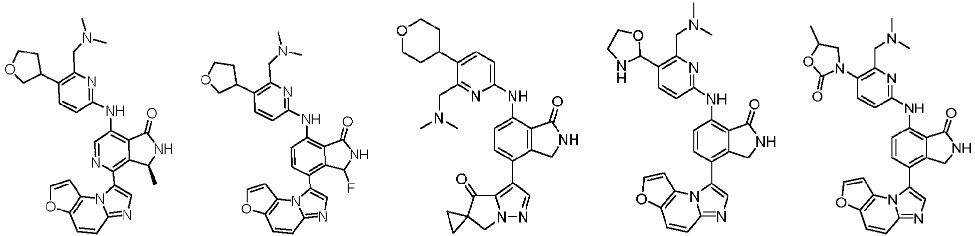

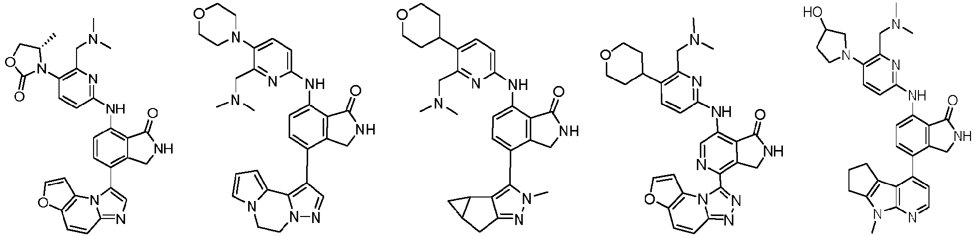

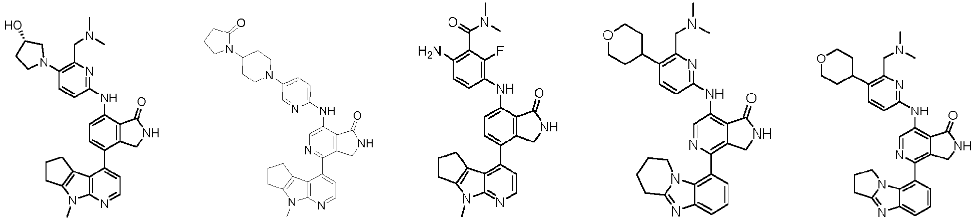

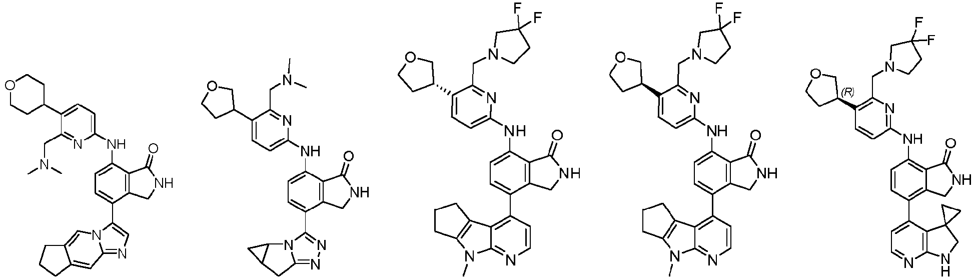

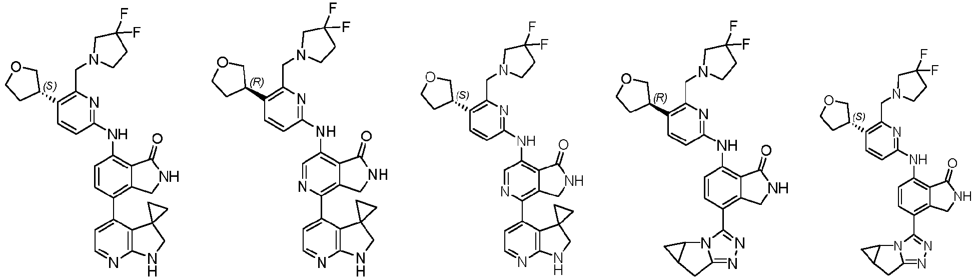

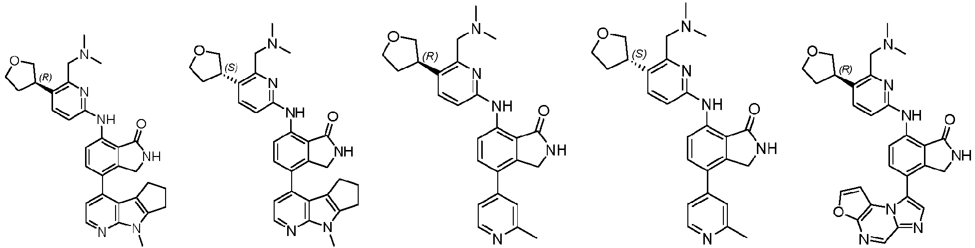

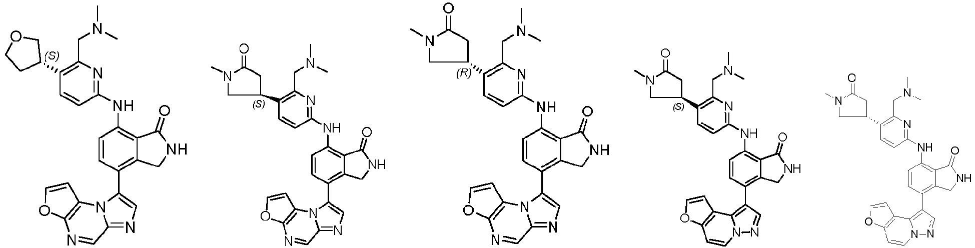

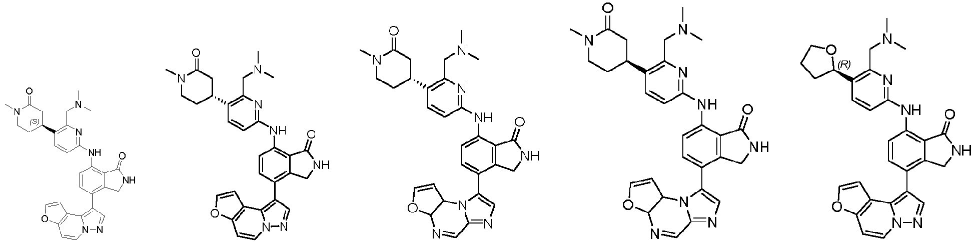

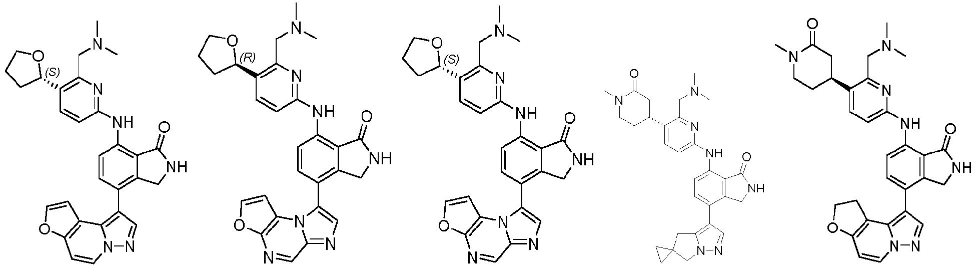

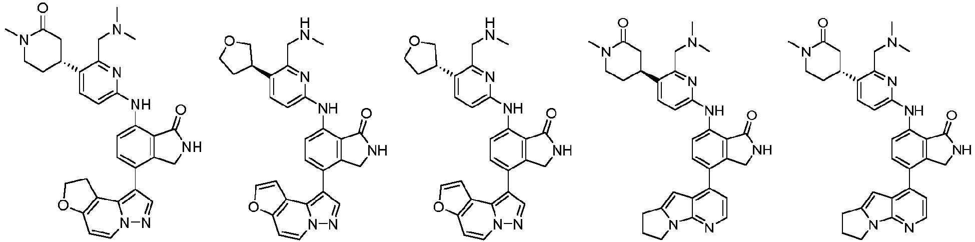

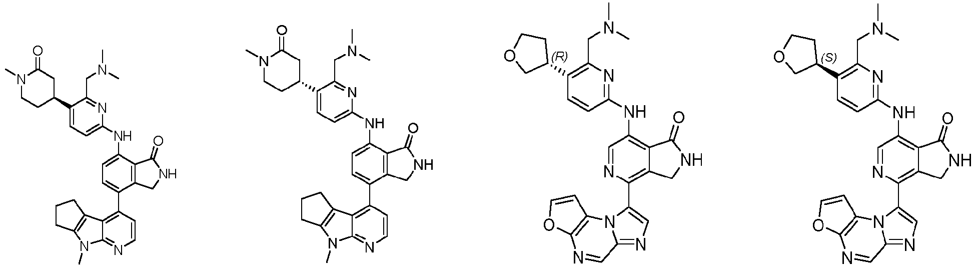

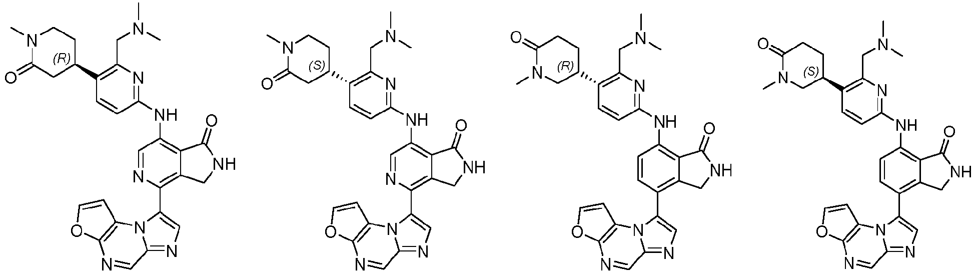

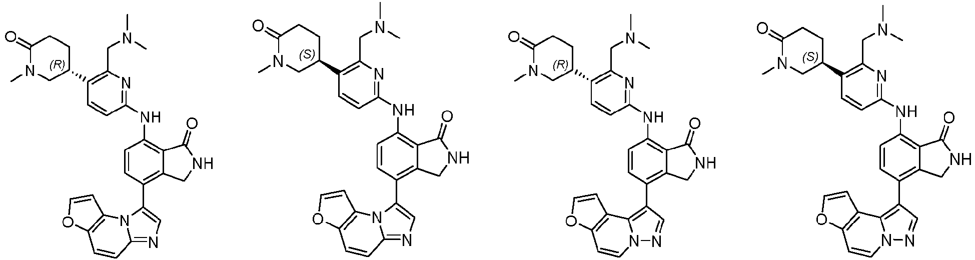

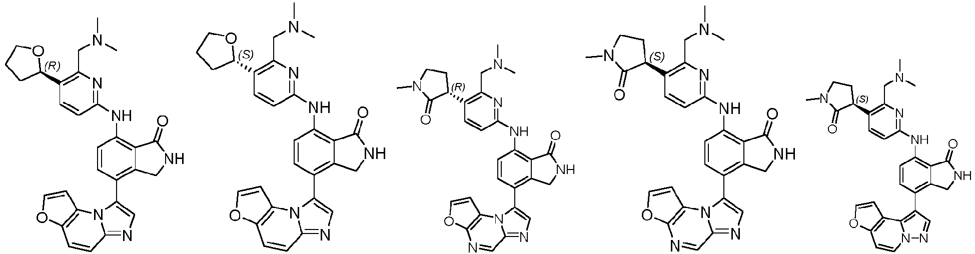

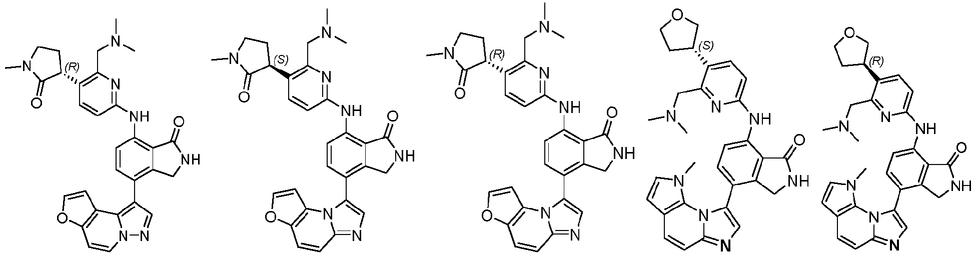

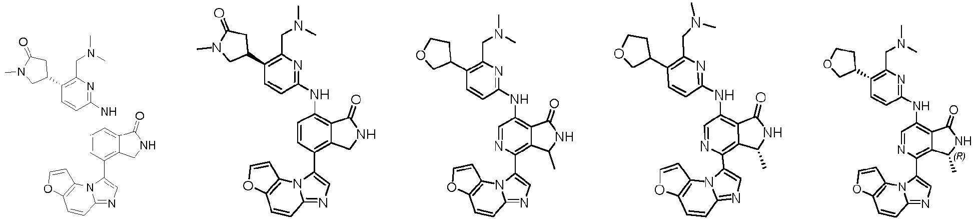

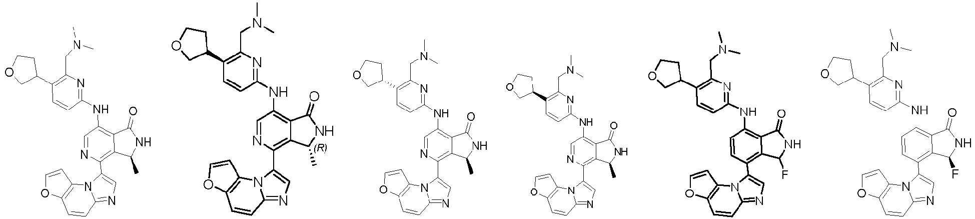

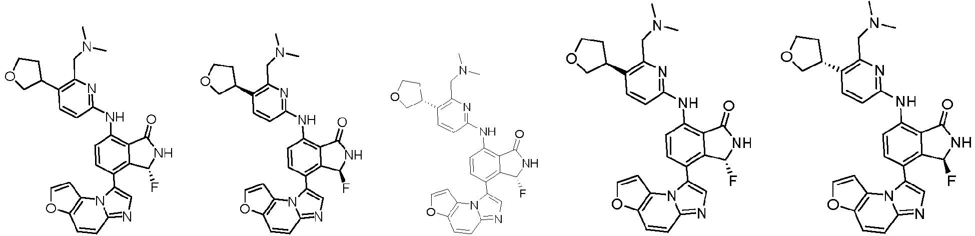

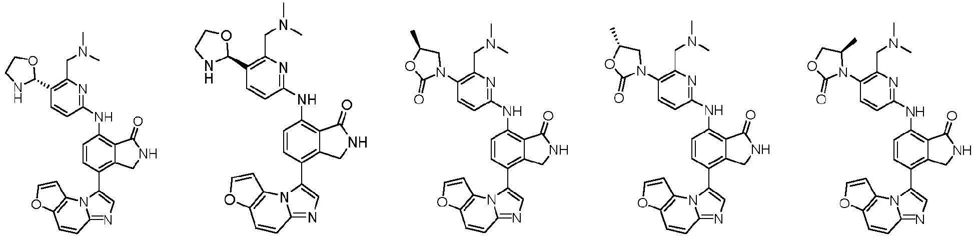

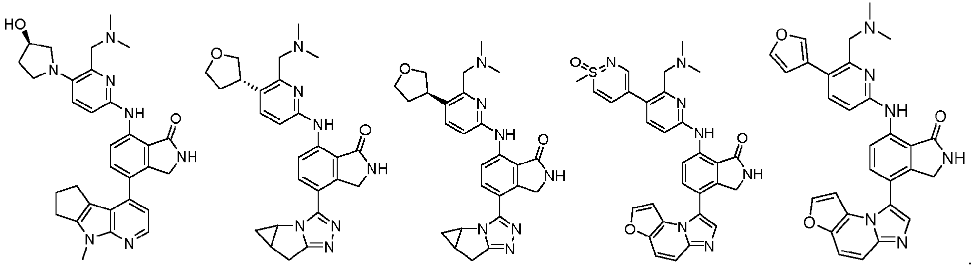

In another aspect, the present application provides a pharmaceutical composition comprising the compound, the stereoisomer thereof, or the pharmaceutically acceptable salt thereof of the present application. In some embodiments, the pharmaceutical composition of the present application further comprises a pharmaceutically acceptable excipient.

In another aspect, the present application provides a method for treating a disease related to HPK1 kinase in a mammal, which comprises administering to a mammal, preferably a human, in need of such treatment a therapeutically effective amount of the compound, the stereoisomer thereof, or the pharmaceutically acceptable salt thereof, or the pharmaceutical composition thereof described above.

In another aspect, the present application provides use of the compound, the stereoisomer, or the pharmaceutically acceptable salt thereof, or the pharmaceutical composition thereof described above for preparing a medicament for treating a disease related to HPK1 kinase.

In another aspect, the present application provides use of the compound, the stereoisomer thereof, or the pharmaceutically acceptable salt thereof, or the pharmaceutical composition thereof described above for treating a disease related to HPK1 kinase.

In another aspect, the present application provides the compound, the stereoisomer thereof, or the pharmaceutically acceptable salt thereof, or the pharmaceutical composition thereof described above for treating a disease related to HPK1 kinase.

In some embodiments, the disease related to HPK1 kinase is selected from cancer. Preferably, the cancer is selected from the group consisting of a solid tumor, leukemia, lymphoma, and the like.

The compound of the present application has at least one of the following effects: improved or excellent inhibitory activity against HPK1 kinase and inhibitory activity against p-SLP76 phosphorylation of Jurkat cells, improved or excellent *in vivo* drug effect, and good *in vitro* and *in vivo* pharmacokinetic properties, for example, stable liver microsome metabolism in mice and humans.

### Definitions

Unless otherwise stated, the following terms used in the present application shall have the following meanings. A certain term, unless otherwise specifically defined, should not be considered uncertain or unclear, but construed according to its common meaning in the art. When referring to a trade name, it is intended to refer to its corresponding commercial product or its active ingredient.

The term "substituted" means that any one or more hydrogen atoms on a specific atom are substituted with substituents, as long as the valence of the specific atom is normal and the resulting compound is stable. When the substituent is oxo (i.e., =O), it means that two hydrogen atoms are substituted and oxo is not possible in an aromatic group.

The "substituent" described herein includes all substituents mentioned in the text, for example, the terms "halogen", "deuterium", "", "-NH₂", "-NH(C₁₋₄ alkyl)", "-N(C₁₋₄ alkyl)₂", "-OH", "-OC₁₋₄ alkyl", "-CN", "C₁₋₄ alkyl", "3-to 6-membered heterocycloalkyl", and the like, and the corresponding non-limiting or exemplary groups mentioned below, wherein some non-limiting examples of the "substituent" include sulfydryl, nitro, nitroso, cyano, an azide group, a sulfoxide group, a sulfone group, a sulfonamide group, carboxyl, an aldehyde group, an imine group, alkyl, halogenated alkyl, cycloalkyl, halogenated cycloalkyl, alkenyl, halogenated alkenyl, cycloalkenyl, halogenated cycloalkenyl, alkynyl, halogenated alkynyl, cycloalkynyl, halogenated cycloalkynyl, heteroalkyl, halogenated heteroalkyl, alkoxy, alkylthio, aryl, aryloxy, arylthio, arylalkylene, arylalkoxy, arylalkylthio, heteroaryl, heteroaryloxy, heteroarylthio, heteroarylalkylene, heteroarylalkoxy, heteroarylalkylthio, heterocyclyl, heterocyclyloxy, heterocyclylthio, heterocyclylalkylene, heterocyclylalkoxy, heterocyclylalkylthio, acyl, acyloxy, a carbamate group, amido, ureido, an epoxy group, an ester group, oxo, and the like, wherein these substituents are optionally substituted with one or more substituents selected from the group consisting of the following substituents: oxo, hydroxy, amino, nitro, halogen, cyano, alkyl, alkenyl, alkynyl, alkoxy, halogenated alkoxy, alkylamino, dialkylamino, halogenated alkylamino, halogenated dialkylamino, carboxy, -C(O)O-alkyl, -OC(O)-alkyl, - C(O)NH₂, -C(O)NH-alkyl, -C(O)N(alkyl)₂, -NHC(O)-alkyl, -C(O)-alkyl, -S(O)-alkyl, -S(O)₂- alkyl, -S(O)₂NH₂, - S(O)zNH-alkyl, -S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

In some embodiments herein, the substituent is selected from the group consisting of deuterium, tritium, hydroxyl, sulfydryl, halogen, amino, nitro, nitroso, cyano, azido, a sulfoxide group, a sulfone group, a sulfonamide group, carboxyl, an aldehyde group, an imine group, C₁₋₁₂ alkyl, halogenated C₁₋₁₂ alkyl, 3- to 12-membered cycloalkyl, halogenated 3- to 12-membered cycloalkyl, C₂₋₁₂ alkenyl, halogenated C₂₋₁₂ alkenyl, 3- to 12-membered cycloalkenyl, halogenated 3- to 12-membered cycloalkenyl, C₂₋₁₂ alkynyl, halogenated C₂₋₁₂ alkynyl, 8- to 12-membered cycloalkynyl, halogenated 8- to 12-membered cycloalkynyl, C₁₋₁₂ heteroalkyl, halogenated C₁₋₁₂ heteroalkyl, C₁₋₁₂ alkoxy, C₁₋₁₂ alkylthio, 6- to 10-membered aryl, 6- to 10-membered aryloxy, 6- to 10-membered arylthio, 6- to 10-membered aryl C₁₋₁₂ alkylene, 6- to 10-membered aryl C₁₋₁₂ alkoxy, 6- to 10-membered aryl C₁₋₁₂ alkylthio, 5- to 10-membered heteroaryl, 5- to 10-membered heteroaryloxy, 5- to 10-membered heteroarylthio, 5-to 10-membered heteroarylalkylene, 5- to 10-membered heteroarylalkoxy, 5- to 10-membered heteroarylalkylthio, 3- to 12-membered heterocyclyl, 3- to 12-membered heterocyclyloxy, 3- to 12-membered heterocyclylthio, 3- to 12-membered heterocyclyl C₁₋₁₂ alkylene, 3-to 12-membered heterocyclyl C₁₋₁₂ alkoxy, 3- to 12-membered heterocyclyl C₁₋₁₂ alkylthio, C₁₋₁₂ acyl, C₁₋₁₂ acyloxy, a carbamate group, C₁₋₁₂ amido, ureido, an epoxy group, a C₂₋₁₂ ester group, and oxo, wherein these substituents are optionally substituted with one or more substituents selected from the group consisting of the following substituents: oxo, hydroxyl, amino, nitro, halogen, cyano, C₁₋₁₂ alkyl, C₂₋₁₂ alkenyl, C₂₋₁₂ alkynyl, C₁₋₁₂ alkoxy, halogenated C₁₋₁₂ alkoxy, C₁₋₁₂ alkylamino, di-C₁₋₁₂ alkylamino, halogenated C₁₋₁₂ alkylamino, halogenated di-C₁₋₁₂ alkylamino, carboxyl, -C(O)O-C₁₋₁₂ alkyl, -OC(O)-C₁₋₁₂ alkyl, - C(O)NH₂, -C(O)NH-C₁₋₁₂ alkyl, -C(O)N(C₁₋₁₂ alkyl)₂, -NHC(O)-C₁₋₁₂ alkyl, -C(O)-C₁₋₁₂ alkyl, -S(O)-C₁₋₁₂ alkyl, - S(O)₂-C₁₋₁₂ alkyl, -S(O)₂NH₂, -S(O)₂NH-C₁₋₁₂ alkyl, -S(O)₂N(C₁₋₁₂ alkyl)₂, 3- to 12-membered cycloalkyl, 3- to 12-membered cycloalkyl C₁₋₁₂ alkylene, 3- to 12-membered cycloalkyloxy, 3- to 12-membered heterocyclyl, 3- to 12-membered heterocyclyl C₁₋₁₂ alkylene, 3- to 12-membered heterocyclyloxy, 3- to 12-membered heterocycloalkyl, 3- to 12-membered heterocycloalkyl C₁₋₁₂ alkylene, 3- to 12-membered heterocycloalkyloxy, 5- to 10-membered heteroaryl, 5- to 10-membered heteroaryl C₁₋₁₂ alkylene, 5- to 10-membered heteroaryloxy, 6- to 10-membered aryl, 6- to 10-membered aryl C₁₋₁₂ alkylene, and 6- to 10-membered aryloxy.

The term "optional" or "optionally" means that the subsequently described event or circumstance may, but does not necessarily, occur. The description includes instances where the event or circumstance occurs and instances where it does not. For example, ethyl being "optionally" substituted with halogen means that the ethyl may be unsubstituted (-CH₂CH₃), monosubstituted (for example, -CH₂CH₂F), polysubstituted (for example, -CHFCH₂F, -CH₂CHF₂, and the like), or fully substituted (-CF₂CF₃). It can be understood by those skilled in the art that for any groups comprising one or more substituents, no substitutions or substitution modes that are impossible to spatially exist and/or synthesize will be introduced.

Cₘ₋ₙ used herein means that the moiety has an integer number of carbon atoms in the given range. For example, "C₁₋₆" means that the group may have 1 carbon atom, 2 carbon atoms, 3 carbon atoms, 4 carbon atoms, 5 carbon atoms or 6 carbon atoms; "C₁₋₃" means that the group may have 1 carbon atom, 2 carbon atoms or 3 carbon atoms. "One or more" used herein refers to an integer ranging from one to ten. For example, "one or more" refers to one, two, three, four, five, six, seven, eight, nine, or ten; or "one or more" refers to one, two, three, four, five, or six; or "one or more" refers to one, two, or three.

When any variable (e.g., R) occurs once or more times in the constitution or structure of a compound, the definition of the variable in each case is independent. Therefore, for example, if a group is substituted with 2 R, the definition of each R is independent.

When a bond of a substituent is cross-linked to two atoms on a ring, the substituent can be bonded to any atom on the ring. For example, structural unit 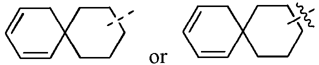 represents that substitution may occur at any one position of cyclohexyl or cyclohexadienyl.

The term "halo-" or "halogen" refers to fluorine, chlorine, bromine, and iodine.

The term "hydroxy" refers to -OH group.

The term "amino" refers to -NH₂ group.

The term "nitro" refers to -NO₂ group.

The term "cyano" refers to -CN group.

The term "alkyl" refers to hydrocarbyl with a general formula of CₙH₂ₙ₊₁. The alkyl may be linear or branched. For example, the term "C₁₋₆ alkyl" refers to alkyl containing 1 to 6 carbon atoms (for example, methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, and the like). The alkyl moieties (i.e., alkyl) of alkoxy, alkylamino, dialkylamino, alkylsulfonyl, and alkylthio are similar as defined above.

The term "alkoxy" refers to -O- alkyl.

The term "monocyclic" refers to a cyclic group containing one ring, which may be fully saturated, partially saturated, or aromatic. The monocyclic ring may consist entirely of C atoms or may contain one or more heteroatoms, for example, selected from the group consisting of N, O, S, and P.

The term "bicyclic" refers to a cyclic group containing two rings, which may be fully saturated, partially saturated, or aromatic. The bicyclic ring may consist entirely of C atoms or may contain one or more heteroatoms, for example, selected from the group consisting of N, O, S, and P. The bicyclic ring may be a fused, bridged, or spiro ring.

The term "tricyclic" refers to a cyclic group containing three rings, which may be fully saturated, partially saturated, or aromatic. The tricyclic ring may consist entirely of C atoms or may contain one or more heteroatoms, for example, selected from the group consisting of N, O, S, and P. Any two adjacent monocyclic rings in the tricyclic ring can form a fused, bridged, or spiro ring.

The term "cycloalkyl" refers to a fully saturated carbocyclic ring. Unless otherwise specified, the carbocyclic ring is usually a 3- to 10-membered ring. Unless otherwise specified, the cycloalkyl may be monocyclic, bicyclic, or tricyclic. Non-limiting examples of the cycloalkyl include, but are not limited to, cyclopropyl, cyclobutyl, cyclopentyl, cyclohexyl, norbomyl (bicyclo[2.2.1]heptyl), bicyclo[2.2.2]octyl, adamantyl, and the like.

The term "heterocyclyl" refers to a fully saturated or partially unsaturated (but not a fully unsaturated heteroaromatic group) nonaromatic ring that may exist in the form of a monocyclic, bridged cyclic, fused cyclic, or spiro cyclic structure. Unless otherwise specified, the heterocyclic ring is usually a 3- to 20-membered ring, a 3- to 15-membered ring, a 3- to 12-membered ring, a 3- to 10-membered ring (e.g., a 3-, 4-, 5-, 6-, 7-, 8-, 9-, or 10-membered ring), a 4- to 8-membered ring, a 5- to 8-membered ring, or a 5- to 6-membered ring containing 1 to 3 heteroatoms (preferably 1 or 2 heteroatoms) independently selected from the group consisting of sulfur, oxygen, nitrogen, phosphorus, silicon, and/or boron. Non-limiting examples of the heterocyclyl include, but are not limited to, oxiranyl, tetrahydrofuranyl, dihydrofuranyl, pyrrolidinyl, N-methylpyrrolidinyl, dihydropyrrolyl, piperidinyl, piperazinyl, pyrazolidinyl, tetrahydropyranyl, morpholinyl, sulfomorpholinyl, tetrahydrothienyl, and the like.

The term "cycloalkenyl" refers to a non-aromatic carbocyclic ring that is not fully saturated and may exist as a monocyclic, bicyclic bridged cyclic, or spiro cyclic structure. Unless otherwise specified, the carbocyclic ring is usually a 4- to 16-membered ring, a 4- to 12-membered ring, a 4- to 10-membered ring, or a 4- to 8-membered ring (specifically, a 5-, 6-, 7-, 8-, 9-, 10-, or 11-membered ring). Non-limiting examples of cycloalkenyl include, but are not limited to, cyclopentenyl, cyclopentadienyl, cyclohexenyl, cyclohexadienyl, cycloheptenyl, cycloheptadienyl, and the like.

The term "monocycloalkyl" refers to a cycloalkyl group that exists as a single ring.

The term "spiro ring" refers to a fully saturated or partially unsaturated polycyclic ring system in which monocyclic rings share one carbon atom (referred to as a spiro atom), including carbocyclic rings and heterocyclic rings. Unless otherwise specified, the spiro ring is 5- to 20-membered, preferably 6- to 14- membered, and more preferably 9- to 14-membered. When the spiro ring is a heterocyclic ring, one or more ring atoms in the polycyclic ring are heteroatoms (preferably 1 or 2 heteroatoms) selected from the group consisting of N, O, S(O)ₙ, and P(O)ₙ (wherein n is 0, 1, or 2), and the remaining ring atoms are carbon atoms.

The term "spirocycloalkyl" refers to a fully saturated all-carbon polycyclic ring in which monocyclic rings share one carbon atom (referred to as a spiro atom). Unless otherwise specified, the spirocycloalkyl is 5- to 20-membered, preferably 6- to 14-membered, and more preferably 9- to 14-membered. According to the number of spiro atoms shared among the rings, the spirocycloalkyl is monospirocycloalkyl, bispirocycloalkyl, or polyspirocycloalkyl, preferably monospirocycloalkyl or bispirocycloalkyl, and more preferably 4 membered/4 membered, 4 membered/5-membered, 4 membered/6-membered, 5-membered/5-membered, or 5-membered/6-membered monospirocycloalkyl. Non-limiting examples of the spirocycloalkyl include

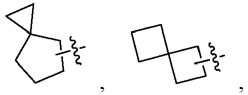

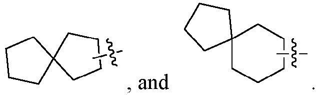

The term "spiro-heterocycloalkyl" refers to a fully saturated polycyclic ring in which monocyclic rings share one carbon atom (referred to as a spiro atom), wherein one or more ring atoms in the polycyclic ring are heteroatoms (preferably 1 or 2 heteroatoms) selected from the group consisting of N, O, S(O)ₙ, and P(O)ₙ (wherein n is 0, 1, or 2), and the remaining ring atoms are carbon atoms. Unless otherwise specified, the spiro-heterocycloalkyl is 5- to 20-membered, preferably 6- to 14-membered, and more preferably 6- to 10-membered. According to the number of spiro atoms shared among the rings, the spiro heterocyclic ring is a monospiro heterocyclic ring, a bispiro heterocyclic ring, or a polyspiro heterocyclic ring, preferably a monospiro heterocyclic ring or a bispiro heterocyclic ring, and more preferably a 4-membered/4-membered, 4-membered/5-membered, 4-membered/6-membered, 5-membered/5-membered, or 5-membered/6-membered monospiro heterocyclic ring. Non-limiting examples of the spiro-heterocycloalkyl include 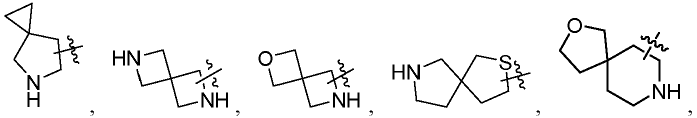 or the like.

The term "bridged ring" refers to a fully saturated or partially unsaturated polycyclic ring system in which two rings share three or more atoms, including carbocyclic rings and heterocyclic rings. Unless otherwise specified, the bridged ring is 5- to 14-membered, preferably 6- to 14-membered, and more preferably 6- to 10-membered. According to the number of the formed rings, the bridged ring may be a bicyclic, tricyclic, tetracyclic, or polycyclic bridged heterocyclic ring, preferably a bicyclic or tricyclic bridged heterocyclic ring, and more preferably a bicyclic bridged heterocyclic ring. When the bridged ring is a heterocyclic ring, one or more ring atoms in the polycyclic ring are heteroatoms (preferably 1 or 2 heteroatoms) selected from the group consisting of N, O, S(O)ₙ, and P(O)ₙ (wherein n is 0, 1, or 2), and the remaining ring atoms are carbon atoms.

The term "bridged cycloalkyl" refers to a fully saturated all-carbon polycyclic ring in which two rings share three or more atoms. Unless otherwise specified, the bridged cycloalkyl is 5- to 14-membered, preferably 6- to 14-membered, and more preferably 6- to 10-membered. According to the number of the formed rings, the bridged cycloalkyl may be a bicyclic, tricyclic, tetracyclic, or polycyclic bridged heterocyclic ring, preferably a bicyclic or tricyclic bridged heterocyclic ring, and more preferably a bicyclic bridged heterocyclic ring. Non-limiting examples of the bridged cycloalkyl include: 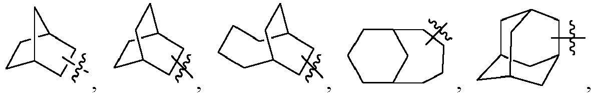 or the like.

The term "bridged heterocycloalkyl" is a fully saturated polycyclic ring in which two rings share three or more atoms, wherein one or more ring atoms are heteroatoms selected from the group consisting of N, O, and S(O)ₙ, P(O)ₙ (wherein n is 0, 1, or 2), and the remaining ring atoms are carbon atoms. Unless otherwise specified, the bridged heterocycloalkyl is 5- to 14-membered, preferably 6- to 14-membered, and more preferably 6- to 10-membered. According to the number of the formed rings, the bridged heterocycloalkyl may be a bicyclic, tricyclic, tetracyclic, or polycyclic bridged heterocyclic ring, preferably a bicyclic or tricyclic bridged heterocyclic ring, and more preferably a bicyclic bridged heterocyclic ring. Non-limiting examples of the bridged heterocycloalkyl include: 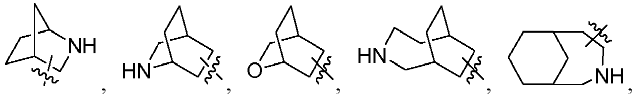 or the like.

The term "heterocycloalkyl" refers to a fully saturated cyclic group containing heteroatoms. Unless otherwise specified, the heterocycloalkyl is usually a ring containing 1-3 heteroatoms (preferably 1 or 2 heteroatoms) independently selected from the group consisting of N, O, S(O)ₙ, and P(O)ₙ (wherein n is 0, 1 or 2). Unless otherwise specified, the heterocycloalkyl may be a monocyclic, bicyclic, or tricyclic group. Unless otherwise specified, the heterocycloalkyl includes, but is not limited to, a 3- to 12-membered ring, a 3- to 8-membered ring, or a 5- to 8-membered ring. Examples of 3-membered heterocycloalkyl include, but are not limited to, oxiranyl, thiiranyl, and aziranyl. Non-limiting examples of 4-membered heterocycloalkyl include, but are not limited to, azetidinyl, oxetanyl, and thietanyl. Examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl. Examples of 6-membered heterocycloalkyl include, but are not limited to, piperidinyl, tetrahydropyranyl, tetrahydrothiopyranyl, morpholinyl, piperazinyl, 1,4-oxathianyl, 1,4-dioxanyl, thiomorpholinyl, 1,3-dithianyl, or 1,4-dithianyl. Examples of 7-membered heterocycloalkyl include, but are not limited to, azepanyl, oxepanyl, and thiepanyl. Monocyclic heterocycloalkyl having 5 or 6 ring atoms is preferred. The term "heterocycloalkenyl" includes cycloalkenyl in which one or more carbon atoms are substituted with a heteroatom, specifically, for example, cycloalkenyl in which up to 3 carbon atoms, up to 2 carbon atoms, and in one embodiment 1 carbon atom, are each independently replaced by N, O, or S(O), provided that at least one cycloalkenyl carbon-carbon double bond is preserved. The heterocycloalkenyl that may exist in the form of a monocyclic, bridged cyclic, or spiro cyclic structure may be a 3-to 16-membered ring (for example, a 3-to 12-membered or 5-to 8-membered ring, specifically, for example, a 5-membered, 6-membered, 7-membered, 8-membered, 9-membered, 10-membered, or 11-membered ring). Examples of the heterocycloalkenyl include, but are not limited to, dihydropyridinyl, dihydropyrrolyl, tetrahydropyridinyl, tetrahydroazepinyl, or azaspirooctene.

The term "monoheterocycloalkyl" refers to a heterocycloalkyl group that exists as a single ring.

The term "fused ring" refers to polycyclic compounds formed when two or more carbocyclic or heterocyclic rings are parallelized through two atoms shared thereby, including fully saturated, partially saturated, and aromatic rings. Unless otherwise specified, the fused rings are 5- to 20-membered, preferably 6- to 14-membered, and more preferably 9- to 14-membered. Non-limiting examples of the fused ring include, but are not limited to, naphthalene, anthracene, phenanthrene, 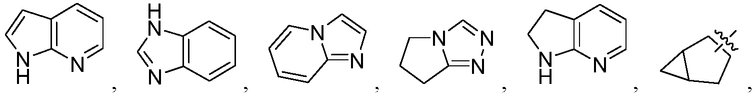 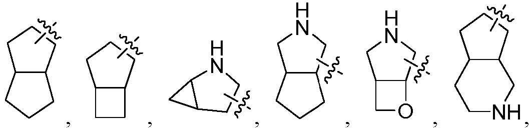 or the like.

As used herein, taking 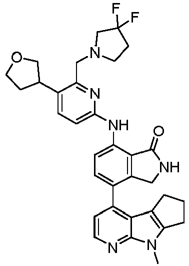 as an example, the "monocyclic ring" in "the monocyclic ring in the tricyclic ring to which the 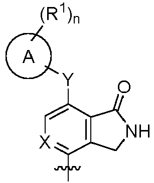 moiety of formula (I) is attached is an aromatic ring" refers to the pyridinyl in the tricyclic ring 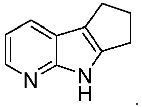 Taking 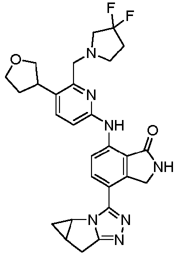 as another example, the "monocyclic ring" in "the monocyclic ring of in tricyclic ring to which the 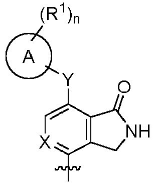 moiety of formula (I) is attached is an aromatic ring" refers to the 1,2,4-triazolyl group in the tricyclic ring

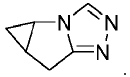

As used herein, the taking 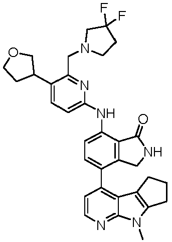 as an example, "the monocyclic ring in the tricyclic ring to which the 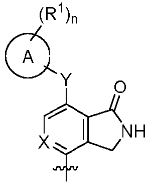 moiety of formula (I) is attached and the ring adjacent thereto form a fused ring" means that the pyridinyl and the pyrrolyl in the tricyclic ring 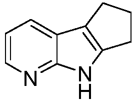 form a fused ring 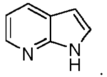 Taking 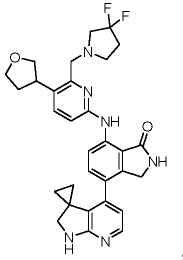 as another example, "the monocyclic ring in the tricyclic ring to which the 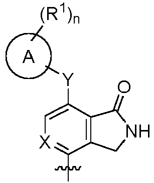 moiety of formula (I) is attached and the ring adjacent thereto form a fused ring" means that the pyridinyl and the tetrahydropyrrolyl in the tricyclic ring 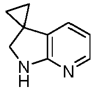 form a fused ring

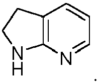

The term "aryl" refers to an all-carbon aromatic monocyclic or fused polycyclic group having a conjugated π-electron system. Unless otherwise specified, aryl may have 6-20 carbon atoms, 6-14 carbon atoms, or 6-12 carbon atoms. Non-limiting examples of the aryl include, but are not limited to, phenyl, naphthyl, anthryl, 1,2,3,4-tetrahydronaphthalene, and the like.

The term "heteroaryl" refers to a monocyclic or polycyclic ring system that contains at least one ring atom selected from the group consisting of N, O, S(O)ₙ, and P(O)ₙ (wherein n is 0, 1, or 2), with the remaining ring atoms being C, and that has at least one aromatic ring. Unless otherwise specified, the heteroaryl may be monocyclic, bicyclic, or tricyclic. Unless otherwise specified, the heteroaryl may have a single 5- to 8-membered ring, or multiple fused rings containing 6 to 14, especially 6 to 10 ring atoms. Non-limiting examples of the heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, and the like.

The term "treat", "treating", or "treatment" refers to administering the compound or formulation described herein to ameliorate or eliminate a disease or one or more symptoms related to the disease, including:
(i) inhibiting a disease or disease state, i.e., arresting its progression; and
(ii) alleviating a disease or disease state, i.e., causing its regression.

The term "therapeutically effective amount" refers to an amount of the compound described herein for (i) treating the specific disease, condition or disorder described herein; (ii) alleviating, ameliorating, or eliminating one or more symptoms of the specific disease, condition or disorder described herein, or (iii) preventing or delaying onset of one or more symptoms of the specific disease, condition or disorder described herein. The amount of the compound of the present application composing the "therapeutically effective amount" varies depending on the compound, the disease state and its severity, the mode of administration, and the age of the mammal to be treated, but can be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

A pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "pharmaceutical composition" refers to a mixture consisting of one or more of the compounds or the salts thereof of the present application and a pharmaceutically acceptable excipient. The pharmaceutical composition is intended to facilitate the administration of the compound of the present application to an organic entity.

The term "pharmaceutically acceptable excipient" refers to those that do not have a significant irritating effect on an organic entity and do not impair the biological activity and properties of the active compound. Suitable excipients are well known to those skilled in the art, for example, carbohydrate, wax, water-soluble and/or water-swellable polymers, hydrophilic or hydrophobic material, gelatin, oil, solvent, or water.

The word "comprise" and variations thereof such as "comprises" or "comprising" will be understood in an open, non-exclusive sense, i.e., "including but not limited to".

Unless otherwise specified, terms in the singular shall be deemed to include the plural and vice versa. Unless otherwise specified, the word "a" or "an" refers to "at least one". Unless otherwise stated, "or" is used to mean "and/or".

The compounds of the present invention may exist in the form of a specific geometric isomer or stereoisomer. All such compounds are contemplated herein, including *cis* and *trans* isomers, (-)- and (+)-enantiomers, (*R*)- and (5)-enantiomers, diastereoisomers, (D)-isomers, (L)-isomers, and racemic mixtures and other mixtures thereof, such as an enantiomer or diastereoisomer enriched mixture, all of which are encompassed within the scope of the present invention. The substituents such as alkyl may have an additional asymmetric carbon atom. All these isomers and mixtures thereof are encompassed within the scope of the present invention.

Unless otherwise stated, "(D)" or "(+)" stands for dextrorotation, "(L)" or "(-)" stands for levorotation, and *"(DL)"* or "(±)" stands for racemization.

Unless otherwise stated, the absolute configuration of a stereogenic center is represented by a wedged solid bond 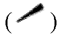 and a wedged dashed bond 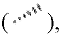 and the relative configuration of a stereogenic center is represented by a straight solid bond 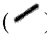 1 and a straight dashed bond

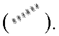

Optically active (*R*)- and (S)-isomers and *D* and *L* isomers can be prepared by chiral synthesis or chiral reagents or other conventional techniques. If one enantiomer of a certain compound of the present invention is to be obtained, the desired pure enantiomer can be prepared by asymmetric synthesis or derivatization using a chiral additive, wherein the resulting diastereoisomeric mixture is separated and the auxiliary group is cleaved. Alternatively, when the molecule contains a basic functional group (for example, amino) or an acidic functional group (for example, carboxyl), the compound reacts with an appropriate optically active acid or base to form a salt of the diastereoisomer, which is then subjected to diastereoisomeric resolution through conventional methods well known in the art to give the pure enantiomer. Furthermore, the enantiomer and the diastereoisomer are generally isolated through chromatography using a chiral stationary phase, optionally in combination with chemical derivatization (e.g., carbamate generated from amines).

The present application also comprises isotopically labeled compounds of the present application which are identical to those recited herein but have one or more atoms replaced by an atom having an atomic mass or mass number different from the atomic mass or mass number generally found in nature. Examples of isotopes that can be incorporated into the compounds of the present application include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, ³⁶Cl, and the like.

Certain isotopically labeled compounds of the present application (e.g., those labeled with ³H and ¹⁴C) can be used to analyze compounds and/or substrate tissue distribution. Tritium (i.e., ³H) and carbon-14 (i.e., ¹⁴C) isotopes are particularly preferred for their ease of preparation and detectability. Positron emitting isotopes, such as ¹⁵O, ¹³N, ¹¹C, and ¹⁸F, can be used in positron emission tomography (PET) studies to determine substrate occupancy. Isotopically labeled compounds of the present application can generally be prepared by following procedures analogous to those disclosed in the schemes and/or examples below while substituting a non-isotopically labeled reagent with an isotopically labeled reagent.

Furthermore, substitution with heavier isotopes such as deuterium (i.e., ²H or D) may afford certain therapeutic advantages resulting from greater metabolic stability (for example, increased *in vivo* half-life or reduced dosage requirements) and hence may be preferred in some circumstances in which deuterium substitution may be partial or complete. Partial deuterium substitution refers to substitution of at least one hydrogen with at least one deuterium, and complete deuterium substitution refers to substitution of all hydrogens of the group with deuterium, for example, methyl (-CH₃) completely substituted with deuterium is -CD₃.

The compounds of the present application may also exist in different tautomeric forms, and all such forms are included within the scope of the present application. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerism and imine-enamine isomerization. A specific example of a proton tautomer may be an imidazole moiety where a proton can transfer between two ring nitrogen atoms.

The pharmaceutical composition of the present application may be prepared by combining the compound of the present application with a suitable pharmaceutically acceptable excipient.

Typical routes of administration of the compound or the pharmaceutically acceptable salt thereof or the pharmaceutical composition thereof of the present application include, but are not limited to, oral, local, inhalation, parenteral, intranasal, intraocular, intramuscular, subcutaneous, and intravenous administration.

The pharmaceutical composition of the present application can be manufactured using methods well known in the art, such as conventional mixing, dissolving, granulating, dragee-making, levigating, emulsifying, lyophilizing, and the like.

In all of the administration methods of the compound of general formula II described herein, the daily dose administered is from 0.001 mg/kg body weight to 2000 mg/kg body weight, given in individual or separated doses. The compounds of the present application can be prepared by a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application.

The chemical reactions of the specific embodiments of the present application are conducted in a proper solvent that must be suitable for the chemical changes in the present application and the reagents and materials required. In order to obtain the compounds of the present application, it is sometimes necessary for those skilled in the art to modify or select a synthesis procedure or a reaction process based on the existing embodiments.

An important consideration in synthetic route planning in the art is the selection of suitable protecting groups for reactive functional groups (e.g., amino in the present application). For example, reference may be made to Greene's Protective Groups in Organic Synthesis (4th Ed.) Hoboken, New Jersey: John Wiley & Sons, Inc. All references cited herein are incorporated by reference in their entirety.

In some embodiments, the compound of formula II of the present application can be prepared by those skilled in the art of organic synthesis according to the following routes:
Route 1: 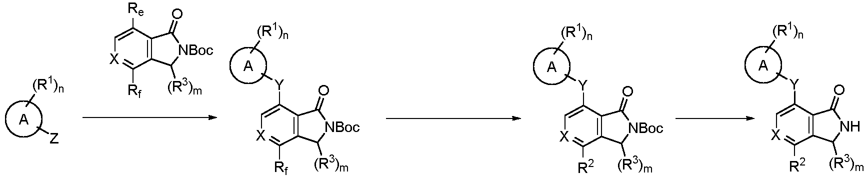
Route 2: 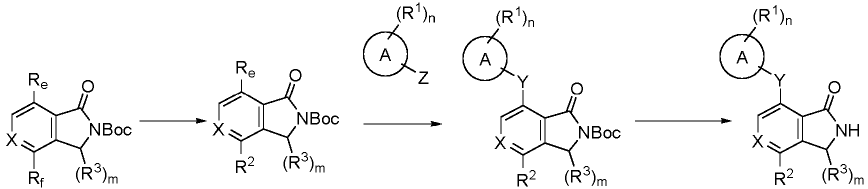
   wherein, Z is selected from the group consisting of halogen, OH, NH₂, NH(C₁₋₆ alkyl), and other groups; R^{e} is selected from the group consisting of halogen, NH₂, boric acid, borate, and other groups;
   R^{f} is selected from the group consisting of halogen, boric acid, borate, and other groups.
   wherein ring A, R¹, X, Y, R², R³, m, and n are as defined herein.

All patents, patent applications and other identified publications are explicitly incorporated herein by reference for the purpose of description and disclosure. These publications are provided solely because they were disclosed prior to the filing date of the present application. All statements as to the dates of these documents or descriptions as to the contents of these documents are based on the information available to the applicant and do not constitute any admission as to the correctness of the dates or the content of these documents. Moreover, in any country or region, any reference to these publications herein shall not be construed as an admission that the publications form part of the common knowledge in the art.

Specific compounds of the present application, which can be prepared by the preparation method of the present application, are listed in the following table:

| Structural formula | Mass spectrometric data | Structural formula | Mass spectrometric data |
|---|---|---|---|
| 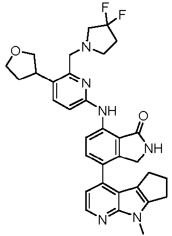 | HRMS (ESI, [M-H]⁻) | 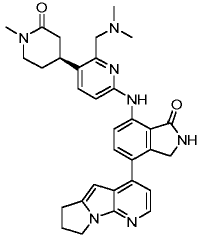 | HRMS (ESI, [M+H]⁺) |
| | m/z: 583.2640 | | *m*/*z:* 550.2933 or 550.2928 |
| 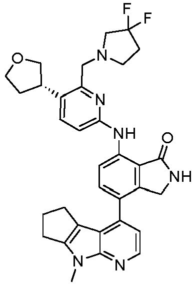 | HRMS (ESI, [M-H]⁻) | 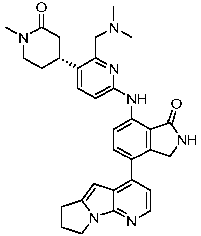 | HRMS (ESI, [M+H]⁺) |
| | m/z: 583.2642 or 583.2641 | | *m*/*z:* 550.2933 or 550.2928 |
| 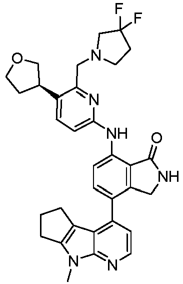 | HRMS (ESI, [M-H]⁻) | 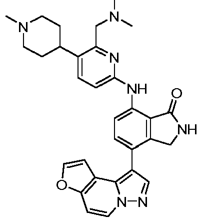 | HRMS (ESI, [M+H]⁺) |
| | m/z: 583.2642 or 583.2641 | | *m*/*z:* 536.2775 |
| 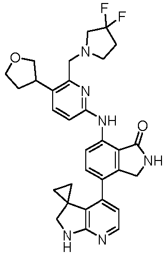 | HRMS (ESI, [M+H]⁺) | 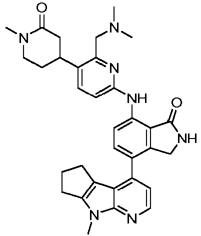 | HRMS (ESI, [M+H]⁺) |
| | *m*/*z:* 559.2629 | | *m*/*z:* 564.3084 |
| 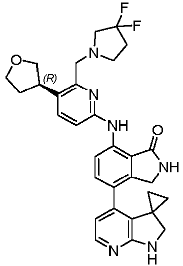 | HRMS (ESI, [M+H]⁺) | 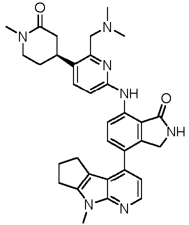 | HRMS (ESI, [M+H]⁺) |
| | *m*/*z*: 559.2631 or 559.2633 | | *m*/*z*: 564.3088 or 564.3079 |
| 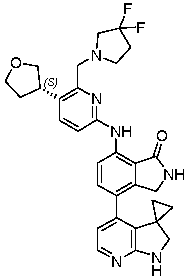 | HRMS (ESI, [M+H]⁺) | 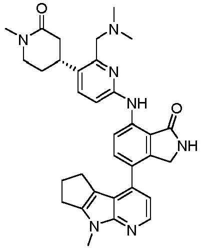 | HRMS (ESI, [M+H]⁺) |
| | *m*/*z*: 559.2631 or 559.2633 | | *m*/*z*: 564.3088 or 564.3079 |
| 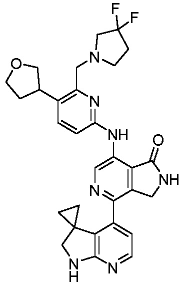 | HRMS (ESI, [M+H]⁺) | 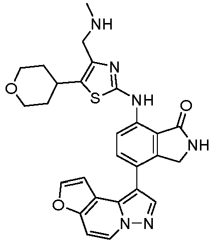 | HRMS (ESI, [M+H]⁺) |
| | *m*/*z*: 560.2588 | | *m*/*z*: 515.1871 |
| 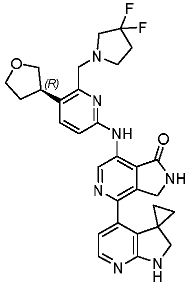 | HRMS (ESI, [M+H]⁺) | 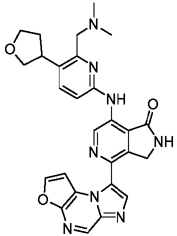 | HRMS (ESI, [M+H]⁺) |
| | *m*/*z*: 560.2589 or 560.2590 | | *m*/*z*: 511.2235 |
| 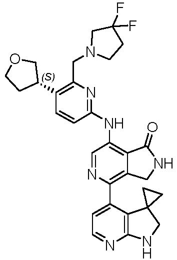 | HRMS (ESI, [M+H]⁺) | 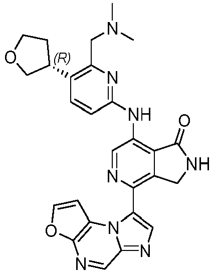 | HRMS (ESI, [M+H]⁺) |
| | *m*/*z*: 560.2589 or 560.2590 | | *m*/*z:* 511.2246 or 511.2232 |
| 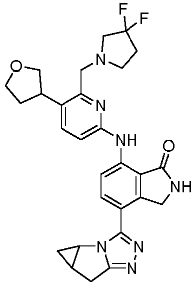 | HRMS (ESI, [M+H]⁺) | 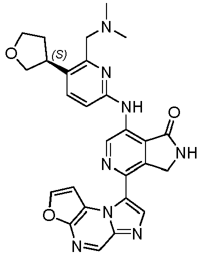 | HRMS (ESI, [M+H]⁺) |
| | *m*/*z*: 534.2424 | | *m*/*z:* 511.2246 or 511.2232 |
| 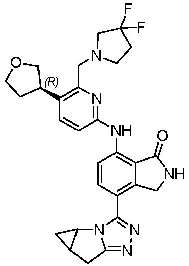 | HRMS (ESI, [M+H]⁺) | 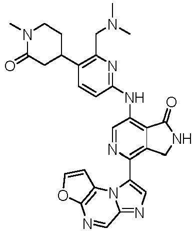 | HRMS (ESI, [M+H]⁺) |
| | *m*/*z*: 534.2428 or 534.2427 | | *m*/*z*: 552.2465 |
| 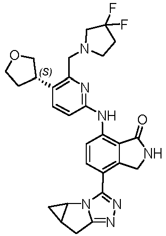 | HRMS (ESI, [M+H]⁺) | 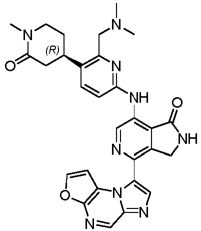 | HRMS (ESI, [M+H]⁺) |
| | *m*/*z*: 534.2428 or 534.2427 | | *m*/*z*: 552.2486 or 552.2476 |
| 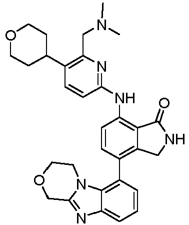 | HRMS (ESI, [M+H]⁺) | 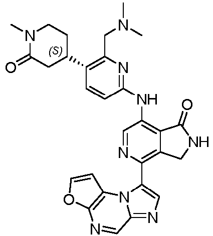 | HRMS (ESI, [M+H]⁺) |
| | *m*/*z*: 539.2762 | | *m*/*z*: 552.2486 or 552.2476 |
| 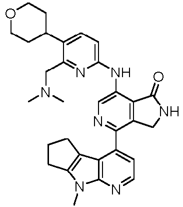 | HRMS (ESI, [M+H]⁺) | 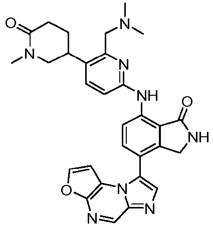 | HRMS (ESI, [M+H]⁺) |
| | *m*/*z*: 538.2923 | | *m*/*z*: 551.2532 |
| 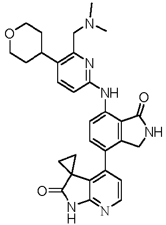 | HRMS (ESI, [M+H]⁺) | 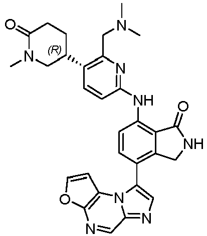 | HRMS (ESI, [M+H]⁺) |
| | *m*/*z:* 525.2609 | | *m*/*z:* 551.2545 or 551.2538 |
| 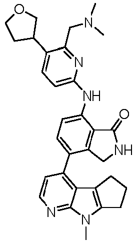 | HRMS (ESI, [M+H]⁺) | 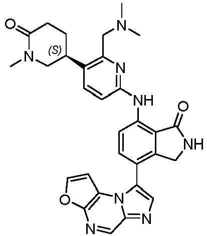 | HRMS (ESI, [M+H]⁺) |
| | *m*/*z:* 523.2814 | | *m*/*z:* 551.2545 or 551.2538 |
| 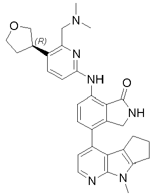 | HRMS (ESI, [M+H]⁺) | 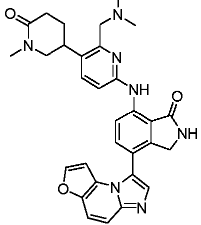 | HRMS (ESI, [M+H]⁺) |
| | *m*/*z:* 523.2810 or 523.2818 | | *m*/*z:* 550.2585 |
| 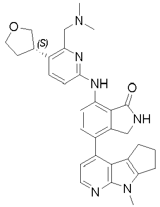 | HRMS (ESI, [M+H]⁺) | 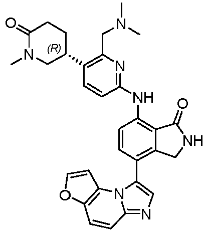 | HRMS (ESI, [M+H]⁺) |
| | *m*/*z:* 523.2810 or 523.2818 | | *m*/*z:* 550.2575 or 550.2580 |
| 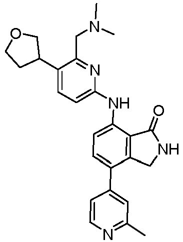 | HRMS (ESI, [M+H]⁺) | 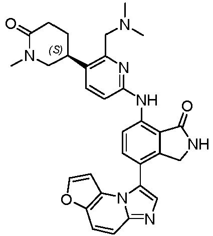 | HRMS (ESI, [M+H]⁺) |
| | *m*/*z:* 444.2396 | | *m*/*z:* 550.2575 or 550.2580 |
| 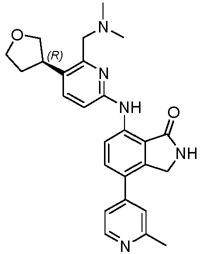 | HRMS (ESI, [M+H]⁺) | 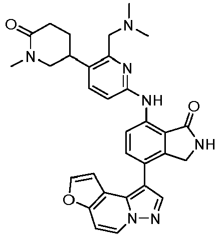 | HRMS (ESI, [M+H]⁺) |
| | *m*/*z*: 444.2398 or 444.2395 | | *m*/*z*: 550.2578 |
| 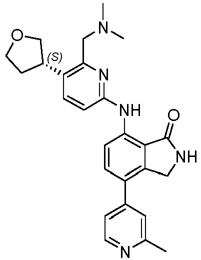 | HRMS (ESI, [M+H]⁺) | 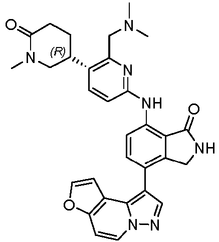 | HRMS (ESI, [M+H]⁺) |
| | *m*/*z*: 444.2398 or 444.2395 | | *m*/*z*: 550.2584 or 550.2574 |
| 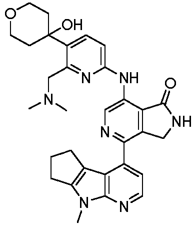 | HRMS (ESI, [M+H]⁺) | 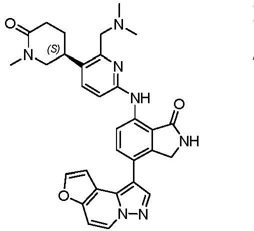 | HRMS (ESI, [M+H]⁺) |
| | 554.2880 | | *m*/*z*: 550.2584 or 550.2574 |
| 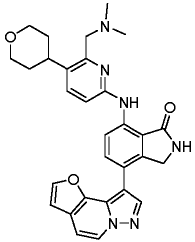 | HRMS (ESI, [M+H]⁺) | 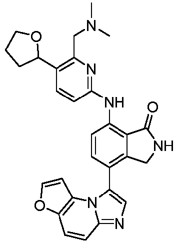 | HRMS (ESI, [M+H]⁺) |
| | *m*/*z*: 523.2455 | | *m*/*z*: 509.2325 |
| 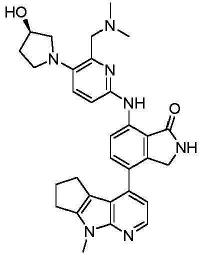 | HRMS (ESI, [M+H]⁺) | 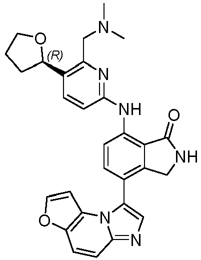 | HRMS (ESI, [M+H]⁺) |
| | *m*/*z*: 538.2923 | | *m*/*z*: 509.2335 or 509.2331 |
| 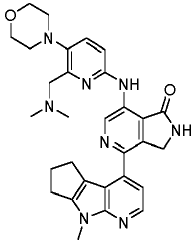 | HRMS (ESI, [M+H]⁺) | 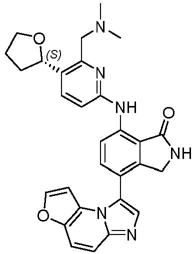 | HRMS (ESI, [M+H]⁺) |
| | *m*/*z*: 539.2878 | | *m*/*z*: 509.2335 or 509.2331 |
| 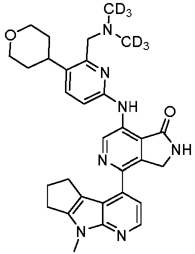 | HRMS (ESI, [M+H]⁺) | 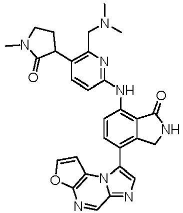 | HRMS (ESI, [M+H]⁺) |
| | *m*/*z*: 544.3300 | | *m*/*z*: 537.2415 |
| 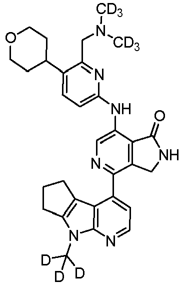 | HRMS (ESI, [M+H]⁺) | 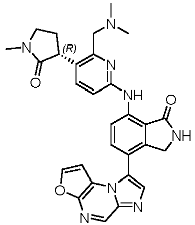 | HRMS (ESI, [M+H]⁺) |
| | *m*/*z*: 547.3490 | | *m*/*z*: 537.2411 or 537.2404 |
| 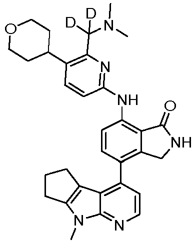 | HRMS (ESI, [M+H]⁺) | 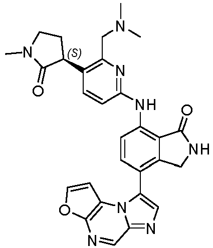 | HRMS (ESI, [M+H]⁺) |
| | *m*/*z*: 539.3097 | | *m*/*z*: 537.2411 or 537.2404 |
| 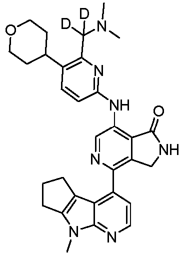 | HRMS (ESI, [M+H]⁺) | 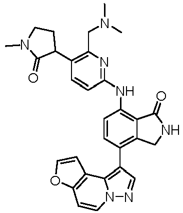 | HRMS (ESI, [M+H]⁺) |
| | *m*/*z*: 540.3049 | | *m*/*z*: 536.2420 |
| 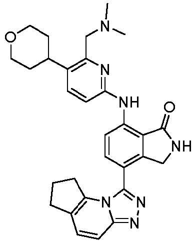 | HRMS (ESI, [M+H]⁺) | 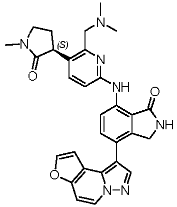 | HRMS (ESI, [M+H]⁺) |
| | *m*/*z*: 524.2768 | | *m*/*z:* 536.2417 or 536.2405 |
| 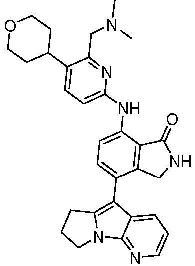 | HRMS (ESI, [M+H]⁺) | 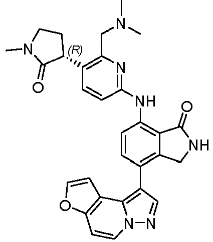 | HRMS (ESI, [M+H]⁺) |
| | *m*/*z:* 523.2818 | | *m*/*z:* 536.2417 or 536.2405 |
| 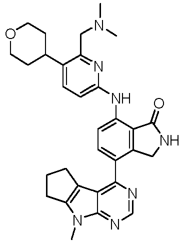 | HRMS (ESI, [M+H]⁺) | 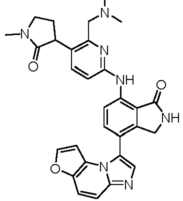 | HRMS (ESI, [M+H]⁺) |
| | *m*/*z:* 538.2919 | | *m*/*z:* 536.2411 |
| 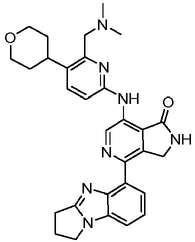 | HRMS (ESI, [M+H]⁺) | 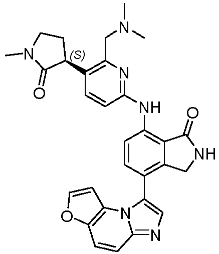 | HRMS (ESI, [M+H]⁺) |
| | *m*/*z:* 524.2767 | | *m*/*z:* 536.2405 or 536.2419 |
| 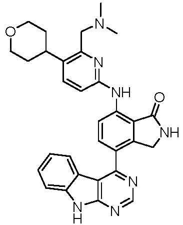 | HRMS (ESI, [M+H]⁺) | 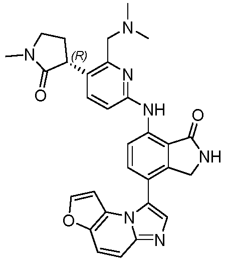 | HRMS (ESI, [M+H]⁺) |
| | m/z: 534.2617 | | *m*/*z:* 536.2405 or 536.2419 |
| 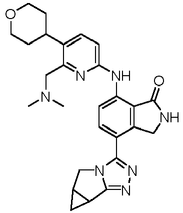 | HRMS (ESI, [M+H]⁺) | 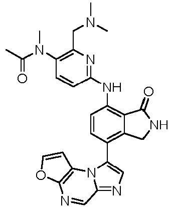 | HRMS (ESI, [M+H]⁺) |
| | *m*/*z:* 486.2614 | | *m*/*z:* 511.2035 |
| 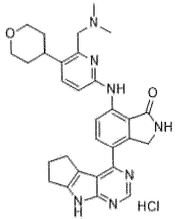 | HRMS (ESI, [M+H]⁺) | 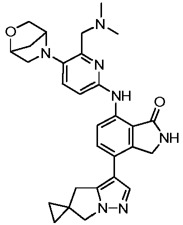 | HRMS (ESI, [M+H]⁺) |
| | *m*/*z:* 524.2770 | | *m*/*z:* 512.2801 |
| 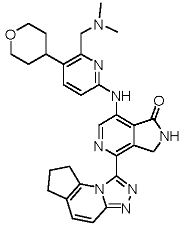 | HRMS (ESI, [M+H]+) | 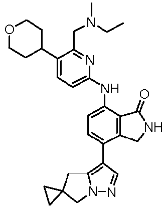 | HRMS (ESI, [M+H]⁺) |
| | m/z: 525.2721 | | *m*/*z:* 513.3024 |
| 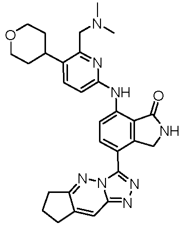 | HRMS (ESI, [M+H]⁺) | 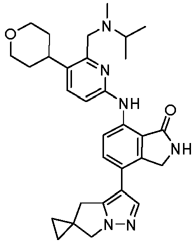 | HRMS (ESI, [M+H]⁺) |
| | *m*/*z:* 525.2725 | | *m*/*z:* 527.3124 |
| 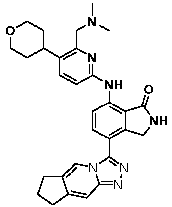 | HRMS (ESI, [M+H]⁺) | 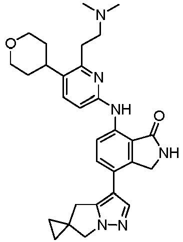 | HRMS (ESI, [M+H]⁺) |
| | *m*/*z:* 524.2754 | | *m*/*z:* 513.3012 |
| 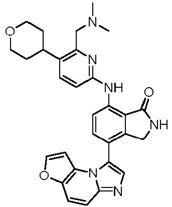 | HRMS (ESI, [M+H]⁺) | 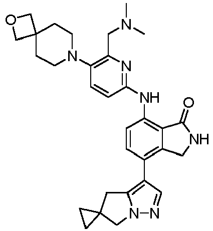 | HRMS (ESI, [M+H]⁺) |
| | *m*/*z:* 523.2457 | | *m*/*z:* 540.3125 |
| 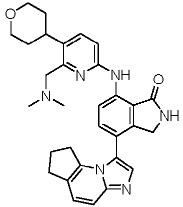 | HRMS (ESI, [M+H]⁺) | 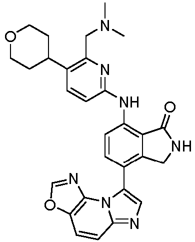 | HRMS (ESI, [M+H]⁺) |
| | *m*/*z:* 523.2984 | | *m*/*z:* 524.2389 |
| 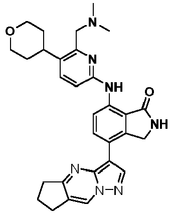 | HRMS (ESI, [M+H]⁺) | 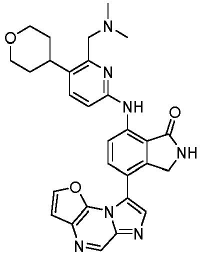 | HRMS (ESI, [M+H]⁺) |
| | *m*/*z:* 524.2754 | | *m*/*z:* 524.2405 |
| 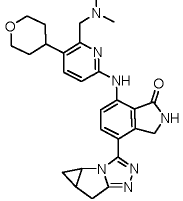 | HRMS (ESI, [M+H]⁺) | 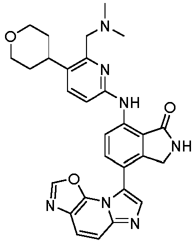 | HRMS (ESI, [M+H]⁺) |
| | *m*/*z:* 486.2615 | | *m*/*z:* 524.2400 |
| 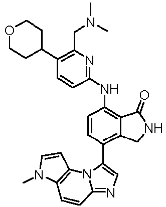 | HRMS (ESI, [M+H]⁺) | 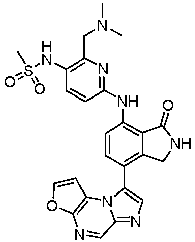 | HRMS (ESI, [M+H]⁺) |
| | *m*/*z:* 536.2776 | | *m*/*z:* 533.1710 |
| 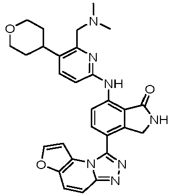 | HRMS (ESI, [M+H]⁺) | 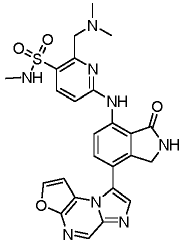 | HRMS (ESI, [M+H]⁺) |
| | *m*/*z:* 524.2407 | | *m*/*z:* 533.1716 |
| 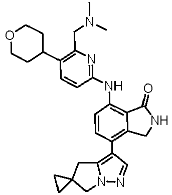 | HRMS (ESI, [M+H]⁺) | 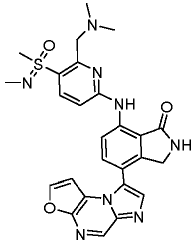 | HRMS (ESI, [M+H]⁺) |
| | *m*/*z:* 499.2818 | | *m*/*z:* 533.1727 |
| 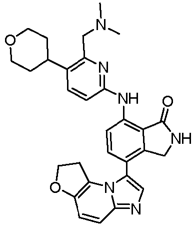 | HRMS (ESI, [M+H]⁺) | 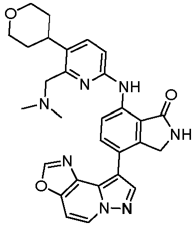 | HRMS (ESI, [M+H]⁺) |
| | *m*/*z:* 525.2621 | | *m*/*z:* 524.2409 |
| 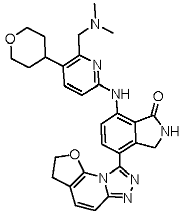 | HRMS (ESI, [M+H]⁺) | 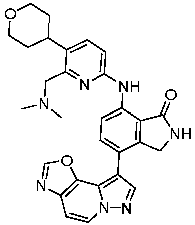 | HRMS (ESI, [M+H]⁺) |
| | *m*/*z:* 526.2566 | | *m*/*z:* 524.2413 |
| 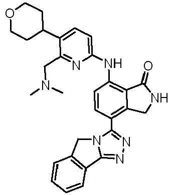 | HRMS (ESI, [M+H]⁺) | 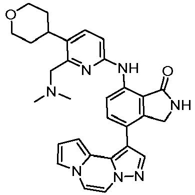 | HRMS (ESI, [M+H]⁺) |
| | *m*/*z:* 522.2616 | | *m*/*z:* 522.2619 |
| 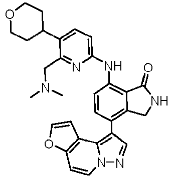 | HRMS (ESI, [M+H]⁺) | 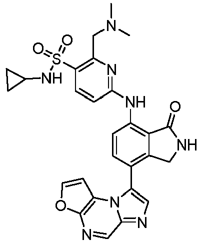 | HRMS (ESI, [M+H]⁺) |
| | *m*/*z:* 523.2455 | | *m*/*z:* 559.1880 |
| 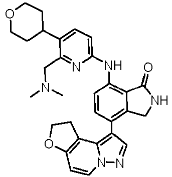 | HRMS (ESI, [M+H]⁺) | 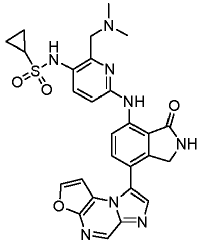 | HRMS (ESI, [M+H]⁺) |
| | *m*/*z:* 525.2606 | | *m*/*z:* 559.1882 |
| 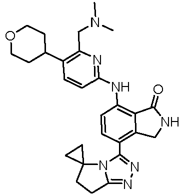 | HRMS (ESI, [M+H]⁺) | 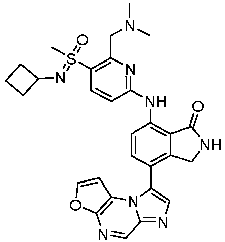 | HRMS (ESI, [M+H]⁺) |
| | *m*/*z*: 500.2767 | | *m*/*z*: 571.2239 |
| 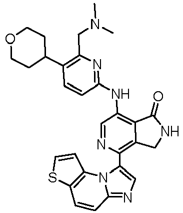 | HRMS (ESI, [M+H]⁺) | 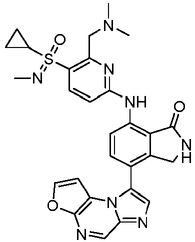 | HRMS (ESI, [M+H]⁺) |
| | *m*/*z*: 540.2182 | | *m*/*z*: 557.2080 |
| 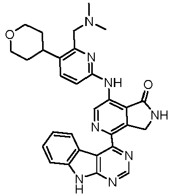 | HRMS (ESI, [M+H]⁺) | 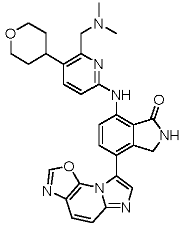 | HRMS (ESI, [M+H]⁺) |
| | *m*/*z*: 535.2570 | | *m*/*z*: 524.2411 |
| 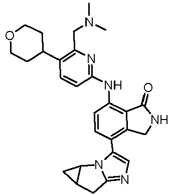 | HRMS (ESI, [M+H]⁺) | 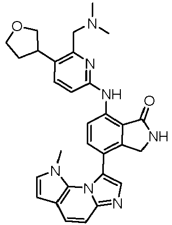 | HRMS (ESI, [M+H]⁺) |
| | *m*/*z*: 485.2662 | | *m*/*z*: 522.2616 |
| 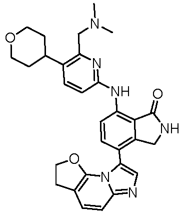 | HRMS (ESI, [M+H]⁺) | 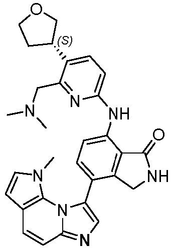 | HRMS (ESI, [M+H]⁺) |
| | *m*/*z*: 525.2630 | | *m*/*z*: 522.2619 or 522.2610 |
| 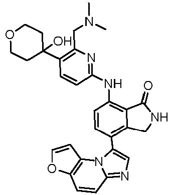 | HRMS (ESI, [M+H]⁺) | 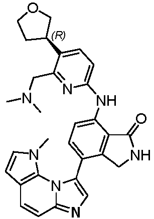 | HRMS (ESI, [M+H]⁺) |
| | *m*/*z*: 539.2407 | | *m*/*z*: 522.2619 or 522.2610 |
| 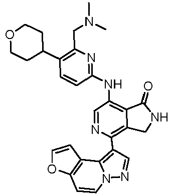 | HRMS (ESI, [M+H]⁺) | 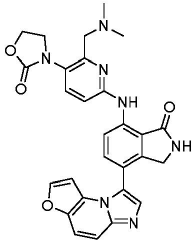 | HRMS (ESI, [M+H]⁺) |
| | *m*/*z:* 524.2407 | | *m*/*z:* 524.2038 |
| 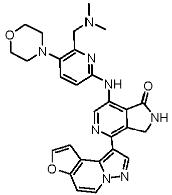 | HRMS (ESI, [M+H]⁺) | 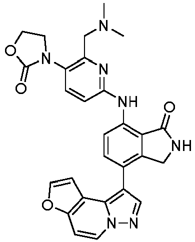 | HRMS (ESI, [M+H]⁺) |
| | *m*/*z:* 525.2359 | | *m*/*z:* 522.2044 |
| | HRMS (ESI, [M+H]⁺) | 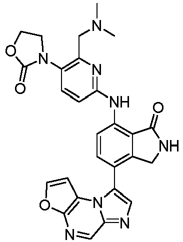 | HRMS (ESI, [M+H]⁺) |
| 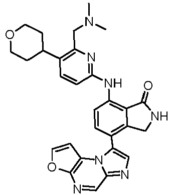 | *m*/*z:* 524.2398 | | *m*/*z:* 525.1999 |
| 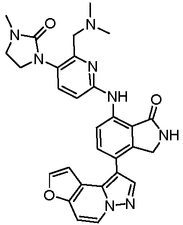 | HRMS (ESI, [M+H]⁺) | 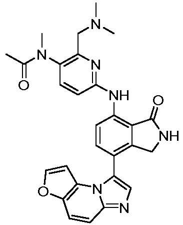 | HRMS (ESI, [M+H]⁺) |
| | *m*/*z:* 537.2365 | | *m*/*z:* 510.2163 |
| 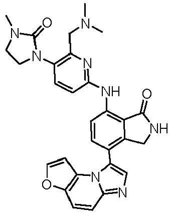 | HRMS (ESI, [M+H]⁺) | 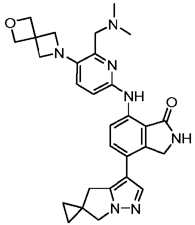 | HRMS (ESI, [M+H]⁺) |
| | *m*/*z:* 537.2365 | | *m*/*z:* 512.2681 |
| 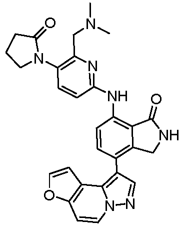 | HRMS (ESI, [M+H]⁺) | 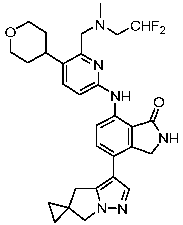 | HRMS (ESI, [M+H]⁺) |
| | *m*/*z:* 522.2254 | | *m*/*z:* 549.2723 |
| 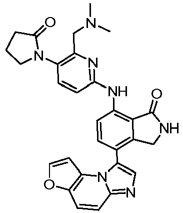 | HRMS (ESI, [M+H]⁺) | 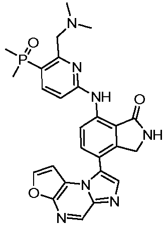 | HRMS (ESI, [M+H]⁺) |
| | *m*/*z:* 522.2254 | | *m*/*z:* 516.1819 |
| 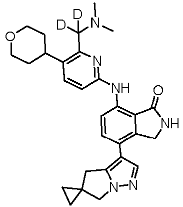 | HRMS (ESI, [M+H]⁺) | 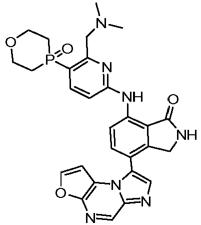 | HRMS (ESI, [M+H]⁺) |
| | *m*/*z:* 501.2944 | | *m*/*z:* 558.1951 |
| 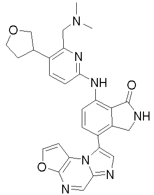 | HRMS (ESI, [M+H]⁺) | 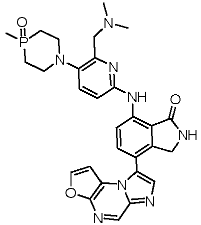 | HRMS (ESI, [M+H]⁺) |
| | *m*/*z:* 510.2242 | | m/z: 571.2273 |
| 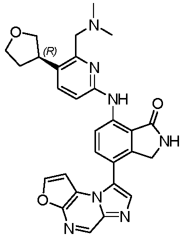 | HRMS (ESI, [M+H]⁺) | 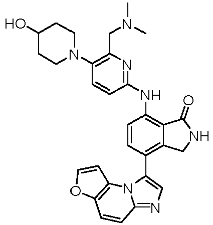 | HRMS (ESI, [M+H]⁺) |
| | *m*/*z:* 510.2240 or 510.2241 | | *m*/*z:* 538.2476 |
| 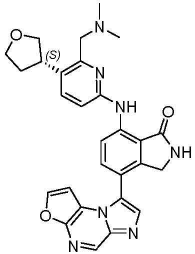 | HRMS (ESI, [M+H]⁺) | 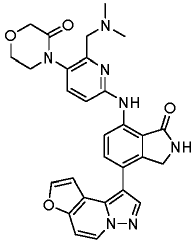 | HRMS (ESI, [M+H]⁺) |
| | *m*/*z:* 510.2240 or 510.2241 | | *m*/*z:* 538.2149 |
| 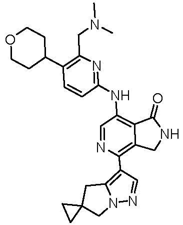 | HRMS (ESI, [M+H]⁺) | 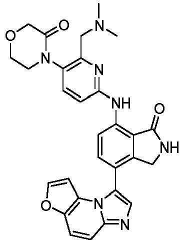 | HRMS (ESI, [M+H]⁺) |
| | *m*/*z:* 500.2768 | | *m*/*z:* 538.2146 |
| 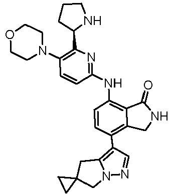 | HRMS (ESI, [M+H]⁺) | 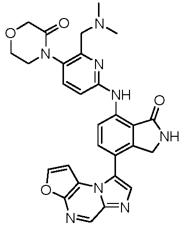 | HRMS (ESI, [M+H]⁺) |
| | *m*/*z:* 512.2766 | | *m*/*z:* 539.2065 |
| 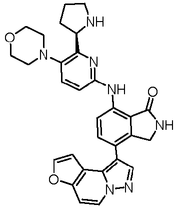 | HRMS (ESI, [M+H]⁺) | 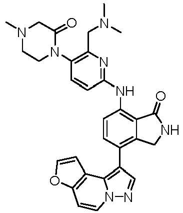 | HRMS (ESI, [M+H]⁺) |
| | *m*/*z:* 536.2407 | | *m*/*z:* 551.2431 |
| 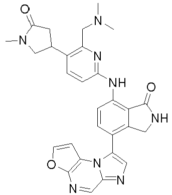 | HRMS (ESI, [M+H]⁺) | 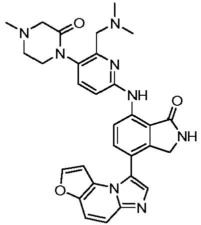 | HRMS (ESI, [M+H]⁺) |
| | *m*/*z:* 537.2354 | | *m*/*z:* 551.2456 |
| 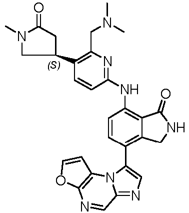 | HRMS (ESI, [M+H]⁺) | 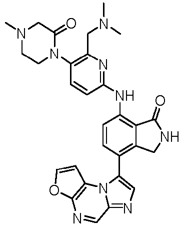 | HRMS (ESI, [M+H]⁺) |
| | *m*/*z:* 537.2359 or 537.2350 | | *m*/*z:* 552.2379 |
| 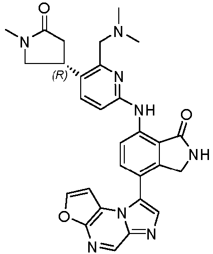 | HRMS (ESI, [M+H]⁺) | 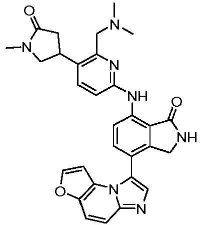 | HRMS (ESI, [M+H]⁺) |
| | *m*/*z:* 537.2359 or 537.2350 | | *m*/*z:* 536.2349 |
| 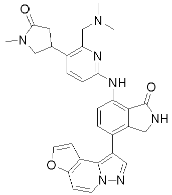 | HRMS (ESI, [M+H]⁺) | 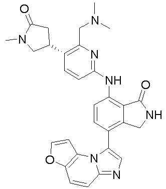 | HRMS (ESI, [M+H]⁺) |
| | *m*/*z:* 536.2408 | | *m*/*z:* 536.2325 |
| 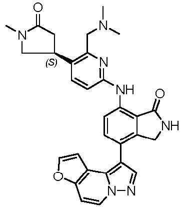 | HRMS (ESI, [M+H]⁺) | 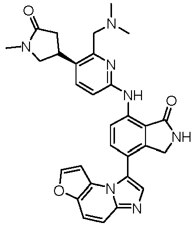 | HRMS (ESI, [M+H]⁺) |
| | *m*/*z*: 536.2410 or 536.2405 | | *m*/*z*: 536.2341 |
| 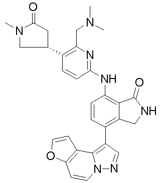 | HRMS (ESI, [M+H]⁺) | 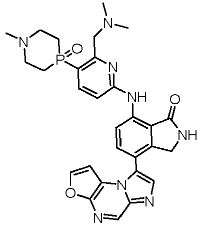 | HRMS (ESI, [M+H]⁺) |
| | *m*/*z*: 536.2410 or 536.2405 | | *m*/*z*: 571.2269 |
| 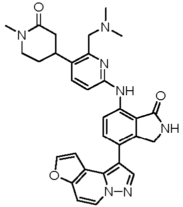 | HRMS (ESI, [M+H]⁺) | 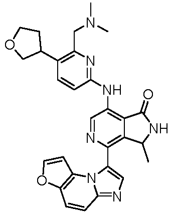 | HRMS (ESI, [M+H]⁺) |
| | *m*/*z*: 550.2567 | | *m*/*z*: 524.2400 |
| 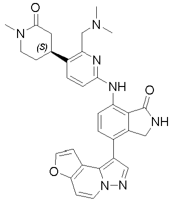 | HRMS (ESI, [M+H]⁺) | 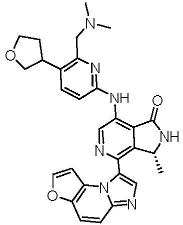 | HRMS (ESI, [M+H]⁺) |
| | *m*/*z*: 550.2565 or 550.2562 | | *m*/*z*: 524.2313 |
| 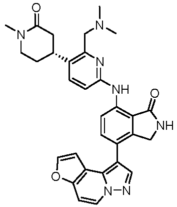 | HRMS (ESI, [M+H]⁺) | 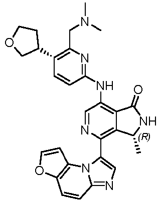 | HRMS (ESI, [M+H]⁺) |
| | *m*/*z*: 550.2565 or 550.2562 | | *m*/*z*: 524.2340 or 524.2355 |
| 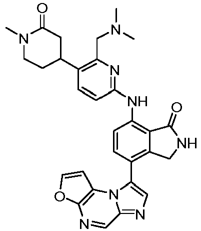 | HRMS (ESI, [M+H]⁺) | 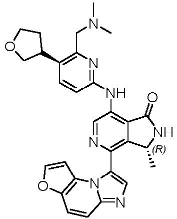 | HRMS (ESI, [M+H]⁺) |
| | *m*/*z*: 551.2505 | | *m*/*z*: 524.2340 or 524.2355 |
| 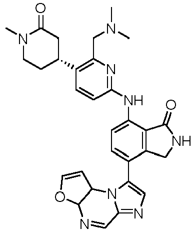 | HRMS (ESI, [M+H]⁺) | 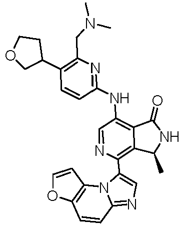 | HRMS (ESI, [M+H]⁺) |
| | *m*/*z:* 551.2501 or 551.2506 | | *m*/*z:* 524.2421 |
| 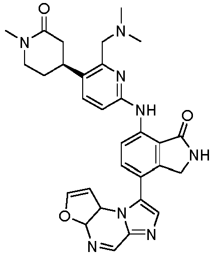 | HRMS (ESI, [M+H]⁺) | 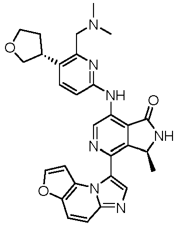 | HRMS (ESI, [M+H]⁺) |
| | *m*/*z:* 551.2501 or 551.2506 | | *m*/*z:* 524.2436 or 524.2412 |
| 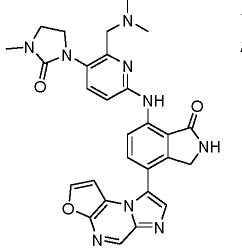 | HRMS (ESI, [M+H]⁺) | 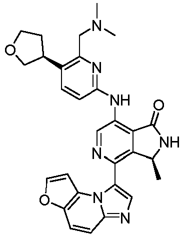 | HRMS (ESI, [M+H]⁺) |
| | *m*/*z:* 538.2311 | | *m*/*z:* 524.2436 or 524.2412 |
| 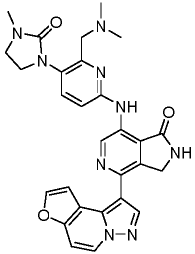 | HRMS (ESI, [M+H]⁺) | 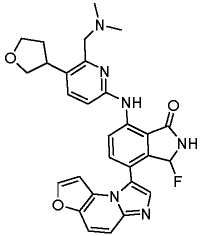 | HRMS (ESI, [M+H]⁺) |
| | *m*/*z:* 538.2313 | | *m*/*z:* 527.2212 |
| 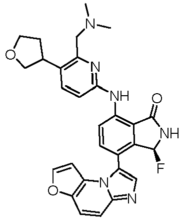 | HRMS (ESI, [M+H]⁺) | 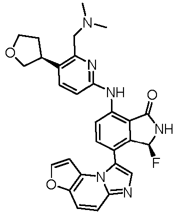 | HRMS (ESI, [M+H]⁺) |
| | *m*/*z:* 527.2210 | | *m*/*z:* 527.2223 or 527.2203 |
| 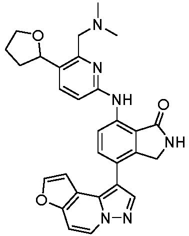 | HRMS (ESI, [M+H]⁺) | 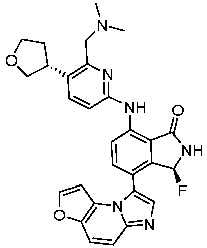 | HRMS (ESI, [M+H]⁺) |
| | *m*/*z:* 509.2311 | | *m*/*z:* 527.2223 or 527.2203 |
| 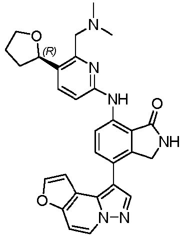 | HRMS (ESI, [M+H]⁺) | 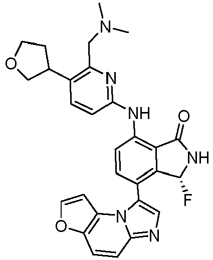 | HRMS (ESI, [M+H]⁺) |
| | *m*/*z:* 509.2314 or 509.2309 | | *m*/*z:* 527.2203 |
| 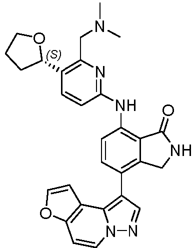 | HRMS (ESI, [M+H]⁺) | 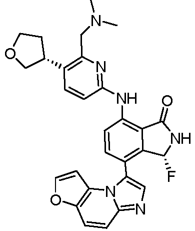 | HRMS (ESI, [M+H]⁺) |
| | *m*/*z:* 509.2314 or 509.2309 | | *m*/*z:* 527.2195 or 527.2186 |
| 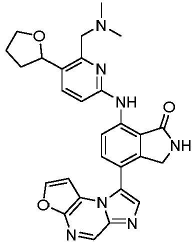 | HRMS (ESI, [M+H]⁺) | 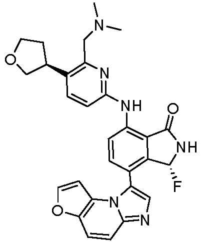 | HRMS (ESI, [M+H]⁺) |
| | *m*/*z:* 510.2246 | | *m*/*z:* 527.2195 or 527.2186 |
| 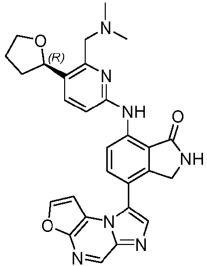 | HRMS (ESI, [M+H]⁺) | 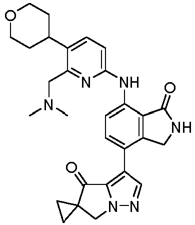 | HRMS (ESI, [M+H]⁺) |
| | *m*/*z:* 510.2248 or 510.2244 | | *m*/*z:* 513.2625 |
| 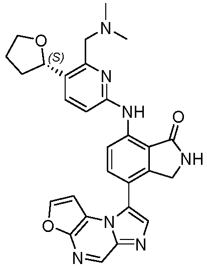 | HRMS (ESI, [M+H]⁺) | 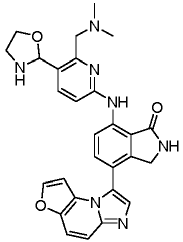 | HRMS (ESI, [M+H]⁺) |
| | *m*/*z*: 510.2248 or 510.2244 | | *m*/*z*: 510.2242 |
| 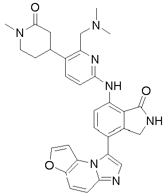 | HRMS (ESI, [M+H]⁺) | 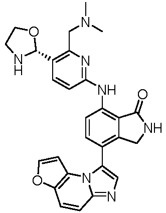 | HRMS (ESI, [M+H]⁺) |
| | *m*/*z:* 550.2546 | | *m*/*z*: 510.2256 or 510.2235 |
| 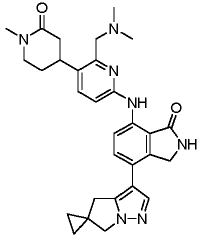 | HRMS (ESI, [M+H]⁺) | 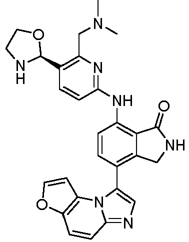 | HRMS (ESI, [M+H]⁺) |
| | *m*/*z:* 526.2932 | | *m*/*z*: 510.2256 or 510.2235 |
| 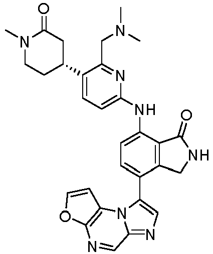 | HRMS (ESI, [M+H]⁺) | 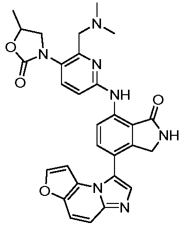 | HRMS (ESI, [M+H]⁺) |
| | *m*/*z:* 551.2515 or 551.2518 | | m/z: 538.2191 |
| 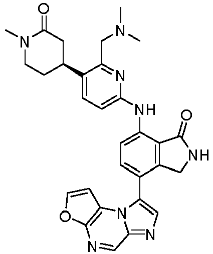 | HRMS (ESI, [M+H]⁺) | 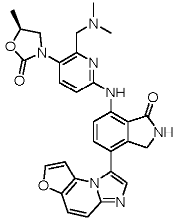 | HRMS (ESI, [M+H]⁺) |
| | *m*/*z:* 551.2515 or 551.2518 | | m/z: 538.2185 or 538.2223 |
| 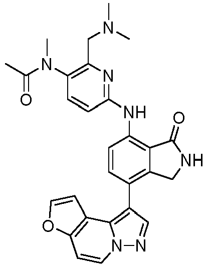 | HRMS (ESI, [M+H]⁺) | 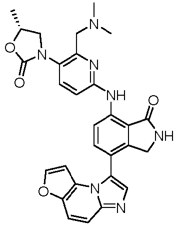 | HRMS (ESI, [M+H]⁺) |
| | *m*/*z:* 510.2255 | | m/z: 538.2185 or 538.2223 |
| 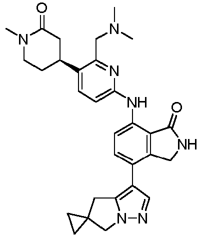 | HRMS (ESI, [M+H]⁺) | 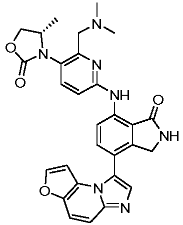 | HRMS (ESI, [M+H]⁺) |
| | *m*/*z:* 526.2926 or 526.2935 | | m/z: 538.2176 or 538.2201 |
| 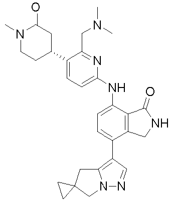 | HRMS (ESI, [M+H]⁺) | 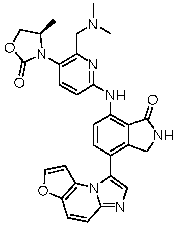 | HRMS (ESI, [M+H]⁺) |
| | *m*/*z:* 526.2926 or 526.2935 | | m/z: 538.2176 or 538.2201 |
| 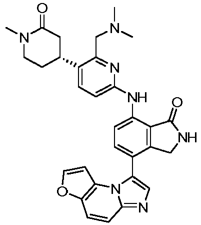 | HRMS (ESI, [M+H]⁺) | 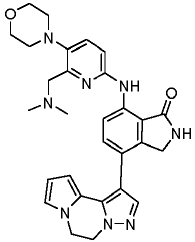 | HRMS (ESI, [M+H]⁺) |
| | *m*/*z:* 550.2558 or 550.2547 | | m/z: 525.2765 |
| 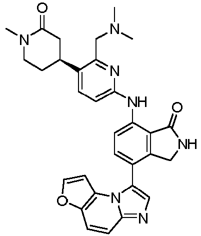 | HRMS (ESI, [M+H]⁺) | 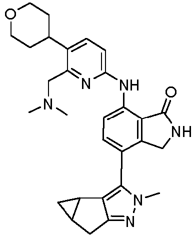 | HRMS (ESI, [M+H]⁺) |
| | *m*/*z:* 550.2558 or 550.2547 | | m/z: 499.2816 |
| 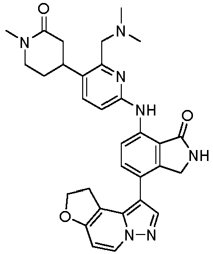 | HRMS (ESI, [M+H]⁺) | 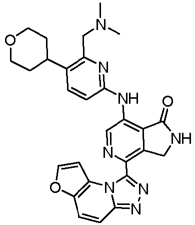 | HRMS (ESI, [M+H]⁺) |
| | *m*/*z:* 552.2725 | | m/z: 525.2369 |
| 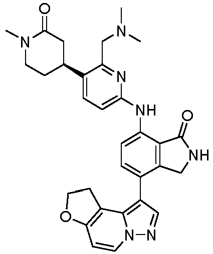 | HRMS (ESI, [M+H]⁺) | 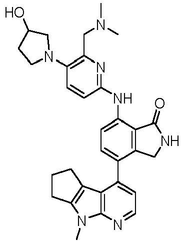 | HRMS (ESI, [M+H]⁺) |
| | *m*/*z:* 552.2729 or 552.2728 | | m/z: 538.2952 |
| 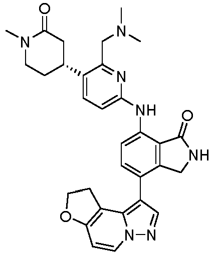 | HRMS (ESI, [M+H]⁺) | 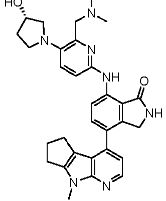 | HRMS (ESI, [M+H]⁺) |
| | *m*/*z:* 552.2729 or 552.2728 | | m/z: 538.2943 or 538.2941 |
| 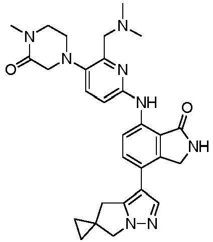 | HRMS (ESI, [M+H]⁺) | 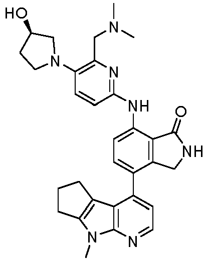 | HRMS (ESI, [M+H]⁺) |
| | *m*/*z:* 527.2887 | | m/z: 538.2943 or 538.2941 |
| 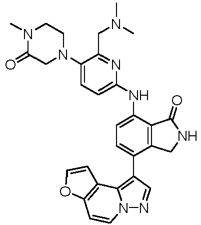 | HRMS (ESI, [M+H]⁺) | 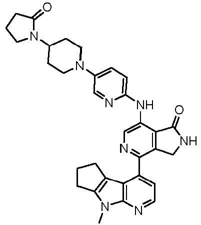 | HRMS (ESI, [M+H]⁺) |
| | *m*/*z:* 551.2519 | | m/z: 563.2842 |
| 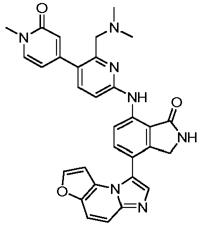 | HRMS (ESI, [M+H]⁺) | 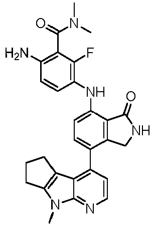 | HRMS (ESI, [M+H]⁺) |
| | *m*/*z:* 546.2251 | | m/z: 499.2243 |
| 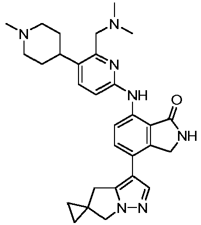 | HRMS (ESI, [M+H]⁺) | 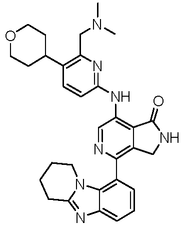 | HRMS (ESI, [M+H]⁺) |
| | *m*/*z:* 512.3127 | | m/z: 538.2942 |
| 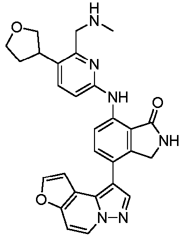 | HRMS (ESI, [M+H]⁺) | 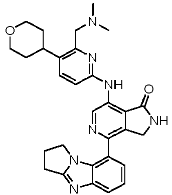 | HRMS (ESI, [M+H]⁺) |
| | *m*/*z:* 495.2142 | | m/z: 524.2796 |
| 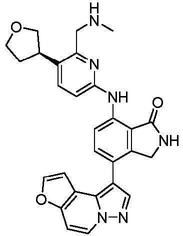 | HRMS (ESI, [M+H]⁺) | 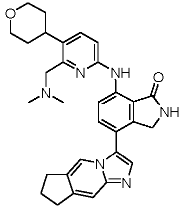 | HRMS (ESI, [M+H]⁺) |
| | *m*/*z:* 495.2140 or 495.2144 | | m/z: 523.2842 |
| 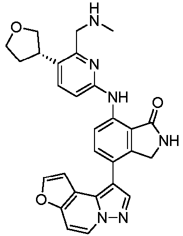 | HRMS (ESI, [M+H]⁺) | 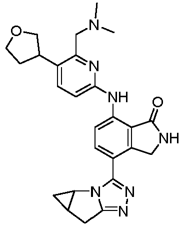 | HRMS (ESI, [M+H]⁺) |
| | *m*/*z:* 495.2140 or 495.2144 | | m/z: 472.2446 |
| 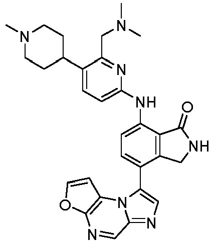 | HRMS (ESI, [M+H]⁺) | 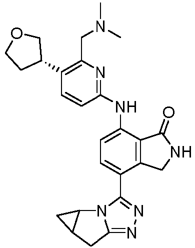 | HRMS (ESI, [M+H]⁺) |
| | *m*/*z:* 537.2718 | | m/z: 472.2455 or 472.2448 |
| 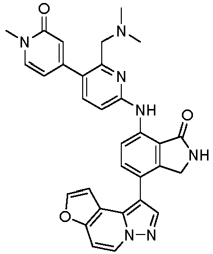 | HRMS (ESI, [M+H]⁺) | 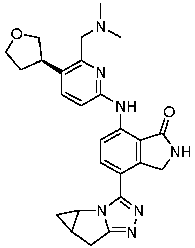 | HRMS (ESI, [M+H]⁺) |
| | *m*/*z:* 546.2261 | | m/z: 472.2455 or 472.2448 |
| 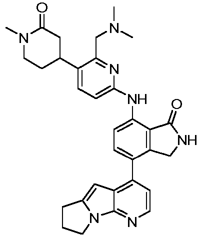 | HRMS (ESI, [M+H]⁺) | 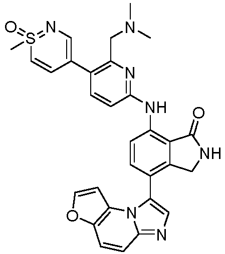 | HRMS (ESI, [M+H]⁺) |
| | *m*/*z:* 550.2931 | | m/z: 566.1972 |
| | | 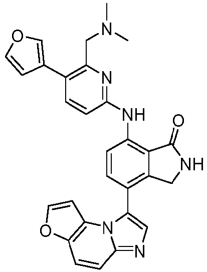 | HRMS (ESI, [M+H]⁺) |
| 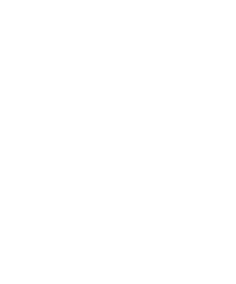 | | | m/z: 505.2054 |

The following abbreviations are used in the present application:
DMSO: dimethyl sulfoxide; THF: tetrahydrofuran; NCS: *N*-chlorosuccinimide; NBS: *N*-bromosuccinimide; CCl₄: carbon tetrachloride; DMF: *N,N*-dimethylformamide; HOVEYDA-GRUBBS: (1,3-bis (2,4,6-trimethylphenyl)-2-imidazolidinylidene)dichloro(*o*-isopropoxybenzylidene)ruthenium; Boc: *tert*-butyloxycarbonyl; Ms: methanesulfonyl;
Ts: *p*-toluenesulfonyl; and Cbz: benzyloxycarbonyl.

### DETAILED DESCRIPTION

For clarity, the present invention is further described with the following examples, which are, however, not intended to limit the scope of the present application. It will be apparent to those skilled in the art that various changes and modifications can be made to the specific embodiments of the present invention without departing from the spirit and scope of the present invention. All reagents used in the present application are commercially available and can be used without further purification.

The compounds of the present application can be obtained by similar preparation methods to those in the examples, including but not limited to, adjustment of structurally similar starting materials, reagents, or process parameters. The structural characterization of the compounds of the present application can be obtained by MS or ¹H NMR. The results for the compounds of the present application can also be obtained by the same effect test method.

### Preparation of Intermediates

### Intermediate Example 1: Preparation of Intermediate 1A

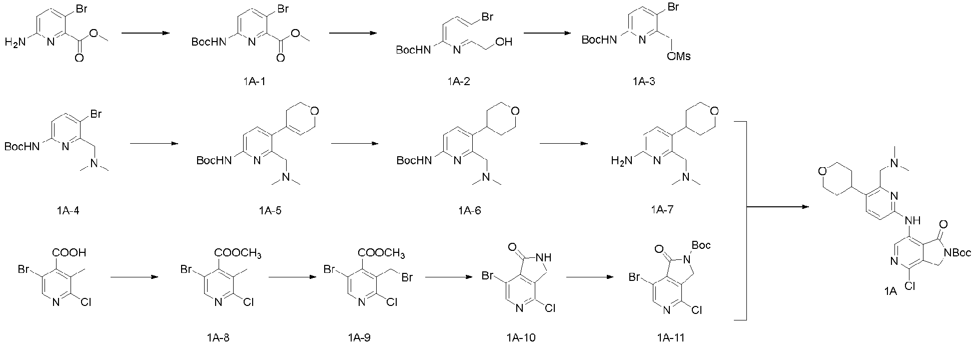

### Step A: Preparation of intermediate 1A-1

To a reaction flask were added the compounds methyl 6-amino-3-bromopicolinate (100 g), *N,N-*dimethylpyridin-4-amine (10.6 g), di-*tert*-butyl dicarbonate (170 g), and tetrahydrofuran (600 mL) in sequence. After the addition, the mixture was stirred at room temperature. After the reaction was completed, 10% citric acid aqueous solution (200 mL, w/w) was added to the reaction solution to quench the reaction, and the resulting mixture was extracted with dichloromethane, washed with saturated brine, dried, and concentrated to give intermediate 1A-1 (120.1 g).

MS (ESI, [M+H]⁺) *m*/*z*: 331.1.

### Step B: Preparation of intermediate 1A-2

To a reaction flask were added intermediate 1A-1 (85 g), tetrahydrofuran (300 mL), and lithium borohydride (6.2 g) in sequence. After the addition, the mixture was stirred at room temperature. After the reaction was completed, a saturated ammonium chloride solution was added to the reaction solution to quench the reaction, and the resulting mixture was extracted with dichloromethane, washed with saturated brine, dried, and concentrated to give intermediate 1A-2 (60.0 g).

MS (ESI, [M+H]⁺) *m*/*z*: 303.1.

### Step C: Preparation of intermediates 1A-3

To a reaction flask were added intermediate 1A-2 (54.2 g), *N,N-*diisopropylethylamine, dichloromethane (200 mL), and methanesulfonic anhydride (78 g) in sequence. After the addition, the mixture was stirred at -15 °C. After the reaction was completed, the reaction solution was directly used in the next step without treatment.

### Step D: Preparation of intermediate 1A-4

To a reaction flask were added a solution of dimethylamine in tetrahydrofuran (446 mL, 2 M), dichloromethane (200 mL), and the reaction solution of intermediate 1A-3 in sequence. The mixture was stirred at room temperature. After the reaction was completed, the reaction solution was extracted with dichloromethane, washed with saturated brine, dried, concentrated, and subjected to column chromatography (dichloromethane/methanol = 5/1) to give intermediate 1A-4 (38.2 g).

MS (ESI, [M+H]⁺) *m*/*z*: 330.1.

### Step E: Preparation of intermediate 1A-5

To a reaction flask were added intermediate 1A-4 (38.2 g), potassium carbonate (36.0 g), tetrakis(triphenylphosphine)palladium (15.0 g), 3,6-dihydro-2*H*-pyran-4-boronic acid pinacol ester (23.7 g), water (80 mL), and dioxane (400 mL) in sequence. After the addition, the mixture was purged with nitrogen and stirred at 100°C. After the reaction was completed, the reaction solution was filtered, concentrated, and subjected to column chromatography (petroleum ether/ethyl acetate = 5/1) to give intermediate 1A-5 (24.5 g).

MS (ESI, [M+H]⁺) *m*/*z*: 334.0.

### Step F: Preparation of intermediate 1A-6

To a reaction flask were added intermediate 1A-5 (24.5 g), 10 wt% palladium hydroxide on carbon (24.5 g), and methanol (100 mL) in sequence. After the addition, the mixture was stirred at 35 °C under hydrogen atmosphere. After the reaction was completed, the reaction solution was filtered and concentrated to give intermediate 1A-6 (22.0 g).

MS (ESI, [M+H]⁺) *m*/*z*: 336.0.

### Step G: Preparation of intermediate 1A-7

To a reaction flask were added intermediate 1A-6 (22.0 g) and a solution of hydrochloric acid in 1,4-dioxane (164 mL, 4 M) in sequence. After the addition, the mixture was stirred at room temperature. After the reaction was completed, the reaction solution was adjusted to alkalinity (about pH 10) with a saturated sodium bicarbonate solution, extracted with dichloromethane, and concentrated to give intermediate 1A-7 (13.0 g).

MS (ESI, [M+H]⁺) *m*/*z*: 236.3.

### Step H: Preparation of intermediate 1A-8

To a reaction flask were added the compounds 5-bromo-2-chloro-3-methylpyridine-4-carboxylic acid (50.0 g), potassium carbonate (54.2 g), iodomethane (14.0 mL), and *N,N*-dimethylformamide (500 mL) in sequence. After the addition, the mixture was stirred at room temperature. After the reaction was completed, water (1000 mL) was added to the reaction solution, and the resulting mixture was extracted with ethyl acetate and concentrated to give intermediate 1A-8 (41.2 g).

MS (ESI, [M+H]⁺) *m*/*z*: 264.2.

### Step I: Preparation of intermediate 1A-9

To a reaction flask were added intermediate 1A-8 (41.2 g), *N*-bromosuccinimide (42.0 g), dibenzoylmethane peroxide (0.6 g), and 1,2-dichloroethane (500 mL) in sequence. After the addition, the mixture was stirred at 90 °C. After the reaction was completed, a saturated sodium sulfite solution (100 mL) was added to the reaction solution to quench the reaction, and the resulting mixture was extracted with dichloromethane, washed with saturated brine, dried, and concentrated to give intermediate 1A-9 (54.5 g).

¹H NMR (500 MHz, CDCl₃) δ 8.51 (s, 1H), 4.53 (s, 2H), 4.05 (s, 3H).

### Step J: Preparation of intermediate 1A-10

To a reaction flask were added intermediate 1A-9 (54.5 g), a solution of ammonia in methanol (60 mL, 7 M), and methanol (500 mL) in sequence. After the addition, the mixture was stirred at 75 °C. After the reaction was completed, the reaction solution was filtered and dried to give intermediate 1A-10 (29.2 g).

¹H NMR (500 MHz, DMSO-*d*₆) δ 9.30 (s, 1H), 8.67 (d, *J=* 2.7 Hz, 1H), 4.41 (s, 2H).

### Step K: Preparation of intermediate 1A-11

To a reaction flask were added intermediate 1A-10 (29.2 g), *N,N*-dimethylpyridin-4-amine (1.5 g), di-*tert*-butyl dicarbonate (28.0 g), and dioxane(200 mL) in sequence. After the addition, the mixture was stirred at room temperature. After the reaction was completed, the reaction solution was concentrated and subjected to column chromatography (petroleum ether/ethyl acetate = 10/1) to give intermediate 1A-11 (35.3 g).

¹H NMR (500 MHz, DMSO-*d*₆) δ 8.75 (s, 1H), 4.73 (s, 2H), 1.54 (s, 9H).

### Step L: Preparation of intermediate 1A

To a reaction flask were sequentially added intermediate 1A-7 (1.2 g), 4,5-bis-diphenylphosphino-9,9-dimethylxanthene (244.6 mg), tris(dibenzylideneacetone)dipalladium(0) (258.3 mg), cesium carbonate (1.8 g), intermediate 1A-11 (1.1 g), and 1,4-dioxane (100 mL) in sequence. After the addition, the mixture was purged with nitrogen and stirred at 100 °C. After the reaction was completed, the reaction solution was filtered and concentrated to give a crude product, which was subjected to column chromatography (dichloromethane/methanol = 20/1) to give intermediate 1A (1.2 g).

MS (ESI, [M+H]⁺) *m*/*z*: 502.3.

### Intermediate Example 2: Preparation of Intermediate 2A

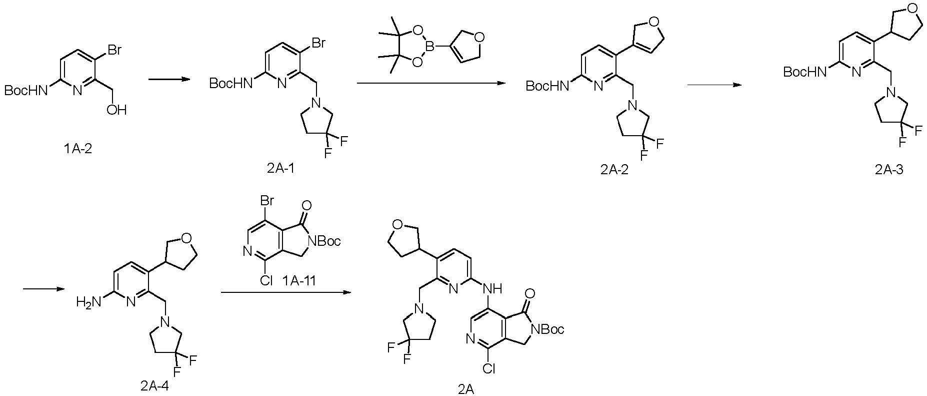

### Step A: Preparation of intermediate 2A-1

To a reaction flask were added intermediate 1A-2 (112.2 g), dichloromethane (450 mL), *N,N-*diisopropylethylamine (162.0 mL), and methanesulfonic anhydride (162.0 g) in sequence. After the addition, the mixture was stirred at - 15 °C. After the reaction was completed, 3,3-difluoropyrrolidine (118.5 g) was added to the reaction solution in batches. After the addition, the mixture was stirred at room temperature. After the reaction was completed, the reaction solution was extracted with dichloromethane, washed with saturated brine, dried, concentrated, and subjected to column chromatography (dichloromethane/methanol = 30/1) to give intermediate 2A-1 (95.0 g).

MS (ESI, [M+H]⁺) *m*/*z*: 392.3.

### Step B: Preparation of intermediate 2A-2

To a reaction flask were added intermediate 2A-1 (30.0 g), potassium carbonate (31.5 g), tetrakis(triphenylphosphine)palladium (10.5 g), 2,5-dihydrofuran-3-boronic acid pinacol ester (19.5 g), water (100 mL), and dioxane (500 mL) in sequence. After the addition, the mixture was purged with nitrogen and stirred at 100 °C. After the reaction was completed, the reaction solution was filtered, concentrated, and subjected to column chromatography (petroleum ether/ethyl acetate = 5/1) to give intermediate 2A-2 (23.2 g).

MS (ESI, [M+H]⁺) *m*/*z*: 382.5.

### Step C: Preparation of intermediate 2A-3

To a reaction flask were added intermediate 2A-2 (15.0 g), 10 wt% palladium hydroxide on carbon (15.0 g), and methanol (225 mL) in sequence. After the addition, the mixture was stirred at 35 °C under hydrogen atmosphere. After the reaction was completed, the reaction solution was filtered and concentrated to give intermediate 2A-3 (14.5 g).

MS (ESI, [M+H]⁺) *m*/*z*: 384.8.

### Step D: Preparation of intermediate 2A-4

To a reaction flask were added intermediate 2A-3 (14.5 g) and a solution of hydrochloric acid in 1,4-dioxane (122.4 mL, 4 M) in sequence. After the addition, the mixture was stirred at room temperature. After the reaction was completed, the reaction solution was adjusted to alkalinity (about pH 10) with a saturated sodium bicarbonate solution, extracted with dichloromethane, and concentrated to give intermediate 2A-4 (7.5 g).

MS (ESI, [M+H]⁺) *m*/*z*: 284.1.

### Step E: Preparation of intermediate 2A

To a reaction flask were added intermediate 2A-4 (1.0 g), 4,5-bis-diphenylphosphino-9,9-dimethylxanthene (244.6 mg), tris(dibenzylideneacetone)dipalladium(0) (258.3 mg), cesium carbonate (1.8 g), intermediate 1A-11 (1.1 g), and 1,4-dioxane (100 mL) in sequence. After the addition, the mixture was purged with nitrogen and stirred at 100 °C. After the reaction was completed, the reaction solution was filtered and concentrated to give a crude product, which was subjected to column chromatography (dichloromethane/methanol = 20/1) to give intermediate 2A (1.0 g).

MS (ESI, [M+H]⁺) *m*/*z*: 550.3.

### Intermediate Example 3: Preparation of Intermediate 3A

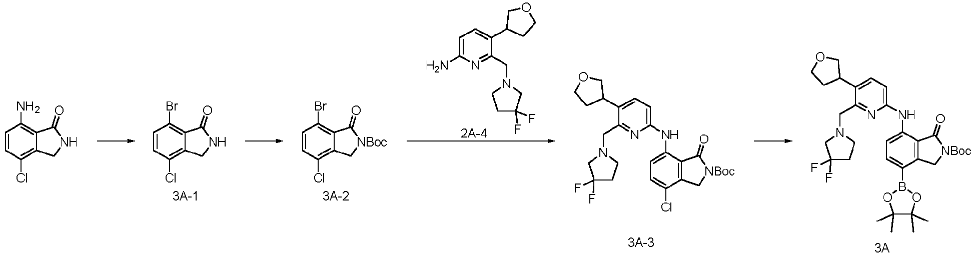

### Step A: Preparation of intermediate 3A-1

To a reaction flask were added 7-amino-4-chloroisoindol-1-one (10 g) and a solution of 48% hydrogen bromide in methanol (90 mL, w/w) in sequence at -10 °C. After the addition was completed, an aqueous solution (7.6 g, 65 mL) in which sodium nitrite was dissolved was slowly added dropwise to the reaction solution. After the addition, the reaction solution was stirred for 1 h. A 48% aqueous hydrogen bromide solution (8.6 g, 90 mL) in which cuprous bromide was dissolved was slowly added dropwise to the reaction solution. After the addition was completed, the reaction solution was stirred at 80 °C. After the reaction was completed, the reaction solution was poured into ice water, and the resulting mixture was stirred sufficiently and filtered. The filter cake was washed and dried to give intermediate 3A-1 (8.5 g).

MS (ESI, [M+H]⁺) *m*/*z*: 245.9.

### Step B: Preparation of intermediate 3A-2

To a reaction flask were added intermediate 3A-1 (8.5 g), tetrahydrofuran (150 mL), 4-dimethylaminopyridine (1.0 g), and di-*tert*-butyl dicarbonate (11.2 g) in sequence. After the addition, the mixture was stirred at room temperature. After the reaction was completed, 10% citric acid aqueous solution (200 mL, w/w) was added to the reaction solution to quench the reaction, and the resulting mixture was extracted with dichloromethane, washed with saturated brine, dried, and concentrated to give intermediate 3A-2 (9.6 g).

MS (ESI, [M+H]⁺) *m*/*z*: 345.9.

### Step C: Preparation of intermediate 3A-3

To a reaction flask were added intermediate 3A-2 (2.5 g), 4,5-bis-diphenylphosphino-9,9-dimethylxanthene (612 mg), tris(dibenzylideneacetone)dipalladium(0) (645.3 mg), cesium carbonate (5.8 g), intermediate 2A-4 (4.6 g), and 1,4-dioxane (100 mL) in sequence. After the addition, the mixture was purged with nitrogen and stirred at 100 °C. After the reaction was completed, the reaction solution was filtered and concentrated to give a crude product, which was subjected to column chromatography (dichloromethane/methanol = 20/1) to give intermediate 3A-3 (4.2 g).

MS (ESI, [M+H]⁺) *m*/*z*: 549.3.

### Step D: Preparation of intermediate 3A

To a microwave tube were added intermediate 3A-3 (2.0 g), chloro(2-dicyclohexylphosphine-2',4',6'-triisopropyl-1,1'-biphenyl)[2-(2'-amino-1,1'-biphenyl)]palladium (II) (573.0 mg), bis(pinacolato)diboron (1.9 g), potassium carbonate (1.0 g), and 1,4-dioxane (60 mL) in sequence. After the addition, the mixture was purged with nitrogen and reacted at 90 °C under microwave irradiation. After the reaction was completed, the reaction solution was filtered, concentrated, and subjected to column chromatography (dichloromethane/methanol = 20/1) to give intermediate 3A (2.4 g).

MS (ESI, [M+H]⁺) *m*/*z*: 641.5.

### Intermediate Example 4: Preparation of Intermediate 4A

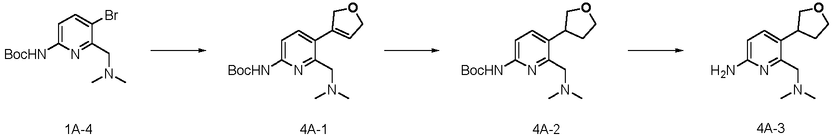

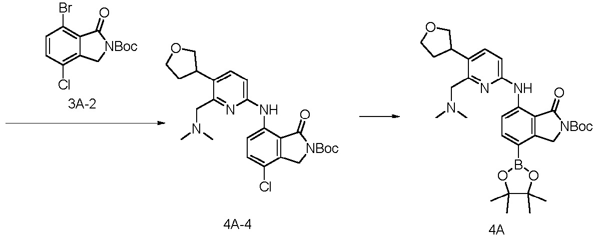

### Step A: Preparation of intermediate 4A-1

To a reaction flask were added intermediate 1A-4 (25.2 g), potassium carbonate (31.5 g), tetrakis(triphenylphosphine)palladium (10.5 g), 2,5-dihydrofuran-3-boronic acid pinacol ester (19.6 g), water (100 mL), and 1,4-dioxane (500 mL) in sequence. After the addition, the mixture was purged with nitrogen and stirred at 100 °C. After the reaction was completed, the reaction solution was filtered, concentrated, and subjected to column chromatography (petroleum ether/ethyl acetate = 5/1) to give intermediate 4A-1 (20.8 g).

MS (ESI, [M+H]⁺) *m*/*z*: 320.5.

### Step B: Preparation of intermediate 4A-2

To a reaction flask were added intermediate 4A-1 (15.0 g), 10 wt% palladium hydroxide on carbon (15.0 g), and methanol (225 mL) in sequence. After the addition, the mixture was stirred at 35 °C under hydrogen atmosphere. After the reaction was completed, the reaction solution was filtered and concentrated to give intermediate 4A-2 (14.8 g).

MS (ESI, [M+H]⁺) *m*/*z*: 322.8.

### Step C: Preparation of intermediate 4A-3

To a reaction flask were added intermediate 4A-2 (14.8 g) and a solution of hydrochloric acid in 1,4-dioxane (122.4 mL, 4 M) in sequence. After the addition, the mixture was stirred at room temperature. After the reaction was completed, the reaction solution was adjusted to alkalinity (about pH 10) with a saturated sodium bicarbonate solution, extracted with dichloromethane, and concentrated to give intermediate 4A-3 (8.2 g).

MS (ESI, [M+H]⁺) *m*/*z*: 222.1.

### Step D: Preparation of intermediate 4A-4

To a reaction flask were added intermediate 3A-2 (2.5 g), 4,5-bis-diphenylphosphino-9,9-dimethylxanthene (612 mg), tris(dibenzylideneacetone)dipalladium(0) (645.3 mg), cesium carbonate (5.8 g), intermediate 4A-3 (3.6 g), and 1,4-dioxane (100 mL) in sequence. After the addition, the mixture was purged with nitrogen and stirred at 100 °C. After the reaction was completed, the reaction solution was filtered and concentrated to give a crude product, which was subjected to column chromatography (dichloromethane/methanol = 20/1) to give intermediate 4A-4 (4.0 g).

MS (ESI, [M+H]⁺) *m*/*z*: 487.4.

### Step E: Preparation of intermediate 4A

To a microwave tube were added intermediate 4A-4 (2.0 g), chloro(2-dicyclohexylphosphine-2',4',6'-triisopropyl-1,1'-biphenyl)[2-(2'-amino-1,1'-biphenyl)]palladium (II) (585.0 mg), bis(pinacolato)diboron (2.1 g), potassium carbonate (1.2 g), and 1,4-dioxane (60 mL) in sequence. After the addition, the mixture was purged with nitrogen and reacted at 90 °C under microwave irradiation. After the reaction was completed, the reaction solution was filtered, concentrated, and subjected to column chromatography (dichloromethane/methanol = 20/1) to give intermediate 4A (2.6 g).

MS (ESI, [M+H]⁺) *m*/*z*: 579.5.

### Intermediate Example 5: Preparation of Intermediate 5A

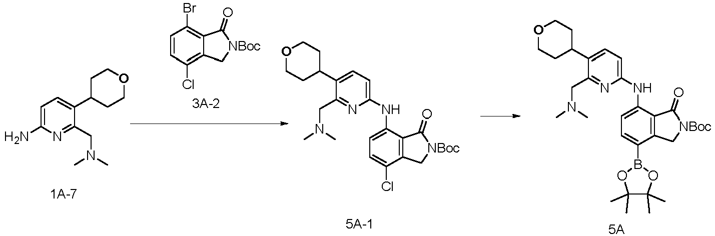

### Step A: Preparation of intermediate 5A-1

To a reaction flask were added intermediate 3A-2 (2.5 g), 4,5-bis-diphenylphosphino-9,9-dimethylxanthene (612 mg), tris(dibenzylideneacetone)dipalladium(0) (645.3 mg), cesium carbonate (5.8 g), intermediate 1A-7 (3.8 g), and 1,4-dioxane (100 mL) in sequence. After the addition, the mixture was purged with nitrogen and stirred at 100 °C. After the reaction was completed, the reaction solution was filtered and concentrated to give a crude product, which was subjected to column chromatography (dichloromethane/methanol = 20/1) to give intermediate 5A-1 (4.4 g).

MS (ESI, [M+H]⁺) *m*/*z*: 501.3.

### Step B: Preparation of intermediate 5A

To a microwave tube were added intermediate 5A-1 (2.1 g), chloro(2-dicyclohexylphosphine-2',4',6'-triisopropyl-1,1'-biphenyl)[2-(2'-amino-1,1'-biphenyl)]palladium (II) (585.0 mg), bis(pinacolato)diboron (2.1 g), potassium carbonate (1.2 g), and 1,4-dioxane (60 mL) in sequence. After the addition, the mixture was purged with nitrogen and reacted at 90 °C under microwave irradiation. After the reaction was completed, the reaction solution was filtered, concentrated, and subjected to column chromatography (dichloromethane/methanol = 20/1) to give intermediate 5A (2.8 g).

MS (ESI, [M+H]⁺) *m*/*z*: 593.3.

### Intermediate Example 6: Preparation of Intermediate 6A

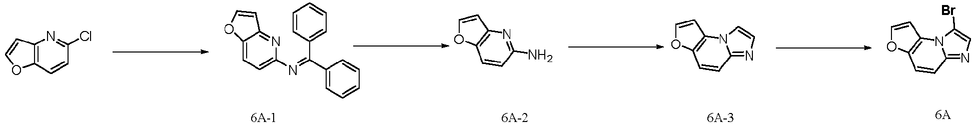

### Step A: Preparation of intermediate 6A-1

To a reaction flask were added the compounds 5-chlorofuro[3,2-*b*]pyridine (9.5 g), toluene (150.0 mL), benzophenone imine (13.5 g), sodium *tert*-butoxide (11.9 g), tris(dibenzylideneadone) (2.8 g), and 1,1-binaphthyl-2,2-bis-diphenylphosphine (1.9 g) in sequence. After the addition, the mixture was stirred at 100 °C. After the reaction was completed, the reaction solution was extracted with dichloromethane. The organic phase was washed with saturated brine, dried, concentrated, and subjected to column chromatography (dichloromethane/methanol = 30/1) to give intermediate 6A-1 (15.2 g).

MS (ESI, [M+H]⁺) *m*/*z*: 299.12.

### Step B: Preparation of intermediate 6A-2

To a reaction flask were added intermediate 6A-1 (15.2 g), dichloromethane (20.0 mL), and a solution of hydrochloric acid in 1,4-dioxane (4 M, 191.0 mL) in sequence. After the addition, the mixture was stirred at 100 °C. After the reaction was completed, the reaction solution was filtered, concentrated, and subjected to column chromatography (petroleum ether/ethyl acetate = 5/1) to give intermediate 6A-2 (5.1 g).

MS (ESI, [M+H]⁺) *m*/*z*: 135.2.

### Step C: Preparation of intermediate 6A-3

To a reaction flask were added intermediate 6A-2 (5.1 g), bromoacetaldehyde diethyl acetal (36.7 g), methanol (10.0 mL), and hydrochloric acid (3 M, 12.2 mL) in sequence. After the addition, the mixture was stirred at 35 °C. After the reaction was completed, the reaction solution was filtered, concentrated, and subjected to column chromatography (petroleum ether/ethyl acetate = 5/1) to give intermediate 6A-3 (5.3 g).

MS (ESI, [M+H]⁺) *m*/*z*: 159.1.

### Step D: Preparation of intermediate 6A

To a reaction flask were sequentially added intermediate 6A-3 (5.3 g), dimethyl sulfoxide (30.0 mL), 2,5-dibromo-4,4-dimethylcyclopentane-1,3-dione (3.8 g), and sodium carbonate (2.8 g) in sequence. After the addition, the mixture was stirred at room temperature under nitrogen atmosphere. After the reaction was completed, the reaction solution was extracted with water and dichloromethane. The organic phase was isolated, concentrated, and subjected to column chromatography (petroleum ether/ethyl acetate = 5/1) to give intermediate 6A-4 (4.3 g).

MS (ESI, [M+H]⁺) *m*/*z*: 237.4.

### Intermediate Example 7: Preparation of Intermediate 7A

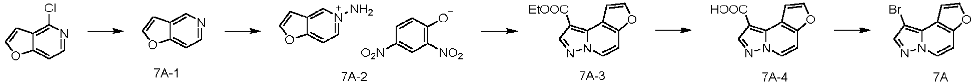

### Step A: Preparation of intermediate 7A-1

To a reaction flask were added compound 4-chlorofuranylpyridine (10 g), acetic acid (130 mL), and zinc powder (10.0 g) in sequence. After the addition, the mixture was stirred at 100 °C. After the reaction was completed, the reaction solution was adjusted to alkalinity (about pH 10) with a saturated sodium bicarbonate solution and extracted with ethyl acetate. The organic phase was isolated, washed, dried, concentrated, and subjected to column chromatography (petroleum ether/ethyl acetate = 9/1)to give intermediate 7A-1 (5.2 g).

MS (ESI, [M+H]⁺) *m*/*z*: 120.0.

### Step B: Preparation of intermediate 7A-2

To a reaction flask were sequentially added intermediate 7A-1 (5 g), acetonitrile (50.0 mL), and 2,4-dinitrophenylhydroxylamine (8.5 g) in sequence. After the addition, the mixture was stirred at 40 °C. After the reaction was completed, the reaction solution was filtered and washed. The residue was dried to give intermediate 7A-2 (9.8 g), which was directly used in the next step.

### Step C: Preparation of intermediate 7A-3

To a reaction flask were sequentially added intermediate 7A-2 (5 g), potassium carbonate (3 g), ethyl propiolate (3.2 g), and *N,N*-dimethylformamide (60 mL) in sequence. After the addition, the mixture was stirred at room temperature. After the reaction was completed, water was added to the reaction solution to quench the reaction, and the resulting mixture was extracted with ethyl acetate. The organic phase was isolated, dried, filtered, concentrated, and subjected to column chromatography (petroleum ether/ethyl acetate = 6/1) to give intermediate 7A-3 (3.4 g).

MS (ESI, [M+H]⁺) *m*/*z*: 231.0.

### Step D: Preparation of intermediate 7A-4

To a reaction flask were added intermediate 7A-3 (3.4 g), lithium hydroxide monohydrate (6 g), methanol (60 mL), and water (6 mL) in sequence. After the addition, the mixture was stirred at 60 °C. After the reaction was completed, the reaction solution was adjusted to acidity (about pH 5) with 1 M diluted hydrochloric acid and extracted with ethyl acetate. The organic phase was isolated, dried, filtered, concentrated, and subjected to column chromatography (petroleum ether/ethyl acetate = 3/1) to give intermediate 7A-4 (2.9 g).

MS (ESI, [M+H]⁺) *m*/*z*: 203.2.

### Step E: Preparation of intermediate 7A

To a reaction flask were added intermediate 7A-4 (2.9 g), N-bromosuccinimide (2.56 g), sodium bicarbonate (3.6 g), and *N,N*-dimethylformamide (40 mL) in sequence. After the addition, the mixture was reacted at room temperature. After the reaction was completed, the reaction solution was extracted with water and ethyl acetate. The organic phase was isolated, dried, filtered, concentrated, and subjected to column chromatography (petroleum ether/ethyl acetate = 6/1) to give intermediate 7A (3.08 g).

MS (ESI, [M+H]⁺) *m*/*z*: 237.0.

### Intermediate Example 8: Preparation of Intermediate 8A

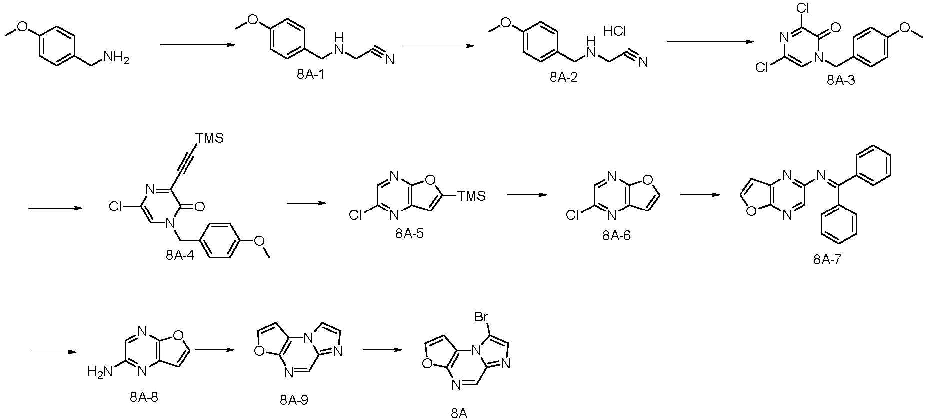

### Step A: Preparation of intermediate 8A-1

To a reaction flask were added 4-methoxybenzylamine (10.00 g), 3,6-dihydro-2*H*-pyran-4-boronic acid pinacol ester (6.60 g), potassium carbonate (20.15 g), chloroacetonitrile (6.05 g), and acetonitrile (100 mL) in sequence. After the addition, the mixture was stirred at 60 °C. After the reaction was completed, the reaction solution was concentrated and subjected to column chromatography (petroleum ether/ethyl acetate = 10/1) to give intermediate 8A-1 (10.72 g).

MS (ESI, [M+H]⁺) *m*/*z*: 177.1*.*

### Step B: Preparation of intermediate 8A-2

To a reaction flask were added intermediate 8A-1 (10.00 g), a solution of hydrochloric acid in dioxane (4 M, 20 mL), and dichloromethane (100 mL) in sequence. After the addition, the mixture was stirred at room temperature. After the reaction was completed, the reaction solution was filtered and dried to give intermediate 8A-2 (11.53 g). MS (ESI, [M+H]⁺) *m*/*z*: 177.1*.*

### Step C: Preparation of intermediate 8A-3

To a reaction flask were added intermediate 8A-2 (10.00 g), chlorobenzene (75 mL), and oxalyl chloride (17.90 g) in sequence at 0 °C. After the addition, the mixture was reacted at room temperature for 0.5 h. Triethylamine hydrochloride (32.40 g) was added to the reaction solution. After the addition, the mixture was stirred at room temperature. After the reaction was completed, the reaction solution was concentrated and extracted with water and dichloromethane. The organic phase was isolated, dried, filtered, concentrated, and subjected to column chromatography (petroleum ether/ethyl acetate = 4/1) to give intermediate 8A-3 (10.60 g).

MS (ESI, [M+H]⁺) *m*/*z*: 285.2.

### Step D: Preparation of intermediate 8A-4

To a reaction flask were added intermediate 8A-3 (8.4 g), copper(I) iodide (0.56 g), bis(triphenylphosphine)palladium(II) dichloride (2.07 g), trimethylsilylacetylene (5.50 g), triethylamine (24 mL), and *N,N*-dimethylformamide (24 mL) in sequence. After the addition, the mixture was purged with nitrogen and stirred at 80 °C. After the reaction was completed, a saturated ammonium chloride solution was added to the reaction solution to quench the reaction, and the resulting mixture was extracted with dichloromethane. The organic phase was isolated, dried, filtered, concentrated, and subjected to column chromatography (petroleum ether/ethyl acetate = 5/1) to give intermediate 8A-4 (9.10 g).

MS (ESI, [M+H]⁺) *m*/*z*: 347.1.

### Step E: Preparation of intermediate 8A-5

To the reaction flask were added intermediate 8A-4 (9.10 g), dichloromethane (150 mL), silver trifluoromethanesulfonate (0.40 g), and trifluoroacetic acid (9.75 mL) in sequence. After the addition, the mixture was stirred at room temperature. After the reaction was completed, the reaction solution was concentrated and subjected to column chromatography (petroleum ether/ethyl acetate = 10/1) to give intermediate 8A-5 (4.26 g).

MS (ESI, [M+H]⁺) *m*/*z*: 227.4.

### Step F: Preparation of intermediate 8A-6

To a reaction flask were added intermediate 8A-5 (1.42 g), tetrahydrofuran (25 mL), and a solution of tetrabutylammonium fluoride in tetrahydrofuran (1 M, 7.52 mL) in sequence at -78 °C. After the addition, the mixture was stirred at -78 °C. After the reaction was completed, a saturated ammonium chloride solution was added to the reaction solution to quench the reaction, and the resulting mixture was extracted with dichloromethane. The organic phase was isolated, dried, filtered, concentrated, and subjected to column chromatography (petroleum ether/ethyl acetate = 10/1) to give intermediate 8A-6 (0.89 g).

MS (ESI, [M+H]⁺) *m*/*z:* 155.1.

### Step G: Preparation of intermediate 8A-7

To a reaction flask were added intermediate 8A-6 (0.57 g), benzophenone imine (0.80 g), sodium *tert*-butoxide (0.71 g), tris(dibenzylideneacetone)dipalladium(0) (0.17 g), 1,1'-binaphthyl-2,2'-bisdiphenylphosphine (0.11 g), and 1,4-dioxane (30 mL) in sequence. After the addition, the mixture was purged with nitrogen and stirred at 80 °C. After the reaction was completed, the reaction solution was filtered, concentrated, and subjected to column chromatography (petroleum ether/ethyl acetate = 5/1) to give intermediate 8A-7 (0.46 g).

MS (ESI, [M+H]⁺) *m*/*z:* 300.1.

### Step H: Preparation of intermediate 8A-8

To a reaction flask were added intermediate 8A-7 (0.46 g), a solution of hydrochloric acid in dioxane (4 M, 5.76 mL), and dichloromethane (10 mL) in sequence. After the addition, the mixture was stirred at room temperature. After the reaction was completed, the reaction solution was concentrated and subjected to column chromatography (petroleum ether/ethyl acetate = 1/1) to give intermediate 8A-8 (0.17 g).

MS (ESI, [M+H]⁺) *m*/*z*: 136.0.

### Step I: Preparation of intermediate 8A-9

To a reaction flask were added bromoacetaldehyde diethyl acetal (2.47 g), methanol (2 mL), and hydrochloric acid (3 M, 4 mL) in sequence. After the addition, the mixture was reacted at 80 °C for 1 h. Then the reaction solution was adjusted to pH 8 with a saturated sodium bicarbonate solution. Intermediate 8A-8 (0.11 g) was added to the reaction solution, and the resulting mixture was stirred at room temperature. After the reaction was completed, the reaction solution was concentrated and subjected to column chromatography (dichloromethane/methanol = 20/1) to give intermediate 8A-9 (0.12 g).

MS (ESI, [M+H]⁺) *m*/*z*: 160.2.

### Step J: Preparation of intermediate 8A

To a reaction flask were added dibromohydantoin (108 mg), dimethyl sulfoxide (2 mL), sodium carbonate (160 mg), and intermediate 8A-9 (120 mg) in sequence. After the addition, the mixture was stirred at 60 °C. After the reaction was completed, the reaction solution was extracted with water and dichloromethane. The organic phase was isolated, dried, filtered, concentrated, and subjected to column chromatography (dichloromethane/methanol = 20/1) to give intermediate 8A (178 mg).

MS (ESI, [M+H]⁺) *m*/*z*: 238.0.

### Intermediate Example 9: Preparation of Intermediate 9A

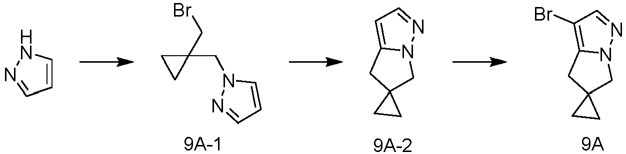

### Step A: Preparation of intermediate 9A-1

To a reaction flask were added pyrazole (10.0 g), 60 wt% sodium hydride (11 g), and *N,N-*dimethylformamide (120 mL), and 1,1-bis-bromomethylcyclopropane (10.0 g) was slowly added at 0 °C. After the addition, the mixture was reacted at room temperature. After the reaction was completed, the reaction solution was adjusted to neutrality with 1 M diluted hydrochloric acid (about pH 7), and the resulting mixture was extracted with ethyl acetate. The organic phase was isolated, dried, filtered, concentrated, and subjected to column chromatography (petroleum ether/ethyl acetate = 6/1) to give intermediate 9A-1 (8.6 g).

MS (ESI, [M+H]⁺) *m*/*z:* 215.1.

### Step B: Preparation of intermediate 9A-2

To a reaction flask containing intermediate 9A-1 (8.6 g) and tetrahydrofuran (100 mL) was slowly added dropwise a solution of lithium diisopropylamide in tetrahydrofuran (2 M, 41 mL) at -78 °C. After the addition, the mixture was reacted at room temperature. After the reaction was completed, the reaction was quenched, and the reaction solution was extracted with ethyl acetate. The organic phase was isolated, dried, filtered, concentrated, and subjected to column chromatography (petroleum ether/ethyl acetate = 9/1) to give intermediate 9A-2 (2.6 g).

MS (ESI, [M+H]⁺) *m*/*z:* 135.1.

### Step C: Preparation of intermediate 9A

To a reaction flask were added intermediate 9A-2 (2.6 g), N-bromosuccinimide (4 g), and dichloromethane (40 mL) under an ice-water bath. After the addition, the mixture was reacted at room temperature. After the reaction was completed, the reaction was quenched, and the reaction solution was extracted with ethyl acetate. The organic phase was isolated, dried, filtered, concentrated, and subjected to column chromatography (petroleum ether/ethyl acetate = 9/1) to give intermediate 9A (2.5 g).

MS (ESI, [M+H]⁺) *m*/*z*: 213.1.

### Example 1: Preparation of Compound 1-1

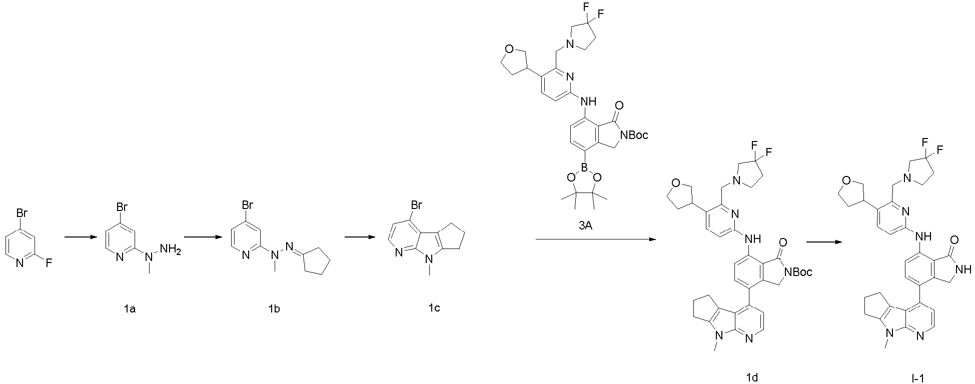

### Step A: Preparation of compound 1a

To a reaction flask were added the compounds 4-bromo-2-fluoropyridine (23.3 g), acetonitrile (300 mL), *N,N-*diisopropylethylamine (70 mL), and methylhydrazine sulfate (19 g) in sequence. After the addition, the mixture was stirred at 90 °C. After the reaction was completed, the reaction solution was concentrated, and then the residue was dissolved in ethyl acetate, washed with water, dried, concentrated, and subjected to column chromatography (petroleum ether/ethyl acetate = 2/1) to give compound 1a (12.09 g).

MS (ESI, [M+H]⁺) *m*/*z*: 201.8.

¹H NMR (500 MHz, CDCl₃) δ 7.93 (d, *J =* 5.4 Hz, 1H), 7.22 (d, *J =* 1.4 Hz, 1H), 6.72 (dd, *J =* 5.4, 1.6 Hz, 1H), 3.98 (s, 2H), 3.27 (s, 3H).

### Step B: Preparation of compound 1b

To a reaction flask were added compound 1a (6.28 g), cyclopentanone (3.02 mL), and methanol (20 mL) in sequence. After the addition, the mixture was stirred at 60 °C. After the reaction was completed, the reaction solution was directly concentrated to give compound 1b (6.34 g).

MS (ESI, [M+H]⁺) *m*/*z*: 268.3.

### Step C: Preparation of compound 1c

To a 35 mL microwave tube were added compound 1b (3 g) and diethylene glycol (18 mL) in sequence. The mixture was stirred at 245 °C for 0.5 h under microwave irradiation. After the reaction was completed, the reaction solution was poured into water, and the resulting mixture was filtered to give compound 1c (1.5 g).

MS (ESI, [M+H]⁺) *m*/*z*: 251.1.

¹H NMR (500 MHz, CDCl₃) δ 7.95 (d, *J =* 5.2 Hz, 1H), 7.15 (d, *J =* 5.2 Hz, 1H), 3.76 (s, 3H), 3.11-3.02 (m, 2H), 2.95-2.84 (m, 2H), 2.49 (dt, *J =* 10.2, 7.1 Hz, 2H).

### Step D: Preparation of compound 1d

To a microwave tube were added compound 1c (0.075 g), intermediate 3A (0.200 g), 2-dicyclohexylphosphine-2',4',6'-triisopropylbiphenyl (0.012 g), chloro(2-dicyclohexylphosphino-2',4',6'-triisopropyl-1,1'-biphenyl)[2-(2'-amino-1,1'-biphenyl)]palladium (II) (0.020 g), tripotassium phosphate (0.212 g), 1,4-dioxane (5 mL), and water (1 mL) in sequence. The mixture was reacted at 100 °C for 3 h. After the reaction was completed, the reaction solution was filtered, concentrated, and subjected to column chromatography (dichloromethane/methanol = 20/1) to give compound 1d (0.082 g).

MS (ESI, [M+H]⁺) *m*/*z:* 685.45.

### Step E: Preparation of compound I-1

To a reaction flask were added compound 1d (0.082 g), dichloromethane (4 mL), and a solution of hydrochloric acid in 1,4-dioxane (2 mL, 4 M) in sequence. The mixture was stirred at room temperature. After the reaction was completed, the reaction solution was adjusted to pH 13 with 10% aqueous sodium hydroxide solution (w/w), extracted with water and dichloromethane, washed, dried, filtered, concentrated, and subjected to column chromatography (dichloromethane/methanol = 10/1) to give compound 1-1 (0.035 g).

HRMS (ESI, [M-H]⁻) *m*/*z*: 583.26408.

¹H NMR (500 MHz, CDCl₃) δ 9.84 (s, 1H), 8.86 (d, *J =* 8.5 Hz, 1H), 8.22 (d, *J =* 4.9 Hz, 1H), 7.57 (dd, *J =* 16.2, 8.5 Hz, 2H), 6.99 (d, *J =* 4.9 Hz, 1H), 6.83 (d, *J =* 8.5 Hz, 1H), 6.38 (s, 1H), 4.38 (s, 2H), 4.08 (dt, *J =* 8.3, 6.5 Hz, 2H), 3.96 - 3.80 (m, 6H), 3.73 (ddd, *J =* 22.7, 14.5, 6.8 Hz, 2H), 3.01 (dd, *J =* 13.2, 11.5 Hz, 2H), 2.95 - 2.82 (m, 4H), 2.55 (t, *J* = 6.7 Hz, 2H), 2.38 (ddd, *J* = 12.7, 11.7, 6.0 Hz, 3H), 2.32 - 2.20 (m, 2H), 1.93 (dd, *J* = 12.4, 7.5 Hz, 1H).

### Example 2: Preparation of Compound 1-2

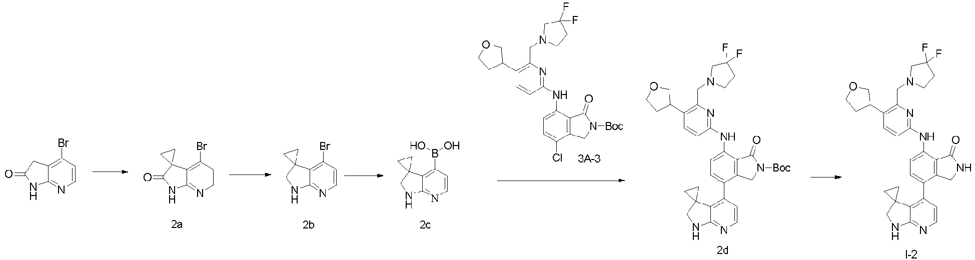

### Step A: Preparation of compound 2a

To a reaction flask were added the compounds 4-bromo-1*H-*pyrrolo[2,3-*b*]pyrimidin-2(3*H*)-one (1 g), diphenyl(vinyl)sulfonium trifluoromethanesulfonate (2 g), 1,8-diazabicyclo[5.4.0]undec-7-ene (2.14 g), and zinc trifluoromethanesulfonate (1.71 g) in sequence. After the addition, the mixture was stirred at room temperature. After the reaction was completed, the reaction was quenched with saturated ammonium chloride solution, and the reaction solution was extracted with ethyl acetate, subjected to liquid separation phase, dried, filtered, concentrated, and subjected to column chromatography (petroleum ether/ethyl acetate = 2/1) to give compound 2a (0.65 g).

MS (ESI, [M+H]⁺) *m*/*z*: 239.0.

### Step B: Preparation of compound 2b

To a reaction flask were added compound 2a (0.55 g), dichloromethane (40 mL), and diisobutylaluminum hydride (21 mL, 1.5 M) in sequence. After the addition, the mixture was stirred at room temperature. After the reaction was completed, the reaction was quenched with methanol, and the reaction solution was filtered. The filtrate was extracted with dichloromethane, dried, filtered, concentrated, and subjected to column chromatography (dichloromethane/methanol = 49/1) to give compound 2b (0.26 g).

MS (ESI, [M+H]⁺) *m*/*z*: 225.0.

### Step C: Preparation of compound 2c

To a microwave tube were added compound 2b (0.26 g), bis(triphenylphosphine)palladium(II) dichloride (100 mg), bis(neopentyl glycolato)diboron (385 mg), potassium acetate (403 mg), and 1,4-dioxane (10 mL) in sequence. The mixture was reacted at 90 °C under microwave irradiation under nitrogen atmosphere. After the reaction was completed, the reaction solution was filtered, concentrated, and subjected to column chromatography (dichloromethane/methanol = 20/1) to give compound 2c (203 mg).

MS (ESI, [M+H]⁺) *m*/*z*: 191.1.

### Step D: Preparation of compound 2d

To a reaction flask were added intermediate 3A-3 (221 mg), compound 2c (102 mg), chloro(2-dicyclohexylphosphino-2',4',6'-triisopropyl-1,1 '-biphenyl)[2-(2'-amino-1,1 '-biphenyl)]palladium (II) (40 mg), potassium phosphate (214 mg), 1,4-dioxane (5 mL), and water (1 mL) in sequence. After the addition, the mixture was stirred at 80 °C under nitrogen atmosphere. After the reaction was completed, the reaction solution was filtered, concentrated, and subjected to column chromatography (dichloromethane/methanol = 19/1) to give compound 2d (85 mg).

MS (ESI, [M+H]⁺) *m*/*z*: 659.43.

### Step E: Preparation of compound I-2

To a reaction flask were added compound 2d (85 mg), dichloromethane (10 mL), and a solution of hydrochloric acid in 1,4-dioxane (2 mL, 4 M) in sequence. The mixture was stirred at room temperature. After the reaction was completed, the reaction solution was adjusted to alkalinity with a saturated sodium bicarbonate solution, extracted with dichloromethane, concentrated, and subjected to column chromatography (dichloromethane/methanol = 10/1) to give compound I-2 (22 mg).

HRMS (ESI, [M+H]⁺) *m*/*z*: 559.26292.

¹H NMR (500 MHz, DMSO-*d*₆) δ 9.75(d, 1H), 8.80(m, 1H), 7.52-7.57(m, 1H), 6.91(s, 1H), 6.80(q, 1H), 6.28(t, 1H), 4.19(d, 2H), 3.84-3.94(m, 6H), 3.68-3.75(m, 4H), 2.96-3.02(m, 2H), 2.25-2.38(m, 3H), 1.88-1.95(m, 1H),0.56-1.25(m, 5H).

### Example 3: Preparation of Compound I-3

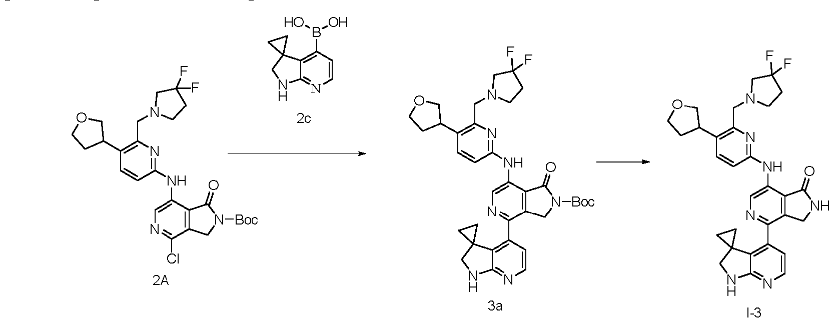

### Step A: Preparation of compound 3a

To a reaction flask were added compound 2c (68 mg), intermediate 2A (185 mg), chloro(2-dicyclohexylphosphino-2',4',6'-triisopropyl-1,1'-biphenyl)[2-(2'-amino-1,1'-biphenyl)]palladium (II) (27 mg), 2-dicyclohexylphosphine-2',4',6'-triisopropylbiphenyl (16 mg), potassium phosphate (142 mg), 1,4-dioxane (5 mL), and water (1 mL) in sequence. After the addition, the mixture was reacted at 85 °C under microwave irradiation under nitrogen atmosphere. After the reaction was completed, the reaction solution was filtered, concentrated, and subjected to column chromatography (dichloromethane/methanol = 20/1) to give compound 3a (98 mg).

MS (ESI, [M+H]⁺) *m*/*z*: 660.44.

### Step E: Preparation of compound I-3

To a reaction flask were added compound 3a (98 mg), dichloromethane (10 mL), and a solution of hydrochloric acid in 1,4-dioxane (2 mL, 4 M) in sequence. The mixture was stirred at room temperature. After the reaction was completed, the reaction solution was adjusted to alkalinity (about pH 10) with a saturated sodium bicarbonate solution, extracted with dichloromethane, concentrated, and subjected to column chromatography (dichloromethane/methanol = 10/1) to give compound I-3 (50 mg).

HRMS (ESI, [M+H]⁺) *m*/*z*: 560.25881.

¹H NMR (500 MHz, DMSO-*d*₆) δ 8.25(s, 1H), 7.56(d, 1H), 7.38(t, 1H), 6.80(d, 1H), 6.36(d, 1H), 4.27(s, 2H), 3.63-3.87(m, 10H), 2.81-2.94(m, 2H), 2.18-2.35(m, 3H), 1.85-1.89(m, 1H), 1.18(s, 3H), 0.82(s, 2H).

### Example 4: Preparation of Compound I-4

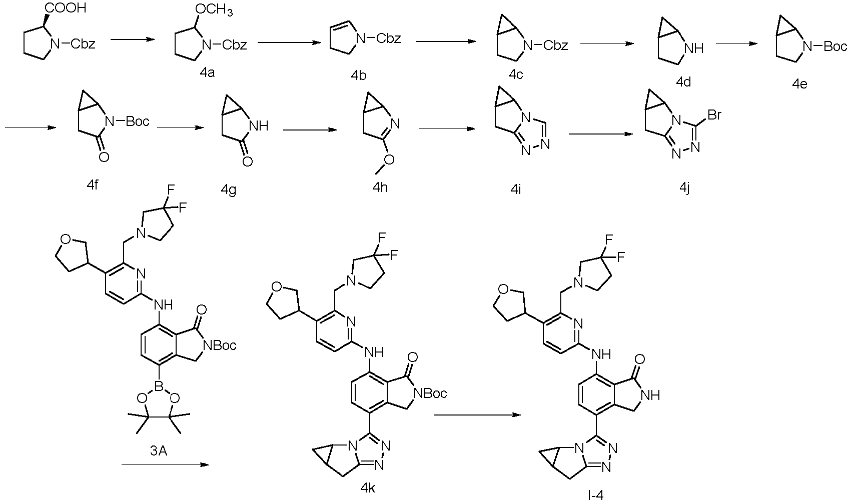

### Step A: Preparation of compound 4a

To a reaction flask were added the compounds *N*-benzyloxycarbonyl-L-proline (20.0 g), iodobenzenediacetic acid (51.7 g), iodine (10.2 g), and dichloromethane (200 mL) in sequence. After the addition, the mixture was stirred at room temperature under nitrogen atmosphere. After the reaction was completed, the reaction solution was concentrated and subjected to column chromatography (petroleum ether/ethyl acetate = 20/1) to give compound 4a (14.8 g).

MS (ESI, [M+H]⁺) *m*/*z*: 236.4.

### Step B: Preparation of compound 4b

To a reaction flask were added compound 4a (14.8 g), dichloromethane (200 mL), *N,N*-diisopropylethylamine (14.0 mL), and trimethylsilyl trifluoromethanesulfonate (14.8 g) in sequence. After the addition, the mixture was stirred at room temperature under nitrogen atmosphere. After the reaction was completed, the reaction solution was concentrated and subjected to column chromatography (petroleum ether/ethyl acetate = 20/1) to give compound 4b (10.6 g).

MS (ESI, [M+H]⁺) *m*/*z*: 204.1.

### Step C: Preparation of compound 4c

To a reaction flask were added compound 4b (10.6 g), diethyl ether (300 mL), chloroiodomethane (7.86 mL), and diethyl zinc (51.2 mL) in sequence. After the addition, the mixture was stirred at 0 °C under nitrogen atmosphere. After the reaction was completed, the reaction solution was concentrated and subjected to column chromatography (petroleum ether/ethyl acetate = 10/1) to give compound 4c (5.00 g).

MS (ESI, [M+H]⁺) *m*/*z*: 218.3.

### Step D: Preparation of compound 4d

To a reaction flask were added compound 4c (5.00 g), ethanol (100 mL), 10 wt% palladium on carbon (2.00 g), and a solution of hydrochloric acid in 1,4-dioxane (2.30 mL, 4 M) in sequence. After the addition, the mixture was stirred at room temperature under hydrogen atmosphere. After the reaction was completed, the reaction solution was filtered, washed, and concentrated to give compound 4d (2.31 g).

MS (ESI, [M+H]⁺) *m*/*z*: 84.1.

### Step E: Preparation of compound 4c

To a reaction flask were added compound 4d (2.31 g), tetrahydrofuran (30.0 mL), sodium carbonate (6.64 g), and di-tert-butyl dicarbonate (7.61 mL) in sequence. After the addition, the mixture was stirred at room temperature. After the reaction was completed, the reaction solution was concentrated and subjected to column chromatography (petroleum ether/ethyl acetate = 10/1) to give compound 4e (3.00 g).

MS (ESI, [M+H]⁺) *m*/*z*: 184.1.

### Step F: Preparation of compound 4f

To a reaction flask were added compound 4e (2.31 g), ruthenium dioxide (0.300 g), sodium periodate (23.3 g), ethyl acetate (30.0 mL), and water (30.0 mL) in sequence. After the addition, the mixture was stirred at room temperature. After the reaction was completed, the reaction solution was concentrated and subjected to column chromatography (petroleum ether/ethyl acetate = 5/1) to give compound 4f (1.47 g).

MS (ESI, [M+H]⁺) *m*/*z*: 198.1.

### Step G: Preparation of compound 4g

To a reaction flask were added compound 4f (1.47 g), a solution of hydrochloric acid in 1,4-dioxane (5.00 mL, 4 M), and dichloromethane (30.0 mL) in sequence. After the addition, the mixture was stirred at room temperature. After the reaction was completed, the reaction solution was concentrated to give compound 4g (1.25 g).

MS (ESI, [M+H]⁺) *m*/*z*: 98.1.

### Step H: Preparation of compound 4h

To a reaction flask were added compound 4g (2.31 g), dichloromethane (20.0 mL), and trimethyloxonium tetrafluoroborate (3.20 g) in sequence. After the addition, the mixture was stirred at room temperature. After the reaction was completed, the reaction solution was concentrated and subjected to column chromatography (dichloromethane/methanol = 10/1) to give compound 4h (0.71 g).

MS (ESI, [M+H]⁺) *m*/*z*: 112.3.

### Step I: Preparation of compound 4i

To a microwave tube were added compound 4h (0.71 g), *n*-butanol (20.0 mL), formylhydrazine (0.36 g), and acetic acid (0.18 g) in sequence. After the addition, the mixture was stirred at 120 °C under nitrogen atmosphere. After the reaction was completed, the reaction solution was concentrated and subjected to column chromatography (dichloromethane/methanol = 2/1) to give compound 4i (0.15 g).

MS (ESI, [M+H]⁺) *m*/*z*: 122.0.

### Step J: Preparation of compound 4j

To a reaction flask were added compound 4i (0.15 g), *N*-bromosuccinimide (0.18 g), carbon tetrachloride (5.00 mL), and acetic acid (5.00 mL) in sequence. After the addition, the mixture was stirred at 80 °C under nitrogen atmosphere. After the reaction was completed, the reaction solution was concentrated and subjected to column chromatography (dichloromethane/methanol = 10/1) to give compound 4j (0.11 g).

MS (ESI, [M+H]⁺) *m*/*z:* 200.0.

### Step K: Preparation of compound 4k

To a reaction flask were added compound 4j (0.15 g), intermediate 3A (0.18 g), 2-dicyclohexylphosphine-2',4',6'-triisopropylbiphenyl (0.02 g), chloro(2-dicyclohexylphosphino-2',4',6'-triisopropyl-1,1'-biphenyl)[2-(2'-amino-1,1'-biphenyl)]palladium (II) (0.03 g), potassium phosphate (0.09 g), water (2.00 mL), and dioxane (8.00 mL) in sequence. After the addition, the mixture was stirred at 85 °C under nitrogen atmosphere. After the reaction was completed, the reaction solution was concentrated and subjected to column chromatography (dichloromethane/methanol = 10/1) to give compound 4k (0.07 g).

MS (ESI, [M+H]⁺) *m*/*z*: 634.4.

### Step L: Preparation of compound 1-4

To a reaction flask were added compound 4k (0.07 g), dichloromethane (10.0 mL), and a solution of hydrochloric acid in 1,4-dioxane (2.00 mL, 4 M) in sequence. After the addition, the mixture was stirred at room temperature. After the reaction was completed, the reaction solution was adjusted to alkalinity (about pH 10) with a saturated sodium bicarbonate solution, extracted with dichloromethane, concentrated, and subjected to column chromatography (dichloromethane/methanol = 10/1) to give compound I-4 (0.036 g).

HRMS (ESI, [M+H]⁺) *m*/*z*: 534.24245.

¹H NMR (500 MHz, CDCl₃)δ10.05 (s, 1H), 8.93 (d, *J =* 8.7 Hz, 1H), 7.92 (d, *J =* 8.7 Hz, 1H), 7.57 (d, *J =* 8.5 Hz, 1H), 6.84 (d,J= 8.5 Hz, 1H), 6.65 (s, 1H), 4.95 (d, *J* = 19.1 Hz, 1H), 4.76 (d, *J* = 19.1 Hz, 1H), 4.17 - 3.99 (m, 3H), 3.97-3.80 (m, 3H), 3.72 (dq, *J* = 14.7, 7.0 Hz, 2H), 3.27 (dd, *J=* 17.3, 6.8 Hz, 1H), 3.16-2.96 (m, 3H), 2.86 (m, 2H), 2.34 (dtt, *J* = 43.8, 14.4, 6.3 Hz, 4H), 1.93 (dq, *J* = 14.8, 7.7 Hz, 1H), 1.42 (q, *J* = 6.7 Hz, 1H), 0.80 (d, *J* = 6.2 Hz, 1H).

### Example 5: Preparation of Compound I-5

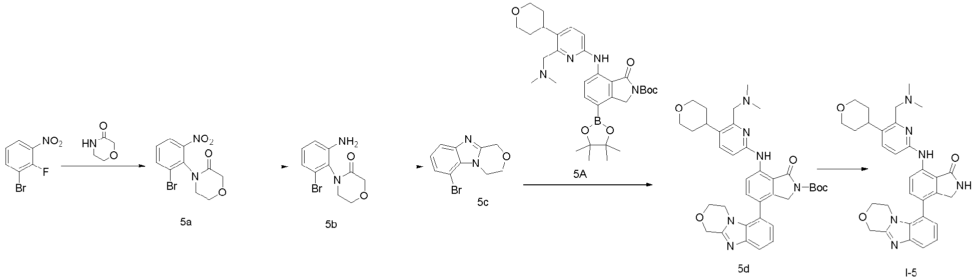

### Step A: Preparation of compound 5a

To a reaction flask were added morpholine-3-one (0.965 g), *N,N*-dimethylformamide (40 mL), and sodium hydride (0.51 g) in sequence at 0 °C under nitrogen atmosphere. After the addition, the mixture was stirred at room temperature for 1 h. 1-Bromo-2-fluoro-3-nitrobenzene (2 g) was added. After the addition, the mixture was stirred at room temperature. After the reaction was completed, the reaction solution was concentrated and subjected to column chromatography (petroleum ether/ethyl acetate = 3/1) to give compound 5a (0.39 g).

MS (ESI, [M+H]⁺) *m*/*z*: 301.0.

### Step B: Preparation of compound 5b

To a reaction flask were added compound 5a (0.39 g), iron powder (0.36 g), ammonium chloride (0.035 g), ethanol (12 mL), and water (3 mL) in sequence. After the addition, the mixture was stirred at 80 °C. After the reaction was completed, the reaction solution was filtered and concentrated to give compound 5b (0.32 g).

MS (ESI, [M+H]⁺) *m*/*z*: 271.0.

### Step C: Preparation of compound 5c

To a reaction flask were added compound 5b (0.32 g) and acetic acid (10 mL) in sequence. After the addition, the mixture was stirred at 110 °C. After the reaction was completed, the reaction solution was concentrated to give compound 5c (0.11 g).

MS (ESI, [M+H]⁺) *m*/*z*: 253.0.

### Step D: Preparation of compound 5d

To a microwave tube were added compound 5c (0.11 g), intermediate 5A (0.33 g), 2-dicyclohexylphosphine-2',4',6'-triisopropylbiphenyl (0.041 g), chloro(2-dicyclohexylphosphino-2',4',6'-triisopropyl-1,1'-biphenyl) [2-(2'-amino-1,1'-biphenyl)]palladium (II) (0.068 g), tripotassium phosphate (0.15 g), 1,4-dioxane (12 mL), and water (3 mL) in sequence. After the addition, the mixture was reacted at 85 °C under microwave irradiation under nitrogen atmosphere. After the reaction was completed, the reaction solution was filtered, concentrated, and subjected to column chromatography (dichloromethane/methanol = 20/1) to give compound 5d (0.20 g).

### Step E: Preparation of compound I-5

To a reaction flask were added compound 5d (0.20 g), dichloromethane (10 mL), and a solution of hydrochloric acid in 1,4-dioxane (2 mL, 4 M) in sequence. The mixture was stirred at room temperature. After the reaction was completed, the reaction solution was adjusted to alkalinity with a saturated sodium bicarbonate solution, extracted with dichloromethane, concentrated, and subjected to column chromatography (dichloromethane/methanol = 20/1) to give compound I-5 (0.035 g).

HRMS (ESI, [M+H]⁺) *m*/*z*: 539.2762.

¹H NMR (500 MHz, CDCl₃) δ 9.72 (s, 1H), 8.88 (d, *J=* 8.5 Hz, 1H), 7.78-7.69 (m, 1H), 7.49 (dd, *J=* 22.7, 8.5 Hz, 2H), 7.32-7.27 (m, 1H), 7.13-7.08 (m, 1H), 6.82 (d, *J =* 8.4 Hz, 1H), 6.53 (s, 1H), 5.06-4.99 (m, 2H), 4.30 (d, *J =* 17.6 Hz, 1H), 4.12-4.01 (m, 3H), 3.98-3.87 (m, 2H), 3.78-3.71 (m, 1H), 3.64 (t, *J=* 11.5 Hz, 1H), 3.56 (dd, *J* = 22.5, 11.8 Hz, 3H), 3.44-3.37 (m, 1H), 3.24-3.15 (m, 1H), 2.31 *(d, J=* 6.3 Hz, 6H), 1.80-1.67 (m, 4H).

### Example I-6: Preparation of Compound I-6

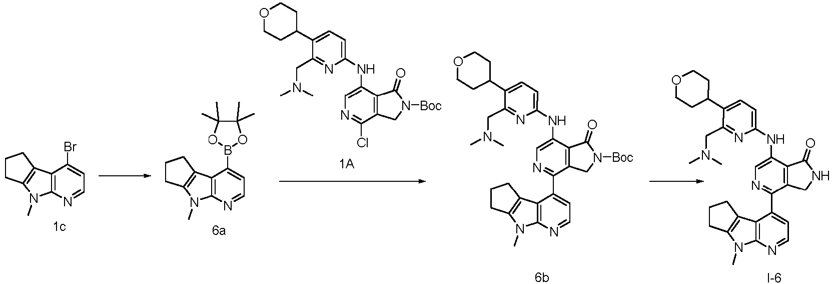

### Step A: Preparation of compound 6a

To a reaction flask were added compound 1c (0.95 g), chloro(2-dicyclohexylphosphino-2',4',6'-triisopropyl-1,1'-biphenyl)[2-(2'-amino-1,1'-biphenyl)]palladium (II) (309 mg), bis(pinacolato)diboron (1.921 g), potassium acetate (743 mg), and 1,4-dioxane (50 mL) in sequence. The mixture was stirred at 100 °C under nitrogen atmosphere. After the reaction was completed, the reaction solution was filtered, concentrated, and subjected to column chromatography (petroleum ether/ethyl acetate = 10/1) to give compound 6a (1.1 g).

MS (ESI, [M+H]⁺) *m*/*z*: 299.19.

### Step B: Preparation of compound 6b

To a reaction flask were added compound 6a (101 mg), intermediate 1A (210 mg), chloro(2-dicyclohexylphosphino-2',4',6'-triisopropyl-1,1'-biphenyl)[2-(2'-amino-1,1'-biphenyl)]palladium (II) (28 mg), potassium phosphate (216 mg), 1,4-dioxane (15 mL), and water (3 mL) in sequence. After the addition, the mixture was stirred at 85 °C under nitrogen atmosphere. After the reaction was completed, the reaction solution was filtered, concentrated, and subjected to column chromatography (dichloromethane/methanol = 18/1) to give compound 6b (110 mg).

MS (ESI, [M+H]⁺) *m*/*z*: 638.45.

### Step C: Preparation of compound I-6

To a reaction flask were added compound 6b (0.11 g), dichloromethane (10 mL), and a solution of hydrochloric acid in 1,4-dioxane (2 mL) in sequence. The mixture was stirred at room temperature. After the reaction was completed, the reaction solution was adjusted to alkalinity (about pH 10) with a saturated sodium bicarbonate solution, extracted with dichloromethane, concentrated, and subjected to column chromatography (dichloromethane/methanol = 15/1) to give compound I-6 (23 mg).

HRMS (ESI, [M+H]⁺) *m*/*z*: 538.2923.

¹H NMR (500 MHz, CDCl₃) δ 10.23(s, 1H), 8.22(d, 1H), 7.58(d, 1H), 7.31(s, 1H), 7.11(s, 1H), 6.88(d, 1H), 4.53(s, 2H), 4.09(dd, 2H), 3.78-3.82(m, 5H), 3.57-3.62(m, 5H), 2.66(t, 2H), 2.45(s, 6H), 1.72-1.83(m, 4H).

### Example 7: Preparation of Compound I-7

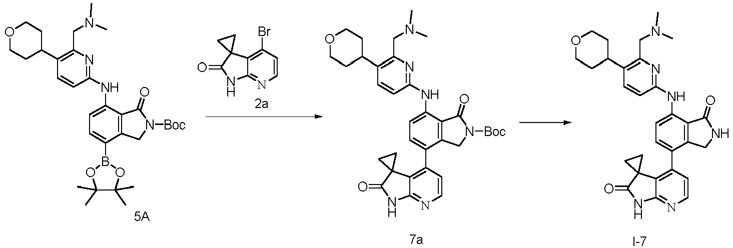

### Step A: Preparation of compound 7a

To a reaction flask were added compound 2a (488 mg), intermediate 5A(500 mg), chloro(2-dicyclohexylphosphino-2',4',6'-triisopropyl-1,1'-biphenyl)[2-(2'-amino-1,1'-biphenyl)]palladium (II) (103 mg), 2-dicyclohexylphosphine-2',4',6'-triisopropylbiphenyl (63 mg), potassium phosphate (553 mg), 1,4-dioxane (50 mL), and water (5 mL) in sequence. After the addition, the mixture was stirred at 85 °C under nitrogen atmosphere. After the reaction was completed, the reaction solution was filtered, concentrated, and subjected to column chromatography (dichloromethane/methanol = 18/1) to give compound 7a (156 mg).

MS (ESI, [M+H]⁺) *m*/*z*: 625.41.

### Step B: Preparation of compound I-7

To a reaction flask were added compound 7a (0.156 g), dichloromethane (10 mL), and a solution of hydrochloric acid in 1,4-dioxane (0.65 mL, 4 M) in sequence. The mixture was stirred at room temperature. After the reaction was completed, the reaction solution was adjusted to alkalinity (about pH 10) with a saturated sodium bicarbonate solution, extracted with dichloromethane, concentrated, and subjected to column chromatography (dichloromethane/methanol = 5/1) to give compound I-7 (30 mg).

HRMS (ESI, [M+H]⁺) *m*/*z*: 525.2609.

¹H NMR (500 MHz, DMSO-*d₆*) δ 11.32(s, 1H), 9.89(s, 1H), 8.73(s, 1H), 8.60(d, 1H), 8.06(d, 1H), 7.64(d, 1H), 7.36(d, 1H), 6.91(d, 1H), 6.85(d, 1H), 4.10(s, 2H), 3.94-3.97(m, 2H), 3.56(s, 2H), 3.40-3.45(m, 2H), 3.15-3.21(m, 1H), 2.21(s, 6H), 1.61-1.71(m, 4H), 1.23-1.33(m, 4H).

### Example 8: Preparation of Compound I-8

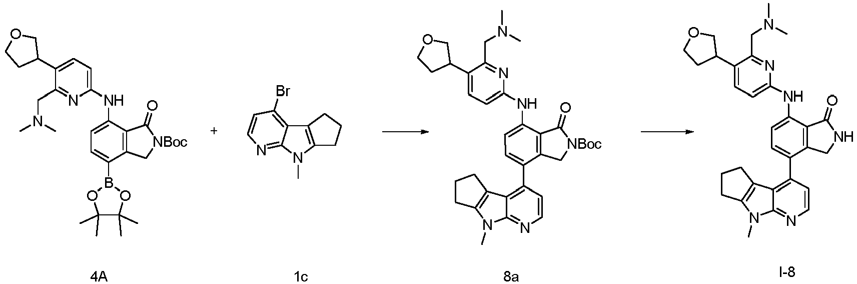

### Step A: Preparation of compound 8a

To a microwave tube were added compound 1c (0.115 g), intermediate 4A (0.300 g), 2-dicyclohexylphosphine-2',4',6'-triisopropylbiphenyl (0.020 g), chloro(2-dicyclohexylphosphino-2',4',6'-triisopropyl-1,1'-biphenyl)[2-(2'-amino-1,1'-biphenyl)]palladium (II) (0.033 g), tripotassium phosphate (0.176 g), 1,4-dioxane (5 mL), and water (1mL) in sequence. The mixture was reacted at 100 °C for 3 h. After the reaction was completed, the reaction solution was filtered, concentrated, and subjected to column chromatography (dichloromethane/methanol = 20/1) to give compound 8a (0.125g).

MS (ESI, [M+H]⁺) *m*/*z*: 623.41.

### Step B: Preparation of compound I-8

To a reaction flask were added compound 8a (0.125 g), dichloromethane (4 mL), and a solution of hydrochloric acid in 1,4-dioxane (1 mL, 4 M) in sequence. The mixture was stirred at room temperature. After the reaction was completed, the reaction solution was adjusted to pH 13 with 10% aqueous sodium hydroxide solution (v/v), extracted with water and dichloromethane, washed, dried, filtered, concentrated, and subjected to column chromatography (dichloromethane/methanol = 10/1) to give compound I-8 (0.045 g).

HRMS (ESI, [M+H]⁺) *m*/*z*: 523.2814.

¹H NMR (500 MHz, CDCl₃) δ 9.78 (s, 1H), 8.86 (d, *J =* 8.5 Hz, 1H), 8.22 (d, *J=* 4.9 Hz, 1H), 7.56 (t, *J=* 8.8 Hz, 2H), 6.98 (d, *J=* 4.9 Hz, 1H), 6.82 (d, *J=* 8.5 Hz, 1H), 6.30 (s, 1H), 4.37 (s, 2H), 4.14-4.04 (m, 2H), 3.97-3.87 (m, 2H), 3.84 (d, *J=* 9.7 Hz, 3H), 3.68 (dd, *J =* 13.5, 6.4 Hz, 2H), 3.58 (d, *J =* 12.3 Hz, 1H), 2.91 (t, *J=* 7.1 Hz, 2H), 2.54 (t, *J=* 6.8 Hz, 2H), 2.43-2.35 (m, 3H), 2.32 (d, *J=* 16.9 Hz, 6H), 1.93 (dq, *J=* 12.5, 7.6 Hz, 1H).

### Example 9: Preparation of Compound I-9

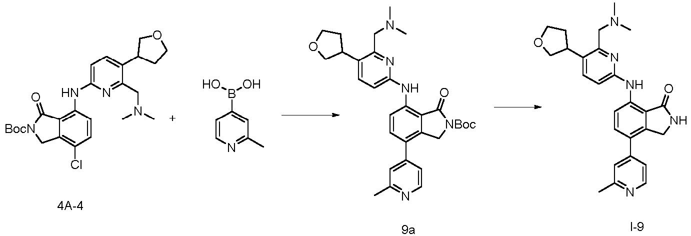

### Step A: Preparation of compound 9a

To a microwave tube were added intermediate 4A-4 (0.127 g), 2-methyl-4-pyridineboronic acid (0.300 g), chloro(2-dicyclohexylphosphino-2',4',6'-triisopropyl-1,1 '-biphenyl)[2-(2'-amino-1,1 '-biphenyl)]palladium(II) (0.048 g), tripotassium phosphate (0.523 g), 1,4-dioxane (5 mL), and water (1 mL) in sequence. The mixture was reacted at 100 °C for 3 h. After the reaction was completed, the reaction solution was filtered, concentrated, and subjected to column chromatography (dichloromethane/methanol = 20/1) to give compound 9a (0.132g).

MS (ESI, [M+H]⁺) *m*/*z*: 544.34.

### Step B: Preparation of compound I-9

To a reaction flask were added compound 9a (0.132 g), dichloromethane (4 mL), and a solution of hydrochloric acid in 1,4-dioxane (1 mL, 4 mol/L) in sequence. The mixture was stirred at room temperature. After the reaction was completed, the reaction solution was adjusted to pH 13 with 10% aqueous sodium hydroxide solution (v/v), extracted with water and dichloromethane, washed, dried, filtered, concentrated, and subjected to column chromatography (dichloromethane/methanol = 10/1) to give compound I-9 (0.043 g).

HRMS (ESI, [M+H]⁺) *m*/*z*: 444.2396.

¹H NMR (500 MHz, CDCl₃)δ 9.86 (s, 1H), 8.90 (d, *J =* 8.7 Hz, 1H), 8.54 (d, *J =* 5.2 Hz, 1H), 7.58 (dd, *J =* 24.4, 8.6 Hz, 2H), 7.23 (s, 1H), 7.17 *(d, J=* 5.1 Hz, 1H), 6.81 *(d, J=* 8.4 Hz, 1H), 6.52 (s, 1H), 4.55 (s, 2H), 4.08 (ddd, *J* = 13.3, 12.1, 6.4 Hz, 2H), 3.98-3.83 (m, 2H), 3.72-3.64 (m, 2H), 3.57 *(d, J=* 12.3 Hz, 1H), 2.61 (s, 3H), 2.42-2.33 (m, 1H), 2.30 (s, 6H), 1.98-1.89 (m, 1H).

### Example 10: Preparation of Compound I-10

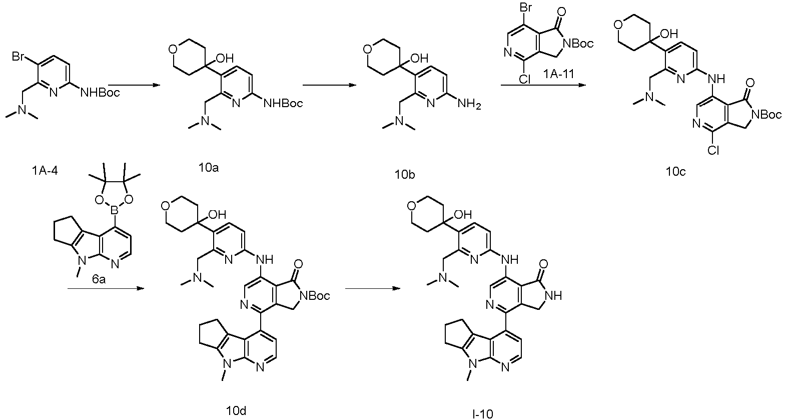

### Step A: Preparation of compound 10a

To a reaction flask were added intermediate 1A-4 (12 g) and tetrahydrofuran (150 mL). After the addition, a solution of *n*-butyllithium in tetrahydrofuran (55 mL, 2 M) was slowly added dropwise at -78 °C under nitrogen atmosphere.

The mixture was stirred for 2 h, and then tetrahydro-4*H*-pyran-4-one (4.73 g) was slowly added. After the addition, the mixture was stirred at room temperature. After the reaction was completed, the reaction was quenched with a saturated ammonium chloride solution, and the reaction solution was extracted with dichloromethane, washed, dried, filtered, and concentrated to give compound 10a (11.75 g).

MS (ESI, [M+H]⁺) *m*/*z*: 352.21.

### Step B: Preparation of compound 10b

To a reaction flask were added compound 10a (1 g), dichloromethane (10 mL), and a solution of hydrochloric acid in 1,4-dioxane (2 mL, 4 M) in sequence. After the addition, the mixture was stirred at room temperature. After the reaction was completed, the reaction solution was adjusted to alkalinity (about pH 10) with a saturated sodium bicarbonate solution, extracted with dichloromethane, and concentrated to give compound 10b (0.626 g).

MS (ESI, [M+H]⁺) *m*/*z*: 252.22.

### Step C: Preparation of compound 10c

To a reaction flask were added compound 10b (0.400 g), intermediate 1A-11 (0.553 g), tris(dibenzylideneacetone) dipalladium(0) (0.146 g), 4,5-bis-diphenylphosphino-9,9-dimethylxanthene (0.111 g), potassium carbonate (0.660 g), and toluene (10 mL) in sequence. The mixture was reacted at 100 °C for 3 h. After the reaction was completed, the reaction solution was filtered, concentrated, and subjected to column chromatography (dichloromethane/methanol = 25/1) to give compound 10c (0.165 g).

MS (ESI, [M+H]⁺) *m*/*z*: 518.30.

### Step D: Preparation of compound 10d

To a microwave tube were added compound 10c (0.165 g), compound 6a (0.114 g), chloro(2-dicyclohexylphosphino-2',4',6'-triisopropyl-1,1'-biphenyl)[2-(2'-amino-1,1'-biphenyl)]palladium(II) (0.025 g), tripotassium phosphate (0.203 g), 1,4-dioxane (5 mL), and water (1 mL) in sequence. The mixture was reacted at 100 °C. After the reaction was completed, the reaction solution was filtered, concentrated, and subjected to column chromatography (dichloromethane/methanol = 20/1) to give compound 10d (0.123 g).

MS (ESI, [M+H]⁺) *m*/*z*: 654.39.

### Step E: Preparation of compound I-10

To a reaction flask were added compound 10d (0.123 g), dichloromethane (4 mL), and a solution of hydrochloric acid in 1,4-dioxane (1 mL, 4 mol/L) in sequence. The mixture was stirred at room temperature. After the reaction was completed, the reaction solution was adjusted to alkalinity (about pH 10) with a saturated sodium bicarbonate solution, extracted with dichloromethane, concentrated, and subjected to column chromatography (dichloromethane/methanol = 10/1) to give compound 1-10 (0.023 g).

HRMS (ESI, [M+H]⁺) *m*/*z*: 554.2880.

¹H NMR (500 MHz, CDCl₃) δ 10.32 (s, 1H), 9.39 (s, 1H), 8.26 (d, *J =* 4.9 Hz, 1H), 7.62 (d, *J =* 8.6 Hz, 1H), 7.09 (d, *J=* 4.9 Hz, 1H), 6.86 (d, *J=* 8.6 Hz, 1H), 6.58 (s, 1H), 4.57 (s, 2H), 4.05 (t, *J=* 11.0 Hz, 2H), 3.94 (s, 2H), 3.86 (dd, *J* = 10.9, 4.3 Hz, 2H), 3.83 (s, 3H), 2.93 (t, *J =* 7.1 Hz, 2H), 2.70 (t, *J =* 6.9 Hz, 2H), 2.43 (dd, *J* = 14.0, 7.1 Hz, 2H), 2.29 (s, 6H), 2.07 (td, *J =* 12.8, 4.9 Hz, 2H), 1.81 (d, *J =* 12.6 Hz, 2H).

### Example 11: Preparation of Compound I-11

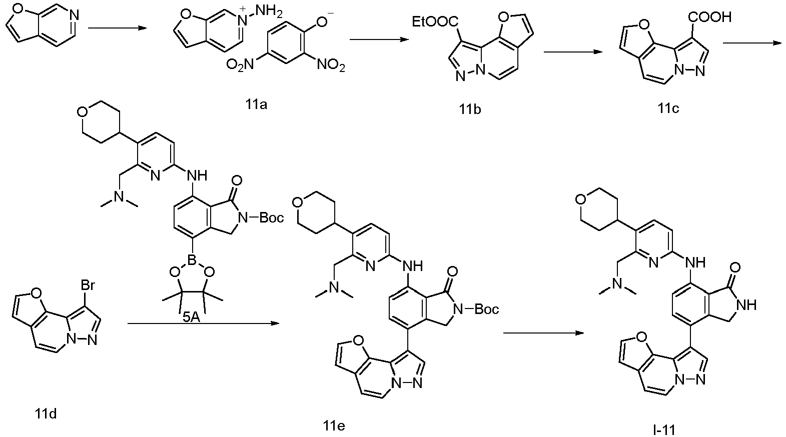

### Step A: Preparation of compound 11a

To a reaction flask were added the compounds furan[2,3-*c*]pyridine (5 g), acetonitrile (50.0 mL), and 2,4-dinitrophenylhydroxylamine (8.5 g) in sequence. After the addition, the mixture was stirred at 40 °C. After the reaction was completed, the reaction solution was filtered and washed to give compound 11a (9.8 g), which was directly used in the next step.

### Step B: Preparation of compound 11b

To a reaction flask were added compound 11a (5 g), potassium carbonate (3 g), ethyl propiolate (3.2 g), and *N,N-*dimethylformamide (60mL) in sequence. After the addition, the mixture was stirred at room temperature. After the reaction was completed, a saturated aqueous sodium chloride solution was added to the reaction solution to quench the reaction, and the resulting mixture was extracted with ethyl acetate. The organic phase was dried, filtered, concentrated, and subjected to column chromatography (petroleum ether/ethyl acetate = 6/1) to give compound 11b (3.4 g).

MS (ESI, [M+H]⁺) *m*/*z*: 231.0.

### Step C: Preparation of compound 11c

To a reaction flask were added compound 11b (3.4 g), lithium hydroxide monohydrate (6 g), methanol (60 mL), and water (6 mL) in sequence. After the addition, the mixture was stirred at 60 °C. After the reaction was completed, the reaction solution was adjusted to acidity (about pH 5) with diluted hydrochloric acid and extracted with ethyl acetate. The organic phase was dried, filtered, concentrated, and subjected to column chromatography (petroleum ether/ethyl acetate = 3/1) to give compound 11c (2.9 g).

MS (ESI, [M+H]⁺) *m*/*z*: 203.2.

### Step D: Preparation of compound 11d

To a reaction flask were added compound 11c (2.9 g), *N*-bromosuccinimide (2.56 g), sodium bicarbonate (3.6 g), and *N,N*-dimethylformamide (40 mL) in sequence. After the addition, the mixture was stirred at room temperature. After the reaction was completed, the reaction solution was extracted with ethyl acetate. The organic phase was dried, filtered, concentrated, and subjected to column chromatography (petroleum ether/ethyl acetate = 6/1) to give intermediate 11d (3.08 g).

MS (ESI, [M+H]⁺) *m*/*z*: 237.0.

### Step E: Preparation of compound 11e

To a reaction flask were added compound 11d (0.2 g), intermediate 5A (0.3 g), potassium phosphate (0.1 g), chloro(2-dicyclohexylphosphino-2',4',6'-triisopropyl-1,1'-biphenyl)[2-(2'-amino-1,1'-biphenyl)]palladium(II) (0.04 g), 1,4-dioxane (10 mL), and water (0.5 mL) in sequence. After the addition, the mixture was stirred at 100 °C under nitrogen atmosphere. After the reaction was completed, the reaction solution was concentrated and subjected to column chromatography (dichloromethane/methanol = 20/1) to give compound 11e (0.11 g).

MS (ESI, [M+H]⁺) *m*/*z*: 623.1.

### Step F: Preparation of compound I-11

To a reaction flask were added compound 11e (0.11 g), a solution of hydrochloric acid in 1,4-dioxane (4 M, 1 mL), and dichloromethane (10 mL) in sequence. After the addition, the mixture was stirred at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 9/1) to give compound 1-11 (0.01 g).

HRMS (ESI, [M+H]⁺) *m*/*z*: 523.2455.

¹H NMR (500 MHz, CD₃Cl:CD₃OD=10:1) δ 8.83 (d, *J =* 8.6 Hz, 1H), 8.36 (d, *J =* 7.2 Hz, 1H), 8.00 (s, 1H), 7.81 (d, *J=* 8.6 Hz, 1H), 7.69 (d, *J=* 2.0 Hz, 1H), 7.52 (d, *J=* 8.4 Hz, 1H), 7.04 (d, *J=* 7.2 Hz, 1H), 6.89 - 6.81 (m, 2H), 4.47 (s, 2H), 4.17 - 4.03 (m, 2H), 3.69 (s, 2H), 3.57 (td, *J =* 11.6, 2.2 Hz, 2H), 3.19 (s, 1H), 2.38 (s, 6H), 1.85 - 1.67 (m, 4H).

### Example 12: Preparation of Compound I-12

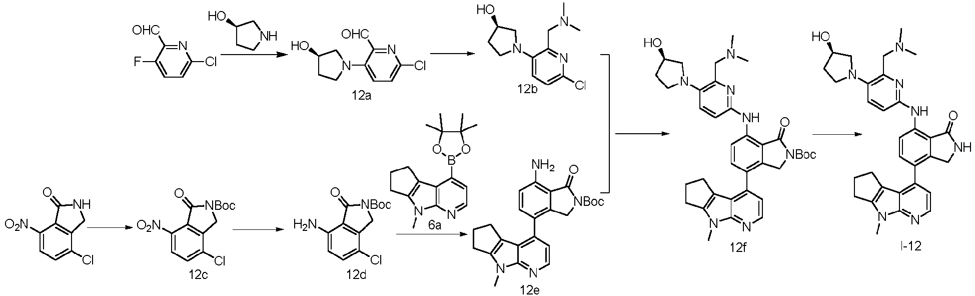

### Step A: Preparation of compound 12a

To a reaction flask were added 2-chloro-5-fluoropyridine-6-carbaldehyde (5.00 g), potassium carbonate (21.66 g), *N,N*-dimethylformamide (100 mL), (*R*)-3-pyrrolidinol (5.46 g), and tetrabutylammonium iodide (2.32 g) in sequence. After the addition, the mixture was stirred at room temperature. After the reaction was completed, the reaction solution was extracted with ethyl acetate. The organic phase was dried, filtered, concentrated, and subjected to column chromatography (petroleum ether/ethyl acetate = 1/1) to give compound 12a (2.56 g).

MS (ESI, [M+H]⁺) m/z: 227. 11.

### Step B: Preparation of compound 12b

To a reaction flask were added compound 12a (2.54 g), methanol (40 mL), acetic acid (0.34 g), and a solution of dimethylamine in tetrahydrofuran (2 M, 8.41 mL) in sequence. After the addition, the mixture was reacted at 0 °C for 0.5 h. Sodium cyanoborohydride (1.41 g) was added to the reaction solution. After the addition, the mixture was stirred at room temperature. After the reaction was completed, the reaction solution was concentrated and subjected to column chromatography (dichloromethane/methanol = 20/1) to give compound 12b (1.57 g).

MS (ESI, [M+H]⁺) m/z: 256.13.

### Step C: Preparation of compound 12c

To a reaction flask were added 4-chloro-7-nitroisoindolin-1-one (5.00 g), 4-dimethylaminopyridine (3.45 g), di*-tert-*butyl dicarbonate (7.70 g), and tetrahydrofuran (100 mL) in sequence. After the addition, the mixture was stirred at room temperature. After the reaction was completed, the reaction solution was extracted with ethyl acetate. The organic phase was dried, filtered, concentrated, and subjected to column chromatography (petroleum ether/ethyl acetate = 5/1) to give compound 12c (2.57 g).

MS (ESI, [M+Na]+) m/z: 335.07.

### Step D: Preparation of compound 12d

To a reaction flask were added compound 12c (2.00 g), zinc powder (2.09 g), ammonium chloride (2.05 g), ethanol (30 mL), and water (6 mL) in sequence. After the addition, the mixture was stirred at 70 °C. After the reaction was completed, the reaction solution was filtered, concentrated, and subjected to column chromatography (petroleum ether/ethyl acetate = 5/1) to give compound 12d (1.77 g).

MS (ESI, [M+Na]+) m/z: 305.09.

### Step E: Preparation of compound 12e

To a reaction flask were added compound 12d (0.27 g), compound 6a (0.30 g), chloro(2-dicyclohexylphosphino-2',4',6'-triisopropyl-1,1'-biphenyl)[2-(2'-amino-1,1'-biphenyl)]palladium (II) (0.08 g), tripotassium phosphate (0.61 mg), 1,4-dioxane (12.0 mL), and water (2.5 mL) in sequence. The mixture was stirred at 85 °C under nitrogen atmosphere. After the reaction was completed, the reaction solution was concentrated and subjected to column chromatography (dichloromethane/methanol = 20/1) to give compound 12e (0.37 g).

MS (ESI, [M+H]⁺) m/z: 419.32.

### Step F: Preparation of compound 12f

To a reaction flask were added compound 12e (150 mg), compound 12b (110 mg), chloro(2-dicyclohexylphosphino-2',4',6'-triisopropyl-1,1'-biphenyl)[2-(2'-amino-1,1'-biphenyl)]palladium (II) (56 mg), 2-dicyclohexylphosphine-2',4',6'-triisopropylbiphenyl (43 mg), potassium carbonate (149 mg) and 1,4-dioxane (15 mL) in sequence. The mixture was stirred at 100 °C under nitrogen atmosphere. After the reaction was completed, the reaction solution was concentrated and subjected to column chromatography (dichloromethane/methanol = 20/1) to give compound 12f (87 mg).

MS (ESI, [M+H]⁺) m/z: 638.44.

### Step G: Preparation of compound I-12

To a reaction flask were added compound 12f (87 mg), dichloromethane (10 mL), and a solution of hydrochloric acid in 1,4-dioxane (4 M, 2 mL) in sequence. The mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 10/1) to give compound I-12 (15 mg).

HRMS (ESI, [M+H]⁺) *m*/*z:* 538.2923.

¹H NMR (500 MHz, CDCl₃) δ 9.66 (s, 1H), 8.73 (d, J = 8.5 Hz, 1H), 8.22 (d, J = 4.9 Hz, 1H), 7.55 (d, J = 8.5 Hz, 1H), 7.30 (d, J = 8.7 Hz, 1H), 6.98 (d, J = 4.9 Hz, 1H), 6.81 (d, J = 8.6 Hz, 1H), 6.13 (s, 1H), 4.43 (t, J = 3.8 Hz, 1H), 4.37 (s, 2H), 3.83 (s, 3H), 3.74 (s, 2H), 3.43 (d, J = 7.5 Hz, 1H), 3.28 (d, J = 9.9 Hz, 1H), 3.20 (dd, J = 9.9, 3.9 Hz, 1H), 3.07 (q, J = 4.1 Hz, 1H), 2.91 (t, J = 7.0 Hz, 2H), 2.55 (t, J = 7.0 Hz, 2H), 2.40 (q, J = 7.0 Hz, 2H), 2.33 (s, 6H), 2.20 (tt, J = 8.9, 5.2 Hz, 2H).

### Example 13: Preparation of Compound I-13

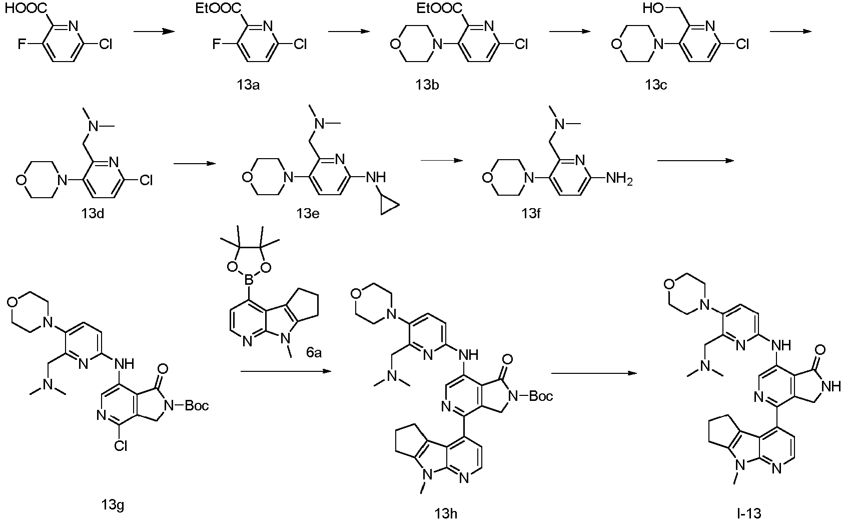

### Step A: Preparation of compound 13a

To a reaction flask were added 2-chloro-5-fluoropyridine-6-carboxylic acid (2 g), ethanol (20 mL), and 2 drops of concentrated sulfuric acid in sequence. After the addition, the mixture was reacted at 80 °C under nitrogen atmosphere. After the reaction was completed, the reaction solution was concentrated and subjected to column chromatography (petroleum ether/ethyl acetate = 9/1) to give compound 13a (2.1 g).

MS (ESI, [M+H]⁺) *m*/*z*: 204.6.

### Step B: Preparation of compound 13b

To a reaction flask were added compound 13a (2.1 g), morpholine (1.72 g), potassium carbonate (4.0 g), and *N,N-*dimethylformamide (20 mL) in sequence. After the addition, the mixture was reacted at 80 °C under nitrogen atmosphere. After the reaction was completed, the reaction solution was concentrated and subjected to column chromatography (petroleum ether/ethyl acetate = 9/1) to give compound 13b (2.2 g).

MS (ESI, [M+H]⁺) *m*/*z*: 271.4.

### Step C: Preparation of compound 13c

To a reaction flask were added compound 13b (2.2 g), sodium borohydride (400 mg), and methanol (40 mL) at 0 °C in sequence. After the addition, the mixture was reacted at 70 °C. After the reaction was completed, a saturated aqueous ammonium chloride solution was added to quench the reaction, and the resulting mixture was extracted with ethyl acetate. The organic phase was dried, filtered, concentrated, and subjected to column chromatography (petroleum ether/ethyl acetate = 4/1) to give compound 13c (1.8 g).

MS (ESI, [M+H]⁺) *m*/*z*: 229.2.

### Step D: Preparation of compound 13d

To a reaction flask were added compound 13c (1.8 g), methanesulfonic anhydride (2.8 g), *N,N-*diisopropylethylamine (3.1 g), and dichloromethane (40 mL) in sequence under an ice-water bath. After the addition, the mixture was reacted under an ice-water bath. After the reaction was completed, a solution of dimethylamine in tetrahydrofuran (2 M, 30 mL) was added to the reaction solution. After the addition, the mixture was reacted at room temperature. After the reaction was completed, the reaction solution was concentrated and subjected to column chromatography (petroleum ether/ethyl acetate = 3/1) to give compound 13d (1.6 g).

MS (ESI, [M+H]⁺) *m*/*z*: 256.2.

### Step E: Preparation of compound 13e

To a reaction flask were added compound 13d (1.6 g), cyclopropylformamide (1.2 g), cesium carbonate (3.8 g), tris(dibenzylideneacetone)dipalladium(0) (0.2 g), 4,5-bis-diphenylphosphino-9,9-dimethylxanthene (0.2 g), and 1,4-dioxane (15 mL) in sequence. After the addition, the mixture was reacted at 100 °C under nitrogen atmosphere. After the reaction was completed, the reaction solution was concentrated and subjected to column chromatography (petroleum ether/ethyl acetate = 4/1) to give compound 13e (1.3 g).

MS (ESI, [M+H]⁺) *m*/*z*: 277.1.

### Step F: Preparation of compound 13f

To a reaction flask were added compound 13e (1.3 g), lithium hydroxide monohydrate (1.9 g), methanol (20 mL), and water (2 mL) in sequence under an ice-water bath. After the addition, the mixture was reacted at 60 °C under nitrogen atmosphere. After the reaction was completed, the reaction solution was adjusted to acidity (about pH 6) with 1 M diluted hydrochloric acid and extracted with ethyl acetate. The organic phase was dried, filtered, concentrated, and subjected to column chromatography (petroleum ether/ethyl acetate = 4/1) to give compound 13f (1.1 g).

MS (ESI, [M+H]⁺) *m*/*z*: 237.4.

### Step G: Preparation of compound 13g

To a reaction flask were added compound 13f (1.1 g), *tert*-butyl 7-bromo-4-chloro-1-oxa-1,3-dihydro-*2H-*pyrrolyl[3,4-c]pyridine-2-carbamate (0.8 g), cesium carbonate (2.1 g), tris(dibenzylideneacetone)dipalladium(0) (0.3 g), 4,5-bis-diphenylphosphino-9,9-dimethylxanthene (0.3 g), and 1,4-dioxane (20 mL) in sequence. After the addition, the mixture was reacted at 100 °C under nitrogen atmosphere. After the reaction was completed, the organic phase was concentrated and subjected to column chromatography (petroleum ether/ethyl acetate = 4/1) to give compound 13g (0.6 g).

MS (ESI, [M+H]⁺) *m*/*z*: 503.1.

### Step H: Preparation of compound 13h

To a reaction flask were added compound 13g (0.4 g), compound 6a (0.8 g), potassium phosphate (0.3 g), chloro(2-dicyclohexylphosphino-2',4',6'-triisopropyl-1,1'-biphenyl)[2-(2'-atnino-1,1'-biphenyl)]palladium (II) (0.08 g), and 1,4-dioxane (20 mL) in sequence. After the addition, the mixture was reacted at 100 °C under nitrogen atmosphere. After the reaction was completed, the organic phase was concentrated and subjected to column chromatography (dichloromethane/methanol = 20/1) to give compound 13h (0.12g).

MS (ESI, [M+H]⁺) *m*/*z*: 639.4.

### Step I: Preparation of compound I-13

To a reaction flask were added compound 13h (0.12 g), a solution of hydrochloric acid in 1,4-dioxane (4 M, 1 mL), and dichloromethane (10 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 9/1) to give compound I-13 (0.042 g).

HRMS (ESI, [M+H]⁺) *m*/*z*: 539.2878.

¹H NMR (500 MHz, CDCl₃:CD₃OD=10:1) δ 10.17 (s, 1H), 8.21 *(d, J=* 5.6 Hz, 1H), 7.52 *(d, J=* 8.6 Hz, 1H), 7.10 (d, *J=* 5.5 Hz, 1H), 6.91 (d, *J* = 8.6 Hz, 1H), 4.54 (s, 2H), 3.88 (s, 5H), 3.82 (s, 4H), 2.95 (d, *J* = 14.0 Hz, 8H), 2.79 (s, 3H), 2.68 (t, J = 6.9 Hz, 3H), 2.45 - 2.38 (m, 3H), 1.24 (d, *J =* 17.1 Hz, 1H).

### Example 14: Preparation of Compound I-14

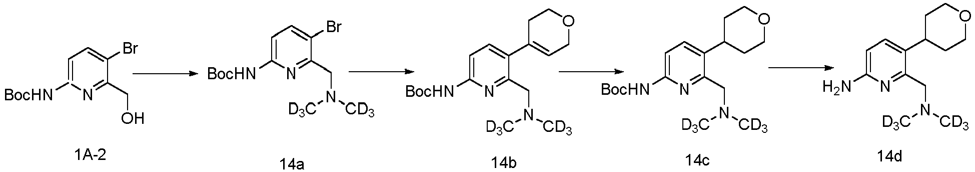

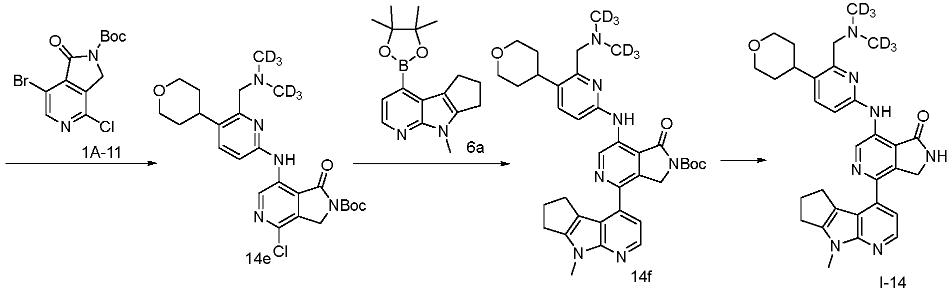

### Step A: Preparation of compound 14a

To a reaction flask were added intermediate 1A-2 (5 g), *N,N-*diisopropylethylamine (4 g), dichloromethane (30 mL), and methanesulfonic anhydride (7.2 g) in sequence. After the addition, the mixture was stirred at -15 °C. After the reaction was completed, dimethyl-d6-amine (10 g) was slowly added to the reaction flask. After the addition, the mixture was stirred at room temperature. After the reaction was completed, the reaction solution was extracted with dichloromethane. The organic phase was washed with a saturated aqueous sodium chloride solution, dried, concentrated, and subjected to column chromatography (dichloromethane/methanol = 5/1) to give compound 14a (3.8 g).

MS (ESI, [M+H]⁺) *m*/*z:* 336.1.

### Step B: Preparation of compound 14b

To a reaction flask were added compound 14a (3.8 g), potassium carbonate (3.6 g), tetrakis(triphenylphosphine)palladium (1.5 g), 3,6-dihydro-2*H*-pyran-4-boronic acid pinacol ester (2.3 g), water (8 mL), and 1,4-dioxane (40 mL) in sequence. After the addition, the mixture was purged with nitrogen and stirred at 100 °C. After the reaction was completed, the reaction solution was filtered, concentrated, and subjected to column chromatography (petroleum ether/ethyl acetate = 5/1) to give compound 14b (2.6 g).

MS (ESI, [M+H]⁺) *m*/*z:* 340.0.

### Step C: Preparation of compound 14c

To a reaction flask were added compound 14b (2.6 g), 10 wt% palladium hydroxide on carbon (2.6 g), and methanol (12 mL) in sequence. After the addition, the mixture was stirred at room temperature under hydrogen atmosphere. After the reaction was completed, the reaction solution was filtered and concentrated to give compound 14c (2.2 g).

MS (ESI, [M+H]⁺) *m*/*z*: 342.0.

### Step D: Preparation of compound 14d

To a reaction flask were added compound 14c (2.2 g) and a solution of hydrochloric acid in 1,4-dioxane (16.4 mL, 4 M) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, and concentrated to give compound 14d (1.4 g).

MS (ESI, [M+H]⁺) *m*/*z*: 242.3.

### Step E: Preparation of compound 14e

To a reaction flask were added compound 14d (1.4 g), 4,5-bis-diphenylphosphino-9,9-dimethylxanthene (246 mg), tris(dibenzylideneacetone)dipalladium(0) (258 mg), cesium carbonate (1.8 g), intermediate 1A-11 (1.3 g), and 1,4-dioxane (100 mL) in sequence. After the addition, the mixture was purged with nitrogen and stirred at 100 °C. After the reaction was completed, the reaction solution was filtered and concentrated to give a crude product, which was subjected to column chromatography (dichloromethane/methanol = 20/1) to give compound 14e (1.3 g).

MS (ESI, [M+H]⁺) *m*/*z*: 508.3.

### Step F: Preparation of compound 14f

To a reaction flask were added compound 6a (101 mg), compound 14e (211 mg), chloro(2-dicyclohexylphosphino-2',4',6'-triisopropyl-1,1'-biphenyl)[2-(2'-amino-1,1'-biphenyl)]palladium (II) (29 mg), potassium phosphate (219 mg), 1,4-dioxane (15 mL), and water (3 mL) in sequence. After the addition, the mixture was stirred at 85 °C under nitrogen atmosphere. After the reaction was completed, the reaction solution was filtered, concentrated, and subjected to column chromatography (dichloromethane/methanol = 18/1) to give compound 14f (101 mg).

MS (ESI, [M+H]⁺) *m*/*z*: 644.4.

### Step G: Preparation of compound I-14

To a reaction flask were added compound 14f (0.101 g), dichloromethane (10 mL), and a solution of hydrochloric acid in 1,4-dioxane (2 mL) in sequence. The mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 15/1) to give compound I-14 (40 mg).

HRMS (ESI, [M+H]⁺) *m*/*z*: 544.3300.

¹H NMR (500 MHz, CDCl₃): δ 10.36 (brs, 1H), 9.81 (s, 1H), 6.60 (s, 2H), 5.20 (s, 1H), 4.81 (s, 1H), 4.25-4.23 (m, 2H), 3.80-3.76 (m, 1H), 3.67 (s, 2H), 3.23-3.16 (m, 1H), 2.93-2.91 (m, 2H), 2.50 (s, 3H), 2.52-2.44 (m, 1H), 2.13-2.09 (m, 1H), 1.94-1.87 (m, 4H), 1.13-1.11 (m, 6H).

### Example 15: Preparation of Compound I-15

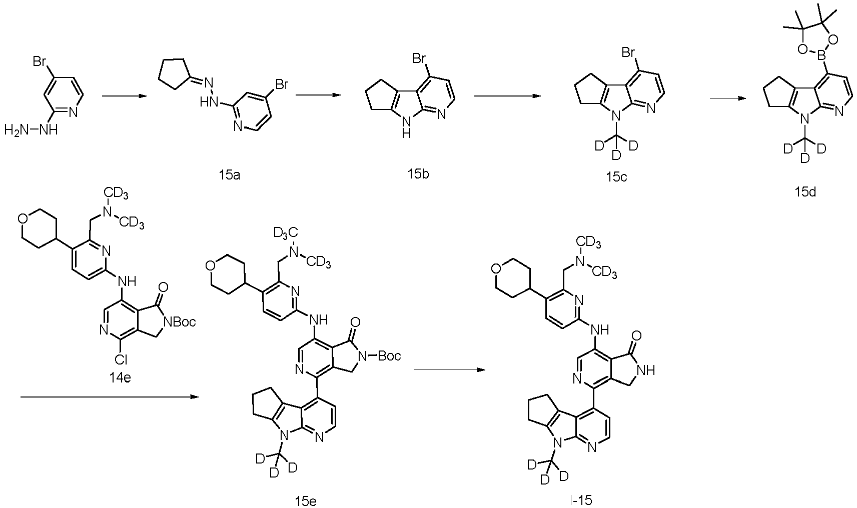

### Step A: Preparation of compound 15a

To a reaction flask were added the compounds 4-bromo-2-hydrazinopyridine (0.92 g), cyclopentanone (0.412 ), and methanol (5 mL) in sequence. The mixture was stirred at room temperature. After the reaction was completed, the reaction solution was concentrated to give compound 15a (1.3 g).

MS (ESI, [M+H]⁺) *m*/*z*: 254.1.

### Step B: Preparation of compound 15b

To a microwave tube were added compound 15a (1.3 g) and diethylene glycol (10 mL) in sequence. After the addition, the mixture was stirred at 245 °C under microwave irradiation. After the reaction was completed, the reaction solution was poured into water, and the resulting mixture was filtered. The residue was subjected to column chromatography (dichloromethane/methanol = 20/1) to give compound 15b (0.17 g).

MS (ESI, [M+H]⁺) *m*/*z*: 237.06.

### Step C: Preparation of compound 15c

To a reaction flask containing compound 15b (0.17 g) and DMF (5 mL) was added 60 wt% sodium hydride (0.215 g) at 0 °C. After the addition, the mixture was stirred for 0.5 h. Deuterated iodomethane (1.039 g) was then added dropwise to the reaction flask. After the addition, the mixture was stirred at room temperature overnight. After the reaction was completed, the reaction was quenched, and the reaction solution was extracted with ethyl acetate. The organic phase was washed with saturated brine, dried, concentrated, and subjected to column chromatography (petroleum ether/ethyl acetate = 4/1) to give compound 15c (0.12 g).

MS (ESI, [M+H]⁺) *m*/*z*: 254.1.

### Step D: Preparation of compound 15d

To a reaction flask were added [1,1'-bis(diphenylphosphino)ferrocene]palladium(II) dichloride (0.039 g), compound 15c (0.12 g), bis(pinacolato)diboron (0.24 g), potassium acetate (0.093g), and 1,4-dioxane (5 mL) in sequence. After the addition, the mixture was purged with nitrogen and stirred at 100 °C. After the reaction was completed, the reaction solution was filtered, concentrated, and subjected to column chromatography (dichloromethane/methanol = 20/1) to give compound 15d (0.107 g).

MS (ESI, [M+H]⁺) *m*/*z*: 302.39.

### Step E: Preparation of compound 15e

To a reaction flask were added compound 14e (0.15 g), compound 15d (0.107 g), chloro(2-dicyclohexylphosphino-2',4',6'-triisopropyl-1,1'-biphenyl)[2-(2'-amino-1,1'-biphenyl)]palladium (II) (0.023 g), tripotassium phosphate (0.188 g), 1,4-dioxane (5 mL), and water (1 mL) in sequence. After the addition, the mixture was purged with nitrogen and stirred at 105 °C. After the reaction was completed, the reaction solution was filtered and concentrated to give a crude product, which was subjected to column chromatography (dichloromethane/methanol = 20/1) to give compound 15e (0.103 g).

MS (ESI, [M+H]⁺) *m*/*z*: 647.51.

### Step F: Preparation of compound I-15

To a reaction flask were added compound 15e (0.103 g), dichloromethane (1 mL), and a solution of hydrochloric acid in 1,4-dioxane (0.5 mL, 4 M) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 10/1) to give compound 1-15 (0.035 g).

HRMS (ESI, [M+H]⁺) *m*/*z*: 547.3490.

¹H NMR (500 MHz, CDCl₃) δ 10.34 (s, 1H), 9.34 (s, 1H), 8.25 (d, *J =* 4.9 Hz, 1H), 7.54 (d, *J =* 8.3 Hz, 1H), 7.08 (d, *J =* 4.9 Hz, 1H), 6.84 (d, *J=* 8.3 Hz, 1H), 6.51 (s, 1H), 4.57 (s, 2H), 4.09 (dd, *J* = 11.3, 2.9 Hz, 2H), 3.56 (t, *J =* 10.7 Hz, 2H), 3.22 (d, *J =* 5.9 Hz, 1H), 3.04 (d, *J =* 7.0 Hz, 2H), 2.93 (t, *J =* 7.0 Hz, 2H), 2.72 (t, *J =* 6.9 Hz, 2H), 2.42 (dd, *J* = 14.0, 7.1 Hz, 2H), 1.78 (dd, *J* = 12.5, 3.2 Hz, 2H), 1.37 (s, 2H).

### Example 16: Preparation of Compound I-16

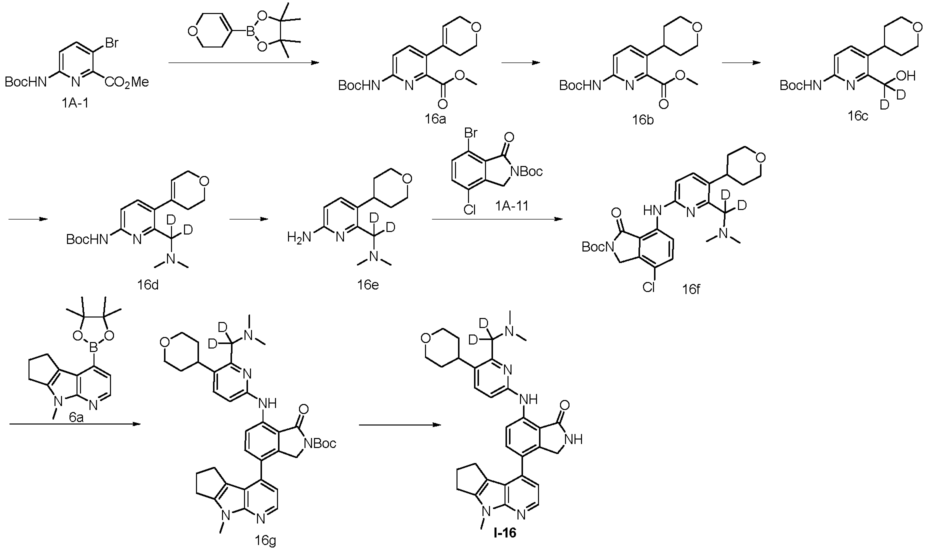

### Step A: Preparation of compound 16a

To a reaction flask were added intermediate 1A-1 (8.00 g), 3,6-dihydro-2*H*-pyran-4-boronic acid pinacol ester (6.60 g), potassium carbonate (10.02 g), tetrakis(triphenylphosphine)palladium (5.58 g), 1,4-dioxane (100 mL), and water (15 mL) in sequence under nitrogen atmosphere. After the addition, the mixture was stirred at 100 °C. After the reaction was completed, the reaction solution was concentrated and subjected to column chromatography (petroleum ether/ethyl acetate = 5/1) to give compound 16a (7.06 g).

MS (ESI, [M+H]⁺) *m*/*z*: 335.11.

### Step B: Preparation of compound 16b

To a reaction flask were added compound 16a (7.06 g), methanol (50 mL), and 10 wt% palladium hydroxide on carbon (7.41 g) in sequence. After the addition, the mixture was stirred at room temperature under hydrogen atmosphere. After the reaction was completed, the reaction solution was filtered and concentrated to give compound 16b (5.19 g).

MS (ESI, [M+H]⁺) *m*/*z*: 337.12.

### Step C: Preparation of compound 16c

To a reaction flask were added compound 16b (5.14 g), tetrahydrofuran (100 mL), and deuterated lithium aluminum hydride (1.60 g) in sequence at 0 °C. After the addition, the mixture was stirred at room temperature. After the reaction was completed, water was added to quench the reaction, and the resulting mixture was filtered and extracted with ethyl acetate. The organic phase was dried, filtered, and concentrated to give compound 16c (2.76 g).

MS (ESI, [M+H]⁺) *m*/*z:* 311.17.

### Step D: Preparation of compound 16d

To a reaction flask were added compound 16c (2.76 g), dichloromethane (60 mL), triethylamine (1.80 g), and methanesulfonic anhydride (3.10 g) in sequence at -10 °C. After the addition, the mixture was stirred at 0 °C. After the reaction was completed, the reaction solution was added dropwise to a 40% aqueous dimethylamine solution (20.04 g) at -10 °C. After the addition, the mixture was stirred at room temperature. After the reaction was completed, the reaction solution was concentrated. The crude product was subjected to column chromatography (dichloromethane:methanol = 20: 1) to give compound 16d (1.17 g).

MS (ESI, [M+H]⁺) *m*/*z*: 336.26.

### Step E: Preparation of compound 16e

To a reaction flask were added compound 16d (1.17 g), dichloromethane (10 mL), and a solution of hydrochloric acid in 1,4-dioxane (4 M, 20 mL) in sequence. After the addition, the mixture was stirred at room temperature. After the reaction was completed, the reaction solution was concentrated, adjusted to alkalinity (about pH 10) with a saturated sodium bicarbonate solution, and extracted with dichloromethane. The organic phase was dried, filtered, and dried under reduced pressure to remove the solvent to give compound 16e (1.13 g).

MS (ESI, [M+H]⁺) *m*/*z*: 238.25.

### Step F: Preparation of compound 16f

To a reaction flask were added compound 16e (1.00 g), intermediate 1A-11 (0.86 g), tris(dibenzylideneacetone)dipalladium(0) (0.26 g), 4,5-bis-diphenylphosphino-9,9-dimethylxanthene (0.20 g), cesium carbonate (2.82 g), and 1,4-dioxane (30 mL) under nitrogen atmosphere. The mixture was stirred at 110 °C. After the reaction was completed, the reaction solution was filtered, concentrated, and subjected to column chromatography (dichloromethane/methanol = 20/1) to give compound 16f (0.85 g).

MS (ESI, [M+H]⁺) *m*/*z*: 503.33.

### Step G: Preparation of compound 16g

To a microwave tube were added compound 16f (150 mg), compound 6a (107 mg), chloro(2-dicyclohexylphosphino-2',4',6'-triisopropyl-1,1'-biphenyl)[2-(2'-amino-1,1'-biphenyl)]palladium (II) (24 mg), tripotassium phosphate (190 mg), 1,4-dioxane (3.0 mL), and water (0.5 mL) in sequence. The mixture was stirred at 85 °C under microwave irradiation under nitrogen atmosphere. After the reaction was completed, the reaction solution was concentrated and subjected to column chromatography (dichloromethane/methanol = 20/1) to give compound 16g (167 mg).

MS (ESI, [M+H]+) m/z: 639.52.

### Step H: Preparation of compound 1-16

To a reaction flask were added compound 16g (167 mg), dichloromethane (10.00 mL), and a solution of hydrochloric acid in 1,4-dioxane (1.00 mL, 4 M) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, and concentrated. The resulted crude product was subjected to column chromatography (dichloromethane/methanol = 10/1) to give compound I-16 (35 mg).

HRMS (ESI, [M+H]⁺) *m*/*z*: 539.3097.

¹H NMR (500 MHz, CDC13) δ 9.77 (s, 1H), 8.86 (d, J = 8.6 Hz, 1H), 8.23 (d, J = 4.9 Hz, 1H), 7.57 (d, J = 8.6 Hz, 1H), 7.51 (d, J = 8.4 Hz, 1H), 6.98 (d, J = 5.0 Hz, 1H), 6.83 (d, J = 8.4 Hz, 1H), 6.08 (s, 1H), 4.37 (s, 2H), 4.14 - 4.06 (m, 2H), 3.83 (s, 3H), 3.58 - 3.53 (m, 2H), 3.22 - 3.18 (m, 1H), 2.96 - 2.87 (m, 2H), 2.54 (dd, J = 8.1, 5.8 Hz, 2H), 2.40 (q, J = 7.1 Hz, 2H), 2.33 (s, 6H), 1.85 - 1.70 (m, 4H).

### Example 17: Preparation of Compound I-17

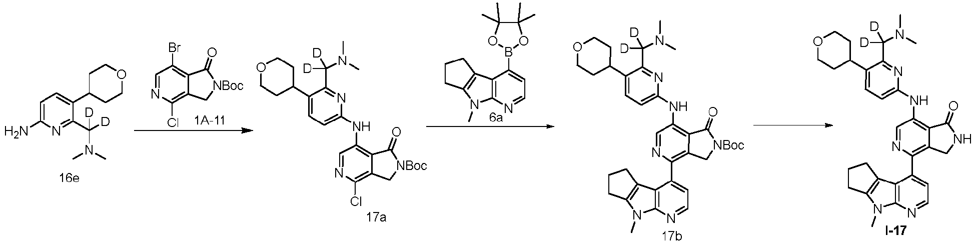

### Step A: Preparation of compound 17a

To a reaction flask were added compound 16e (879 mg), intermediate 1A-11 (500 mg), tris(dibenzylideneacetone) dipalladium(0) (193 mg), 4,5-bis-diphenylphosphino-9,9-dimethylxanthene (146 mg), potassium carbonate (873 mg), and toluene (20 mL) in sequence under nitrogen atmosphere. The mixture was stirred at 100°C. After the reaction was completed, the reaction solution was filtered, concentrated, and subjected to column chromatography (dichloromethane/methanol = 20/1) to give compound 17a (237 mg).

MS (ESI, [M+H]⁺) *m*/*z:* 504.27.

### Step B: Preparation of compound 17b

To a microwave tube were added compound 17a (150 mg), compound 6a (106 mg), chloro(2-dicyclohexylphosphino-2',4',6'-triisopropyl-1,1'-biphenyl)[2-(2'-amino-1,1'-biphenyl)]palladium (II) (23 mg), tripotassium phosphate (190 mg), 1,4-dioxane (8.0 mL), and water (1.5 mL) in sequence. The mixture was stirred at 85 °C under microwave irradiation under nitrogen atmosphere. After the reaction was completed, the reaction solution was concentrated and subjected to column chromatography (dichloromethane/methanol = 20/1) to give compound 17b (103 mg).

MS (ESI, [M+H]⁺) m/z: 640.40.

### Step C: Preparation of compound I-17

To a reaction flask were added compound 17b (103 mg), dichloromethane (10.00 mL), and a solution of hydrochloric acid in 1,4-dioxane (1.00 mL, 4 M) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 10/1) to give compound 1-17 (25 mg).

HRMS (ESI, [M+H]⁺) *m*/*z*: 540.3049.

¹H NMR (500 MHz, CDCl₃) δ 10.35 (s, 1H), 9.34 (s, 1H), 8.25 (d, J = 4.9 Hz, 1H), 7.54 (d, J = 8.4 Hz, 1H), 7.08 (d, J = 4.9 Hz, 1H), 6.84 (d, J = 8.4 Hz, 1H), 6.52 (s, 1H), 4.57 (s, 2H), 4.15 - 4.05 (m, 2H), 3.83 (s, 3H), 3.60 - 3.52 (m, 2H), 3.25 - 3.19 (m, 1H), 2.93 (t, J = 7.1 Hz, 2H), 2.72 (t, J = 7.0 Hz, 2H), 2.42 (p, J = 7.3 Hz, 2H), 2.33 (s, 6H), 1.84 - 1.71 (m, 4H).

### Example 18: Preparation of Compound I-18

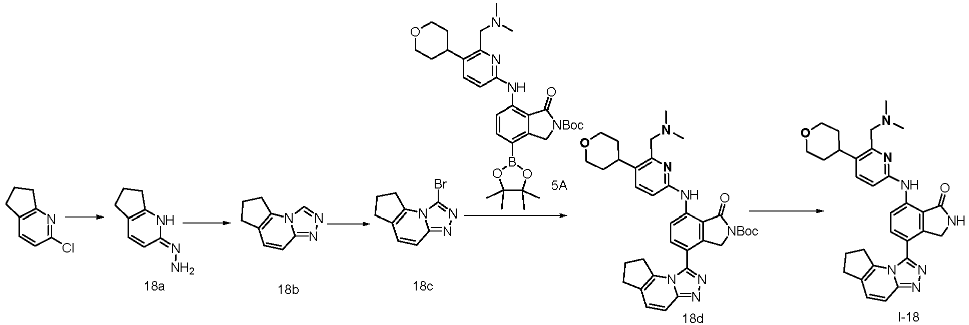

### Step A: Preparation of compound 18a

To a reaction flask were added 2-chloro-6,7-dihydro-5*H*-2,3-cyclopentenopyridine (5.5 g) and 85% hydrazine hydrate (10 mL) in sequence. After the addition, the mixture was stirred at 120 °C. After the reaction was completed, the reaction solution was extracted with dichloromethane. The organic phase was dried, filtered, and concentrated to give compound 18a (5.2 g).

MS (ESI, [M+H]⁺) m/z: 150.2.

### Step B: Preparation of compound 18b

To a reaction flask were added compound 18a (5.2 g), *N,N*-dimethylformamide (25 mL), and imidazole hydrochloride (0.4 g) in sequence. After the addition, the mixture was stirred at 170 °C. After the reaction was completed, the reaction solution was concentrated and subjected to column chromatography (dichloromethane/methanol = 10/1) to give compound 18b (4.9 g).

MS (ESI, [M+H]⁺) m/z: 160.0.

### Step C: Preparation of compound 18c

To a reaction flask were added compound 18b (4.9 g), NBS (6.1 g), and carbon tetrachloride (50 mL) in sequence. After the addition, the mixture was stirred at 60 °C. After the reaction was completed, the reaction was quenched, and the reaction solution was extracted with dichloromethane. The organic phase was dried, filtered, concentrated, and subjected to column chromatography (dichloromethane/methanol = 10/1) to give compound 18c (5.2 g).

MS (ESI, [M+H]⁺) m/z: 238.4.

### Step D: Preparation of compound 18d

To a reaction flask were added compound 18c (0.18 g), intermediate 5A (0.17 g), 2-dicyclohexylphosphine-2',4',6'-triisopropylbiphenyl (0.02 g), chloro(2-dicyclohexylphosphino-2',4',6'-triisopropyl-1,1'-biphenyl)[2-(2'-amino-1,1'-biphenyl)]palladium (II) (0.03 g), potassium phosphate (0.09 g), water (2.00 mL), and 1,4-dioxane (15 mL) in sequence. After the addition, the mixture was stirred at 85 °C under nitrogen atmosphere. After the reaction was completed, the reaction solution was concentrated and subjected to column chromatography (dichloromethane/methanol = 10/1) to give compound 18d (0.12g).

MS (ESI, [M+H]⁺) *m*/*z*: 624.4.

### Step E: Preparation of compound I-18

To a reaction flask were added compound 18d (0.12 g), dichloromethane (10 mL), and a solution of hydrochloric acid in 1,4-dioxane (2 mL, 4 M) in sequence. The mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 20/1) to give compound 1-18 (40 mg).

HRMS (ESI, [M+H]⁺) *m*/*z*: 524.2768.

¹H NMR (500 MHz, DMSO-*d*₆) δ 10.02 (s, 1H), 8.75 (s, 1H), 8.71 (d, *J =* 8.5 Hz, 1H), 7.67 (dt, *J =* 8.5, 3.1 Hz, 3H), 7.39 (d, *J =* 9.3 Hz, 1H), 6.95 (d, *J =* 8.4 Hz, 1H), 4.30 (s, 2H), 3.97 (dd, *J =* 10.6, 4.1 Hz, 2H), 3.59 (s, 2H), 3.44 (td, *J =* 11.4, 2.6 Hz, 2H), 3.25 - 3.14 (m, 1H), 2.87 (t, *J =* 7.4 Hz, 2H), 2.69 (t, *J =* 7.6 Hz, 2H), 2.23 (s, 6H), 2.11 - 1.99 (m, 2H), 1.66 (dt, *J=* 15.5, 11.6 Hz, 4H).

### Example 19: Preparation of Compound I-19

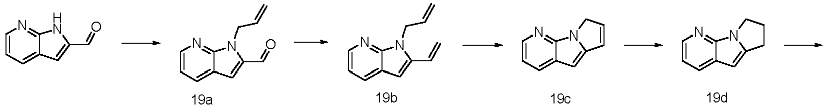

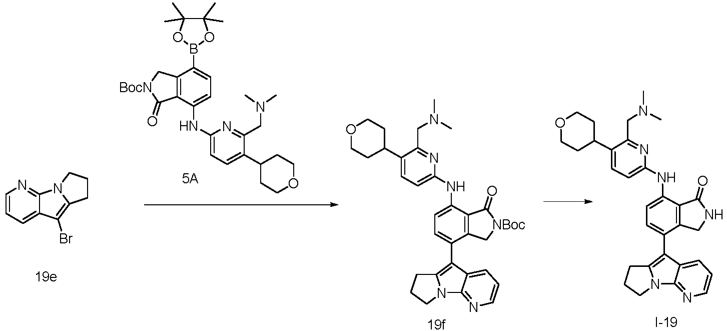

### Step A: Preparation of compound 19a

To a reaction flask were added the compounds 1*H*-pyrrolo[2,3-*b*]pyridine-2-carbaldehyde (4 g), cesium carbonate (13.38 g), *N,N*-dimethylformamide (50.0 mL), and 3-bromopropene (4.30 g) in sequence. After the addition, the mixture was stirred at room temperature. After the reaction was completed, 100 mL of water was added, and the resulting mixture was extracted with ethyl acetate. The organic phase was dried, filtered, concentrated, and subjected to column chromatography (petroleum ether/ethyl acetate = 17/3) to give compound 19a (3.4 g).

MS (ESI, [M+H]⁺) *m*/*z:* 187.15.

### Step B: Preparation of compound 19b

To a reaction flask containing methyltriphenylphosphine bromide (7.3 g) and tetrahydrofuran (100 mL) was slowly added dropwise potassium bis(trimethylsilyl)amide (5.0 g) under nitrogen atmosphere. After the addition, the mixture was stirred at room temperature for 30 min. After the reaction was completed, compound 19a (3.4 g) was added. The mixture was stirred at room temperature. After the reaction was completed, 100 mL of water was added, and the resulting mixture was extracted with ethyl acetate. The organic phase was dried, filtered, concentrated, and subjected to column chromatography (petroleum ether/ethyl acetate = 10/1) to give compound 19b (2.85 g).

MS (ESI, [M+H]⁺) *m*/*z*: 185.14.

### Step C: Preparation of compound 19c

To a reaction flask were added compound 19b (2.35 g), HOVEYDA-GRUBBS catalyst (1.42 g), and dichloromethane (40 mL) in sequence. After the addition, the mixture was stirred at 40°C under nitrogen atmosphere. After the reaction was completed, the reaction solution was filtered, concentrated, and subjected to column chromatography (dichloromethane/methanol = 99/1) to give compound 19c (0.56 g).

MS (ESI, [M+H]⁺) *m*/*z*: 157.13.

### Step D: Preparation of compound 19d

To a reaction flask were added compound 19c (0.56 g), 10 wt% palladium on carbon (0.38 g), ammonium formate (6.6 g), and ethanol (20 mL) in sequence. After the addition, the mixture was stirred at 70°C under nitrogen atmosphere. After the reaction was completed, the reaction solution was filtered, concentrated, and subjected to column chromatography (dichloromethane/methanol = 99/1) to give compound 19d (0.392 g).

MS (ESI, [M+H]⁺) *m*/*z:* 159.1.

### Step E: Preparation of compound 19e

To a reaction flask were added compound 19d (0.392 g), *N*-bromosuccinimide (0.485 g), and dichloromethane (20 mL) in sequence under an ice bath. After the addition, the mixture was stirred under nitrogen atmosphere. After the reaction was completed, 50 mL of water was added, and the resulting mixture was extracted with dichloromethane. The organic phase was dried, filtered, and concentrated to give compound 19e (0.307 g).

MS (ESI, [M+H]⁺) *m*/*z*: 237.1.

### Step F: Preparation of compound 19f

To a reaction flask were added compound 19e (0.17 g), 2-dicyclohexylphosphine-2',4',6'-triisopropylbiphenyl (16 mg), chloro(2-dicyclohexylphosphino-2',4',6'-trilsopropyl-1,1'-biphenyl)[2-(2'-amino-1,1'-biphenyl)]palladium (II) (27 mg), tripotassium phosphate (0.143 g), intermediate 5A (0.25 g), 1,4-dioxane (20 mL), and water (4 mL) in sequence. After the addition, the mixture was purged with nitrogen and stirred at 85 °C under microwave irradiation. After the reaction was completed, the reaction solution was filtered, concentrated, and subjected to column chromatography (dichloromethane/methanol = 3/7) to give compound 19f (0.185 g).

MS (ESI, [M+H]⁺) *m*/*z*: 623.44.

### Step F: Preparation of compound 1-19

To a reaction flask were added compound 19f (0.185 g), dichloromethane (10 mL), and a solution of hydrochloric acid in 1,4-dioxane (1 mL, 4 M) in sequence. The mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 3/2) to give compound I-19 (50 mg).

HRMS (ESI, [M+H]⁺) *m*/*z*: 523.2818.

¹H NMR (500 MHz, DMSO-*d*₆) δ 9.72 (s, 1H), 8.82 (d, J = 8.5 Hz, 1H), 8.27 (dd, J = 4.8, 1.5 Hz, 1H), 7.71 (dd, J = 7.9, 1.6 Hz, 1H), 7.51 (dd, J = 16.0, 8.4 Hz, 2H), 7.04 (dd, J = 7.9, 4.8 Hz, 1H), 6.83 (d, J = 8.4 Hz, 1H), 6.33 (s, 1H), 4.36 - 4.26 (m, 4H), 4.09 (dd, J = 11.1, 4.1 Hz, 2H), 3.64 (s, 2H), 3.56 (td, J = 11.6, 2.3 Hz, 2H), 3.20 (tt, J = 11.8, 4.0 Hz, 1H), 3.04 (t, J = 7.3 Hz, 2H), 2.69 (p, J = 7.2 Hz, 2H), 2.34 (s, 6H), 1.78 - 1.71 (m, 4H).

### Example 20: Preparation of Compound 1-20

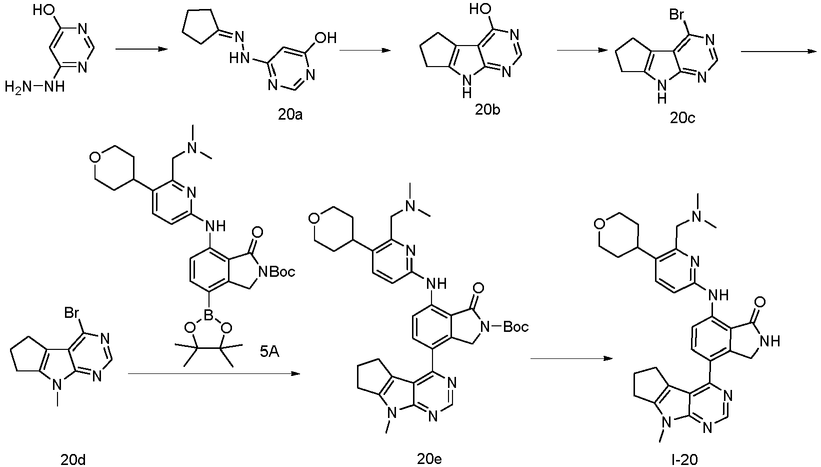

### Step A: Preparation of compound 20a

To a reaction flask were added the compounds 4-hydrazino-6-hydroxypyrimidine (5.0 g), cyclopentanone (3.33 g), and methanol (25 mL) in sequence. The mixture was stirred at room temperature. After the reaction was completed, the reaction solution was concentrated to give compound 20a (8.3 g).

MS (ESI, [M+H]⁺) *m*/*z*: 193.16.

### Step B: Preparation of compound 20b

To a microwave tube were added compound 20a (8.3 g) and diethylene glycol (50 mL) in sequence. After the addition, the mixture was stirred at 245 °C under microwave irradiation. After the reaction was completed, the reaction solution was poured into water, and the resulting mixture was filtered. The residue was subjected to column chromatography (dichloromethane/methanol = 20/1) to give compound 20b (2.3 g).

MS (ESI, [M+H]⁺) *m*/*z*: 176.10.

### Step C: Preparation of compound 20c

To a reaction flask were added compound 20b (1.0 g), DMF (8 mL), and phosphorus oxybromide (3.48 mL) in sequence. After the addition, the mixture was stirred at 80 °C. After the reaction was completed, the reaction solution was adjusted to alkalinity (about pH 10) with a saturated sodium bicarbonate solution and extracted with ethyl acetate. The organic phase was dried, filtered, concentrated, and subjected to column chromatography (dichloromethane/methanol = 25/1) to give compound 20c (0.54 g).

MS (ESI, [M+H]⁺) *m*/*z*: 238.0.

### Step D: Preparation of compound 20d

To a reaction flask containing compound 20c (0.50 g) and DMF (5 mL) was slowly added 60 wt% sodium hydride (0.126g) at 0 °C. After the addition, the mixture was stirred for 0.5 h. Iodomethane (0.328 g) was then added dropwise to the reaction solution. After the addition, the mixture was stirred at room temperature. After the reaction was completed, the reaction was quenched. The reaction solution was extracted with ethyl acetate. The organic phase was dried, filtered, concentrated, and subjected to column chromatography (petroleum ether/ethyl acetate = 4/1) to give compound 20d (0.203 g).

MS (ESI, [M+H]⁺) *m*/*z*: 252.02.

### Step E: Preparation of compound 20e

To a microwave tube were added 2-dicyclohexylphosphine-2',4',6'-triisopropylbiphenyl (0.020 g), chloro(2-dicyclohexylphosphino-2',4',6'-triisopropyl-1,1 '-biphenyl)[2-(2'-amino-1,1 '-biphenyl)]palladium (II) (0.033 g), tripotassium phosphate (0.179 g), compound 20d (0.117 g), intermediate 5A (0.250 g), 1,4-dioxane (20 mL), and water (4 mL) in sequence. After the addition, the mixture was purged with nitrogen and stirred at 85 °C under microwave irradiation. After the reaction was completed, the reaction solution was filtered and concentrated to give a crude product, which was subjected to column chromatography (dichloromethane/methanol = 20/1) to give compound 20e (0.106 g).

MS (ESI, [M+H]⁺) *m*/*z*: 638.47.

### Step F: Preparation of compound 1-20

To a reaction flask were added compound 20e (0.106 g), dichloromethane (2 mL), and a solution of hydrochloric acid in 1,4-dioxane (0.5 mL, 4 M) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 10/1) to give compound I-20 (40 mg).

HRMS (ESI, [M+H]⁺) *m*/*z*: 538.2919.

### Example 21: Preparation of Compound I-21

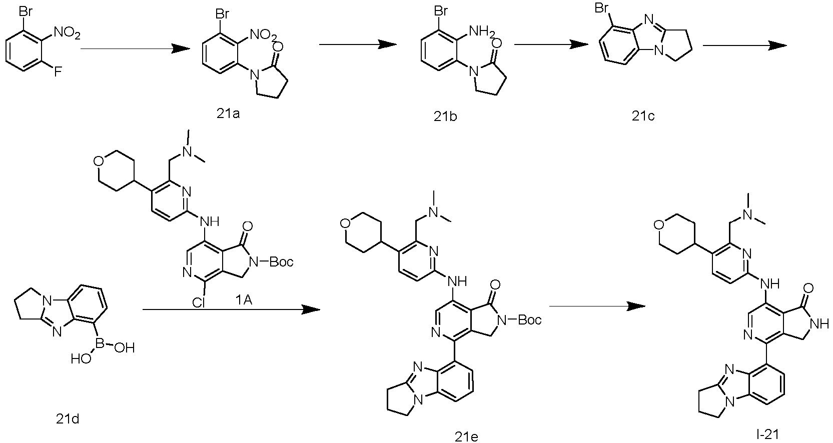

### Step A: Preparation of compound 21a

To a reaction flask were added pyrrolidine-2-one (0.85 g), *N,N*-dimethylformamide (40 mL), and 60 wt% sodium hydride (0.55 g) in sequence at 0 °C under nitrogen atmosphere. After the addition, the mixture was stirred at 0 °C for 1 h. 1-Bromo-3-fluoro-2-nitrobenzene (2.0 g) was added to the reaction flask. After the addition, the mixture was stirred at 0 °C. After the reaction was completed, water was added to quench the reaction, and the resulting mixture was extracted with ethyl acetate. The organic phase was dried, filtered, concentrated, and subjected to column chromatography (petroleum ether/ethyl acetate = 3/1) to give compound 21a (1.0 g).

MS (ESI, [M+H]⁺) m/z: 285.0.

### Step B: Preparation of compound 21b

To a reaction flask were added compound 21a (1.0 g), reduced iron powder (0.9 g), ammonium chloride (0.9 g), ethanol (12 mL), and water (3 mL) in sequence. After the addition, the mixture was stirred at 80 °C. After the reaction was completed, the reaction solution was filtered and concentrated to give compound 21b (0.8 g).

MS (ESI, [M+H]⁺) m/z: 255.0.

### Step C: Preparation of compound 21c

To a reaction flask were added compound 21b (0.8 g) and acetic acid (20 mL) in sequence. After the addition, the mixture was stirred at 110 °C. After the reaction was completed, the reaction solution was concentrated, adjusted to pH 8-9 with a saturated aqueous sodium bicarbonate solution, and extracted with dichloromethane. The organic phase was dried, filtered, and concentrated to give compound 21c (0.7 g).

MS (ESI, [M+H]⁺) m/z: 237.1.

### Step D: Preparation of compound 21d

To a microwave tube were added compound 21c (0.4 g), 5,5,5',5'-tetramethyl-2,2'-bis(1,3,2-dioxetane) (0.57 g), [1,1'-bis(diphenylphosphino)ferrocene]palladium(II) dichloride (0.24 g), potassium acetate (0.33 g), and 1,4-dioxane (15 mL) in sequence. After the addition, the mixture was stirred at 125 °C under microwave irradiation. After the reaction was completed, the reaction solution was filtered and concentrated to give compound 21d (0.3 g). MS (ESI, [M+H]⁺) m/z: 203.2.

### Step E: Preparation of compound 21e

To a reaction flask were added intermediate 1A (0.22 g), compound 21d (0.3 g), chloro(2-dicyclohexylphosphino-2',4',6'-triisopropyl-1,1'-biphenyl)[2-(2'-amino-1,1'-biphenyl)]palladium (II) (0.3 g), tripotassium phosphate (0.28 g), 1,4-dioxane (10 mL), and water (2 mL) in sequence. After the addition, the mixture was stirred at 100 °C. After the reaction was completed, the reaction solution was filtered, concentrated, and subjected to column chromatography (dichloromethane/methanol = 40/1) to give compound 21e (120 mg).

MS (ESI, [M+H]⁺) m/z: 624.4.

### Step F: Preparation of compound I-21

To a reaction flask were added compound 21e (120 mg), dichloromethane (10 mL), and a solution of hydrochloric acid in 1,4-dioxane (2 mL, 4 M) in sequence. The mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 20/1) to give compound I-21 (8 mg).

HRMS (ESI, [M+H]⁺) *m*/*z*: 524.2767.

¹H NMR (500 MHz, CDCl₃) δ 10.29 (d, *J* = 16.1 Hz, 1H), 9.34 (d, *J* = 14.7 Hz, 1H), 7.52 (s, 2H), 7.34 (s, 1H), 7.23 (s, 1H), 6.80 (s, 1H), 6.36 (d, *J =* 14.1 Hz, 1H), 4.73 (d, *J =* 16.2 Hz, 2H), 4.12 (d, *J =* 46.4 Hz, 4H), 3.59 (d, *J* = 42.9 Hz, 4H), 3.22 (s, 1H), 3.07 (s, 2H), 2.74 (s, 2H), 2.29 (s, 6H), 1.25 (s, 3H), 0.84 (s, 1H).

### Example 22: Preparation of Compound I-22

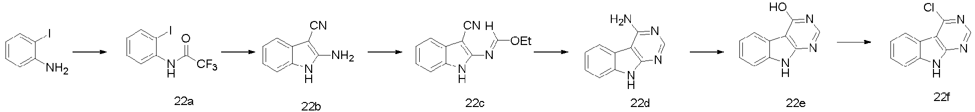

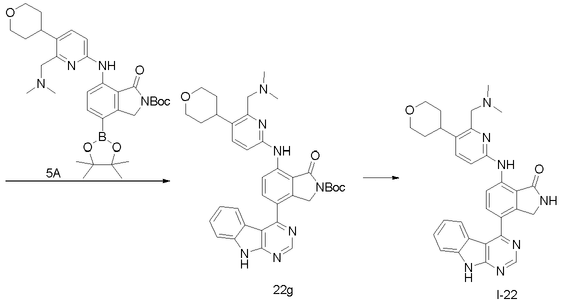

### Step A: Preparation of compound 22a

To a reaction flask were added compound 2-iodoaniline (25.0 g), dichloromethane (500.0 mL), triethylamine (17.3 g), and trifluoroacetic anhydride (28.8 g) in sequence. After the addition, the mixture was stirred at room temperature. After the reaction was completed, the reaction solution was extracted with dichloromethane. The organic phase was dried, filtered, concentrated, and subjected to column chromatography (dichloromethane/methanol = 30/1) to give compound 22a (34.2 g).

MS (ESI, [M+H]⁺) *m*/*z*: 316.2.

### Step B: Preparation of compound 22b

To a reaction flask were added compound 22a (34.2 g), dimethyl sulfoxide (500.0 mL), potassium carbonate (29.8 mL), L-proline (2.4 mL), malononitrile (8.6 mL), and copper(I) iodide (2.0 mL) in sequence. After the addition, the mixture was purged with nitrogen and stirred at 60 °C. After the reaction was completed, the reaction solution was filtered, concentrated, and subjected to column chromatography (petroleum ether/ethyl acetate = 5/1) to give compound 22b (15.9 g).

MS (ESI, [M+H]⁺) *m*/*z*: 158.2.

### Step C: Preparation of compound 22c

To a reaction flask were added compound 22b (15.9 g), triethyl orthoformate (105.0 g), and acetonitrile (150.0 mL) in sequence. After the addition, the mixture was stirred at 95 °C. After the reaction was completed, the reaction solution was filtered, concentrated, and subjected to column chromatography (petroleum ether/ethyl acetate = 5/1) to give compound 22c (14.4 g).

MS (ESI, [M+H]⁺) *m*/*z*: 214.2.

### Step D: Preparation of compound 22d

To a reaction flask were added compound 22c (14.4 g), a solution of ammonia in methanol (96.0 mL, 7 M), and methanol (10 mL) in sequence. After the addition, the mixture was stirred at 105 °C. After the reaction was completed, the reaction solution was extracted with dichloromethane. The organic phase was dried, filtered, concentrated, and subjected to column chromatography (petroleum ether/ethyl acetate = 5/1) to give compound 22d (14.4 g).

MS (ESI, [M+H]⁺) *m*/*z*: 185.2.

### Step E: Preparation of compound 22e

To a reaction flask were added compound 22d (14.4 g), water (500.0 mL), acetic acid (150.0 mL), and sodium nitrite (26.9 g) in sequence. After the addition, the mixture was stirred at 60 °C. After the reaction was completed, the reaction solution was extracted with dichloromethane. The organic phase was dried, filtered, concentrated, and subjected to column chromatography (dichloromethane/methanol = 30/1) to give compound 22e (12.0 g).

MS (ESI, [M+H]⁺) *m*/*z*: 186.3.

### Step F: Preparation of compound 22f

To a reaction flask were added compound 22e (12.0 g) and phosphorus oxychloride (150.0 mL) in sequence. After the addition, the mixture was stirred at 120 °C. After the reaction was completed, the reaction solution was concentrated under reduced pressure and extracted with dichloromethane. The organic phase was dried, filtered, concentrated, and subjected to column chromatography (petroleum ether/ethyl acetate = 5/1) to give compound 22f (6.8 g).

MS (ESI, [M+H]⁺) *m*/*z*: 204.2.

### Step G: Preparation of compound 22g

To a reaction flask were added compound 22f (0.16 g), 2-dicyclohexylphosphine-2',4',6'-triisopropylbiphenyl (0.02 g), potassium phosphate (0.3 g), chloro(2-dicyclohexylphosphino-2',4',6'-trilsopropyl-1,1'-biphenyl)[2-(2'-amino-1,1'-biphenyl)]palladium (II) (0.07 g), intermediate 5A (0.4 g), water (3.0 mL), and 1,4-dioxane (12.0 mL) in sequence. After the addition, the mixture was stirred at 85 °C. After the reaction was completed, the reaction solution was filtered, concentrated, and subjected to column chromatography (petroleum ether/ethyl acetate = 5/1) to give compound 22g (0.08 g).

MS (ESI, [M+H]⁺) *m*/*z*: 634.4.

### Step H: Preparation of compound 1-22

To a reaction flask were added compound 22g (0.08 g), dichloromethane (10 mL), and a solution of hydrochloric acid in 1,4-dioxane (2 mL, 4 M) in sequence. The mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 20/1) to give compound I-22 (0.013 g).

HRMS (ESI, [M+H]⁺) m/z: 534.2617.

¹H NMR (500 MHz, CDCl₃) δ 9.00 - 8.85 (m, 2H), 8.04 (d, J = 8.6 Hz, 1H), 7.85 (d, J = 8.0 Hz, 1H), 7.61 - 7.56 (m, 2H), 7.53 (t, J = 7.7 Hz, 1H), 7.35 (s, 2H), 7.22 (t, J = 7.6 Hz, 1H), 6.91 (d, J = 8.4 Hz, 1H), 4.65 (s, 2H), 4.11 (dd, J = 11.6, 3.9 Hz, 2H), 3.67 (s, 2H), 3.60 (d, J = 11.6 Hz, 2H), 3.38 (s, 1H), 3.26 - 3.13 (m, 1H), 2.37 (s, 6H), 2.02 (t, J = 6.6 Hz, 1H), 0.87 (dd, J = 15.8, 8.7 Hz, 2H).

### Example 23: Preparation of Compound I-23

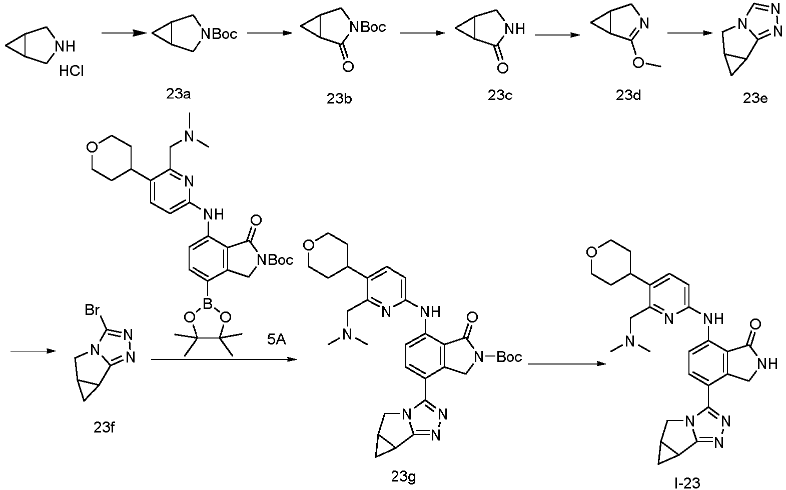

### Step A: Preparation of compound 23a

To a reaction flask were added 3-azabicyclo[3.1.0]hexane hydrochloride (20 g), triethylamine (30 g), di-*tert*-butyl dicarbonate (52 g), and dichloromethane (300 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (petroleum ether/ethyl acetate = 45/1) to give compound 23a (32 g).

MS (ESI, [M+H]⁺) *m*/*z*: 184.3.

### Step B: Preparation of compound 23b

To a reaction flask were added compound 23a (15 g), ruthenium dioxide (2.5 g), sodium periodate (90 g), ethyl acetate (300 mL), and water (100 mL) in sequence. After the addition, the mixture was stirred at room temperature. After the reaction was completed, the reaction solution was filtered and extracted with ethyl acetate. The organic phase was dried, filtered, concentrated, and subjected to column chromatography (petroleum ether/ethyl acetate = 45/1) to give compound 23b (12 g).

MS (ESI, [M+H]⁺) *m*/*z*: 198.2.

### Step C: Preparation of compound 23c

To a reaction flask were added compound 23b (10 g), a solution of hydrochloric acid in 1,4-dioxane (4 M, 10 mL), and dichloromethane (100 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, and concentrated to give compound 23c (6 g).

MS (ESI, [M+H]⁺) *m*/*z*: 98.3.

### Step D: Preparation of compound 23d

To a reaction flask were added compound 23c (6 g), trimethyloxonium tetrafluoroborate (12 g), dichloromethane (80 mL) in sequence under an ice-water bath. After the addition, the mixture was stirred at room temperature. After the reaction was completed, water was added to the reaction solution to quench the reaction, and the resulting mixture was extracted with dichloromethane. The organic phase was dried, filtered, and concentrated to give compound 23d (5.9 g). The crude product was concentrated and directly used in the next step.

### Step E: Preparation of compound 23e

To a reaction flask were added compound 23d (5 g), hydrazine formate (2.7 g), acetic acid (0.6 mL), and *n*-butanol (25 mL) in sequence. After the addition, the mixture was stirred at 120 °C under nitrogen atmosphere. After the reaction was completed, the reaction solution was concentrated and subjected to column chromatography (dichloromethane/methanol = 45/1) to give compound 23e (2.1 g).

MS (ESI, [M+H]⁺) *m*/*z*: 122.3.

### Step F: Preparation of compound 23f

To a reaction flask were added compound 23e (2 g), *N*-bromosuccinimide (3.2 g), sodium carbonate (1.8 g), and chloroform (30 mL) in sequence. After the addition, the mixture was stirred at room temperature. After the reaction was completed, the reaction solution was concentrated and extracted with dichloromethane. The organic phase was dried, filtered, and concentrated to give compound 23f (1.2 g).

MS (ESI, [M+H]⁺) *m*/*z*: 200.0.

### Step G: Preparation of compound 23g

To a reaction flask were added compound 23f (0.5 g), intermediate 5A (1.2 g), potassium phosphate (0.4 g), chloro(2-dicyclohexylphosphino-2',4',6'-triisopropyl-1,1'-biphenyl)[2-(2'-amino-1,1'-biphenyl)]palladium (II) (0.22 g), and 1,4-dioxane (20 mL) in sequence. After the addition, the mixture was stirred at 100 °C under nitrogen atmosphere. After the reaction was completed, the reaction solution was filtered, concentrated, and subjected to column chromatography (dichloromethane/methanol = 20/1) to give compound 23g (0.6 g).

MS (ESI, [M+H]⁺) *m*/*z*: 586.3.

### Step H: Preparation of compound I-23

To a reaction flask were added compound 23g (0.6 g), a solution of hydrochloric acid in 1,4-dioxane (4 M, 2 mL), and dichloromethane (10 mL) in sequence. After the addition, the mixture was stirred at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 9/1) to give compound I-23 (0.24 g).

HRMS (ESI, [M+H]⁺) *m*/*z*: 486.2614.

¹H NMR (500 MHz, CDCl₃:CD₃OD=10:1) δ 8.85 (d, J = 8.7 Hz, 1H), 7.60 (d, J = 8.7 Hz, 1H), 7.54 (d, J = 8.5 Hz, 1H), 6.84 (d, J = 8.4 Hz, 1H), 4.85 - 4.68 (m, 2H), 4.45 - 4.31 (m, 2H), 4.10 (dd, J = 11.3, 4.0 Hz, 2H), 3.69 (s, 1H), 3.61 - 3.54 (m, 4H), 3.29 (s, 1H), 3.21 - 3.12 (m, 1H), 2.70 - 2.55 (m, 2H), 2.39 (s, 6H), 1.79 (dd, J = 12.7, 4.3 Hz, 2H), 1.75 - 1.65 (m, 2H), 1.53 - 1.43 (m, 1H), 0.88 (d, J = 4.8 Hz, 1H).

### Example 24: Preparation of Compound 1-24

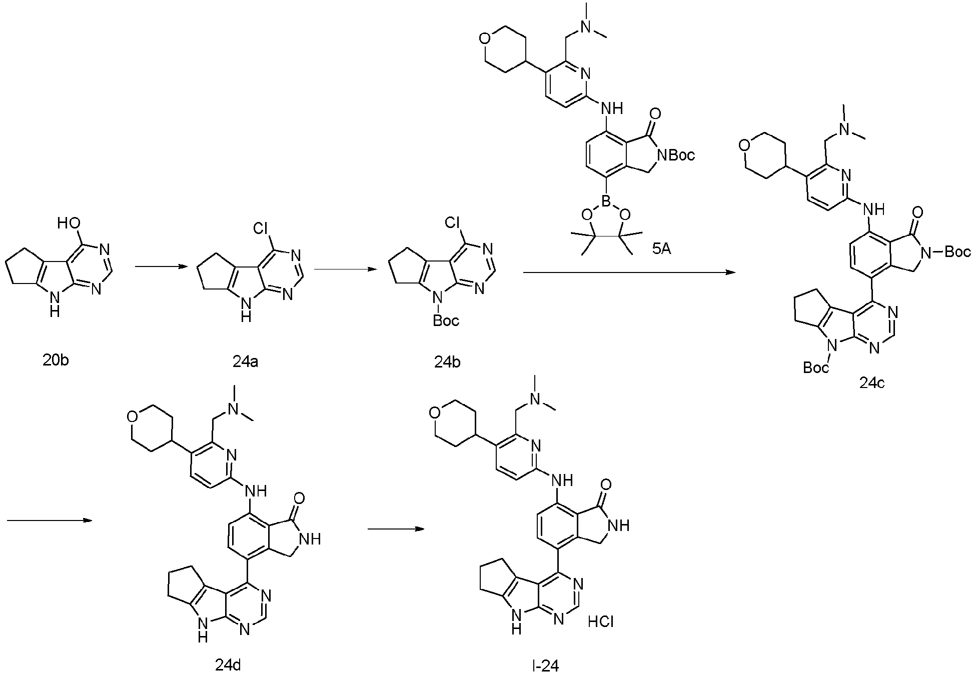

### Step A: Preparation of compound 24a

To a reaction flask were added compound 20b (0.6 g) and phosphorus oxychloride (9.58 mL) in sequence. The mixture was stirred at 100 °C. After the reaction was completed, the reaction solution was concentrated and diluted with dichloromethane. The reaction was quenched, and the reaction solution was extracted with dichloromethane. The organic phase was dried, filtered, concentrated, and subjected to column chromatography (petroleum ether/ethyl acetate = 4/1) to give compound 24a (0.623 g).

MS (ESI, [M+H]⁺) *m*/*z*: 194.04.

### Step B: Preparation of compound 24b

To a reaction flask were added compound 24a (0.2 g), 4-dimethylaminopyridine (0.025 g), DMF (5 mL), and di-*tert*-butyl dicarbonate (0.2 g) in sequence. The mixture was stirred at room temperature. After the reaction was completed, the reaction solution was concentrated and subjected to column chromatography (petroleum ether/ethyl acetate = 4/1) to give compound 24b (0.24 g).

MS (ESI, [M+H]⁺) *m*/*z*: 294.4.

### Step C: Preparation of compound 24c

To a reaction flask were added compound 24b (0.099 g), intermediate 5A (0.2 g), 2-dicyclohexylphosphine-2',4',6'-triisopropylbiphenyl (0.016 g), chloro(2-dicyclohexylphosphino-2',4',6'-triisopropyl-1,1'-biphenyl)[2-(2'-amino-1,1'-biphenyl)]palladium (II) (0.027 g), tripotassium phosphate (0.143 g), 1,4-dioxane (5 mL), and water (1 mL) in sequence. The mixture was purged with nitrogen and reacted at 85 °C under microwave irradiation. After the reaction was completed, the reaction solution was filtered and concentrated to give a crude product, which was subjected to column chromatography (dichloromethane/methanol = 20/1) to give compound 24c (0.250 g).

MS (ESI, [M+H]⁺) *m*/*z*: 724.45.

### Step D: Preparation of compound 24d

To a reaction flask were added compound 24c (0.250 g), dichloromethane (2 mL), and a solution of hydrochloric acid in 1,4-dioxane (0.5 mL, 4 M) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 10/1) to give compound 24d (0.020 g).

MS (ESI, [M+H]⁺) *m*/*z*: 524.30.

### Step E: Preparation of compound 1-24

To a reaction flask were added compound 24d (0.020 g), dichloromethane (5 mL), and a solution of hydrochloric acid in 1,4-dioxane (0.4 mL, 0.1 M) in sequence. After the addition, the mixture was stirred at room temperature. After the reaction was completed, the reaction solution was concentrated, slurried with methanol, and purified to give compound I-24 (0.008 g).

HRMS (ESI, [M+H]⁺) *m*/*z*: 524.2770.

### Example 25: Preparation of Compound 1-25

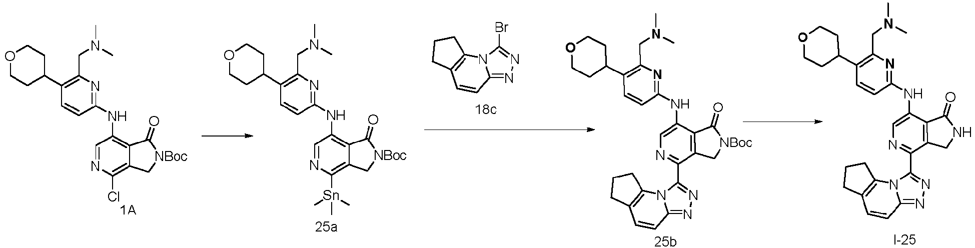

### Step A: Preparation of compound 25a

To a reaction flask were added intermediate 1A (0.5 g), hexamethylditin (0.46 g), bis(triphenylphosphine)palladium(II) dichloride (0.07 g), and toluene (30 mL) in sequence. After the addition, the mixture was stirred at 110 °C under nitrogen atmosphere. After the reaction was completed, the reaction solution was extracted with dichloromethane. The organic phase was dried, filtered, concentrated, and subjected to column chromatography (dichloromethane/methanol = 20/1) to give compound 25a (0.32 g).

MS (ESI, [M+H]⁺) *m*/*z*: 632.4.

### Step B: Preparation of compound 25b

To a reaction flask were added compound 25a (0.20 g), compound 18c (0.10 g), copper(I) iodide (0.2 mg), tetrakis(triphenylphosphine)palladium (0.01 g), and 1,4-dioxane (45 mL) in sequence. After the addition, the mixture was stirred at 100 °C under nitrogen atmosphere. After the reaction was completed, the reaction solution was extracted with dichloromethane. The organic phase was dried, filtered, concentrated, and subjected to column chromatography (dichloromethane/methanol = 10/1) to give compound 25b (0.06 g).

MS (ESI, [M+H]⁺) *m*/*z*: 625.1.

### Step C: Preparation of compound 1-25

To a reaction flask were added compound 25b (0.06 g), dichloromethane (10 mL), and a solution of hydrochloric acid in 1,4-dioxane (2 mL, 4 M) in sequence. The mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 20/1) to give compound I-25 (10 mg).

HRMS (ESI, [M+H]+) m/z: 525.2721.

¹H NMR (500 MHz, CDCl₃) δ 10.31 (s, 1H), 9.46 (s, 1H), 7.68 (d, *J =* 9.1 Hz, 1H), 7.56 (d, *J =* 8.5 Hz, 1H), 7.30 (d, *J =* 9.2 Hz, 1H), 6.86 (d, *J* = 8.4 Hz, 1H), 6.65 (s, 1H), 4.88 (s, 2H), 4.10 (dd, *J* = 10.9, 4.4 Hz, 2H), 3.65 (s, 2H), 3.56 (td, *J =* 11.7, 2.4 Hz, 2H), 3.27 (t, *J =* 7.7 Hz, 2H), 2.98 (t, *J =* 7.5 Hz, 2H), 2.32 (s, 6H), 2.25 - 2.14 (m, 2H), 1.80 - 1.69 (m, 5H).

### Example 26: Preparation of Compound I-26

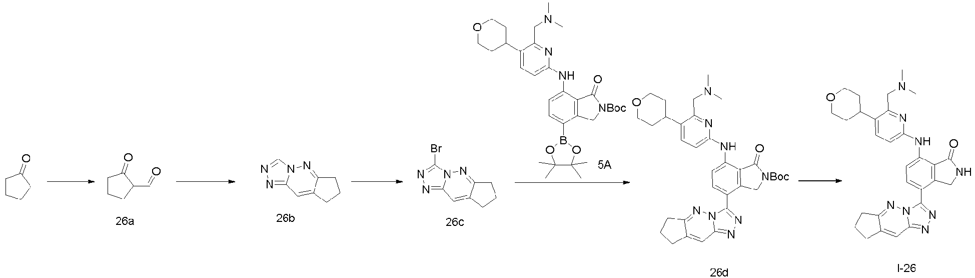

### Step A: Preparation of compound 26a

To a reaction flask were added tetrahydrofuran (40 mL) and 60 wt% sodium hydride (7.13 g) in sequence at room temperature, and a solution of cyclohexanone (6 g) in tetrahydrofuran (15 mL) was added dropwise. After the addition, the mixture was left to stand at 0 °C, and a solution of ethyl formate in ethanol (0.75mL) was added dropwise. After the addition, the mixture was reacted at 0 °C. After the reaction was completed, the reaction was quenched, water and ethyl acetate were added, and the resulting mixture was subjected to liquid separation. The aqueous phase was taken, adjusted to pH 3 with hydrochloric acid, and extracted with ethyl acetate. The organic phase was dried, filtered, concentrated, and subjected to column chromatography (petroleum ether/ethyl acetate = 9/1) to give compound 26a (4.1 g).

MS (ESI, [M-H]⁻) *m*/*z*: 111.01.

### Step B: Preparation of compound 26b

To a reaction flask were added compound 26a (1.5 g), ethanol (15 mL), trifluoroacetic acid (0.5 mL), and 4-amino-1,2,4-triazole (1.125 g) in sequence at 0 °C. After the addition, the mixture was stirred at 0 °C for 1 h, and then reacted at 100 °C under microwave irradiation. After the reaction was completed, the reaction solution was concentrated and subjected to column chromatography (dichloromethane/methanol = 20/1) to give compound 26b (1.2 g).

MS (ESI, [M+H]⁺) *m*/*z*: 161.04.

### Step C: Preparation of compound 26c

To a reaction flask were added compound 26b (0.2 g), dichloromethane (5 mL), and N-bromosuccinimide (0.333 g) in sequence. After the addition, the mixture was stirred at room temperature. After the reaction was completed, the reaction solution was concentrated and subjected to column chromatography (dichloromethane/methanol = 20/1) to give compound 26c (0.195 g).

MS (ESI, [M+H]⁺) *m*/*z*: 239.0.

### Step D: Preparation of compound 26d

To a reaction flask were added compound 26c (0.089 g), intermediate 5A (0.22 g), 2-dicyclohexylphosphine-2',4',6'-triisopropylbiphenyl (0.018 g), chloro(2-dicyclohexylphosphino-2',4',6'-triisopropyl-1,1'-biphenyl)[2-(2'-amino-1,1'-biphenyl)]palladium (II) (0.029 g), tripotassium phosphate (0.158g), 1,4-dioxane (20 mL), and water (4 mL) in sequence. The mixture was purged with nitrogen and stirred at 85 °C under microwave irradiation. After the reaction was completed, the reaction solution was filtered and concentrated to give a crude product, which was subjected to column chromatography (dichloromethane/methanol = 20/1) to give compound 26d (0.106 g).

MS (ESI, [M+H]⁺) *m*/*z*: 625.39.

### Step E: Preparation of compound I-26

To a reaction flask were added compound 26d (0.106 g), dichloromethane (2 mL), and a solution of hydrochloric acid in 1,4-dioxane (0.4 mL, 4 M) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 10/1) to give compound I-26 (0.023 g).

HRMS (ESI, [M+H]⁺) *m*/*z*: 525.2725.

¹H NMR (500 MHz, DMSO-*d*₆) δ 10.16 (s, 1H), 8.90 (s, 1H), 8.71 (dd, *J =* 22.4, 8.8 Hz, 2H), 8.10 (s, 1H), 7.66 (d, *J =* 8.4 Hz, 1H), 6.94 (d, *J =* 8.4 Hz, 1H), 4.70 (s, 2H), 3.96 (d, *J =* 10.3 Hz, 2H), 3.59 (s, 2H), 3.44 (dd, *J =* 11.3, 9.2 Hz, 2H), 3.20 (dd, *J* = 13.2, 9.1 Hz, 1H), 3.07 (t, *J =* 7.4 Hz, 2H), 2.99 (t, *J =* 7.1 Hz, 2H), 2.24 (s, 6H), 2.20 - 2.12 (m, 2H), 1.74 - 1.59 (m, 4H).

### Example 27: Preparation of Compound I-27

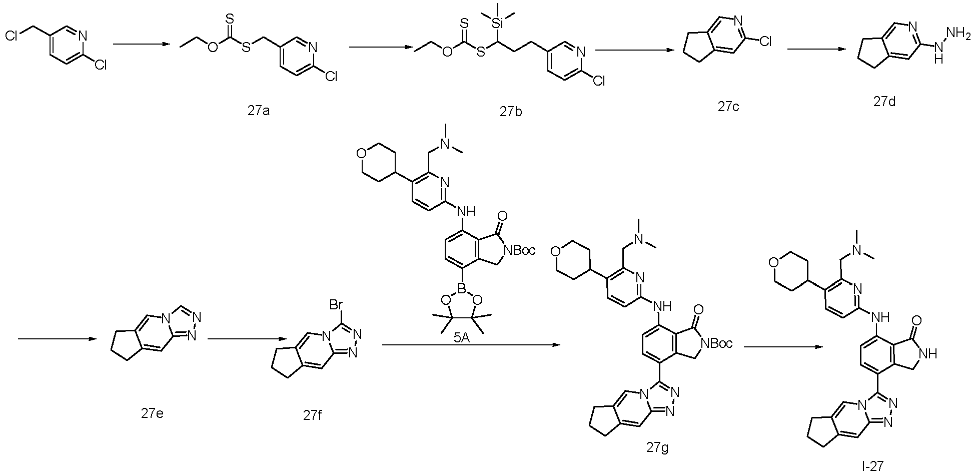

### Step A: Preparation of compound 27a

To a reaction flask were added the compounds 2-chloro-5-chloromethylpyridine (15.0 g), acetone (120.0 mL), and potassium ethylxanthate (14.8 g) in sequence. After the addition, the mixture was stirred at room temperature. After the reaction was completed, the reaction solution was extracted with dichloromethane. The organic phase was dried, filtered, concentrated, and subjected to column chromatography (dichloromethane/methanol = 30/1) to give compound 27a (23.1 g).

MS (ESI, [M+H]⁺) *m*/*z*: 248.0.

### Step B: Preparation of compound 27b

To a reaction flask were added compound 27a (23.1 g), 1,2-dichloroethane (50.0 mL), vinyltrimethylsilane (54.4 mL), and dilauroyl peroxide (16.3 mL) in sequence. After the addition, the mixture was purged with nitrogen and stirred at 40 °C. After the reaction was completed, the reaction solution was filtered, concentrated, and subjected to column chromatography (petroleum ether/ethyl acetate = 5/1) to give compound 27b (13.0 g).

MS (ESI, [M+H]⁺) *m*/*z*: 348.1.

### Step C: Preparation of compound 27c

To a reaction flask were added compound 27b (13.0 g), dilauroyl peroxide (2.9 g), acetonitrile (80.0 mL), and trifluoroacetic acid (3.1 mL) in sequence. After the addition, the mixture was stirred at 80 °C. After the reaction was completed, the reaction solution was filtered, concentrated, and subjected to column chromatography (petroleum ether/ethyl acetate = 5/1) to give compound 27c (2.1 g).

MS (ESI, [M+H]⁺) *m*/*z*: 154.2.

### Step D: Preparation of compound 27d

To a reaction flask were added compound 27c (2.1 g) and hydrazine hydrate (1.9 mL) in sequence. After the addition, the mixture was stirred at 120 °C. After the reaction was completed, the reaction solution was extracted with dichloromethane. The organic phase was dried, filtered, concentrated, and subjected to column chromatography (petroleum ether/ethyl acetate = 5/1) to give compound 27d (1.3 g).

MS (ESI, [M+H]⁺) *m*/*z*: 150.0.

### Step E: Preparation of compound 27e

To a reaction flask were added compound 27d (1.2 g), imidazole hydrochloride (1.2 g), and *N,N*-dimethylformamide (50.0 mL) in sequence. After the addition, the mixture was stirred at 160 °C. After the reaction was completed, the reaction solution was extracted with dichloromethane. The organic phase was dried, filtered, concentrated, and subjected to column chromatography (dichloromethane/methanol = 30/1) to give compound 27e (0.8 g).

MS (ESI, [M+H]⁺) *m*/*z*: 160.0.

### Step F: Preparation of compound 27f

To a reaction flask were added compound 27e (0.8 g), dichloromethane (20.0 mL), and *N*-bromosuccinimide (0.59 mL) in sequence. After the addition, the mixture was stirred at 45 °C. After the reaction was completed, the reaction solution was concentrated and subjected to column chromatography (petroleum ether/ethyl acetate = 5/1) to give compound 27f (0.09 g).

MS (ESI, [M+H]⁺) *m*/*z*: 238.1.

### Step G: Preparation of compound 27g

To a reaction flask were added compound 27f (0.09 g), 2-dicyclohexylphosphine-2',4',6'-triisopropylbiphenyl (0.02 g), potassium phosphate (0.3 g), chloro(2-dicyclohexylphosphino-2',4',6'-trilsopropyl-1,1'-biphenyl)[2-(2'-amino-1,1'-biphenyl)]palladium (II) (0.07 g), intermediate 5A (0.4 g), water (3.0 mL), and 1,4-dioxane (12.0 mL) in sequence. After the addition, the mixture was stirred at 85 °C. After the reaction was completed, the reaction solution was filtered, concentrated, and subjected to column chromatography (petroleum ether/ethyl acetate = 5/1) to give compound 27g (0.13 g).

MS (ESI, [M+H]⁺) *m*/*z*: 624.3.

### Step H: Preparation of compound I-27

To a reaction flask were added compound 27g (0.13 g), dichloromethane (2 mL), and a solution of hydrochloric acid in 1,4-dioxane (0.4 mL, 4 M) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 10/1) to give compound I-27 (0.03 g).

HRMS (ESI, [M+H]⁺) *m*/*z*: 524.2754.

¹H NMR (500 MHz, CDCl₃) δ 9.95 (s, 1H), 8.99 (d, *J* = 8.7 Hz, 1H), 8.15 - 8.10 (m, 1H), 7.77 (d, *J* = 8.6 Hz, 1H), 7.59 - 7.55 (m, 1H), 7.53 (d, *J =* 8.5 Hz, 1H), 6.84 (d, *J =* 8.4 Hz, 1H), 6.40 (s, 1H), 4.73 (s, 2H), 4.09 (dd, *J* = 11.1, 4.1 Hz, 2H), 3.64 (s, 2H), 3.55 (td, *J* = 11.7, 2.2 Hz, 2H), 3.21 (m, 1H), 2.93 (td, *J =* 7.3, 1.7 Hz, 2H), 2.33 (s, 6H), 2.22 - 2.13 (m, 2H), 1.76 - 1.68 (m, 4H).

### Example 28: Preparation of Compound 28

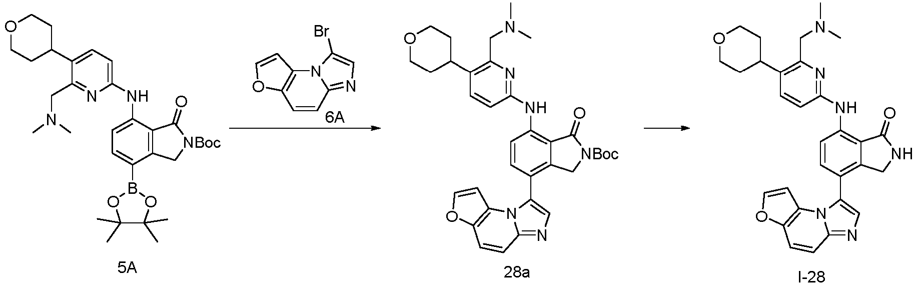

### Step A: Preparation of compound 28a

To a microwave tube were added intermediate 5A (230 mg), intermediate 6A (110 mg), chloro(2-dicyclohexylphosphino-2',4',6'-triisopropyl-1,1'-biphenyl)[2-(2'-atnino-1,1'-biphenyl)]palladium (II) (31 mg), 2-dicyclohexylphosphine-2',4',6'-triisopropylbiphenyl (19 mg), tripotassium phosphate (165 mg), 1,4-dioxane (3.5 mL), and water (0.5 mL) in sequence. The mixture was stirred at 85 °C under microwave irradiation under nitrogen atmosphere. After the reaction was completed, the reaction solution was filtered, concentrated, and subjected to column chromatography (dichloromethane/methanol = 20/1) to give compound 28a (72 mg).

MS (ESI, [M+H]+) m/z: 623.34.

### Step B: Preparation of compound I-28

To a reaction flask were added compound 28a (99 mg), dichloromethane (5.00 mL), and methanesulfonic acid (0.1 mL) in sequence. After the addition, the mixture was reacted at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 10/1) to give compound I-28 (20 mg).

HRMS (ESI, [M+H]⁺) *m*/*z:* 523.2457.

¹H NMR (500 MHz, CDCl₃) δ 9.83 (s, 1H), 8.96 (d, J = 8.5 Hz, 1H), 7.66 (s, 1H), 7.62 - 7.51 (m, 5H), 6.86 (d, J = 8.4 Hz, 1H), 6.34 - 6.27 (m, 2H), 4.24 (s, 2H), 4.13 - 4.06 (m, 2H), 3.64 (s, 2H), 3.58 - 3.53 (m, 2H), 3.24 - 3.19 (m, 1H), 2.33 (s, 6H), 1.80 - 1.69 (m, 4H).

### Example 29: Preparation of Compound I-29

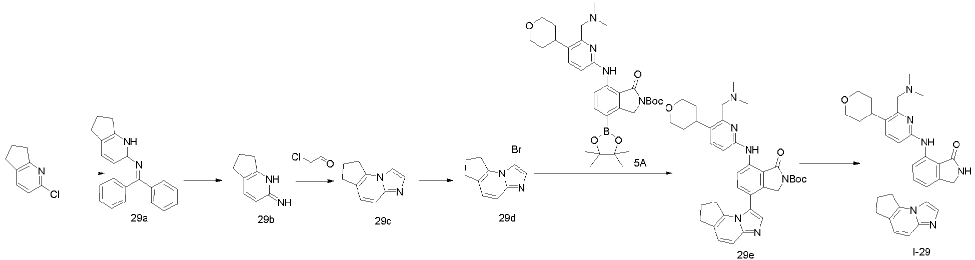

### Step A: Preparation of compound 29a

To a reaction flask were added 2-chloro-6,7-dihydro-5*H*-2,3-cyclopentenopyridine (5.0 g), tris(dibenzylideneacetone)dipalladium(0) (0.6 g), benzophenone imine (2.83 g), sodium *tert*-butoxide (6.25 g), 1,1'-binaphthyl-2,2'-bisdiphenylphosphine (0.4 g), and toluene (80 mL) in sequence. After the addition, the mixture was stirred at 80 °C. After the reaction was completed, the reaction solution was extracted with dichloromethane. The organic phase was dried, filtered, concentrated, and subjected to column chromatography (dichloromethane/methanol = 10/1) to give compound 29a (4.1 g).

MS (ESI, [M+H]⁺) m/z: 301.2.

### Step B: Preparation of compound 29b

To a reaction flask were added compound 29a (4.1 g), dichloromethane (50 mL), and concentrated hydrochloric acid (20 mL) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 20/1) to give compound 29b (1.6 g).

MS (ESI, [M+H]⁺) m/z: 135.3.

### Step C: Preparation of compound 29c

To a reaction flask were added compound 29b (1.6 g), ethanol (30 mL), and 2-chloroacetaldehyde (10.6 g) in sequence. After the addition, the mixture was stirred at 80 °C. After the reaction was completed, the reaction solution was concentrated, adjusted to alkalinity (about pH 10) with a saturated sodium bicarbonate solution, and extracted with dichloromethane. The organic phase was dried, filtered, concentrated, and subjected to column chromatography (dichloromethane/methanol = 20/1) to give compound 29c (1.3 g).

MS (ESI, [M+H]⁺) m/z: 159.4.

### Step D: Preparation of compound 29d

To a reaction flask were added compound 29c (1.3 g), CClₐ (30 mL), and NBS (0.4 g) in sequence. After the addition, the mixture was stirred at room temperature. After the reaction was completed, a saturated sodium thiosulfate solution was added to quench the reaction, and the resulting mixture was extracted with dichloromethane. The organic phase was dried, filtered, concentrated, and subjected to column chromatography (dichloromethane/methanol = 20/1) to give compound 29d (1.6 g).

MS (ESI, [M+H]⁺) m/z: 237.0.

### Step E: Preparation of compound 29e

To a reaction flask were added compound 29d (0.17 g), intermediate 5A (0.17 g), 2-dicyclohexylphosphine-2',4',6'-triisopropylbiphenyl (0.02 g), chloro(2-dicyclohexylphosphino-2',4',6'-triisopropyl-1,1'-biphenyl)[2-(2'-amino-1,1'-biphenyl)]palladium (II) (0.03 g), potassium phosphate (0.09 g), water (2.00 mL), and 1,4-dioxane (15 mL) in sequence. After the addition, the mixture was stirred at 85 °C under nitrogen atmosphere. After the reaction was completed, the reaction solution was concentrated and subjected to column chromatography (dichloromethane/methanol = 10/1) to give compound 29e (0.11 g).

MS (ESI, [M+H]⁺) *m*/*z*: 623.4.

### Step F: Preparation of compound I-29

To a reaction flask were added compound 29e (0.11 g), dichloromethane (10 mL), and a solution of hydrochloric acid in 1,4-dioxane (2 mL, 4 M) in sequence. The mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 20/1) to give compound I-29 (52 mg).

HRMS (ESI, [M+H]⁺) *m*/*z*: 523.2984.

¹H NMR (500 MHz, CDCl₃) δ 9.75 (s, 1H), 8.86 (d, *J =* 8.5 Hz, 1H), 7.55 (s, 1H), 7.52 (dd, *J =* 8.8, 4.2 Hz, 2H), 7.46 (d, *J =* 8.5 Hz, 1H), 7.16 (d, *J =* 9.0 Hz, 1H), 6.83 (d, *J =* 8.5 Hz, 1H), 6.27 (s, 1H), 4.13 - 4.05 (m, 2H), 3.61 (d, *J =* 9.3 Hz, 2H), 3.55 (td, *J =* 11.6, 2.4 Hz, 2H), 3.20 (tt, *J =* 11.7, 3.9 Hz, 1H), 2.88 (t, *J =* 7.5 Hz, 2H), 2.31 (s, 6H), 2.05 (s, 3H), 1.83 - 1.68 (m, 7H).

### Example 30: Preparation of Compound 1-30

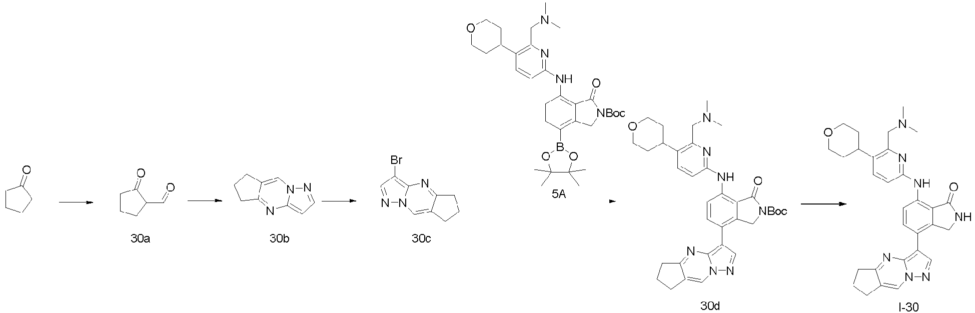

### Step A: Preparation of compound 30a

To a reaction flask were added the compounds cyclopentanone (6.0 g), 60 wt% sodium hydride (7.2 g), and ethyl formate (21.1 g) in sequence. After the addition, the mixture was stirred at 0 °C. After the reaction was completed, water was added to the reaction solution to quench the reaction, and the resulting mixture was extracted with dichloromethane. The organic phase was dried, filtered, concentrated, and subjected to column chromatography (dichloromethane/methanol = 30/1) to give compound 30a (2.0 g).

MS (ESI, [M+H]⁺) *m*/*z*: 113.0.

### Step B: Preparation of compound 30b

To a reaction flask were added compound 30a (2.0 g), trifluoroacetic acid (1.1 mL), 5-amino-1*H*-pyrazole (1.2 g), and ethanol (20.0 mL) in sequence at 0 °C. After the addition, the mixture was stirred at 0 °C. After the reaction was completed, the reaction solution was filtered, concentrated, and subjected to column chromatography (petroleum ether/ethyl acetate = 5/1) to give compound 30b (1.6 g).

MS (ESI, [M+H]⁺) *m*/*z*: 160.0.

### Step C: Preparation of compound 30c

To a reaction flask were added compound 30b (1.6 g), N-bromosuccinimide (1.3 g), and acetonitrile (40.0 mL) in sequence at 0 °C. After the addition, the mixture was stirred at 0 °C. After the reaction was completed, the reaction solution was filtered, concentrated, and subjected to column chromatography (petroleum ether/ethyl acetate = 5/1) to give compound 30c (1.2 g).

MS (ESI, [M+H]⁺) *m*/*z*: 238.0.

### Step D: Preparation of compound 30d

To a reaction flask were added compound 30c (0.2 g), 2-dicyclohexylphosphine-2',4',6'-triisopropylbiphenyl (0.03 g), potassium phosphate (1.2 g), chloro(2-dicyclohexylphosphino-2',4',6'-trilsopropyl-1,1'-biphenyl)[2-(2'-amino-1,1'-biphenyl)]palladium (II) (0.07 g), intermediate 5A (0.4 g), water (3.0 mL), and 1,4-dioxane (12.0 mL) in sequence. After the addition, the mixture was purged with nitrogen and stirred at 85 °C. After the reaction was completed, the reaction solution was filtered, concentrated, and subjected to column chromatography (petroleum ether/ethyl acetate = 5/1) to give compound 30d (0.2 g).

MS (ESI, [M+H]⁺) *m*/*z*: 624.7.

### Step E: Preparation of compound I-30

To a reaction flask were added compound 30d (0.2 g), dichloromethane (10 mL), and a solution of hydrochloric acid in 1,4-dioxane (2 mL, 4 M) in sequence. The mixture was stirred at room temperature. After the reaction was completed, the reaction solution was adjusted to alkalinity (about pH 10) with a saturated sodium bicarbonate solution and extracted with dichloromethane. The organic phase was concentrated and subjected to column chromatography (dichloromethane/methanol = 20/1) to give compound I-30 (0.05 g).

HRMS (ESI, [M+H]⁺) *m*/*z*: 524.2754.

¹H NMR (500 MHz, CDCl₃) δ 9.75 (s, 1H), 8.80 (d, J = 8.6 Hz, 1H), 8.45 (d, J = 1.5 Hz, 1H), 8.12 (s, 1H), 7.96 (d, J = 8.6 Hz, 1H), 7.49 (d, J = 8.5 Hz, 1H), 6.83 (d, J = 8.5 Hz, 1H), 6.41 (s, 1H), 4.62 (s, 2H), 4.14 - 4.03 (m, 2H), 3.66 (s, 2H), 3.56 (td, J = 11.6, 2.3 Hz, 2H), 3.20 (td, J = 12.0, 6.2 Hz, 1H), 3.11 - 2.96 (m, 4H), 2.35 (s, 6H), 2.26 (p, J = 7.5 Hz, 2H), 1.83 - 1.75 (m, 2H), 1.74 - 1.67 (m, 2H).

### Example 31: Preparation of Compound I-31

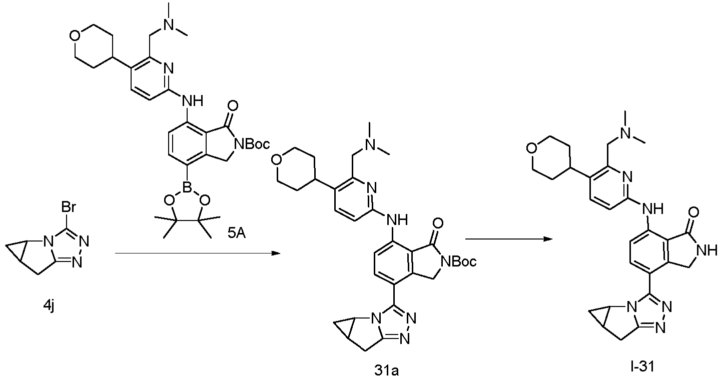

### Step A: Preparation of compound 31a

To a reaction flask were added compound 4j (0.15 g), intermediate 5A (0.17 g), 2-dicyclohexylphosphine-2',4',6'-triisopropylbiphenyl (0.02 g), chloro(2-dicyclohexylphosphino-2',4',6'-triisopropyl-1,1'-biphenyl)[2-(2'-amino-1,1'-biphenyl)]palladium (II) (0.03 g), potassium phosphate (0.09 g), water (2.00 mL), and 1,4-dioxane (15 mL) in sequence. After the addition, the mixture was stirred at 85 °C under nitrogen atmosphere. After the reaction was completed, the reaction solution was concentrated and subjected to column chromatography (dichloromethane/methanol = 10/1) to give compound 31a (0.11 g).

MS (ESI, [M+H]⁺) *m*/*z*: 586.4.

### Step B: Preparation of compound I-31

To a reaction flask were added compound 31a (0.11 g), dichloromethane (10 mL), and a solution of hydrochloric acid in 1,4-dioxane (2 mL, 4 M) in sequence. The mixture was stirred at room temperature. After the reaction was completed, the reaction solution was adjusted to alkalinity (about pH 10) with a saturated sodium bicarbonate solution and extracted with dichloromethane. The organic phase was concentrated and subjected to column chromatography (dichloromethane/methanol = 20/1) to give compound I-31 (36 mg).

HRMS (ESI, [M+H]⁺) *m*/*z*: 486.2615.

¹H NMR (500 MHz, CDCl₃) δ 9.99 (s, 1H), 8.92 (d, *J* = 8.7 Hz, 1H), 7.89 (d, *J =* 8.7 Hz, 1H), 7.52 (d, *J =* 8.4 Hz, 1H), 6.83 *(d, J=* 8.4 Hz, 1H), 6.30 (s, 1H), 4.93 *(d, J=* 19.0 Hz, 1H), 4.73 *(d, J=* 19.1 Hz, 1H), 4.09 (dd, *J* = 10.9, 4.4 Hz, 2H), 4.03 (td, *J* = 5.9, 2.2 Hz, 1H), 3.69 - 3.58 (m, 2H), 3.55 (td, *J =* 11.6, 2.3 Hz, 2H), 3.33 - 3.16 (m, 2H), 3.10 (d, *J* = 17.3 Hz, 1H), 2.39 (dq, *J* = 12.1, 6.2 Hz, 1H), 2.31 (s, 5H), 1.88 - 1.67 (m, 4H), 1.42 (dt, *J* = 8.8, 6.1 Hz, 1H), 1.25 (s, 1H).

### Example 32: Preparation of Compound 1-32

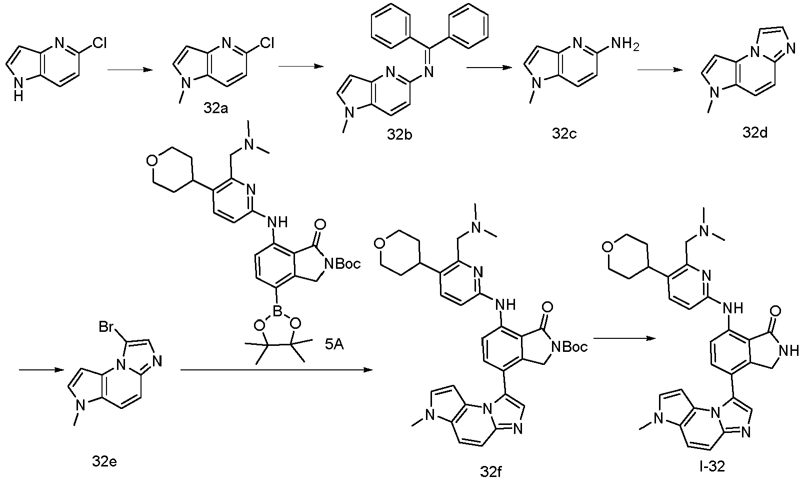

### Step A: Preparation of compound 32a

To a reaction flask were added 5-chloro-1*H*-pyrrolo[3,2-*b*]pyridine (6 g), cesium carbonate (12.81 g), DMF (120 mL), and iodomethane (5.58 g) in sequence. After the addition, the mixture was stirred at 60 °C. After the reaction was completed, water and ethyl acetate were added to the reaction solution, and the resulting mixture was extracted and subjected to liquid separation. The organic phase was collected, dried, filtered, concentrated, and subjected to column chromatography (petroleum ether/ethyl acetate = 5/1) to give compound 32a (4.4 g).

MS (ESI, [M-H]⁻) *m*/*z*: 167.19.

### Step B: Preparation of compound 32b

To a reaction flask were added sodium *tert*-butoxide (4.33 g), 1,1'-binaphthyl-2,2'-bisdiphenylphosphine (2.242 g), tris(dibenzylideneacetone)dipalladium(0) (2.473 g), benzophenone imine (4.90 g), compound 32a (3.0 g), and toluene (50 mL) in sequence. After the addition, the mixture was purged with nitrogen and stirred at 80 °C. After the reaction was completed, the reaction solution was filtered, concentrated, and subjected to column chromatography (petroleum ether/ethyl acetate = 1/4) to give compound 32b (2.0 g).

MS (ESI, [M-H]⁻) *m*/*z:* 312.36.

### Step C: Preparation of compound 32c

To a reaction flask were added compound 32b (2.0 g), dichloromethane (20 mL), and a solution of hydrochloric acid in 1,4-dioxane (8 mL, 4 M) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 10/1) to give compound 32c (0.70 g).

MS (ESI, [M-H]⁻) *m*/*z:* 148.11.

### Step D: Preparation of compound 32d

To a reaction flask were added compound 32c (0.7 g), ethanol (20 mL), potassium carbonate (1.315 g), and chloroacetaldehyde (1.12 g) in sequence. After the addition, the mixture was stirred at 90 °C. After the reaction was completed, the reaction solution was concentrated and subjected to column chromatography (dichloromethane/methanol = 20/1) to give compound 32d (0.68 g).

MS (ESI, [M-H]⁻) *m*/*z*: 172.27.

### Step E: Preparation of compound 32e

To a reaction flask were added compound 32d (0.25 g), DMSO (5 mL), sodium carbonate (0.310 g), and dibromohydantoin (0.209 g) in sequence. After the addition, the mixture was stirred at room temperature. After the reaction was completed, the reaction solution was extracted with water and ethyl acetate. The organic phase was dried, filtered, and concentrated. The residue was subjected to column chromatography (dichloromethane/methanol = 20/1) to give compound 32e (0.223 g).

MS (ESI, [M+H]⁺) *m*/*z:* 250.05.

### Step F: Preparation of compound 32f

To a reaction flask were added compound 32e (0.106 g), intermediate 5A (0.25 g), 2-dicyclohexylphosphine-2',4',6'-triisopropylbiphenyl (0.02 g), chloro(2-dicyclohexylphosphino-2',4',6'-triisopropyl-1,1'-biphenyl)[2-(2'-amino-1,1'-biphenyl)]palladium (II) (0.033 g), tripotassium phosphate (0.179 g), 1,4-dioxane (5 mL), and water (1 mL) in sequence. The mixture was purged with nitrogen and stirred at 85 °C under microwave irradiation. After the reaction was completed, the reaction solution was filtered and concentrated to give a crude product, which was subjected to column chromatography (dichloromethane/methanol = 20/1) to give compound 32f (0.106 g).

MS (ESI, [M+H]⁺) *m*/*z:* 636.44.

### Step G: Preparation of compound I-32

To a reaction flask were added compound 32f (0.106 g), dichloromethane (2 mL), and a solution of hydrochloric acid in 1,4-dioxane (0.4 mL, 4 M) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 10/1) to give compound I-32 (0.025 g).

HRMS (ESI, [M+H]⁺) *m*/*z:* 536.2776.

¹H NMR (500 MHz, DMSO-*d*₆) δ 9.98 (s, 1H), 8.70 (d, *J =* 6.3 Hz, 2H), 7.71 - 7.54 (m, 4H), 7.34 (d, *J =* 9.5 Hz, 1H), 7.17 (d, *J =* 3.0 Hz, 1H), 6.96 (d, *J =* 8.4 Hz, 1H), 5.70 (d, *J =* 3.0 Hz, 1H), 4.18 (s, 2H), 4.00 - 3.92 (m, 2H), 3.84 (s, 3H), 3.61 (s, 2H), 3.44 (dd, *J* = 11.4, 9.4 Hz, 2H), 3.18 (dd, *J* = 13.3, 9.4 Hz, 1H), 2.25 (s, 6H), 1.72 - 1.59 (m, 4H).

### Example 33: Preparation of Compound I-33

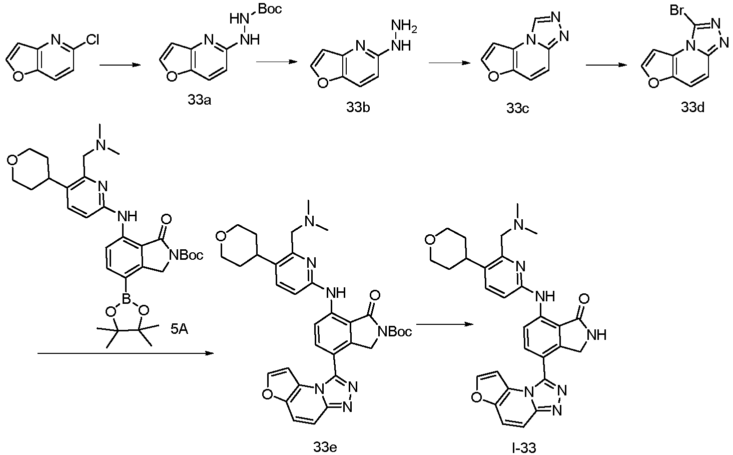

### Step A: Preparation of compound 33a

To a reaction flask were added 5-chlorofuro[3,2-*b*]pyridine (1.5 g), tert-butyl carbazate (1.6 g), cesium carbonate (4.4 g), tris(dibenzylideneacetone)dipalladium(0) (0.92 g), 1,1'-bis(diphenylphosphino)ferrocene (1.10 g), and toluene (20 mL) in sequence. After the addition, the mixture was stirred at 100 °C under nitrogen atmosphere. After the reaction was completed, the reaction solution was concentrated, and subjected to column chromatography (dichloromethane/methanol = 6/1) to give compound 33a (1.6 g).

MS (ESI, [M+H]⁺) *m*/*z*: 250.2.

### Step B: Preparation of compound 33b

To a reaction flask were added compound 33a (1.6 g), a solution of hydrochloric acid in 1,4-dioxane (4 M, 2 mL), and dichloromethane (10 mL) in sequence. After the addition, the mixture was stirred at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 9/1) to give compound 33b (1.2 g).

MS (ESI, [M+H]⁺) *m*/*z*: 150.2.

### Step C: Preparation of compound 33c

To a reaction flask were added compound 33b (1.2 g), imidazole (80 mg), and *N,N*-dimethylformamide (10 mL) in sequence. After the addition, the mixture was stirred at 170 °C under nitrogen atmosphere. After the reaction was completed, a saturated aqueous ammonium chloride solution was added to the reaction solution to quench the reaction, and the resulting mixture was extracted with ethyl acetate. The organic phase was dried, filtered, concentrated, and subjected to column chromatography (petroleum ether/ethyl acetate = 4/1) to give compound 33c (0.8 g).

MS (ESI, [M+H]⁺) *m*/*z*: 160.2.

### Step D: Preparation of compound 33d

To a reaction flask were added compound 33c (0.8 g), *N*-bromosuccinimide (0.75 g), and dichloromethane (10 mL) in sequence. After the addition, the mixture was stirred at room temperature. After the reaction was completed, an aqueous sodium bicarbonate solution was added to the reaction solution to quench the reaction, and the resulting mixture was extracted with dichloromethane. The organic phase was dried, filtered, concentrated, and subjected to column chromatography (petroleum ether/ethyl acetate = 4/1) to give compound 33d (0.7 g).

MS (ESI, [M+H]⁺) *m*/*z*: 238.1.

### Step E: Preparation of compound 33c

To a reaction flask were added compound 33d (0.7 g), intermediate 5A (0.9 g), potassium phosphate (0.2 g), chloro(2-dicyclohexylphosphino-2',4',6'-triisopropyl-1,1'-biphenyl)[2-(2'-amino-1,1'-biphenyl)]palladium (II) (0.15 g), 1,4-dioxane (20 mL), and water (1 mL) in sequence. After the addition, the mixture was stirred at 100 °C under nitrogen atmosphere. After the reaction was completed, the reaction solution was concentrated and subjected to column chromatography (dichloromethane/methanol = 15/1) to give compound 33e (0.2 g).

MS (ESI, [M+H]⁺) *m*/*z*: 624.5.

### Step F: Preparation of compound I-33

To a reaction flask were added compound 33e (0.2 g), a solution of hydrochloric acid in 1,4-dioxane (4 M, 1 mL), and dichloromethane (10 mL) in sequence. After the addition, the mixture was stirred at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 9/1) to give compound I-33 (0.11 g).

HRMS (ESI, [M+H]⁺) *m*/*z*: 524.2407.

¹H NMR (500 MHz, CDCl₃:CD₃OD=10:1) δ 9.00(s, 1H) 7.76 (d, *J =* 8.6 Hz, 1H), 7.70 *(d, J=* 6.3 Hz, 3H), 7.57 (d, *J =* 8.5 Hz, 1H), 6.90 (d, *J =* 8.5 Hz, 1H), 6.77 (d, *J* = 2.2 Hz, 1H), 4.58 (s, 2H), 4.11 (dd, *J =* 11.4, 4.0 Hz, 2H), 3.68 (s, 2H), 3.58 (t, *J =* 11.5 Hz, 2H), 3.28 - 3.08 (m, 1H), 2.36 (s, 6H), 1.80 (dd, *J =* 12.6, 4.1 Hz, 2H), 1.74(dd, *J* = 12.6, 4.1 Hz, 2H).

### Example 34: Preparation of Compound I-34

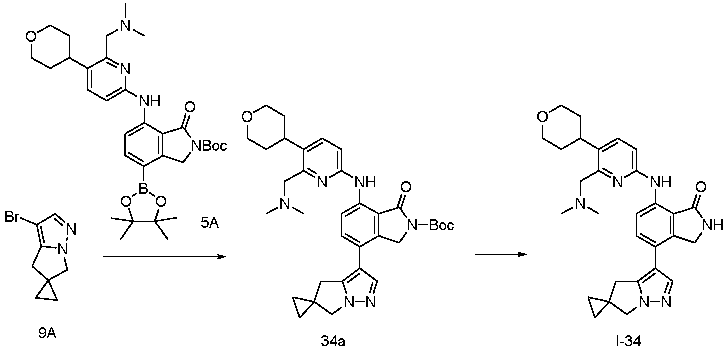

### Step A: Preparation of compound 34a

To a reaction flask were added intermediate 9A (0.7 g), intermediate 5A (0.9 g), potassium phosphate (0.2 g), chloro(2-dicyclohexylphosphino-2',4',6'-triisopropyl-1,1'-biphenyl)[2-(2'-amino-1,1'-biphenyl)]palladium (II) (0.15 g), 1,4-dioxane (20 mL), and water (1 mL) in sequence. After the addition, the mixture was stirred at 100 °C under nitrogen atmosphere. After the reaction was completed, the reaction solution was concentrated and subjected to column chromatography (dichloromethane/methanol = 15/1) to give compound 34a (0.2 g).

MS (ESI, [M+H]⁺) *m*/*z*: 599.5.

### Step B: Preparation of compound 1-34

To a reaction flask were added compound 34a (0.2 g), a solution of hydrochloric acid in dioxane (4 M, 1 mL), and dichloromethane (10 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 9/1) to give compound I-34 (0.08 g).

HRMS (ESI, [M+H]⁺) *m*/*z*: 499.2818.

¹H NMR (500 MHz, CDCl₃:CD₃OD=10:1) δ 8.72 (dd, *J* = 8.6, 2.5 Hz, 1H), 7.64 *(d, J=* 2.4 Hz, 1H), 7.47 (ddd, *J* = 26.6, 8.6, 2.4 Hz, 2H), 6.81 (dd, *J =* 8.5, 2.2 Hz, 1H), 4.51 (d, *J =* 2.2 Hz, 2H), 4.16 - 4.05 (m, 4H), 3.63 (s, 2H), 3.56 (t, *J =* 11.8 Hz, 2H), 3.20(t, *J* = 2.5Hz, 2H), 3.10 (d, *J =* 2.3 Hz, 2H), 2.36(s, 6H), 1.83 - 1.74 (m, 2H), 1.72 (s, 2H), 0.89 (d, *J* = 10.0 Hz, 4H).

### Example 35: Preparation of Compound 1-35

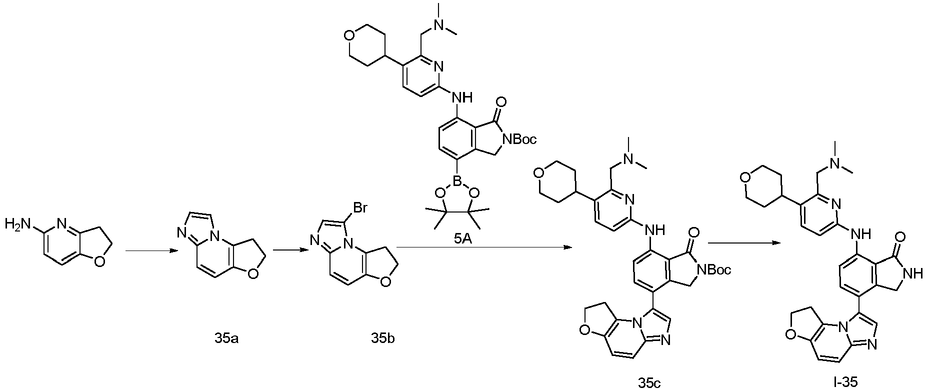

### Step A: Preparation of compound 35a

To a reaction flask were added the compounds 2,3-dihydrofuro[3,2-*b*]pyridin-5-amine (0.7 g), ethanol (20 mL), potassium carbonate (1.766 g), and chloroacetaldehyde (2.018 g) in sequence. After the addition, the mixture was stirred at 90 °C. After the reaction was completed, the reaction solution was concentrated and subjected to column chromatography (dichloromethane/methanol = 20/1) to give compound 35a (0.60 g).

MS (ESI, [M+H]⁺) *m*/*z*: 161.23.

### Step B: Preparation of compound 35b

To a reaction flask were added compound 35a (0.30 g), DMSO (5 mL), sodium carbonate (0.199 g), and dibromohydantoin (0.268 g) in sequence. After the addition, the mixture was stirred at room temperature. After the reaction was completed, the reaction solution was extracted with water and ethyl acetate. The organic phase was dried, filtered, concentrated, and subjected to column chromatography (dichloromethane/methanol = 20/1) to give compound 35b (0.340 g).

MS (ESI, [M+H]⁺) *m*/*z*: 239.21.

### Step C: Preparation of compound 35c

To a reaction flask were added compound 35b (0.101 g), intermediate 5A (0.25 g), 2-dicyclohexylphosphine-2',4',6'-triisopropylbiphenyl (0.02 g), chloro(2-dicyclohexylphosphino-2',4',6'-triisopropyl-1,1'-biphenyl)[2-(2'-amino-1,1'-biphenyl)]palladium (II) (0.033 g), tripotassium phosphate (0.179 g), 1,4-dioxane (5 mL), and water (1 mL) in sequence. The mixture was purged with nitrogen and stirred at 85 °C under microwave irradiation. After the reaction was completed, the reaction solution was filtered and concentrated to give a crude product, which was subjected to column chromatography (dichloromethane/methanol = 20/1) to give compound 35c (0.156 g).

MS (ESI, [M+H]⁺) *m*/*z*: 625.49.

### Step D: Preparation of compound I-35

To a reaction flask were added compound 35c (0.106 g), dichloromethane (2 mL), and a solution of hydrochloric acid in 1,4-dioxane (0.4 mL, 4 M) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 10/1) to give compound I-35 (0.012 g).

HRMS (ESI, [M+H]⁺) *m*/*z*: 525.2621.

¹H NMR (500 MHz, CDCl₃) δ 8.81 (d,J = 8.4 Hz, 1H), 7.54 - 7.39 (m, 4H), 7.00 (d, *J =* 9.6 Hz, 1H), 6.81 (d, *J =* 8.3 Hz, 1H), 4.52 (t, *J =* 8.7 Hz, 2H), 4.18 (s, 2H), 4.06 (d, *J =* 9.3 Hz, 2H), 3.60 (s, 2H), 3.53 (t, *J =* 11.2 Hz, 2H), 3.14 (t, *J=* 11.5 Hz, 1H), 2.88 (s, 2H), 2.30 (s, 6H), 1.72 (dt, *J =* 27.0, 11.4 Hz, 4H).

### Example 36: Preparation of Compound I-36

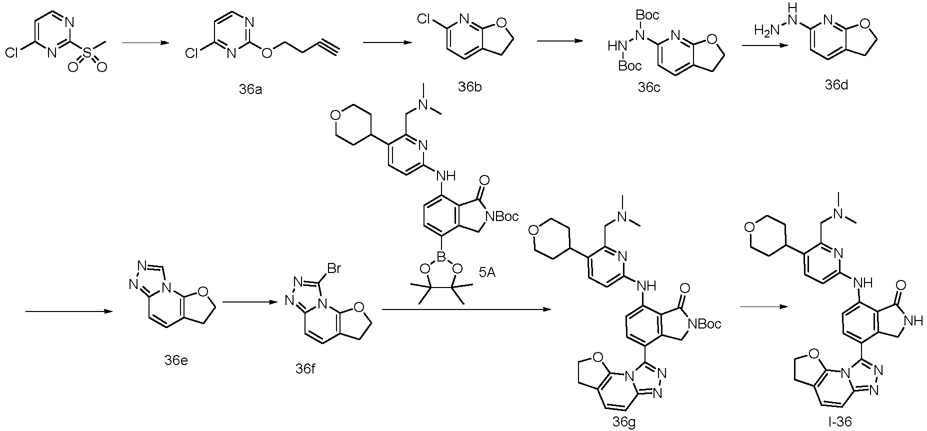

### Step A: Preparation of compound 36a

To a reaction flask were added 4-chloro-2-methanesulfonylpyrimidine (6 g), THF (120 mL), 3-butyn-1-ol (2.2 g), and 60 wt% sodium hydride (1.246 g) in sequence. After the addition, the mixture was stirred at room temperature. After the reaction was completed, water was added to the reaction solution to quench the reaction, and the resulting mixture was extracted with water and ethyl acetate. The organic phase was dried, filtered, concentrated, and subjected to column chromatography (petroleum ether/ethyl acetate = 9/1) to give compound 36a (2.0 g).

MS (ESI, [M+H]⁺) *m*/*z*: 183.0.

### Step B: Preparation of compound 36b

To a reaction flask were added compound 36a (2 g) and diphenyl ether (20 mL) in sequence. After the addition, the mixture was stirred at 240 °C under microwave irradiation. After the reaction was completed, the reaction solution was concentrated and subjected to column chromatography (petroleum ether/ethyl acetate = 9/1) to give compound 36b (1.03 g).

MS (ESI, [M+H]⁺) *m*/*z*: 156.03.

### Step C: Preparation of compound 36c

To a reaction flask were added compound 36b (0.884 g), di-tert-butyl azidodicarboxylate (1.2 g), cesium carbonate (2.52 g), tris(dibenzylideneacetone)dipalladium(0) (0.473 g), 1,1'-bis(diphenylphosphino)ferrocene (0.573 g), and toluene (5 mL) in sequence. After the addition, the mixture was purged with nitrogen and stirred at 110 °C. After the reaction was completed, the reaction solution was concentrated and subjected to column chromatography (petroleum ether/ethyl acetate = 4/1) to give compound 36c (1.4 g).

MS (ESI, [M+H]⁺) *m*/*z*: 352.2.

### Step D: Preparation of compound 36d

To a reaction flask were added compound 36c (0.5 g), dichloromethane (5 mL), and methanesulfonic acid (0.6 g) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, and concentrated to give crude compound 36d (1.0 g). MS (ESI, [M+H]⁺) *m*/*z*: 152.0.

### Step E: Preparation of compound 36e

To a reaction flask were added compound 36d (1.0 g) and triethyl orthoformate (10 mL) in sequence. After the addition, the mixture was stirred at 80 °C. After the reaction was completed, the reaction solution was concentrated to give crude compound 36e (1.1 g).

MS (ESI, [M+H]⁺) *m*/*z*: 162.07.

### Step F: Preparation of compound 36f

To a reaction flask were added compound 36e (1.1 g), DMSO (5 mL), sodium carbonate (2.1 g), and dibromohydantoin (0.6 g) in sequence. After the addition, the mixture was stirred at room temperature. After the reaction was completed, the reaction solution was extracted with water and ethyl acetate. The organic phase was dried, filtered, concentrated, and subjected to column chromatography (dichloromethane/methanol = 20/1) to give compound 36f (0.150 g).

MS (ESI, [M+H]⁺) *m*/*z*: 240.05.

### Step G: Preparation of compound 36g

To a reaction flask were added compound 36f (0.101 g), intermediate 5A (0.25 g), 2-dicyclohexylphosphine-2',4',6'-triisopropylbiphenyl (0.02 g), chloro(2-dicyclohexylphosphino-2',4',6'-triisopropyl-1,1'-biphenyl)[2-(2'-amino-1,1'-biphenyl)]palladium (II) (0.033 g), tripotassium phosphate (0.179 g), 1,4-dioxane (5 mL), and water (1 mL) in sequence. The mixture was purged with nitrogen and stirred at 85 °C under microwave irradiation. After the reaction was completed, the reaction solution was concentrated to give a crude product, which was subjected to column chromatography (dichloromethane/methanol = 20/1) to give compound 36g (0.106 g).

MS (ESI, [M+H]⁺) *m*/*z*: 626.62.

### Step D: Preparation of compound I-36

To a reaction flask were added compound 36g (0.106 g), dichloromethane (2 mL), and a solution of hydrochloric acid in 1,4-dioxane (0.4 mL, 4 M) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 10/1) to give compound I-36 (0.008 g).

HRMS (ESI, [M+H]⁺) *m*/*z*: 526.2566.

### Example 37: Preparation of Compound I-37

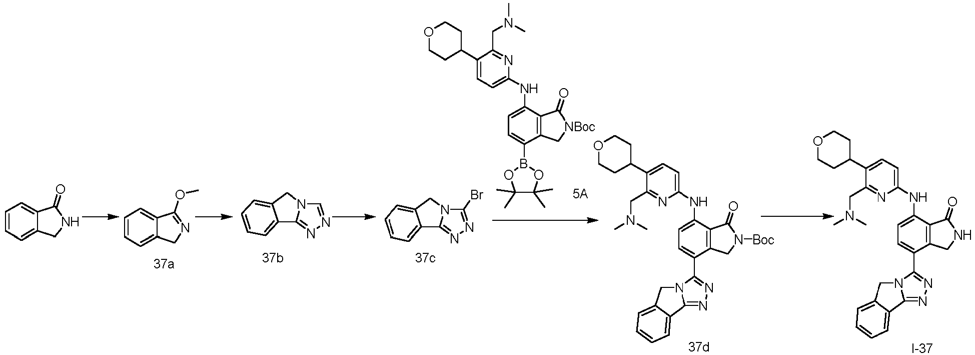

### Step A: Preparation of compound 37a

To a reaction flask were added 1 -isoindolinone (1 g), trimethyloxonium tetrafluoroborate (2 g), and dichloromethane (30 mL) in sequence under an ice-water bath. After the addition, the mixture was stirred at room temperature. After the reaction was completed, a saturated aqueous sodium bicarbonate solution was added to the reaction solution to quench the reaction, and the resulting mixture was extracted with dichloromethane. The organic phase was dried, filtered, and concentrated to give compound 37a (1.1 g).

MS (ESI, [M+H]⁺) *m*/*z*: 148.2.

### Step B: Preparation of compound 37b

To a reaction flask were added compound 37a (1.1 g), hydrazine formate (0.6 g), acetic acid (0.1 mL), and n-butanol (12 mL) in sequence. After the addition, the mixture was stirred at 120 °C under nitrogen atmosphere. After the reaction was completed, the reaction solution was concentrated and subjected to column chromatography (dichloromethane/methanol = 45/1) to give compound 37b (0.8 g).

MS (ESI, [M+H]⁺) *m*/*z*: 158.2.

### Step C: Preparation of compound 37c

To a reaction flask were added compound 37b (0.8 g), N-bromosuccinimide (0.75 g), and dichloromethane (10 mL) in sequence. After the addition, the mixture was stirred at room temperature. After the reaction was completed, the reaction solution was extracted with dichloromethane and water. The organic phase was dried, filtered, concentrated, and subjected to column chromatography (petroleum ether/ethyl acetate = 4/1) to give compound 37c (0.6 g).

MS (ESI, [M+H]⁺) *m*/*z*: 236.0.

### Step D: Preparation of compound 37d

To a reaction flask were added compound 37c (0.5 g), intermediate 5A (0.7g), potassium phosphate (0.12g), chloro(2-dicyclohexylphosphino-2',4',6'-triisopropyl-1,1'-biphenyl)[2-(2'-amino-1,1'-biphenyl)]palladium (II) (0.08g), 1,4-dioxane (10 mL), and water (0.5 mL) in sequence. After the addition, the mixture was stirred at 100 °C under nitrogen atmosphere. After the reaction was completed, the reaction solution was concentrated and subjected to column chromatography (dichloromethane/methanol = 15/1) to give compound 37d (0.11 g).

MS (ESI, [M+H]⁺) *m*/*z*: 622.7.

### Step E: Preparation of compound I-37

To a reaction flask were added compound 37d (0.11 g), a solution of hydrochloric acid in dioxane (4 M, 0.6 mL), and dichloromethane (10 mL) in sequence. After the addition, the mixture was stirred at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 9/1) to give compound I-37 (0.02 g).

HRMS (ESI, [M+H]⁺) *m*/*z*: 522.2616.

### Example 38: Preparation of Compound I-38

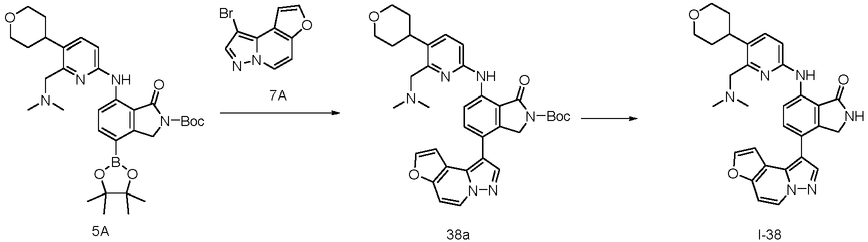

### Step A: Preparation of compound 38a

To a reaction flask were added intermediate 7A (0.4 g), intermediate 5A (0.6 g), potassium phosphate (0.18 g), chloro(2-dicyclohexylphosphino-2',4',6'-triisopropyl-1,1'-biphenyl)[2-(2'-amino-1,1'-biphenyl)]palladium (II) (0.06 g), 1,4-dioxane (10 mL), and water (0.5 mL) in sequence. After the addition, the mixture was stirred at 100 °C under nitrogen atmosphere. After the reaction was completed, the reaction solution was concentrated and subjected to column chromatography (dichloromethane/methanol = 15/1) to give compound 38a (0.21 g).

MS (ESI, [M+H]⁺) *m*/*z*: 623.4.

### Step B: Preparation of compound I-38

To a reaction flask were added compound 38a (0.21 g), a solution of hydrochloric acid in 1,4-dioxane (4 M, 0.9 mL), and dichloromethane (10 mL) in sequence. After the addition, the mixture was stirred at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 9/1) to give compound I-38 (0.10 g).

HRMS (ESI, [M+H]⁺) *m*/*z*: 523.2455.

¹H NMR (500 MHz, CDCl₃:CD₃OD=10:1) δ 8.81 (t, *J =* 6.7 Hz, 1H), 8.38 (s, 1H), 7.99 (dd, *J =* 6.3, 2.2 Hz, 1H), 7.63 (t, *J =* 7.3 Hz, 2H), 7.57 - 7.49 (m, 1H), 7.32 - 7.27 (m, 1H), 7.11 (s, 1H), 6.86 (s, 1H), 6.72 (d, J = 6.1 Hz, 1H), 4.36 (dd, *J* = 6.2, 2.2 Hz, 2H), 4.09 (p, *J* = 4.8 Hz, 2H), 3.69 (d, *J =* 5.9 Hz, 2H), 3.58 (tt, *J* = 11.3, 4.2 Hz, 2H), 3.20 - 3.14 (m, 1H), 2.39 (d, *J =* 6.0 Hz, 6H), 1.85 - 1.67 (m, 4H).

### Example 39: Preparation of Compound 1-39

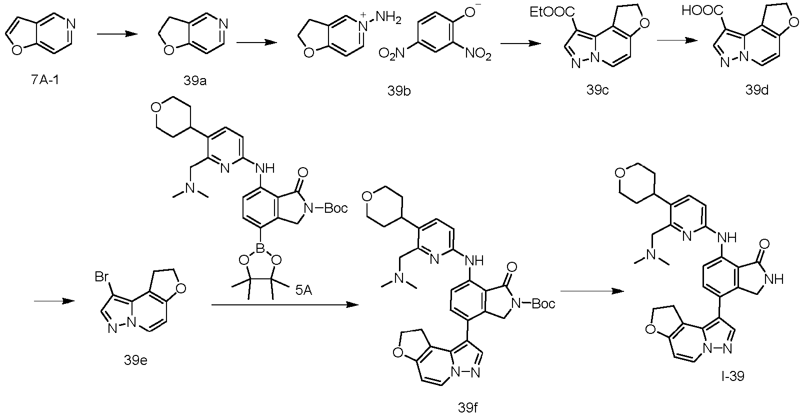

### Step A: Preparation of compound 39a

To a reaction flask were added intermediate 7A-1 (5 g), methanol (50.0 mL), and 10 wt% wet palladium on carbon (0.8 g) in sequence. After the addition, the mixture was stirred at room temperature under hydrogen atmosphere. After the reaction was completed, the reaction solution was filtered and concentrated to give compound 39a (4.2 g). MS (ESI, [M+H]⁺) *m*/*z*: 122.2.

### Step B: Preparation of compound 39b

To a reaction flask were added compound 39a (4.2 g), acetonitrile (50 mL), and 2,4-dinitrophenylhydroxylamine (8 g) in sequence. After the addition, the mixture was stirred at 40 °C under nitrogen atmosphere. After the reaction was completed, the reaction solution was filtered and washed to give compound 39b (4 g), which was directly used in the next step.

### Step C: Preparation of compound 39c

To a reaction flask were added compound 39b (4 g), potassium carbonate (3 g), ethyl propiolate (3.2 g), and DMF (60 mL) in sequence. After the addition, the mixture was stirred at room temperature under nitrogen atmosphere. After the reaction was completed, the reaction solution was extracted with ethyl acetate and water. The organic phase was dried, filtered, concentrated, and subjected to column chromatography (petroleum ether/ethyl acetate = 6/1) to give compound 39c (2 g).

MS (ESI, [M+H]⁺) *m*/*z*: 233.2.

### Step D: Preparation of compound 39d

To a reaction flask were added compound 39c (2 g), lithium hydroxide monohydrate (4 g), methanol (60 mL), and water (6 mL) in sequence. After the addition, the mixture was stirred at 60 °C under nitrogen atmosphere. After the reaction was completed, the reaction solution was adjusted to acidity (about pH 5) with diluted hydrochloric acid and extracted with ethyl acetate. The organic phase was dried, filtered, concentrated, and subjected to column chromatography (petroleum ether/ethyl acetate = 3/1) to give compound 39d (1.4 g).

MS (ESI, [M+H]⁺) *m*/*z*: 205.2.

### Step E: Preparation of compound 39e

To a reaction flask were added compound 39d (1.4 g), *N*-bromosuccinimide (1.2 g), sodium bicarbonate (1.6 g), and *N,N*-dimethylformamide (40 mL) in sequence. After the addition, the mixture was stirred at room temperature. After the reaction was completed, the reaction solution was extracted with ethyl acetate and water. The organic phase was dried, filtered, concentrated, and subjected to column chromatography (petroleum ether/ethyl acetate = 6/1) to give compound 39e (1.0 g).

MS (ESI, [M+H]⁺) *m*/*z*: 239.0.

### Step F: Preparation of compound 39f

To a reaction flask were added compound 39e (0.4 g), intermediate 5A (0.6g), potassium phosphate (0.18g), chloro(2-dicyclohexylphosphino-2',4',6'-triisopropyl-1,1'-biphenyl)[2-(2'-amino-1,1'-biphenyl)]palladium (II) (0.06g), 1,4-dioxane (10 mL), and water (0.5 mL) in sequence. After the addition, the mixture was stirred at 100 °C under nitrogen atmosphere. After the reaction was completed, the reaction solution was concentrated and subjected to column chromatography (dichloromethane/methanol = 20/1) to give compound 39f (0.15 g).

MS (ESI, [M+H]⁺) *m*/*z*: 625.8.

### Step G: Preparation of compound I-39

To a reaction flask were added compound 39f (0.15 g), a solution of hydrochloric acid in dioxane (4 M, 0.9 mL), and dichloromethane (10 mL) in sequence. After the addition, the mixture was stirred at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 9/1) to give compound I-39 (0.04 g).

HRMS (ESI, [M+H]⁺) *m*/*z*: 525.2606.

¹H NMR (500 MHz, CDCl₃:CD₃OD=10:1) δ 8.74 (dd, *J =* 8.5, 3.2 Hz, 1H), 8.29 (s, 1H), 7.88(s, 1H), 7.55 (ddd, *J* = 24.3, 8.5, 3.1 Hz, 2H), 6.90 - 6.80 (m, 2H), 4.71 (td, *J =* 8.2, 3.2 Hz, 2H), 4.42 (d, *J =* 3.1 Hz, 2H), 4.17 - 4.06 (m, 2H), 3.76 - 3.66 (m, 2H), 3.59 *(t, J=* 11.6 Hz, 2H), 3.38 (s, 1H), 3.32 *(t, J=* 8.3 Hz, 2H), 3.17 (ddq, *J* = 11.7, 7.9, 4.0 Hz, 1H), 2.39 (d, *J =* 3.1 Hz, 6H), 1.85 - 1.69 (m, 4H).

### Example 40: Preparation of Compound I-40

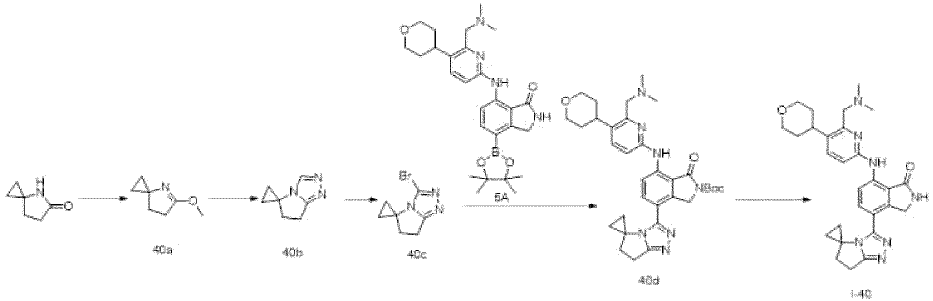

### Step A: Preparation of compound 40a

To a reaction flask were added the compounds 4-azaspiro[2.4]heptan-5-one (1.0 g), trimethyloxonium tetrafluoroborate (7.2 g), and dichloromethane (20.0 g) in sequence. After the addition, the mixture was stirred at room temperature. After the reaction was completed, the reaction solution was extracted with dichloromethane and water. The organic phase was dried, filtered, concentrated, and subjected to column chromatography (dichloromethane/methanol = 30/1) to give compound 40a (1.2 g).

MS (ESI, [M+H]⁺) *m*/*z*: 126.3.

### Step B: Preparation of compound 40b

To a reaction flask were added compound 40a (1.2 g), acetic acid (0.3 mL), 5-amino-1*H*-pyrazole (1.2 g), formylhydrazine (0.5 mL), and n-butanol (20 mL) in sequence. After the addition, the mixture was stirred at 120 °C. After the reaction was completed, the reaction solution was filtered, concentrated, and subjected to column chromatography (petroleum ether/ethyl acetate = 5/1) to give compound 40b (0.3 g).

MS (ESI, [M+H]⁺) *m*/*z*: 136.1.

### Step C: Preparation of compound 40c

To a reaction flask were added compound 40b (0.3 g), N-bromosuccinimide (0.3 g), deuterated chloroform (10.0 mL), and sodium carbonate (0.1 g) in sequence at 0 °C. After the addition, the mixture was stirred at 0 °C. After the reaction was completed, the reaction solution was filtered, concentrated, and subjected to column chromatography (petroleum ether/ethyl acetate = 5/1) to give compound 40c (0.2 g).

MS (ESI, [M+H]⁺) *m*/*z*: 214.2.

### Step D: Preparation of compound 40d

To a reaction flask were added compound 40c (0.2 g), 2-dicyclohexylphosphine-2',4',6'-triisopropylbiphenyl (0.04 g), potassium phosphate (0.3 g), chloro(2-dicyclohexylphosphino-2',4',6'-trilsopropyl-1,1'-biphenyl)[2-(2'-amino-1,1'-biphenyl)]palladium (II) (0.07 g), intermediate 5A (0.4 g), water (3.0 mL), and 1,4-dioxane (12.0 mL) in sequence. After the addition, the mixture was stirred at 85 °C. After the reaction was completed, the reaction solution was filtered, concentrated, and subjected to column chromatography (petroleum ether/ethyl acetate = 5/1) to give compound 40d (0.1 g).

MS (ESI, [M+H]⁺) *m*/*z*: 600.6.

### Step H: Preparation of compound I-40

To a reaction flask were added compound 40d (0.1 g), a solution of hydrochloric acid in dioxane (4 M, 0.9 mL), and dichloromethane (10 mL) in sequence. After the addition, the mixture was stirred at room temperature. After the reaction was completed, the reaction solution was adjusted to alkalinity (about pH 9) with a saturated aqueous sodium bicarbonate solution. The organic phase was dried, filtered, concentrated, and subjected to column chromatography (dichloromethane/methanol = 5/1) to give compound I-40 (0.05 g).

HRMS (ESI, [M+H]⁺) *m*/*z*: 500.2767.

¹H NMR (500 MHz, DMSO-*d*₆) δ 10.00 (s, 1H), 8.78 (s, 1H), 8.63 (d, *J =* 8.6 Hz, 1H), 7.66 (d, *J =* 8.4 Hz, 1H), 7.45 (d, *J =* 8.5 Hz, 1H), 6.93 (d, *J =* 8.4 Hz, 1H), 4.33 (s, 2H), 3.96 (dd, *J =* 10.5, 3.9 Hz, 2H), 3.58 (s, 2H), 3.43 (td*, J =* 11.4, 2.6 Hz, 2H), 3.19 (s, 1H), 3.02 (t, *J =* 7.5 Hz, 2H), 2.70 (t, *J =* 7.5 Hz, 2H), 2.22 (s, 6H), 1.66 (dt, *J =* 24.1, 8.1 Hz, 4H), 1.07 (d, *J =* 6.3 Hz, 2H), 0.92 (d, *J =* 6.3 Hz, 2H).

### Example 41: Preparation of Compound I-41

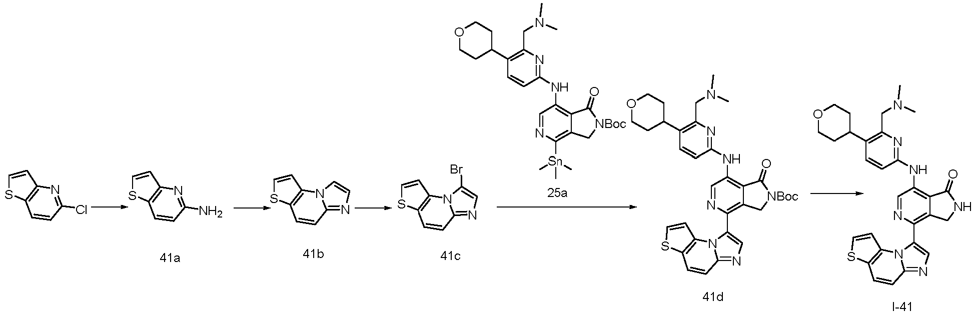

### Step A: Preparation of compound 41a

To a reaction flask were added the compounds 5-chlorothieno[3,2-b]pyridine (2.5 g), toluene (150.0 mL), benzophenone imine (4.5 g), sodium *tert*-butoxide (2.9 g), tris(dibenzylideneadone) (0.8 g), 1,1-binaphthyl-2,2-bis-diphenylphosphine (0.9 g) in sequence. After the addition, the mixture was stirred at 110 °C under nitrogen atmosphere. After the reaction was completed, concentrated hydrochloric acid was added to the reaction solution, and the resulting mixture was stirred for 1 h. After the reaction was completed, the reaction solution was adjusted to alkalinity (about pH 9) with a saturated aqueous sodium carbonate solution and extracted with dichloromethane. The organic phase was dried, filtered, concentrated, and subjected to column chromatography (dichloromethane/methanol = 30/1) to give compound 41a (1.3 g).

MS (ESI, [M+H]⁺) *m*/*z*: 151.2.

### Step B: Preparation of compound 41b

To a reaction flask were added compound 41a (1.3 g), potassium carbonate (4.0 g), chloroacetaldehyde (2.9 g), and ethanol (25 mL) in sequence. After the addition, the mixture was stirred at 120 °C. After the reaction was completed, the reaction solution was filtered, concentrated, and subjected to column chromatography (petroleum ether/ethyl acetate = 5/1) to give compound 41b (1.2 g).

MS (ESI, [M+H]⁺) *m*/*z*: 175.2.

### Step C: Preparation of compound 41c

To a reaction flask were added compound 41b (1.2 g), N-bromosuccinimide (1.3 g), and acetonitrile (20.0 mL) in sequence. After the addition, the mixture was stirred at room temperature. After the reaction was completed, the reaction solution was filtered, concentrated, and subjected to column chromatography (petroleum ether/ethyl acetate = 5/1) to give compound 41c (0.8 g).

MS (ESI, [M+H]⁺) *m*/*z*: 253.1.

### Step D: Preparation of compound 41d

To a reaction flask were added compound 41c (0.2 g), compound 25a (0.5 g), tetrakis(triphenylphosphine)palladium (0.2 g), copper(I) iodide (0.07 g), and toluene (12.0 mL) in sequence. After the addition, the mixture was purged with nitrogen and stirred at 110 °C. After the reaction was completed, the reaction solution was filtered, concentrated, and subjected to column chromatography (petroleum ether/ethyl acetate = 5/1) to give compound 41d (0.2 g).

MS (ESI, [M+H]⁺) *m*/*z*: 640.4.

### Step H: Preparation of compound I-41

To a reaction flask were added compound 40d (0.2 g), a solution of hydrochloric acid in dioxane (4 M, 0.9 mL), and dichloromethane (10 mL) in sequence. After the addition, the mixture was stirred at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 5/1) to give compound I-41 (0.05 g).

HRMS (ESI, [M+H]⁺) *m*/*z*: 540.2182.

¹H NMR (500 MHz, CDCl₃) δ 10.36 (s, 1H), 8.08 (dd, *J =* 5.7, 2.5 Hz, 1H),7.82 (dd, *J =* 9.5, 2.5 Hz, 1H), 7.72 (dd, *J =* 9.6, 2.5 Hz, 1H), 7.62 (ddd, *J =* 16.8, 7.1, 2.5 Hz, 2H), 7.32 (d, *J =* 2.5 Hz, 2H), 6.91 (d, *J =* 8.4 Hz, 1H), 4.88 (d, *J* = 2.4 Hz, 2H), 4.18 - 4.04 (m, 2H), 3.69 (s, 2H), 3.59 (td, *J* = 11.5, 5.6 Hz, 2H), 3.39 (dd, *J* = 3.4,1.9 Hz, 1H), 2.35 (d, *J* = 2.4 Hz, 6H), 1.81 (dd, *J* = 12.5, 3.6 Hz, 2H), 1.76 (d, *J* = 11.1Hz, 2H).

### Example 42: Preparation of Compound I-42

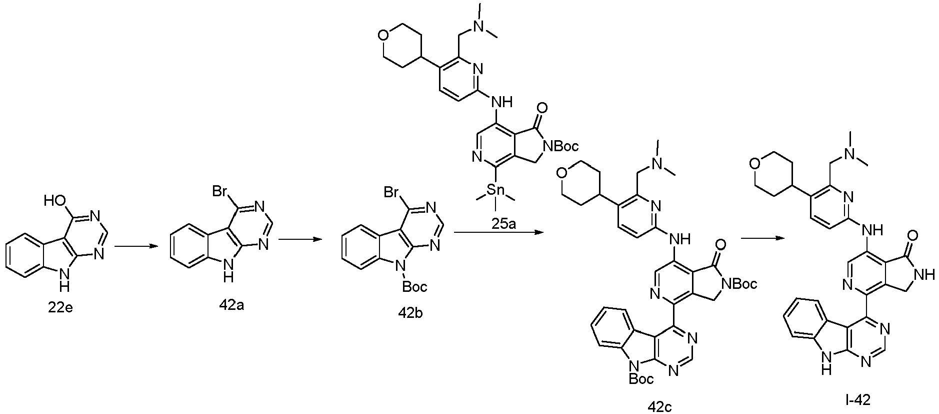

### Step A: Preparation of compound 42a

To a reaction flask were added compound 22e (1.2 g), phosphorus oxybromide (7.7 g), and toluene (50.0 mL) in sequence. After the addition, the mixture was stirred at room temperature. After the reaction was completed, the reaction solution was extracted with dichloromethane and water. The organic phase was dried, filtered, concentrated, and subjected to column chromatography (dichloromethane/methanol = 30/1) to give compound 42a (1.3 g).

MS (ESI, [M+H]⁺) *m*/*z*: 248.2.

### Step B: Preparation of compound 42b

To a reaction flask were added compound 42a (1.2 g), tetrahydrofuran (30 mL), *N,N*-diisopropylethylamine (2 g), di-tert-butyl dicarbonate (1.3 g), and 4-dimethylaminopyridine (0.05 g) in sequence. After the addition, the mixture was stirred at room temperature. After the reaction was completed, the reaction solution was concentrated and subjected to column chromatography (petroleum ether/ethyl acetate = 5/1) to give compound 42b (0.6 g).

MS (ESI, [M+H]⁺) *m*/*z:* 348.2.

### Step C: Preparation of compound 42c

To a reaction flask were added compound 42b (0.6 g), compound 25a (0.7 g), tetrakis(triphenylphosphine) palladium (0.2 g), copper(I) iodide (0.07 g), and toluene (12.0 mL) in sequence. After the addition, the mixture was stirred at 110 °C. After the reaction was completed, the reaction solution was filtered, concentrated, and subjected to column chromatography (petroleum ether/ethyl acetate = 5/1) to give compound 42c (0.3 g).

MS (ESI, [M+H]⁺) *m*/*z:* 735.7.

### Step H: Preparation of compound I-42

To a reaction flask were added compound 42c (0.1 g), a solution of hydrochloric acid in dioxane (4 M, 0.9 mL), and dichloromethane (10 mL) in sequence. After the addition, the mixture was stirred at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 5/1) to give compound I-42 (0.007 g).

HRMS (ESI, [M+H]⁺) *m*/*z:* 535.2570.

### Example 43: Preparation of Compound I-43

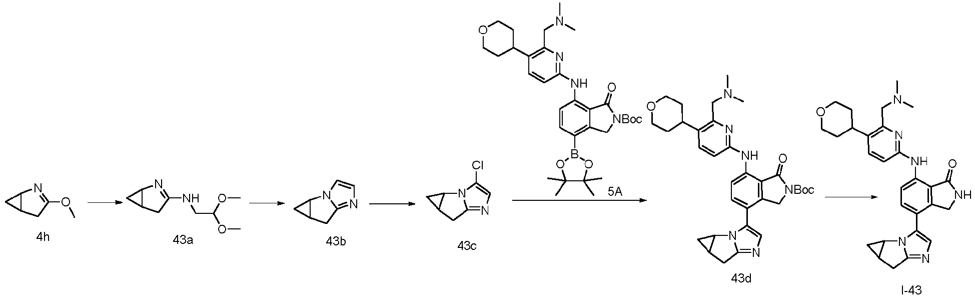

### Step A: Preparation of compound 43a

To a reaction flask were added compound 4h (3.5 g), 2,2-dimethoxyethylamine (4.9 g), and toluene (80 mL) in sequence. After the addition, the mixture was stirred at 120 °C. After the reaction was completed, the reaction solution was extracted with dichloromethane and water. The organic phase was dried, filtered, concentrated, and subjected to column chromatography (dichloromethane/methanol = 20/1) to give compound 43a (3.9 g).

MS (ESI, [M+H]⁺) m/z: 185.2.

### Step B: Preparation of compound 43b

To a reaction flask were added compound 43a (3.9 g), concentrated hydrochloric acid (15 mL), and water (10 mL) in sequence. After the addition, the mixture was stirred at 100 °C. After the reaction was completed, the reaction solution was adjusted to alkalinity (about pH 9) with a saturated sodium carbonate solution and extracted with dichloromethane. The organic phase was dried, filtered, concentrated, and subjected to column chromatography (dichloromethane/methanol = 20/1) to give compound 43b (3.3 g).

MS (ESI, [M+H]⁺) m/z: 121.2.

### Step C: Preparation of compound 43c

To a reaction flask were added compound 43b (3.3 g), dichloromethane (50 mL), and NCS (9.7 g) in sequence. After the addition, the mixture was stirred at room temperature. After the reaction was completed, a saturated sodium thiosulfate solution was added to the reaction solution to quench the reaction, and the resulting mixture was extracted with dichloromethane. The organic phase was dried, filtered, concentrated, and subjected to column chromatography (dichloromethane/methanol = 20/1) to give compound 43c (3.0 g).

MS (ESI, [M+H]⁺) m/z: 155.0.

### Step D: Preparation of compound 43d

To a reaction flask were added compound 43c (0.11 g), intermediate 5A (0.17 g), 2-dicyclohexylphosphine-2',4',6'-triisopropylbiphenyl (0.02 g), chloro(2-dicyclohexylphosphino-2',4',6'-triisopropyl-1,1'-biphenyl)[2-(2'-amino-1,1'-biphenyl)]palladium (II) (0.03 g), potassium phosphate (0.09 g), water (2.00 mL), and 1,4-dioxane (15 mL) in sequence. After the addition, the mixture was stirred at 85 °C under nitrogen atmosphere. After the reaction was completed, the reaction solution was concentrated and subjected to column chromatography (dichloromethane/methanol = 10/1) to give compound 43d (0.12 g).

MS (ESI, [M+H]⁺) *m*/*z:* 585.4.

### Step E: Preparation of compound I-43

To a reaction flask were added compound 43d (0.12 g), dichloromethane (10 mL), and a solution of hydrochloric acid in 1,4-dioxane (2 mL, 4 M) in sequence. The mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 10/1) to give compound I-43 (30 mg).

HRMS (ESI, [M+H]⁺) *m*/*z*: 485.2662.

### Example 44: Preparation of Compound I-44

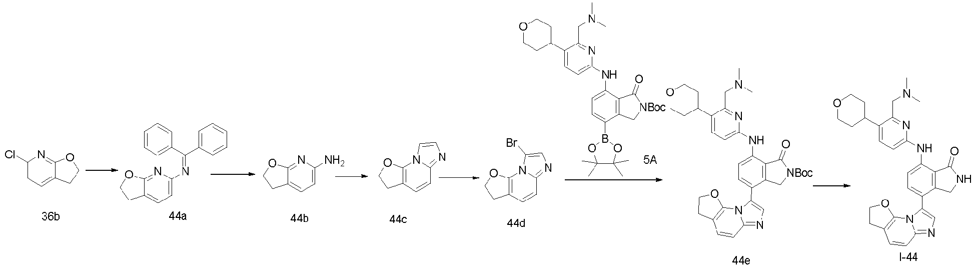

### Step A: Preparation of compound 44a

To a reaction flask were added sodium *tert*-butoxide (0.556 g), 1,1'-binaphthyl-2,2'-bisdiphenylphosphine (0.288 g), tris(dibenzylideneacetone)dipalladium(0) (0.318 g), benzophenone imine (0.629 g), compound 36b (0.36 g), and toluene (10 mL) in sequence. After the addition, the mixture was purged with nitrogen and stirred at 80 °C. After the reaction was completed, the reaction solution was filtered, concentrated, and subjected to column chromatography (petroleum ether/ethyl acetate = 1/4) to give compound 44a (0.5 g).

MS (ESI, [M+H]⁺) *m*/*z*: 301.35.

### Step B: Preparation of compound 44b

To a reaction flask were added compound 44a (0.5 g), dichloromethane (5 mL), and a solution of hydrochloric acid in 1,4-dioxane (2 mL, 4 M) in sequence. After the addition, the mixture was stirred at room temperature. After the reaction was completed, the reaction solution was adjusted to alkalinity (about pH 8) with a saturated sodium bicarbonate solution and extracted with dichloromethane. The organic phase was dried, filtered, concentrated, and subjected to column chromatography (dichloromethane/methanol = 10/1) to give compound 44b (0.15 g).

MS (ESI, [M+H]⁺) *m*/*z*: 137.00.

### Step C: Preparation of compound 44c

To a reaction flask were added compound 44b (0.15 g), ethanol (5 mL), potassium carbonate (0.305 g), and chloroacetaldehyde (0.324 g) in sequence. After the addition, the mixture was stirred at 90 °C. After the reaction was completed, the reaction solution was concentrated and subjected to column chromatography (dichloromethane/methanol = 20/1) to give compound 44c (0.12 g).

MS (ESI, [M+H]⁺) *m*/*z*: 161.01.

### Step D: Preparation of compound 44d

To a reaction flask were added compound 44c (0.12 g), DMSO (3 mL), sodium carbonate (0. 11g), and dibromohydantoin (0.075 g) in sequence. After the addition, the mixture was stirred at room temperature. After the reaction was completed, the reaction solution was extracted with water and ethyl acetate. The organic phase was dried, filtered, concentrated, and subjected to column chromatography (dichloromethane/methanol = 20/1) to give compound 44d (0.105 g).

MS (ESI, [M+H]⁺) *m*/*z*: 239.07.

### Step E: Preparation of compound 44e

To a reaction flask were added compound 44d (0.101 g), intermediate 5A (0.25 g), 2-dicyclohexylphosphine-2',4',6'-triisopropylbiphenyl (0.02 g), chloro(2-dicyclohexylphosphino-2',4',6'-triisopropyl-1,1'-biphenyl)[2-(2'-amino-1,1'-biphenyl)]palladium (II) (0.033 g), tripotassium phosphate (0.179 g), 1,4-dioxane (5 mL), and water (1 mL) in sequence. After the addition, the mixture was purged with nitrogen and stirred at 85 °C under microwave irradiation. After the reaction was completed, the reaction solution was filtered and concentrated to give a crude product, which was subjected to column chromatography (dichloromethane/methanol = 20/1) to give compound 44e (0.101 g).

MS (ESI, [M+H]⁺) *m*/*z*: 625.47.

### Step F: Preparation of compound I-44

To a reaction flask were added compound 44e (0.101 g), dichloromethane (2 mL), and a solution of hydrochloric acid in 1,4-dioxane (0.4 mL, 4 M) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 5/1) to give compound I-44 (0.012 g).

HRMS (ESI, [M+H]⁺) *m*/*z*: 525.2630.

¹H NMR (500 MHz, CDCl₃) δ 8.65 (d, *J =* 8.4 Hz, 1H), 7.46 (d, *J =* 8.5 Hz, 1H), 7.41 (dd, *J =* 5.9, 2.1 Hz, 2H), 7.26 *(d, J=* 2.1 Hz, 1H), 7.16 (dd, *J* = 24.5, 8.7 Hz, 2H), 6.79 *(d, J=* 8.4 Hz, 1H), 4.66 *(t, J=* 8.8 Hz, 2H), 4.22 (s, 2H), 4.02 (d, *J =* 8.7 Hz, 2H), 3.59 (s, 2H), 3.50 (t, *J =* 11.6 Hz, 2H), 3.23 (t, *J =* 8.8 Hz, 2H), 3.09 (t, *J =* 11.4 Hz, 1H), 2.29 (s, 6H), 1.74 - 1.62 (m, 4H).

### Example 45: Preparation of Compound I-45

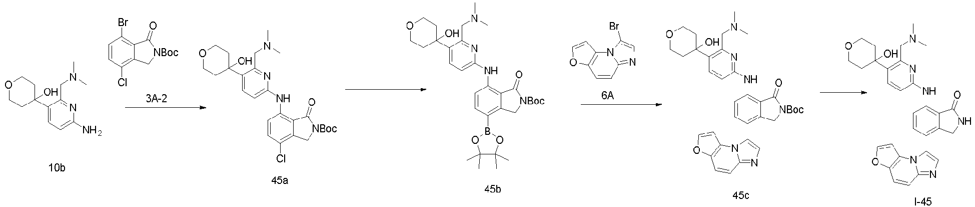

### Step A: Preparation of compound 45a

To a reaction flask were added intermediate 3A-2 (1.3 g), compound 10b (1.1 g), 1,4-dioxane (50.0 mL), 4,5-bis-diphenylphosphino-9,9-dimethylxanthene (0.4 g), cesium carbonate (2.2 g), and tris(dibenzylideneadone) (0.4 g) in sequence. After the addition, the mixture was stirred at 110 °C under nitrogen atmosphere. After the reaction was completed, the reaction solution was filtered, concentrated, and subjected to column chromatography (dichloromethane/methanol = 30/1) to give compound 45a (0.7 g).

MS (ESI, [M+H]⁺) m/z: 517.3.

### Step B: Preparation of compound 45b

To a reaction flask were added compound 45a (0.7 g), potassium acetate (0.4 g), bis(pinacolato)diboron (0.6 g), chloro(2-dicyclohexylphosphino-2',4',6'-triisopropyl-1,1'-biphenyl)[2-(2'-amino-1,1'-biphenyl)]palladium (II) (0.2 g), and 1,4-dioxane (20.0 mL) in sequence. After the addition, the mixture was purged with nitrogen and stirred at 90 °C. After the reaction was completed, the reaction solution was filtered, concentrated, and subjected to column chromatography (petroleum ether/ethyl acetate = 5/1) to give compound 45b (0.4 g).

MS (ESI, [M+H]⁺) *m*/*z*: 609.4.

### Step C: Preparation of compound 45c

To a reaction flask were added compound 45b (0.4 g), 2-dicyclohexylphosphine-2',4',6'-triisopropylbiphenyl (0.05 g), potassium phosphate (0.3 g), chloro(2-dicyclohexylphosphino-2',4',6'-triisopropyl-1,1'-biphenyl)[2-(2'-amino-1,1'-biphenyl)]palladium (II) (0.07 g), intermediate 6A (0.3 g), water (3.0 mL), and 1,4-dioxane (12.0 mL) in sequence. After the addition, the mixture was purged with nitrogen and stirred at 85 °C. After the reaction was completed, the reaction solution was filtered, concentrated, and subjected to column chromatography (petroleum ether/ethyl acetate = 5/1) to give compound 45c (0.2 g).

MS (ESI, [M+H]⁺) *m*/*z*: 639.4.

### Step D: Preparation of compound I-45

To a reaction flask were added compound 45c (0.1 g), dichloromethane (2 mL), and a solution of hydrochloric acid in 1,4-dioxane (0.4 mL, 4 M) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 10/1) to give compound I-45 (0.05 g).

HRMS (ESI, [M+H]⁺) *m*/*z*: 539.2407.

¹H NMR (500 MHz, CDCl₃) δ 9.90 (s, 1H), 8.90 (d, *J =* 8.5 Hz, 1H), 7.67 (s, 1H), 7.62 (t, *J =* 8.1 Hz, 2H), 7.59 - 7.52 (m, 3H), 6.89 (d, *J =* 8.5 Hz, 1H), 6.49 (s, 1H), 6.28 (d, *J =* 2.2 Hz, 1H), 4.91 - 4.49 (m, 1H), 4.25 (s, 2H), 4.05 (t, *J =* 11.5 Hz, 2H), 3.95 (s, 2H), 3.87 (dd, *J =* 11.1, 4.7 Hz, 2H), 2.33 (s, 6H), 2.08 (td, *J =* 12.7, 4.9 Hz, 3H).

### Example 46: Preparation of Compound I-46

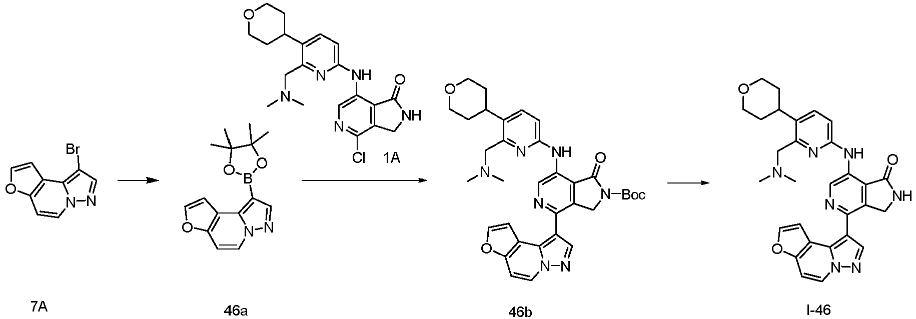

### Step A: Preparation of compound 46a

To a reaction flask were added intermediate 7A (1 g), bis(pinacolato)diboron (1.6 g), potassium carbonate (0.8 g), palladium acetate (0.1 g), tricyclohexylphosphine (0.18 g), diethylene glycol dimethyl ether (15 mL), and water (1 mL) in sequence. After the addition, the mixture was stirred at 100 °C under nitrogen atmosphere. After the reaction was completed, the reaction solution was concentrated and subjected to column chromatography (petroleum ether/ethyl acetate = 20/1) to give compound 46a (0.8 g).

MS (ESI, [M+H]⁺) *m*/*z:* 285.3.

### Step B: Preparation of compound 46b

To a reaction flask were added compound 46a (0.1 g), intermediate 1A (0.2 g), potassium phosphate (0.2 g), chloro(2-dicyclohexylphosphino-2',4',6'-triisopropyl-1,1'-biphenyl)[2-(2'-amino-1,1'-biphenyl)]palladium (II) (0.03 g), 1,4-dioxane (10 mL), and water (0.5 mL) in sequence. After the addition, the mixture was stirred at 100 °C under nitrogen atmosphere. After the reaction was completed, the reaction solution was concentrated and subjected to column chromatography (dichloromethane/methanol = 20/1) to give compound 46b (0.12 g).

MS (ESI, [M+H]⁺) *m*/*z*: 624.7.

### Step C: Preparation of compound 1-46

To a reaction flask were added compound 46b (0.12 g), a solution of hydrochloric acid in 1,4-dioxane (4 M, 9 mL), and dichloromethane (10 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 9/1) to give compound I-46 (0.03 g).

HRMS (ESI, [M+H]⁺) *m*/*z*: 524.2407.

¹H NMR(500 MHz, CDCl₃:CD₃OD=10:1) δ 8.32 (dd, *J* = 11.4, 7.2 Hz, 1H), 8.07 (d*, J =* 11.1 Hz, 1H), 7.64 (d*, J* = 10.1 Hz, 1H), 7.48 (s, 2H), 7.11 (dd, *J* = 11.4, 7.2 Hz, 1H), 6.83 - 6.77 (m, 1H), 4.57 (d, *J =* 10.8 Hz, 2H), 4.02 (dt, *J =* 11.5, 5.6 Hz, 2H), 3.64 (d, *J =* 9.9 Hz, 2H), 3.51 (q, *J =* 11.1 Hz, 2H), 3.29 (d, *J =* 10.1 Hz, 1H), 3.10 (dd, *J* = 10.2, 5.8 Hz, 1H), 2.33 (d, *J =* 10.3 Hz, 6H), 1.76 - 1.61 (m, 4H).

### Example 47: Preparation of Compound I-47

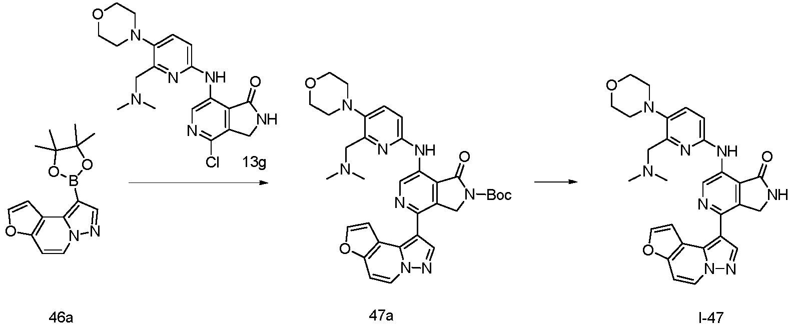

### Step A: Preparation of compound 47a

To a reaction flask were added compound 46a (0.1 g), compound 13g (0.2 g), potassium phosphate (0.2 g), chloro(2-dicyclohexylphosphino-2',4',6'-triisopropyl-1,1'-biphenyl)[2-(2'-atnino-1,1'-biphenyl)]palladium (II) (0.03 g), 1,4-dioxane (10 mL), and water (0.5 mL) in sequence. After the addition, the mixture was stirred at 100 °C under nitrogen atmosphere. After the reaction was completed, the reaction solution was concentrated and subjected to column chromatography (dichloromethane/methanol = 15:1) to give compound 47a (0.1 g).

MS (ESI, [M+H]⁺) *m*/*z*: 625.7.

### Step B: Preparation of compound I-47

To a reaction flask were added compound 47a (0.1 g), a solution of hydrochloric acid in 1,4-dioxane (4 M, 1.9 mL), and dichloromethane (10 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 9/1) to give compound I-47 (0.02 g).

HRMS (ESI, [M+H]⁺) *m*/*z:* 525.2359.

¹H NMR (500 MHz, CDCl₃:CD₃OD=10:1) δ 10.03(s, 1H), 8.32 (d, J = 7.3 Hz, 1H), 7.65 (s, 1H), 7.52 (s, 1H), 7.44 (d, J = 8.6 Hz, 1H), 7.12 (d, J = 7.3 Hz, 1H), 6.82 (d, J = 8.5 Hz, 1H), 4.58 (s, 1H), 4.40(s, 2H), 3.88 - 3.71 (m, 6H), 2.91 - 2.76 (m, 4H), 2.50 - 2.33 (m, 6H).

### Example 48: Preparation of Compound I-48

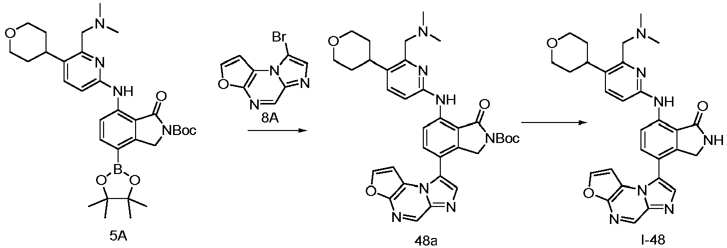

### Step A: Preparation of compound 48a

To a microwave tube were added intermediate 5A (205 mg), intermediate 8A (91 mg), chloro(2-dicyclohexylphosphino-2',4',6'-triisopropyl-1,1'-biphenyl)[2-(2'-atnino-1,1'-biphenyl)]palladium (II) (27 mg), 2-dicyclohexylphosphine-2',4',6'-triisopropylbiphenyl (17 mg), tripotassium phosphate (147 mg), 1,4-dioxane (3 mL), and water (0.5 mL) in sequence. After the addition, the mixture was stirred at 85 °C under microwave irradiation under nitrogen atmosphere. After the reaction was completed, the reaction solution was filtered, concentrated, and subjected to column chromatography (dichloromethane/methanol = 20/1) to give compound 48a (72 mg).

MS (ESI, [M+H]+) m/z: 624.61.

### Step B: Preparation of compound I-48

To a reaction flask were added compound 48a (72 mg), a solution of hydrochloric acid in 1,4-dioxane (4 M, 1.9 mL), and dichloromethane (10 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 10/1) to give compound I-48 (35 mg).

HRMS (ESI, [M+H]⁺) *m*/*z*: 524.2398.

¹H NMR (500 MHz, CDCl₃) δ 9.86 (s, 1H), 9.01 (d, J = 8.6 Hz, 1H), 8.83 (s, 1H), 7.87 (s, 1H), 7.65 - 7.59 (m, 2H), 7.55 (d, J = 8.4 Hz, 1H), 6.86 (d, J = 8.4 Hz, 1H), 6.47 (d, J = 2.4 Hz, 1H), 6.41 (s, 1H), 4.30 (s, 2H), 4.10 (dd, J = 11.3, 4.1 Hz, 2H), 3.63 (s, 2H), 3.58 - 3.53 (m, 2H), 3.25 - 3.19 (m, 1H), 2.32 (s, 6H), 1.84 - 1.72 (m, 4H).

### Example 49: Preparation of Compound I-49

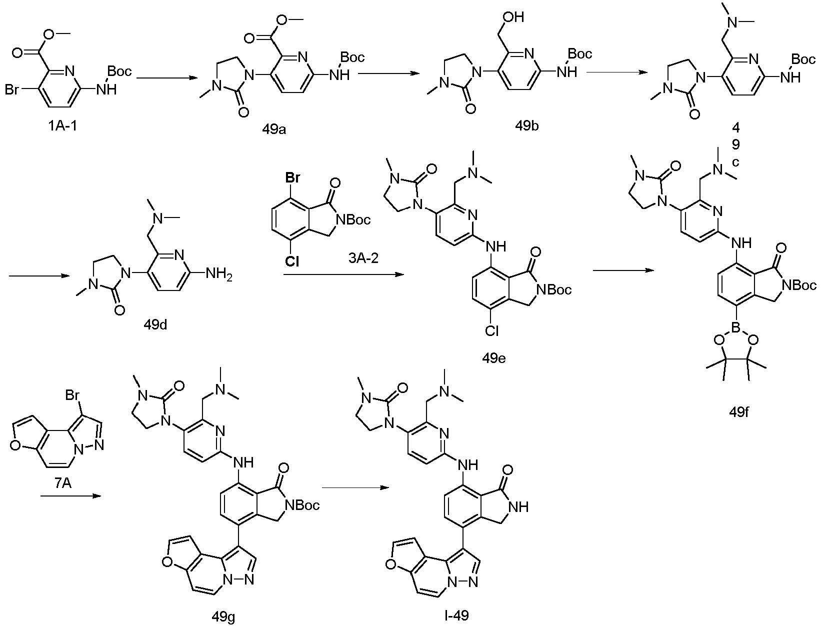

### Step A: Preparation of compound 49a

To a reaction flask were added intermediate 1A-1 (6.0 g), 1,4-dioxane (50.0 mL), 4,5-bis-diphenylphosphino-9,9-dimethylxanthene (1.6 g), cesium carbonate (11.81 g), tris(dibenzylideneadone) (1.6 g), and 1-methyl-2-imidazolidinone (2.2 g) in sequence. After the addition, the mixture was purged with nitrogen and stirred at 110 °C. After the reaction was completed, the reaction solution was filtered, concentrated, and subjected to column chromatography (dichloromethane/methanol = 30/1) to give compound 49a (4.3 g).

MS (ESI, [M+H]⁺) *m*/*z*: 351.1.

### Step B: Preparation of compound 49b

To a reaction flask were added compound 49a (4.3 g), tetrahydrofuran (50.0 mL), and lithium borohydride (0.5 g) in sequence. After the addition, the mixture was stirred at room temperature. After the reaction was completed, a saturated aqueous ammonium chloride solution was added to the reaction solution to quench the reaction, and the resulting mixture was extracted with dichloromethane. The organic phase was dried, filtered, concentrated, and subjected to column chromatography (petroleum ether/ethyl acetate = 5/1) to give compound 49b (2.1 g).

MS (ESI, [M+H]⁺) *m*/*z*: 323.2.

### Step C: Preparation of compound 49c

To a reaction flask were added compound 49b (2.1 g), dichloromethane (30.0 mL), *N,N*-diisopropylethylamine (3.45 mL), and methanesulfonic anhydride (4.5 mL) in sequence under an ice-water bath. After the addition, the mixture was reacted under an ice-water bath. After the reaction was completed, a solution of dimethylamine in tetrahydrofuran (2 M, 30 mL). After the addition, the mixture was reacted at room temperature. After the reaction was completed, the reaction solution was concentrated and subjected to column chromatography (petroleum ether/ethyl acetate = 5/1) to give compound 49c (1.5 g).

MS (ESI, [M+H]⁺) *m*/*z*: 350.0.

### Step D: Preparation of compound 49d

To a reaction flask were added compound 49c (1.5 g), dichloromethane (30.0 mL), and a solution of hydrochloric acid in 1,4-dioxane (4 M, 11 mL) in sequence. After the addition, the mixture was stirred at room temperature. The reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 5/1) to give compound 49d (1.0 g).

MS (ESI, [M+H]⁺) *m*/*z*: 250.0.

### Step E: Preparation of compound 49e

To a reaction flask were added intermediate 3A-2 (3.0 g), compound 49d (1.0 g), 1,4-dioxane (50.0 mL), 4,5-bis-diphenylphosphino-9,9-dimethylxanthene (0.9 g), cesium carbonate (6.6 g), and tris(dibenzylideneadone) (1.2 g) in sequence. After the addition, the mixture was stirred at 110 °C. After the reaction was completed, the reaction solution was filtered, concentrated, and subjected to column chromatography (dichloromethane/methanol = 30/1) to give compound 49e (0.7 g).

MS (ESI, [M+H]⁺) *m*/*z*: 515.0.

### Step F: Preparation of compound 49f

To a reaction flask were added compound 49e (0.7 g), potassium acetate (0.8 g), bis(pinacolato)diboron (0.5 g), chloro(2-dicyclohexylphosphino-2',4',6'-triisopropyl-1,1'-biphenyl)[2-(2'-amino-1,1'-biphenyl)]palladium (II) (0.1 g), and 1,4-dioxane (30.0 mL) in sequence. After the addition, the mixture was purged with nitrogen and stirred at 90 °C. After the reaction was completed, the reaction solution was filtered, concentrated, and subjected to column chromatography (petroleum ether/ethyl acetate = 5/1) to give compound 49f (0.7 g).

MS (ESI, [M+H]⁺) *m*/*z*: 607.4.

### Step G: Preparation of compound 49g

To a reaction flask were added compound 49f (0.3 g), 2-dicyclohexylphosphine-2',4',6'-triisopropylbiphenyl (0.07 g), potassium phosphate (0.5 g), chloro(2-dicyclohexylphosphino-2',4',6'-triisopropyl-1,1'-biphenyl)[2-(2'-amino-1,1'-biphenyl)]palladium (II) (0.1 g), intermediate 7A (0.4 g), water (3.0 mL), and 1,4-dioxane (12.0 mL) in sequence. After the addition, the mixture was stirred at 85 °C. After the reaction was completed, the reaction solution was filtered, concentrated, and subjected to column chromatography (petroleum ether/ethyl acetate = 5/1) to give compound 49g (0.2 g).

MS (ESI, [M+H]⁺) *m*/*z*: 637.4.

### Step H: Preparation of compound I-49

To a reaction flask were added compound 49g (0.2 g), a solution of hydrochloric acid in 1,4-dioxane (4 M, 1.9 mL), and dichloromethane (10 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 5/1) to give compound I-49 (0.07 g).

HRMS (ESI, [M+H]⁺) *m*/*z*: 537.2365.

¹H NMR (500 MHz, CDCl₃) δ 8.85 (dd, *J =* 8.6, 3.0 Hz, 1H), 8.39 (d, *J =* 7.5 Hz, 1H), 8.00 (d, *J =* 3.5 Hz, 1H), 7.69 - 7.62 (m, 2H), 7.44 (d, *J =* 8.6 Hz, 1H), 7.12 (d, *J* = 7.5 Hz, 1H), 6.88 (d, *J* = 8.6 Hz, 1H), 6.74 *(d, J=* 2.1 Hz, 1H), 4.37 (s, 2H), 3.77 (dd, *J =* 9.1, 6.7 Hz, 2H), 3.70 (s, 2H), 3.53 (dd, *J =* 9.1, 6.7 Hz, 2H), 2.90 (s, 3H), 2.42 (s, 6H).

### Example 50: Preparation of Compound I-50

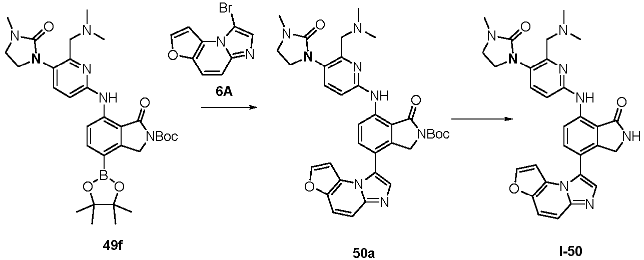

### Step A: Preparation of compound 50a

To a reaction flask were added compound 49f (0.3 g), 2-dicyclohexylphosphine-2',4',6'-triisopropylbiphenyl (0.07 g), potassium phosphate (1.4 g), chloro(2-dicyclohexylphosphino-2',4',6'-trilsopropyl-1,1'-biphenyl)[2-(2'-amino-1,1'-biphenyl)]palladium (II) (0.1 g), intermediate 6A (0.1 g), water (3.0 mL), and 1,4-dioxane (12.0 mL) in sequence. After the addition, the mixture was stirred at 85 °C. After the reaction was completed, the reaction solution was filtered, concentrated, and subjected to column chromatography (petroleum ether/ethyl acetate = 5/1) to give compound 50a (0.2 g).

MS (ESI, [M+H]⁺) *m*/*z*: 637.4.

### Step H: Preparation of compound 1-50

To a reaction flask were added compound 50a (0.1 g), a solution of hydrochloric acid in 1,4-dioxane (4 M, 1.9 mL), and dichloromethane (10 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 5/1) to give compound I-50 (0.1 g).

HRMS (ESI, [M+H]⁺) *m*/*z*: 537.2365.

¹H NMR (500 MHz, CDCl₃) δ 9.97 (s, 1H), 8.95 (d, *J =* 8.5 Hz, 1H), 7.65 (s, 1H), 7.58 (d, *J =* 8.6 Hz, 1H), 7.54 (dd, *J =* 6.6, 2.9 Hz, 3H), 7.45 (d, *J =* 8.5 Hz, 1H), 6.86 (d, *J =* 8.6 Hz, 1H), 6.64 (s, 1H), 6.29 (d, *J =* 2.3 Hz, 1H), 4.22 (s, 2H), 3.77 (dd, *J =* 8.9, 6.8 Hz, 2H), 3.64 (s, 2H), 3.49 (dd, *J =* 8.7, 6.9 Hz, 2H), 2.90 (s, 3H), 2.36 (s, 6H).

### Example 51: Preparation of Compound I-51

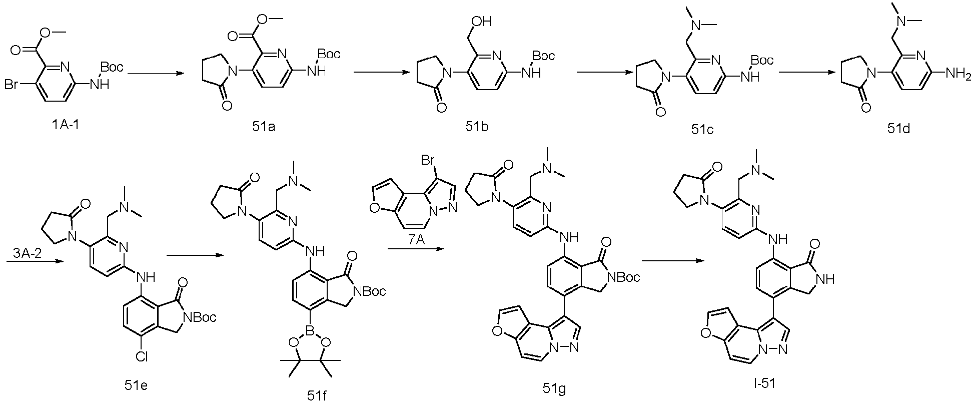

### Step A: Preparation of compound 51a

To a reaction flask were added intermediate 1A-1 (10.0 g), 1,4-dioxane (150.0 mL), 4,5-bis-diphenylphosphino-9,9-dimethylxanthene (2.6 g), cesium carbonate (19.6 g), tris(dibenzylideneadone) (2.7 g), and 2-pyrrolidone (3.0 g) in sequence. After the addition, the mixture was purged with nitrogen and stirred at 110 °C. After the reaction was completed, the reaction solution was filtered, concentrated, and subjected to column chromatography (dichloromethane/methanol = 30/1) to give compound 51a (10.5 g).

MS (ESI, [M+H]⁺) *m*/*z*: 336.1.

### Step B: Preparation of compound 51b

To a reaction flask were added compound 51a (10.5 g), tetrahydrofuran (50.0 mL), and lithium borohydride (1.3 mL) in sequence. After the addition, the mixture was stirred at room temperature. After the reaction was completed, water was added to the reaction solution to quench the reaction, and the resulting mixture was extracted with water and dichloromethane. The organic phase was dried, filtered, concentrated, and subjected to column chromatography (petroleum ether/ethyl acetate = 5/1) to give compound 51b (5.0 g).

MS (ESI, [M+H]⁺) *m*/*z*: 308.2.

### Step C: Preparation of compound 51c

To a reaction flask were added compound 51b (5.0 g), dichloromethane (30.0 mL), *N,N*-diisopropylethylamine (3.45 mL), and methanesulfonic anhydride (4.5 mL) in sequence under an ice-water bath. After the addition, the mixture was reacted under an ice-water bath. After the reaction was completed, a solution of dimethylamine in tetrahydrofuran (2 M, 30 mL). After the addition, the mixture was reacted at room temperature. After the reaction was completed, the reaction solution was concentrated and subjected to column chromatography (petroleum ether/ethyl acetate = 5/1) to give compound 51c (4.0 g).

MS (ESI, [M+H]⁺) *m*/*z*: 335.2.

### Step D: Preparation of compound 51d

To a reaction flask were added compound 51c (4 g), dichloromethane (30.0 mL), and a solution of hydrochloric acid in 1,4-dioxane (4 M, 15 mL) in sequence. After the addition, the mixture was stirred at room temperature. The reaction solution was adjusted to alkalinity (about pH 10) with a saturated sodium bicarbonate solution and extracted with dichloromethane. The organic phase was dried, filtered, concentrated, and subjected to column chromatography (dichloromethane/methanol = 5/1) to give compound 51d (1.5 g).

MS (ESI, [M+H]⁺) *m*/*z*: 235.0.

### Step E: Preparation of compound 51e

To a reaction flask were added intermediate 3A-2 (2.9 g), compound 51d (1.4 g), 1,4-dioxane (80.0 mL), 4,5-bis-diphenylphosphino-9,9-dimethylxanthene (0.9 g), cesium carbonate (6.3 g), and tris(dibenzylideneadone) (1.1 g) in sequence. After the addition, the mixture was purged with nitrogen and stirred at 110 °C. After the reaction was completed, the reaction solution was filtered, concentrated, and subjected to column chromatography (dichloromethane/methanol = 30/1) to give compound 51e (1.1 g).

MS (ESI, [M+H]⁺) *m*/*z*: 500.0.

### Step F: Preparation of compound 51f

To a reaction flask were added compound 51e (1.1 g), potassium acetate (0.5 g), bis(pinacolato)diboron (1.2 g), chloro(2-dicyclohexylphosphino-2',4',6'-triisopropyl-1,1'-biphenyl)[2-(2'-amino-1,1'-biphenyl)]palladium (II) (0.2 g), and 1,4-dioxane (30.0 mL) in sequence. After the addition, the mixture was purged with nitrogen and stirred at 90 °C. After the reaction was completed, the reaction solution was filtered, concentrated, and subjected to column chromatography (dichloromethane/methanol = 20/1) to give compound 51f (0.7 g).

MS (ESI, [M+H]⁺) *m*/*z*: 592.4.

### Step G: Preparation of compound 51g

To a reaction flask were added compound 51f (0.3 g), 2-dicyclohexylphosphine-2',4',6'-triisopropylbiphenyl (0.04 g), potassium phosphate (0.3 g), chloro(2-dicyclohexylphosphino-2',4',6'-trilsopropyl-1,1'-biphenyl)[2-(2'-amino-1,1'-biphenyl)]palladium (II) (0.1 g), intermediate 7A (0.4 g), water (3.0 mL), and 1,4-dioxane (12.0 mL) in sequence. After the addition, the mixture was stirred at 85 °C. After the reaction was completed, the reaction solution was filtered, concentrated, and subjected to column chromatography (dichloromethane/methanol = 10/1) to give compound 51g (0.2 g).

MS (ESI, [M+H]⁺) *m*/*z*: 622.1.

### Step H: Preparation of compound I-51

To a reaction flask were added compound 51g (0.2 g), a solution of hydrochloric acid in 1,4-dioxane (4 M, 1.9 mL), and dichloromethane (10 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 5/1) to give compound I-51 (0.09 g).

HRMS (ESI, [M+H]⁺) *m*/*z*: 522.2254.

¹H NMR (500 MHz, CDCl₃) δ 8.87 (dd, *J =* 8.7, 1.9 Hz, 1H), 8.42 - 8.35 (m, 1H), 8.00 (s, 1H), 7.69 - 7.62 (m, 2H), 7.40 (dd, *J* = 8.6, 1.9 Hz, 1H), 7.13 (d, *J =* 7.6 Hz, 1H), 6.87 (d, *J =* 8.6 Hz, 1H), 6.74 (d, *J* = 2.2 Hz, 1H), 4.38 (d, *J =* 2.0 Hz, 2H), 3.81 (t, *J =* 7.0 Hz, 2H), 3.60 (d, *J =* 1.8 Hz, 2H), 2.58 (td, *J =* 8.1, 1.9 Hz, 2H), 2.35 (d, *J =* 2.0 Hz, 6H), 2.24 (p, *J =* 8.0, 7.6 Hz, 2H).

### Example 52: Preparation of Compound I-52

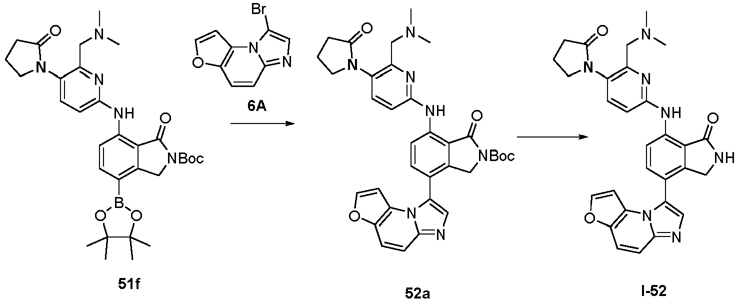

### Step A: Preparation of compound 52a

To a reaction flask were added compound 51f (0.3 g), 2-dicyclohexylphosphine-2',4',6'-triisopropylbiphenyl (0.04 g), potassium phosphate (0.9 g), chloro(2-dicyclohexylphosphino-2',4',6'-trilsopropyl-1,1'-biphenyl)[2-(2'-amino-1,1'-biphenyl)]palladium (II) (0.1 g), intermediate 6A (0.1 g), water (3.0 mL), and 1,4-dioxane (12.0 mL) in sequence. After the addition, the mixture was stirred at 85 °C. After the reaction was completed, the reaction solution was filtered, concentrated, and subjected to column chromatography (petroleum ether/ethyl acetate = 5/1) to give compound 52a (0.2 g).

MS (ESI, [M+H]⁺) *m*/*z*: 622.4.

### Step H: Preparation of compound I-52

To a reaction flask were added compound 52a (0.2 g), a solution of hydrochloric acid in 1,4-dioxane (4 M, 1.9 mL), and dichloromethane (10 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 5/1) to give compound I-52 (0.1 g).

HRMS (ESI, [M+H]⁺) *m*/*z*: 522.2254.

¹H NMR (500 MHz, CDCl₃) δ 8.92 (dd, *J =* 8.6, 3.7 Hz, 1H), 7.69 - 7.55 (m, 4H), 7.53 (dd, *J =* 9.8, 3.9 Hz, 1H), 7.43 (dd, *J* = 8.6, 3.8 Hz, 1H), 7.37 - 7.29 (m, 1H), 6.91 (dd, *J =* 8.6, 3.8 Hz, 1H), 6.29 (t, *J* = 3.0 Hz, 1H), 4.24 (s, 2H), 3.88 - 3.75 (m, 2H), 3.62 (d, *J =* 3.9 Hz, 2H), 2.37 (d, *J =* 3.9 Hz, 6H), 2.30 - 2.19 (m, 2H), 1.56 (q, *J =* 5.2 Hz, 1H), 1.32 - 1.20 (m, 1H).

### Example 53: Preparation of Compound I-53

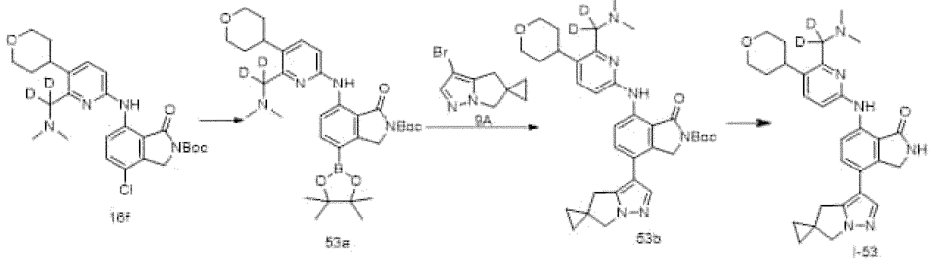

### Step A: Preparation of compound 53a

To a microwave tube were added compound 16f (0.85 g), bis(pinacolato)diboron (0.86 g), chloro(2-dicyclohexylphosphino-2',4',6'-triisopropyl-1,1 '-biphenyl)[2-(2'-amino-1,1 '-biphenyl)]palladium (II) (0.27 g), potassium acetate (0.50 g), and 1,4-dioxane (20 mL) in sequence. After the addition, the mixture was stirred at 90 °C under microwave irradiation under nitrogen atmosphere. After the reaction was completed, the reaction solution was filtered, concentrated, and subjected to column chromatography (dichloromethane/methanol = 20/1) to give compound 53a (0.54 g).

MS (ESI, [M+H]⁺) m/z: 595.43.

### Step B: Preparation of compound 53b

To a microwave tube were added compound 53a (110 mg), intermediate 9A (51 mg), chloro(2-dicyclohexylphosphino-2',4',6'-triisopropyl-1,1'-biphenyl)[2-(2'-atnino-1,1'-biphenyl)]palladium (II) (14 mg), 2-dicyclohexylphosphine-2',4',6'-triisopropylbiphenyl (9 mg), tripotassium phosphate (79 mg), 1,4-dioxane (3 mL), and water (0.5 mL) in sequence. After the addition, the mixture was stirred at 85 °C under microwave irradiation under nitrogen atmosphere. After the reaction was completed, the reaction solution was filtered, concentrated, and subjected to column chromatography (dichloromethane/methanol = 20/1) to give compound 53b (44 mg).

MS (ESI, [M+H]⁺) m/z: 601.56.

### Step C: Preparation of compound I-53

To a reaction flask were added compound 53b (44 mg), a solution of hydrochloric acid in 1,4-dioxane (4 M, 1.9 mL), and dichloromethane (10 mL) in sequence. After the addition, the mixture was reacted at room temperature.

After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 5/1) to give compound I-53 (10 mg).

HRMS (ESI, [M+H]⁺) *m*/*z*: 501.2944.

¹H NMR (500 MHz, CDCl₃) δ 9.74 (s, 1H), 8.73 (d, J = 8.6 Hz, 1H), 7.64 (s, 1H), 7.47 (dd, J = 24.5, 8.6 Hz, 2H), 6.81 (d, J = 8.5 Hz, 1H), 6.12 (s, 1H), 4.53 (s, 2H), 4.14 (s, 2H), 4.08 (dd, J = 11.2, 4.1 Hz, 2H), 3.61 - 3.50 (m, 2H), 3.23 - 3.13 (m, 1H), 3.10 (s, 2H), 2.41 (s, 6H), 1.80 - 1.70 (m, 2H), 0.90 - 0.87 (m, 4H).

### Example 54: Preparation of Compound I-54

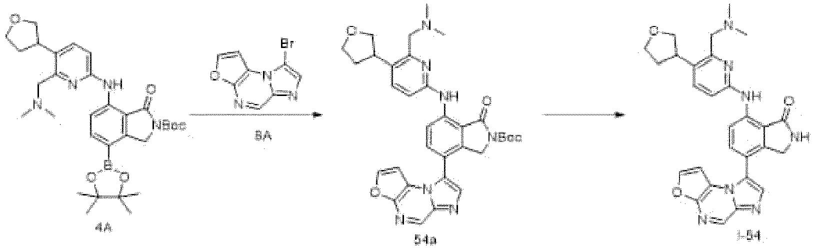

### Step A: Preparation of compound 54a

To a microwave tube were added intermediate 4A (205 mg), intermediate 8A (51 mg), chloro(2-dicyclohexylphosphino-2',4',6'-triisopropyl-1,1'-biphenyl)[2-(2'-atnino-1,1'-biphenyl)]palladium (II) (28 mg), 2-dicyclohexylphosphine-2',4',6'-triisopropylbiphenyl (17 mg), tripotassium phosphate (150 mg), 1,4-dioxane (3 mL), and water (0.5 mL) in sequence. After the addition, the mixture was stirred at 85 °C under microwave irradiation under nitrogen atmosphere. After the reaction was completed, the reaction solution was filtered, concentrated, and subjected to column chromatography (dichloromethane/methanol = 20/1) to give compound 54a (162 mg).

MS (ESI, [M+H]⁺) m/z: 610.43.

### Step B: Preparation of compound I-54

To a reaction flask were added compound 54a (162 mg), a solution of hydrochloric acid in 1,4-dioxane (4 M, 1.9 mL), and dichloromethane (10 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 5/1) to give compound I-54 (70 mg).

HRMS (ESI, [M+H]⁺) *m*/*z*: 510.2242.

¹H NMR (500 MHz, CDCl₃) δ 9.88 (s, 1H), 9.01 (d, J = 8.6 Hz, 1H), 8.83 (s, 1H), 7.87 (s, 1H), 7.68 - 7.58 (m, 3H), 6.86 (d, J = 8.4 Hz, 1H), 6.47 (d, J = 2.5 Hz, 1H), 6.33 (s, 1H), 4.30 (s, 2H), 4.14 - 4.04 (m, 2H), 3.96 - 3.86 (m, 2H), 3.73 - 3.66 (m, 2H), 3.60 (d, J = 12.4 Hz, 1H), 2.42 - 2.36 (m, 1H), 2.31 (s, 6H), 1.97 - 1.90 (m, 1H).

### Example 55: Preparation of Compound I-55

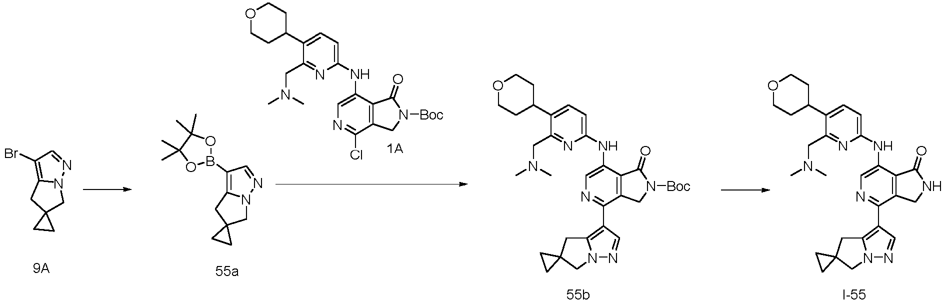

### Step A: Preparation of compound 55a

To a reaction flask containing intermediate 9A (0.25 g) and tetrahydrofuran (20 mL) was slowly added dropwise a solution of n-butyllithium in n-hexane (0.8 mL, 2 M) at -78 °C. After the addition, the mixture was stirred for 1 h. Isopropanol pinacol borate (0.6 g) was slowly added. After the addition, the mixture was heated to room temperature and stirred. After the reaction was completed, a saturated aqueous ammonium chloride solution was added to the reaction solution to quench the reaction, and the resulting mixture was extracted with ethyl acetate. The organic phase was dried, filtered, concentrated, and subjected to column chromatography (petroleum ether/ethyl acetate = 7/3) to give compound 55a (0.1 g).

MS (ESI, [M+H]⁺) *m*/*z*: 261.2.

### Step B: Preparation of compound 55b

To a reaction flask were added compound 55a (0.1 g), intermediate 1A (0.12 g), potassium phosphate (0.2 g), chloro(2-dicyclohexylphosphino-2',4',6'-triisopropyl-1,1'-biphenyl)[2-(2'-amino-1,1'-biphenyl)]palladium (II) (0.15 g), 1,4-dioxane (20 mL), and water (1 mL) in sequence. After the addition, the mixture was stirred at 100 °C under nitrogen atmosphere. After the reaction was completed, the reaction solution was concentrated and subjected to column chromatography (dichloromethane/methanol = 15/1) to give compound 55b (0.1 g).

MS (ESI, [M+H]⁺) *m*/*z*: 600.4.

### Step C: Preparation of compound 1-55

To a reaction flask were added compound 55b (0.2 g), a solution of hydrochloric acid in 1,4-dioxane (4 M, 1.9 mL), and dichloromethane (10 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 5/1) to give compound I-55 (0.08 g).

HRMS (ESI, [M+H]⁺) *m*/*z*: 500.2768.

### Example 56: Preparation of Compound I-56

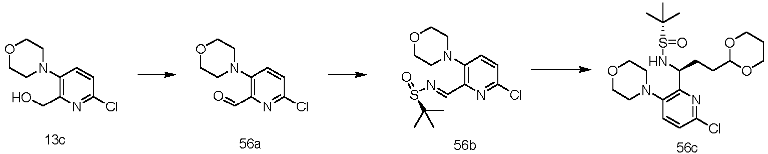

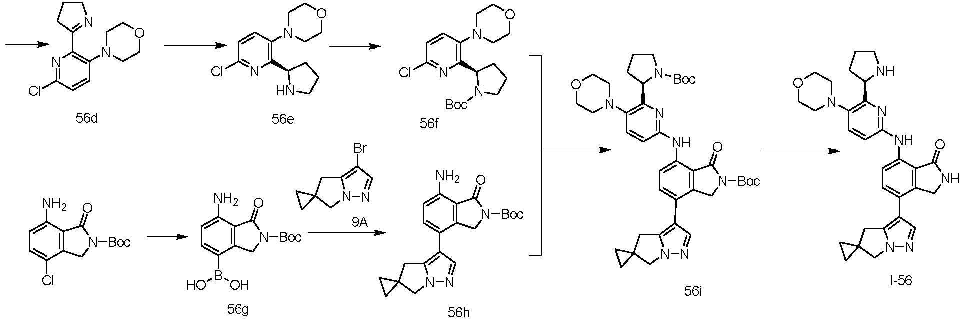

### Step A: Preparation of compound 56a

To a reaction flask were added compound 13c (1.98 g), Dess-Martin periodinane (5.5 g), and dichloromethane (20 mL) in sequence. After the addition, the mixture was stirred at room temperature. After the reaction was completed, a saturated aqueous ammonium chloride solution was added to the reaction solution to quench the reaction, and the resulting mixture was extracted with ethyl acetate. The organic phase was dried, filtered, concentrated, and subjected to column chromatography (petroleum ether/ethyl acetate = 6/1) to give compound 56a (1.5 g).

MS (ESI, [M+H]⁺) *m*/*z*: 227.6.

### Step B: Preparation of compound 56b

To a reaction flask were added 56a (1.5 g), (*R*)-*tert*-butylsulfinylimine (0.98 g), tetraethyl titanate (5.8 g), and tetrahydrofuran (30 mL) in sequence. After the addition, the mixture was stirred at room temperature. After the reaction was completed, a saturated aqueous ammonium chloride solution was added to the reaction solution to quench the reaction, and the resulting mixture was extracted with ethyl acetate. The organic phase was dried, filtered, concentrated, and subjected to column chromatography (petroleum ether/ethyl acetate = 9/1) to give compound 56b (1.82 g).

MS (ESI, [M+H]⁺) *m*/*z*: 330.1.

### Step C: Preparation of compound 56c

To a reaction flask containing compound 56b (1.8 g) and tetrahydrofuran (30 mL) were slowly added dropwise (1,3-dioxane-2-ethyl)magnesium bromide (20 mL, 1 M) at -78 °C. After the addition, the mixture was stirred at room temperature. After the reaction was completed, a saturated aqueous ammonium chloride solution was added to the reaction solution to quench the reaction, and the resulting mixture was extracted with ethyl acetate. The organic phase was dried, filtered, concentrated, and subjected to column chromatography (petroleum ether/ethyl acetate = 4/1) to give compound 56c (1.2 g).

MS (ESI, [M+H]⁺) *m*/*z*: 446.2.

### Step D: Preparation of compound 56d

To a reaction flask were added compound 56c (1.2 g), trifluoroacetic acid (10 mL), and water (0.5 mL) in sequence. After the addition, the mixture was stirred at room temperature. After the reaction was completed, the reaction solution was adjusted to alkalinity (about pH 8) with a saturated aqueous sodium bicarbonate solution and extracted with dichloromethane. The organic phase was dried, filtered, and concentrated to give compound 56d (1.82 g), which was directly used in the next step.

### Step E: Preparation of compound 56e

To a reaction flask containing compound 56d (1.82 g) and trifluoroacetic acid (10 mL) was slowly added dropwise triethylsilane (2.8 g) under an ice-water bath. After the addition, the mixture was stirred at room temperature. After the reaction was completed, the reaction solution was concentrated and subjected to column chromatography (dichloromethane/methanol = 100/1) to give compound 56e (0.8 g).

MS (ESI, [M+H]⁺) *m*/*z*: 268.1.

### Step F: Preparation of compound 56f

To a reaction flask were added compound 56e (1.2 g), di-*tert*-butyl dicarbonate (3.2 g), 4-dimethylaminopyridine (0.3 g), and dichloromethane (15 mL) in sequence. After the addition, the mixture was stirred at room temperature. After the reaction was completed, the reaction solution was extracted with ethyl acetate and water. The organic phase was dried, filtered, concentrated, and subjected to column chromatography (petroleum ether/ethyl acetate = 20:1) to give compound 56f (1.1 g).

MS (ESI, [M+H]⁺) *m*/*z*: 368.1.

### Step G: Preparation of compound 56g

To a reaction flask were added 7-amino-4-chloro-1-oxyisoindole-2-*tert*-butyl carbamate (1.2 g), tetrahydroxydiboron (0.95 g), chloro(2-dicyclohexylphosphino-2',4',6'-triisopropyl-1,1'-biphenyl)[2-(2'-amino-1,1'-biphenyl)]palladium (II) (0.35 g), 2-dicyclohexylphosphine-2',4',6'-triisopropylbiphenyl (0.45 g), potassium phosphate (2.2 g), ethylene glycol (0.8 g), and ethanol (20 mL) in sequence. After the addition, the mixture was stirred at 100 °C under nitrogen atmosphere. After the reaction was completed, the reaction solution was concentrated and subjected to column chromatography (dichloromethane/methanol = 15/1) to give compound 56g (0.8 g).

MS (ESI, [M+H]⁺) *m*/*z*: 293.2.

### Step H: Preparation of compound 56h

To a reaction flask were added compound 56g (0.8 g), intermediate 9A (0.9 g), chloro(2-dicyclohexylphosphino-2',4',6'-triisopropyl-1,1'-biphenyl)[2-(2'-amino-1,1'-biphenyl)]palladium (II) (0.24 g), potassium phosphate (0.8 g), 1,4-dioxane (15 mL), and water (0.5 mL) in sequence. After the addition, the mixture was stirred at 100 °C under nitrogen atmosphere. After the reaction was completed, the reaction solution was concentrated and subjected to column chromatography (petroleum ether/ethyl acetate = 6/1) to give compound 56h (0.8 g).

MS (ESI, [M+H]⁺) *m*/*z*: 381.4.

### Step I: Preparation of compound 56i

To a reaction flask were added compound 56f (0.4 g), compound 56h (0.5 g), cesium carbonate (2.1 g), tris(dibenzylideneacetone)dipalladium(0) (0.1 g), 4,5-bis-diphenylphosphino-9,9-dimethylxanthene (0.15 g), and 1,4-dioxane (20 mL) in sequence. After the addition, the mixture was stirred at 100 °C under nitrogen atmosphere. After the reaction was completed, the reaction solution was concentrated and subjected to column chromatography (petroleum ether/ethyl acetate = 4/1) to give compound 56i (0.24 g).

MS (ESI, [M+H]⁺) *m*/*z*: 712.1*.*

### Step J: Preparation of compound I-56

To a reaction flask were added compound 56i (0.2 g), a solution of hydrochloric acid in 1,4-dioxane (4 M, 1.5 mL), and dichloromethane (15 mL) in sequence. After the addition, the mixture was stirred at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 15/1) to give compound I-56 (0.10 g).

HRMS (ESI, [M+H]⁺) *m*/*z*: 512.2766.

¹H NMR (500 MHz, CDCl₃:CD₃OD=10:1) δ 9.57(s, 1H), 7.87 *(d, J=* 8.1 Hz, 1H), 7.52 - 7.44 (m, 2H), 7.34 (d, *J* = 8.2 Hz, 1H), 6.78 (d, *J* = 8.6 Hz, 1H), 4.36 (s, 2H), 4.05 (s, 2H), 3.81 (q, *J =* 9.1, 7.4 Hz, 4H), 3.67 (s, 1H), 3.49 (s, 1H), 3.02 (s, 2H), 2.96 - 2.89 (m, 2H), 2.69 (t, *J =* 7.9 Hz, 2H), 2.63 - 2.55 (m, 1H), 2.20 - 2.13 (m, 1H), 2.10 (s, 1H), 1.95 (dt, *J =* 14.0, 4.6 Hz, 1H), 1.24 - 1.14 (m, 3H), 0.83 (s, 4H).

### Example 57: Preparation of Compound I-57

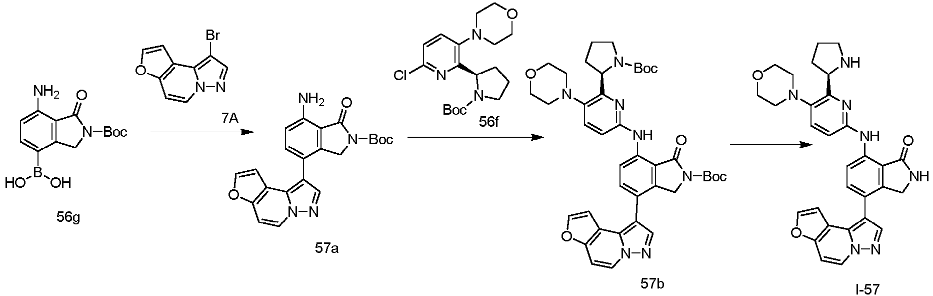

### Step A: Preparation of compound 57a

To a reaction flask were added compound 56g (0.8 g), intermediate 7A (0.9 g), chloro(2-dicyclohexylphosphino-2',4',6'-triisopropyl-1,1'-biphenyl)[2-(2'-amino-1,1'-biphenyl)]palladium (II) (0.24 g), potassium phosphate (0.8 g), 1,4-dioxane (15 mL), and water (0.5 mL) in sequence. After the addition, the mixture was stirred at 100 °C under nitrogen atmosphere. After the reaction was completed, the reaction solution was concentrated and subjected to column chromatography (petroleum ether/ethyl acetate = 6/1) to give compound 57a (0.9 g).

MS (ESI, [M+H]⁺) *m*/*z:* 405.4.

### Step B: Preparation of compound 57b

To a reaction flask were added compound 57a (0.4 g), compound 56f (0.5 g), cesium carbonate (2.1 g), tris(dibenzylideneacetone)dipalladium(0) (0.1 g), 4,5-bis-diphenylphosphino-9,9-dimethylxanthene (0.1 g), and 1,4-dioxane (20 mL) in sequence. After the addition, the mixture was stirred at 100 °C under nitrogen atmosphere. After the reaction was completed, the reaction solution was concentrated and subjected to column chromatography (petroleum ether/ethyl acetate = 4/1) to give 57b (0.32 g).

MS (ESI, [M+H]⁺) *m*/*z*: 736.1.

### Step C: Preparation of compound 1-57

To a reaction flask were added compound 57b (0.2 g), a solution of hydrochloric acid in 1,4-dioxane (4 M, 1.5 mL), and dichloromethane (15 mL) in sequence. After the addition, the mixture was stirred at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 6/1) to give compound I-57 (0.11 g).

HRMS (ESI, [M+H]⁺) *m*/*z*: 536.2407.

¹H NMR (500 MHz, CD₃Cl:CD₃OD=10:1) δ 8.37 (d, *J=* 7.5 Hz, 1H), 8.03 (d*, J* = 8.0 Hz, 1H), 7.99 (s, 1H), 7.70 - 7.56 (m, 3H), 7.12 (d, *J =* 7.4 Hz, 1H), 7.04 (d, *J =* 8.2 Hz, 1H), 6.70 (s, 1H), 4.40 (s, 2H), 3.88 (q, *J =* 8.9, 7.8 Hz, 4H), 3.74 (s, 1H), 3.53 (s, 1H), 3.01 (t*, J* = 7.8 Hz, 2H), 2.78 (t, *J =* 8.3 Hz, 2H), 2.67 (s, 1H), 2.26 (s, 1H), 2.18 (s, 1H), 2.04 (s, 2H), 1.27 (d, *J=* 15.2 Hz, 2H).

### Example 58: Preparation of Compound I-58

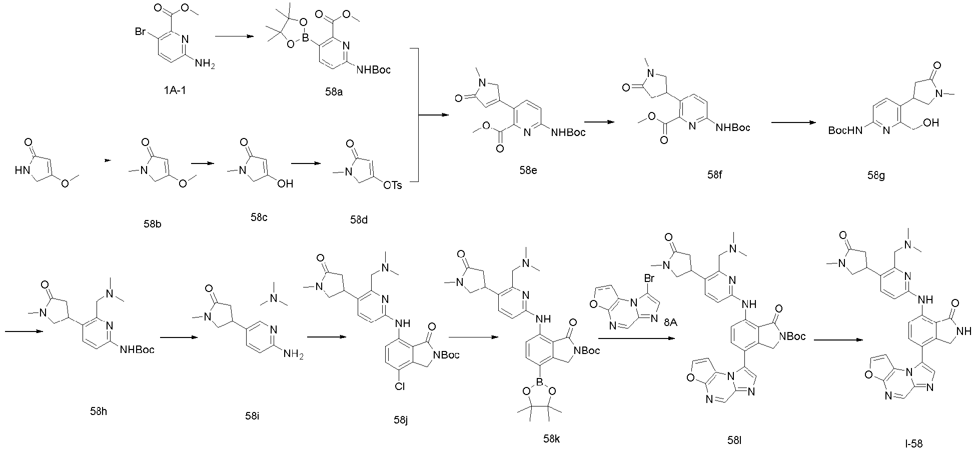

### Step A: Preparation of compound 58a

To a reaction flask were added intermediate 1A-1 (12 g), bis(pinacolato)diboron (13.8 g), [1,1'-bis(diphenylphosphino)ferrocene]palladium(II) dichloride dichloromethane complex (2.96 g), potassium acetate (10.67 g), and 1,4-dioxane (200 mL) in sequence. The mixture was heated and stirred at 90 °C. After the reaction was completed, the reaction solution was filtered, concentrated, and subjected to column chromatography (dichloromethane/methanol = 20/1) to give compound 58a (9.0 g).

MS (ESI, [M+H]⁺) *m*/*z:* 379.5.

### Step B: Preparation of compound 58b

To a reaction flask were added the compounds 4-methoxy-3-pyrrolin-2-one (10 g), potassium hydroxide (7.44 g), tetrabutylammonium hydrogen sulfate (1.50 g), potassium acetate (10.67 g), tetrahydrofuran (150 mL), and iodomethane (18.82 g) in sequence. The mixture was stirred at room temperature. After the reaction was completed, the reaction solution was filtered, concentrated, and subjected to column chromatography (dichloromethane/methanol = 40/1) to give compound 58b (9.1 g).

MS (ESI, [M+H]⁺) *m*/*z*: 128.0.

### Step C: Preparation of compound 58c

To a reaction flask were added compound 58b (9.1 g), dichloromethane (10 mL), and trifluoroacetic acid (35 g) in sequence. The mixture was stirred at room temperature. After the reaction was completed, the reaction solution was concentrated to give compound 58c (8.0 g).

MS (ESI, [M+H]⁺) *m*/*z*: 114.0.

### Step D: Preparation of compound 58d

To a reaction flask were added compound 58c (8.0 g), dichloromethane (100 mL),*p*-toluenesulfonyl chloride (13.48 g), and *N,N-*diisopropylethylamine (13.71 g) in sequence. The mixture was stirred at room temperature. After the reaction was completed, the reaction solution was extracted with water and dichloromethane. The organic phase was isolated, dried, filtered, concentrated, and subjected to column chromatography (petroleum ether/ethyl acetate = 4/1) to give compound 58d (5.2 g).

MS (ESI, [M+H]⁺) *m*/*z*: 268.3.

### Step E: Preparation of compound 58e

To a microwave reaction flask were added compound 58a (12.75 g), compound 58d (5.0 g), palladium acetate (0.42 g), tricyclohexylphosphine fluoroborate (1.38 g), tripotassium phosphate (11.92 g), water (38mL), and 1,4-dioxane (150 mL) in sequence. The mixture was reacted at 85 °C under microwave irradiation. After the reaction was completed, the reaction solution was filtered, concentrated, and subjected to column chromatography (dichloromethane/methanol = 20/1) to give compound 58e (5.0 g).

MS (ESI, [M+H]⁺) *m*/*z*: 348.3.

### Step F: Preparation of compound 58f

To a reaction flask were added compound 58e (5.0 g), methanol (70 mL), and 10 wt% palladium hydroxide on carbon (5.49 g) in sequence. The mixture was stirred at room temperature under hydrogen atmosphere. After the reaction was completed, the reaction solution was filtered, concentrated, and subjected to column chromatography (dichloromethane/methanol = 40/1) to give compound 58f (3.1 g).

MS (ESI, [M+Na]⁺) *m*/*z*: 372.4.

### Step G: Preparation of compound 58g

To a reaction flask were added compound 58f (3.1 g), tetrahydrofuran (30 mL), and a 2 M solution of lithium borohydride in tetrahydrofuran (8.87 mL) in sequence at 0 °C. After the addition, the mixture was stirred at 0 °C. After the reaction was completed, a saturated ammonium chloride solution was added to the reaction solution to quench the reaction, and the resulting mixture was extracted with dichloromethane. The organic phase was isolated, dried, filtered, concentrated, and subjected to column chromatography (dichloromethane/methanol = 20/1) to give compound 58g (1.5 g).

MS (ESI, [M+H]⁺) *m*/*z*: 322.4.

### Step H: Preparation of compound 58h

To a reaction flask were added compound 58g (1.5 g), dichloromethane (20.0 mL), *N,N*-diisopropylethylamine (1.63 mL), and methanesulfonic anhydride (2.44 g) in sequence at -10 °C. After the addition, the mixture was stirred at - 10 °C. After the reaction was completed, the reaction solution was added to a solution of dimethylamine in tetrahydrofuran (2 M, 45 mL). After the addition, the mixture was stirred at room temperature. After the reaction was completed, the reaction solution was concentrated and subjected to column chromatography (petroleum ether/ethyl acetate = 5/1) to give compound 58h (0.90 g).

MS (ESI, [M+H]⁺) *m*/*z*: 349.4.

### Step I: Preparation of compound 58i

To a reaction flask were added compound 58h (0.9 g), dichloromethane (5.0 mL), and a solution of hydrochloric acid in 1,4-dioxane (4 M, 11 mL) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was isolated, dried, filtered, concentrated, and subjected to column chromatography (dichloromethane/methanol = 5/1) to give compound 58i (0.48 g).

MS (ESI, [M+H]⁺) *m*/*z*: 249.4.

### Step J: Preparation of compound 58j

To a reaction flask were added intermediate 3A-2 (0.804 g), compound 58i (0.48 g), 1,4-dioxane (3 mL), 4,5-bis-diphenylphosphino-9,9-dimethylxanthene (0.224 g), cesium carbonate (1.88 g), and tris(dibenzylideneadone) (0.266 g) in sequence. After the addition, the mixture was stirred at 110 °C. After the reaction was completed, the reaction solution was filtered, concentrated, and subjected to column chromatography (dichloromethane/methanol = 30/1) to give compound 58j (0.55 g).

MS (ESI, [M+H]⁺) *m*/*z*: 514.4.

### Step K: Preparation of compound 58k

To a reaction flask were added compound 58j (0.5 g), potassium acetate (0.29 g), bis(pinacolato)diboron (0.45 g), chloro(2-dicyclohexylphosphino-2',4',6'-triisopropyl-1,1'-biphenyl)[2-(2'-amino-1,1'-biphenyl)]palladium (II) (0.15 g), and 1,4-dioxane (8.0 mL) in sequence. After the addition, the mixture was purged with nitrogen and stirred at 90 °C. After the reaction was completed, the reaction solution was filtered, concentrated, and subjected to column chromatography (dichloromethane/methanol = 30/1) to give compound 58k (0.50 g).

MS (ESI, [M+H]⁺) *m*/*z*: 606.6.

### Step L: Preparation of compound 58l

To a reaction flask were added compound 58k (0.25 g), 2-dicyclohexylphosphine-2',4',6'-triisopropylbiphenyl (0.19 g), potassium phosphate (0.18 g), chloro(2-dicyclohexylphosphino-2',4',6'-triisopropyl-1,1'-biphenyl)[2-(2'-amino-1,1'-biphenyl)]palladium (II) (0.32 g), intermediate 8A (0.1 g), water (3.0 mL), and 1,4-dioxane (12.0 mL) in sequence. After the addition, the mixture was stirred at 85 °C. After the reaction was completed, the reaction solution was filtered, concentrated, and subjected to column chromatography (dichloromethane/methanol = 20/1) to give compound **58l** (0.1 g).

MS (ESI, [M+H]⁺) *m*/*z*: 637.4.

### Step M: Preparation of compound 1-58

To a reaction flask were added compound **58l** (0.1 g), a solution of hydrochloric acid in 1,4-dioxane (4 M, 1.9 mL), and dichloromethane (2 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was isolated, dried, filtered, concentrated, and subjected to column chromatography (dichloromethane/methanol = 5/1) to give compound I-58 (0.03 g).

HRMS (ESI, [M+H]⁺) *m*/*z*: 537.2354.

¹H NMR (500 MHz, CDCl₃) δ 9.93 (s, 1H), 8.99 (d, *J =* 8.6 Hz, 1H), 8.83 (s, 1H), 7.86 (s, 1H), 7.62 (t, *J =* 5.5 Hz, 2H), 7.54 (d, *J =* 8.5 Hz, 1H), 6.87 (d, *J =* 8.5 Hz, 1H), 6.54 - 6.44 (m, 2H), 4.30 (s, 2H), 4.11 - 4.04 (m, 1H), 3.78 (dd, *J =* 9.6, 8.6 Hz, 1H), 3.68 (d, *J =* 12.4 Hz, 1H), 3.56 (d, *J =* 12.4 Hz, 1H), 3.31 (dd, *J =* 9.9, 6.1 Hz, 1H), 2.92 (s, 3H), 2.84 (dd, *J =* 17.0, 9.4 Hz, 1H), 2.45 (dd, *J =* 17.0, 7.2 Hz, 1H), 2.27 (s, 6H).

### Example 59: Preparation of Compound I-59

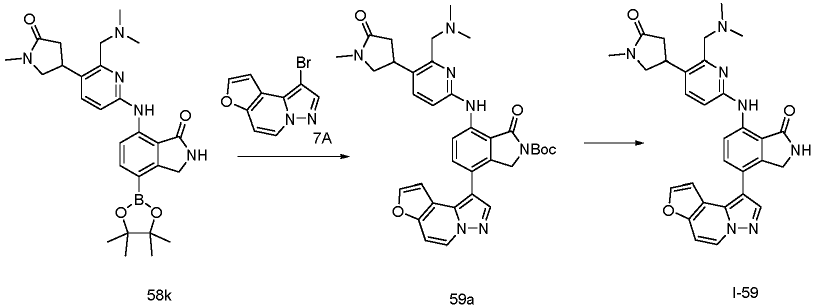

### Step A: Preparation of compound 59a

To a reaction flask were added compound 58k (0.25 g), 2-dicyclohexylphosphine-2',4',6'-triisopropylbiphenyl (0.19 g), potassium phosphate (0.18 g), chloro(2-dicyclohexylphosphino-2',4',6'-triisopropyl-1,1'-biphenyl)[2-(2'-amino-1,1'-biphenyl)]palladium (II) (0.32 g), intermediate 7A (0.1 g), water (3.0 mL), and 1,4-dioxane (12.0 mL) in sequence. After the addition, the mixture was stirred at 85 °C. After the reaction was completed, the reaction solution was filtered, concentrated, and subjected to column chromatography (dichloromethane/methanol = 20/1) to give compound 59a (0.1 g).

MS (ESI, [M+H]⁺) *m*/*z*: 636.40.

### Step B: Preparation of compound I-59

To a reaction flask were added compound 59a (0.1 g), a solution of hydrochloric acid in 1,4-dioxane (4 M, 3 mL), and dichloromethane (5 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was isolated, dried, filtered, concentrated, and subjected to column chromatography (dichloromethane/methanol = 5/1) to give compound I-59 (0.03 g).

HRMS (ESI, [M+H]⁺) *m*/*z*: 536.2408.

¹H NMR (500 MHz, CDCl₃) δ 9.81 (s, 1H), 8.88 (d, *J =* 8.5 Hz, 1H), 8.39 (d, *J =* 7.6 Hz, 1H), 8.00 (s, 1H), 7.63 (dd, *J =* 6.8, 5.4 Hz, 2H), 7.49 (d, *J =* 8.5 Hz, 1H), 7.09 (d, *J =* 7.6 Hz, 1H), 6.84 (d, *J =* 8.5 Hz, 1H), 6.72 *(d, J =* 1.4 Hz, 1H), 6.36 (s, 1H), 4.38 (s, 2H), 4.12 - 4.01 (m, 1H), 3.76 (dd, *J =* 9.7, 8.6 Hz, 1H), 3.68 (d, *J =* 12.3 Hz, 1H), 3.56 (d, *J =* 12.3 Hz, 1H), 3.31 (dd, *J* = 9.9, 6.2 Hz, 1H), 2.92 (s, 3H), 2.83 (dd, *J =* 17.1, 9.4 Hz, 1H), 2.45 (dd, *J =* 17.0, 7.3 Hz, 1H), 2.27 (s, 6H).

### Example 60: Preparation of Compound 1-60

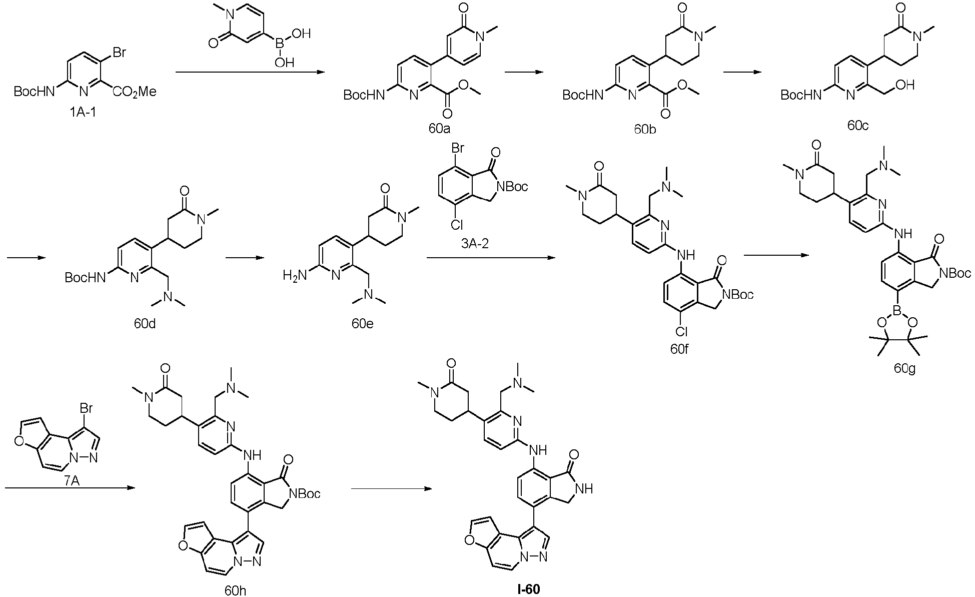

### Step A: Preparation of compound 60a

To a reaction flask were added intermediate 1A-1 (7.22 g), 1-methyl-2-oxo-1,2-dihydropyridine-4-boronic acid (5.00 g), sodium carbonate (4.62 g), [1,1'-bis(diphenylphosphino)ferrocene]palladium(II) dichloride dichloromethane complex (1.78 g), 1,4-dioxane (90 mL), and water (15 mL) in sequence under nitrogen atmosphere. After the addition, the mixture was stirred at 100 °C. After the reaction was completed, the reaction solution was concentrated and subjected to column chromatography (petroleum ether/ethyl acetate = 1/3) to give compound 60a (4.92 g).

MS (ESI, [M+H]⁺) *m*/*z*: 360.45.

### Step B: Preparation of compound 60b

To a reaction flask were added compound 60a (4.92 g), ammonium formate (3.45 g), methanol (25 mL), tetrahydrofuran (20 mL), 10wt% palladium hydroxide on carbon (5.41 g), and acetic acid (3.45 mL) in sequence. After the addition, the mixture was stirred at room temperature under hydrogen atmosphere. After the reaction was completed, the reaction solution was filtered and concentrated to give compound 60b (4.30 g).

MS (ESI, [M+H]⁺) *m*/*z*: 364.26.

### Step C: Preparation of compound 60c

To a reaction flask were added compound 60b (5.14 g), tetrahydrofuran (100 mL), and a solution of lithium borohydride in tetrahydrofuran (14.79 mL, 2 M) in sequence at 0 °C. After the addition, the mixture was stirred at room temperature. After the reaction was completed, the reaction was quenched with a saturated ammonium chloride solution, and the reaction solution was extracted with ethyl acetate. The organic phase was isolated, dried, filtered, and concentrated to give compound 60c (3.68 g).

MS (ESI, [M+H]⁺) *m*/*z*: 336.38.

### Step D: Preparation of compound 60d

To a reaction flask were added compound 60c (2.43 g), dichloromethane (30 mL), *N,N*-diisopropylethylamine (1.87 g), and methanesulfonic anhydride (2.52 g) in sequence at -10 °C. After the addition, the mixture was stirred at 0 °C.

After the reaction was completed, the reaction solution was added dropwise to a solution of dimethylamine in tetrahydrofuran (2 M, 36.20 mL) at -10 °C. After the addition, the mixture was stirred at room temperature. After the reaction was completed, the reaction solution was concentrated and subjected to column chromatography (dichloromethane:methanol = 20:1) to give compound 60d (2.58 g).

MS (ESI, [M+H]⁺) *m*/*z*: 363.50.

### Step E: Preparation of compound 60e

To a reaction flask were added compound 60d (2.58 g), dichloromethane (40 mL), and a solution of hydrochloric acid in 1,4-dioxane (17.80 mL, 2 M) in sequence. After the addition, the mixture was stirred at room temperature. After the reaction was completed, the reaction solution was concentrated, adjusted to alkalinity (about pH 10) with a saturated sodium bicarbonate solution, and extracted with dichloromethane. The organic phase was isolated, dried, filtered, and concentrated to give compound 60e (1.85 g).

MS (ESI, [M+H]⁺) *m*/*z*: 263.19.

### Step F: Preparation of compound 60f

To a reaction flask were added compound 60e (1.17 g), intermediate 3A-2 (1.55 g), tris(dibenzylideneacetone) dipalladium(0) (0.41 g), 4,5-bis-diphenylphosphino-9,9-dimethylxanthene (0.31 g), cesium carbonate (4.37 g), and 1,4-dioxane (50 mL) under nitrogen atmosphere. The mixture was stirred at 110 °C. After the reaction was completed, the reaction solution was filtered, concentrated, and subjected to column chromatography (dichloromethane/methanol = 20/1) to give compound 60f (1.13 g).

MS (ESI, [M+H]⁺) *m*/*z*: 528.29.

### Step G: Preparation of compound 60g

To a microwave reaction flask were added compound 60f (1.13 g), bis(pinacolato)diboron (0.97 g), chloro(2-dicyclohexylphosphino-2',4',6'-triisopropyl-1,1 '-biphenyl)[2-(2'-amino-1,1 '-biphenyl)]palladium (II) (0.34 g), potassium acetate (0.63 g), and 1,4-dioxane (15 mL) in sequence. The mixture was purged with nitrogen and stirred at 90 °C under microwave irradiation. After the reaction was completed, the reaction solution was filtered, concentrated, and subjected to column chromatography (dichloromethane/methanol = 20/1) to give compound 60g (0.54 g).

MS (ESI, [M+H]⁺) m/z: 620.72.

### Step H: Preparation of compound 60h

To a microwave reaction flask were added compound 60g (284 mg), intermediate 7A (109 mg), chloro(2-dicyclohexylphosphino-2',4',6'-triisopropyl-1,1 '-biphenyl)[2-(2'-amino-1,1 '-biphenyl)]palladium (II) (36 mg), tripotassium phosphate (195 mg), 1,4-dioxane (7.0 mL), and water (1.0 mL) in sequence. The mixture was stirred at 85 °C under microwave irradiation under nitrogen atmosphere. After the reaction was completed, the reaction solution was concentrated and subjected to column chromatography (dichloromethane/methanol = 20/1) to give compound 60h (94 mg).

MS (ESI, [M+H]⁺) m/z: 650.49.

### Step I: Preparation of compound 1-60

To a reaction flask were added compound 60h (94 mg), a solution of hydrochloric acid in 1,4-dioxane (4 M, 1.9 mL), and dichloromethane (2 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 5/1) to give compound I-60 (38 mg).

HRMS (ESI, [M+H]⁺) *m*/*z*: 550.2567.

¹H NMR (500 MHz, CDCl₃) δ 9.79 (s, 1H), 8.90 (d, J = 8.5 Hz, 1H), 8.39 (d, J = 7.5 Hz, 1H), 8.00 (s, 1H), 7.66 - 7.60 (m, 2H), 7.44 (d, J = 8.4 Hz, 1H), 7.10 (d, J = 7.5 Hz, 1H), 6.84 (d, J = 8.5 Hz, 1H), 6.73 (d, J = 2.0 Hz, 1H), 6.09 (s, 1H), 4.38 (s, 2H), 3.66 (d, J = 12.3 Hz, 1H), 3.60 - 3.53 (m, 2H), 3.49 - 3.43 (m, 1H), 3.38 - 3.34 (m, 1H), 3.02 (s, 3H), 2.75 - 2.70 (m, 1H), 2.40 (dd, J = 17.4, 11.6 Hz, 1H), 2.28 (s, 6H), 2.07 - 1.94 (m, 2H).

### Example 61: Preparation of Compound I-61

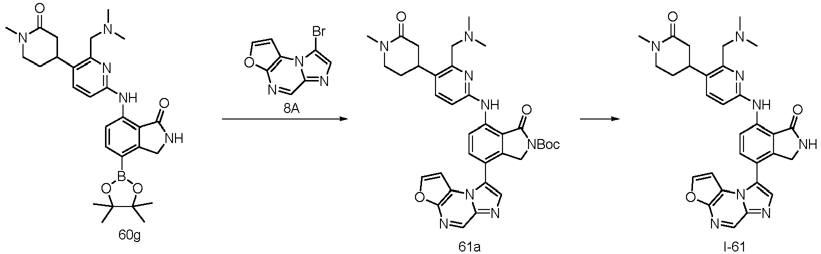

### Step A: Preparation of compound 61a

To a microwave reaction flask were added compound 60g (300 mg), intermediate 8A (115 mg), chloro(2-dicyclohexylphosphino-2',4',6'-triisopropyl-1,1 '-biphenyl)[2-(2'-amino-1,1 '-biphenyl)]palladium (II) (38 mg), tripotassium phosphate (206 mg), 1,4-dioxane (7.0 mL), and water (1.0 mL) in sequence. The mixture was stirred at 85 °C under microwave irradiation under nitrogen atmosphere. After the reaction was completed, the reaction solution was concentrated and subjected to column chromatography (dichloromethane/methanol = 20/1) to give compound 61a (124 mg).

MS (ESI, [M+H]⁺) m/z: 651.42.

### Step B: Preparation of compound I-61

To a reaction flask were added compound 61a (124 mg), a solution of hydrochloric acid in 1,4-dioxane (4 M, 1.9 mL), and dichloromethane (2 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 5/1) to give compound I-61 (60 mg).

HRMS (ESI, [M+H]⁺) *m*/*z*: 551.2505.

¹H NMR (500 MHz, CDCl₃) δ 9.91 (s, 1H), 9.02 (d, J = 8.6 Hz, 1H), 8.84 (s, 1H), 7.87 (s, 1H), 7.65 - 7.58 (m, 2H), 7.48 (d, J = 8.5 Hz, 1H), 6.87 (d, J = 8.5 Hz, 1H), 6.46 (d, J = 2.4 Hz, 1H), 6.19 (s, 1H), 4.30 (s, 2H), 3.66 (d, J = 12.3 Hz, 1H), 3.61 - 3.53 (m, 2H), 3.50 - 3.44 (m, 1H), 3.39 - 3.35 (m, 1H), 3.02 (s, 3H), 2.76 - 2.71 (m, 1H), 2.43 - 2.38 (m, 1H), 2.28 (s, 6H), 2.08 - 1.93 (m, 2H).

### Example 62: Preparation of Compound 1-62

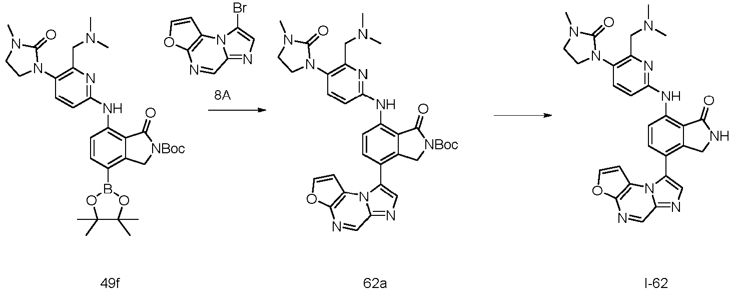

### Step A: Preparation of compound 62a

To a microwave tube were added compound 49f (140 mg), intermediate 8A (50 mg), chloro(2-dicyclohexylphosphino-2',4',6'-triisopropyl-1,1'-biphenyl)[2-(2'-atnino-1,1'-biphenyl)]palladium (II) (25 mg), 2-dicyclohexylphosphine-2',4',6'-triisopropylbiphenyl (15 mg), tripotassium phosphate (134 mg), 1,4-dioxane (3 mL), and water (0.5 mL) in sequence. After the addition, the mixture was stirred at 85 °C under microwave irradiation under nitrogen atmosphere. After the reaction was completed, the reaction solution was filtered, concentrated, and subjected to column chromatography (dichloromethane/methanol = 20/1) to give compound 62a (138 mg).

MS (ESI, [M+H]⁺) m/z: 638.3.

### Step B: Preparation of compound 1-62

To a reaction flask were added compound 62a (138 mg), a solution of hydrochloric acid in 1,4-dioxane (4 M, 1.9 mL), and dichloromethane (2 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 5/1) to give compound I-62 (32 mg).

HRMS (ESI, [M+H]⁺) *m*/*z*: 538.2311.

¹H NMR (500 MHz, CDCl₃) δ 8.99 (dd, J = 8.5, 1.7 Hz, 1H), 8.82 (d, J = 1.5 Hz, 1H), 7.87 (d, J = 1.6 Hz, 1H), 7.69 - 7.61 (m, 2H), 7.48 (dd, J = 8.6, 1.6 Hz, 1H), 7.30 (d, J = 1.6 Hz, 1H), 6.90 (dd, J = 8.5, 1.6 Hz, 1H), 6.49 (t, J = 2.0 Hz, 1H), 4.29 (s, 2H), 3.78 (ddd, J = 8.3, 6.8, 1.5 Hz, 2H), 3.71 (s, 2H), 3.57 - 3.49 (m, 2H), 2.91 (d, J= 1.6 Hz, 3H), 2.36 (s, 6H).

### Example 63: Preparation of Compound I-63

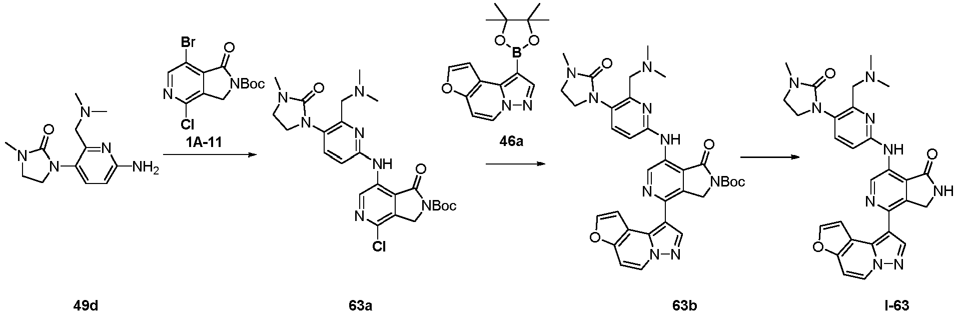

### Step A: Preparation of compound 63a

To a reaction flask were added intermediate 1A-11 (2.9 g), compound 49d (1.9 g), 1,4-dioxane (50.0 mL), 4,5-bis-diphenylphosphino-9,9-dimethylxanthene (1.3 g), cesium carbonate (3.1 g), and tris(dibenzylideneadone) (0.7 g) in sequence. After the addition, the mixture was stirred at 110 °C. After the reaction was completed, the reaction solution was filtered, concentrated, and subjected to column chromatography (dichloromethane/methanol = 30/1) to give compound 63a (3.1 g).

MS (ESI, [M+H]⁺) *m*/*z*: 516.0.

### Step B: Preparation of compound 63b

To a microwave tube were added compound 63a (250 mg), compound 46a (125 mg), chloro(2-dicyclohexylphosphino-2',4',6'-triisopropyl-1,1'-biphenyl)[2-(2'-atnino-1,1'-biphenyl)]palladium (II) (31 mg), 2-dicyclohexylphosphine-2',4',6'-triisopropylbiphenyl (52 mg), tripotassium phosphate (280 mg), 1,4-dioxane (6 mL), and water (1 mL) in sequence. After the addition, the mixture was stirred at 85 °C under microwave irradiation under nitrogen atmosphere. After the reaction was completed, the reaction solution was filtered, concentrated, and subjected to column chromatography (dichloromethane/methanol = 20/1) to give compound 63b (138 mg).

MS (ESI, [M+H]⁺) m/z: 638.3.

### Step C: Preparation of compound 1-63

To a reaction flask were added compound 63b (138 mg), a solution of hydrochloric acid in 1,4-dioxane (4 M, 2.1 mL), and dichloromethane (2 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 5/1) to give compound I-63 (48 mg).

HRMS (ESI, [M+H]⁺) m/z: 538.2313.

¹H NMR (500 MHz, CDCl₃) δ 10.15 (s, 1H), 8.32 (d, J = 7.5 Hz, 1H), 8.07 (s, 1H), 7.72 - 7.62 (m, 2H), 7.45 (d, J = 8.6 Hz, 1H), 7.30 (s, 1H), 7.11 (d, J = 7.5 Hz, 1H), 6.84 (d, J = 8.6 Hz, 1H), 4.59 (s, 2H), 3.79 (t, J = 7.8 Hz, 2H), 3.73 (s, 2H), 3.53 (t, J = 7.8 Hz, 2H), 2.92 (s, 3H), 2.45 (s, 6H).

### Example 64: Preparation of Compound I-64

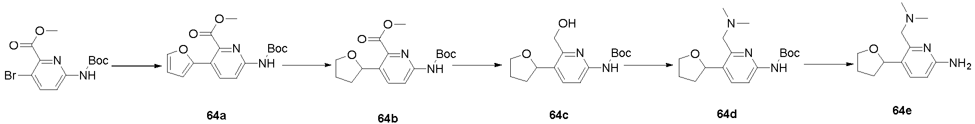

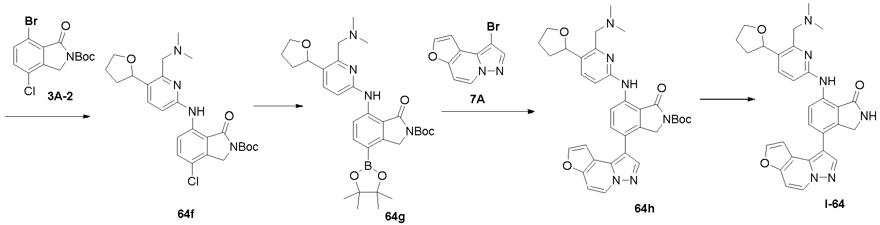

### Step A: Preparation of compound 64a

To a reaction flask were added the compounds methyl 3-bromo-6-((*tert*-butoxycarbonyl)amino)picolinate (10.0 g), 1,4-dioxane (150.0 mL), water (30.0 mL), 4,5-bis-diphenylphosphino-9,9-dimethylxanthene (3.5 g), potassium carbonate (12.5 g), and furan-2-boronic acid pinacol ester (7.0 g) in sequence. After the addition, the mixture was purged with nitrogen and stirred at 90 °C. After the reaction was completed, the reaction solution was filtered, concentrated, and subjected to column chromatography (dichloromethane/methanol = 30/1) to give compound 64a (8.2 g).

MS (ESI, [M+H]⁺) *m*/*z*: 319.2.

### Step B: Preparation of compound 64b

To a reaction flask were added compound 64a (8.2 g), tetrahydrofuran (50.0 mL), methanol (50.0 mL), 10 wt% palladium hydroxide on carbon (8.6 g), acetic acid (5.5 mL), and ammonium formate (5.0 g) in sequence. After the addition, the mixture was purged with hydrogen and stirred at room temperature. After the reaction was completed, the reaction solution was filtered and concentrated. The residue was adjusted to alkalinity (about pH 8) with a saturated aqueous sodium bicarbonate solution and extracted with dichloromethane. The organic phase was dried, filtered, and concentrated to give compound 64b (7.0 g).

MS (ESI, [M+H]⁺) *m*/*z*: 323.2.

### Step C: Preparation of compound 64c

To a reaction flask were added compound 64b (6.0 g), tetrahydrofuran (50.0 mL), and lithium borohydride (1.0 g) in sequence at 0 °C. After the addition, the mixture was purged with nitrogen and stirred at 0 °C. After the reaction was completed, water was added to the reaction solution to quench the reaction, and the resulting mixture was extracted with dichloromethane. The organic phase was isolated, dried, filtered, concentrated, and subjected to column chromatography (petroleum ether/ethyl acetate = 5/1) to give compound 64c (5.0 g).

MS (ESI, [M+H]⁺) *m*/*z*: 295.2.

### Step D: Preparation of compound 64d

To a reaction flask were added compound 64c (5.0 g), dichloromethane (30.0 mL), *N,N*-diisopropylethylamine (2.97 mL), and methanesulfonic anhydride (5.2 mL) in sequence at 0 °C. After the addition, the mixture was stirred at 0 °C. After the reaction was completed, the reaction solution was slowly added to a solution of dimethylamine in tetrahydrofuran (2 M, 15 mL) at 0 °C. After the addition, the mixture was stirred at 0 °C. After the reaction was completed, the reaction solution was concentrated and subjected to column chromatography (petroleum ether/ethyl acetate = 5/1) to give compound 64d (4.9 g).

MS (ESI, [M+H]⁺) *m*/*z*: 322.2.

### Step E: Preparation of compound 64e

To a reaction flask were added compound 64d (4.9 g), dichloromethane (30.0 mL), and a solution of hydrochloric acid in 1,4-dioxane (4 M, 38 mL) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was isolated, dried, filtered, concentrated, and subjected to column chromatography (dichloromethane/methanol = 5/1) to give compound 64e (1.4 g).

MS (ESI, [M+H]⁺) *m*/*z*: 222.4.

### Step F: Preparation of compound 64f

To a reaction flask were added intermediate 3A-2 (1.2 g), compound 64e (0.7 g), 1,4-dioxane (30.0 mL), 4,5-bis-diphenylphosphino-9,9-dimethylxanthene (0.4 g), cesium carbonate (2.9 g), and tris(dibenzylideneadone) (0.5 g) in sequence. After the addition, the mixture was purged with nitrogen and stirred at 110 °C. After the reaction was completed, the reaction solution was filtered, concentrated, and subjected to column chromatography (dichloromethane/methanol = 30/1) to give compound 64f (1.3 g).

MS (ESI, [M+H]⁺) *m*/*z*: 487.2.

### Step G: Preparation of compound 64g

To a reaction flask were added compound 64f (1.3 g), potassium acetate (0.7 g), bis(pinacolato)diboron (0.8 g), chloro(2-dicyclohexylphosphino-2',4',6'-triisopropyl-1,1'-biphenyl)[2-(2'-amino-1,1'-biphenyl)]palladium (II) (0.2 g), and 1,4-dioxane (50.0 mL) in sequence. After the addition, the mixture was purged with nitrogen and stirred at 90 °C. After the reaction was completed, the reaction solution was filtered, concentrated, and subjected to column chromatography (dichloromethane/methanol = 20/1) to give compound 64g (435 mg).

MS (ESI, [M+H]⁺) *m*/*z*: 579.6.

### Step H: Preparation of compound 64h

To a reaction flask were added compound 64g (215 mg), 2-dicyclohexylphosphine-2',4',6'-triisopropylbiphenyl (43 mg), potassium phosphate (314 mg), chloro(2-dicyclohexylphosphino-2',4',6'-triisopropyl-1,1'-biphenyl)[2-(2'-amino-1,1'-biphenyl)]palladium (II) (72 mg), intermediate 7A (105 mg), water (3.0 mL), and 1,4-dioxane (12.0 mL) in sequence. After the addition, the mixture was stirred at 85 °C. After the reaction was completed, the reaction solution was filtered, concentrated, and subjected to column chromatography (dichloromethane/methanol = 10/1) to give compound 64h (132 mg).

MS (ESI, [M+H]⁺) *m*/*z*: 609.1.

### Step I: Preparation of compound 1-64

To a reaction flask were added compound 64h (132 mg), a solution of hydrochloric acid in 1,4-dioxane (4 M, 2.1 mL), and dichloromethane (3 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 5/1) to give compound I-64 (18 mg).

HRMS (ESI, [M+H]⁺) *m*/*z*: 509.2311.

¹H NMR (500 MHz, CDCl₃) δ 8.85 (dd, J = 8.4, 3.2 Hz, 1H), 8.39 (dd, J = 7.4, 2.8 Hz, 1H), 8.00 (d, J = 2.6 Hz, 1H), 7.71 (dd, J = 8.2, 2.8 Hz, 1H), 7.67 - 7.61 (m, 2H), 7.32 (d, J = 2.5 Hz, 1H), 7.12 (dd, J = 7.4, 2.8 Hz, 1H), 6.87 (d, J = 8.5 Hz, 1H), 6.74 (d, J = 2.3 Hz, 1H), 5.27 - 5.18 (m, 1H), 4.37 (s, 2H), 4.14 (td, J = 7.8, 7.1, 5.4 Hz, 1H), 3.94 (td, J = 7.8, 4.8 Hz, 1H), 3.73 (d, J = 12.6 Hz, 1H), 3.62 - 3.53 (m, 1H), 2.45 - 2.37 (m, 1H), 2.34 (d, J = 2.7 Hz, 6H), 2.07 (qd, J = 6.8, 3.7 Hz, 2H), 1.79 - 1.65 (m, 1H).

### Example 65: Preparation of Compound I-65

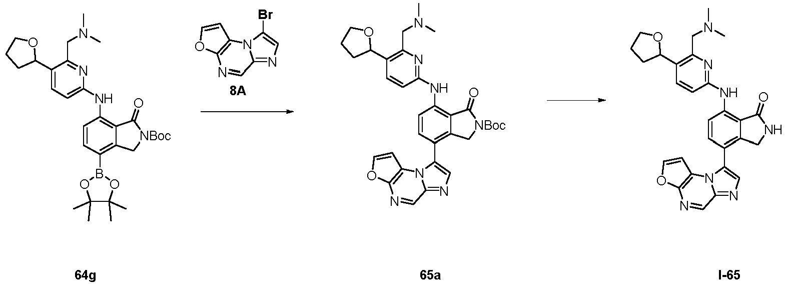

### Step A: Preparation of compound 65a

To a microwave tube were added compound 64g (153 mg), intermediate 8A (60 mg), chloro(2-dicyclohexylphosphino-2',4',6'-triisopropyl-1,1'-biphenyl)[2-(2'-atnino-1,1'-biphenyl)]palladium (II) (29 mg), 2-dicyclohexylphosphine-2',4',6'-triisopropylbiphenyl (19 mg), tripotassium phosphate (161 mg), 1,4-dioxane (6 mL), and water (1 mL) in sequence. After the addition, the mixture was stirred at 85 °C under microwave irradiation under nitrogen atmosphere. After the reaction was completed, the reaction solution was filtered, concentrated, and subjected to column chromatography (dichloromethane/methanol = 20/1) to give compound 65a (200 mg).

MS (ESI, [M+H]⁺) m/z: 610.1.

### Step B: Preparation of compound 1-65

To a reaction flask were added compound 65a (200 mg), a solution of hydrochloric acid in 1,4-dioxane (4 M, 1.9 mL), and dichloromethane (2 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 5/1) to give compound I-65 (9 mg).

HRMS (ESI, [M+H]⁺) *m*/*z*: 510.2246.

¹H NMR (500 MHz, CDCl₃) δ 8.99 (dd, J = 8.5, 1.7 Hz, 1H), 8.82 (d, J = 1.5 Hz, 1H), 7.87 (d, J = 1.6 Hz, 1H), 7.69 - 7.61 (m, 2H), 7.48 (dd, J = 8.6, 1.6 Hz, 1H), 7.30 (d, J = 1.6 Hz, 1H), 6.90 (dd, J = 8.5, 1.6 Hz, 1H), 6.49 (t, J = 2.0 Hz, 1H), 4.29 (s, 2H), 3.78 (ddd, J = 8.3, 6.8, 1.5 Hz, 2H), 3.71 (s, 2H), 3.57 - 3.49 (m, 2H), 2.91 (d, J = 1.6 Hz, 3H), 2.36 (s, 6H).

### Example 66: Preparation of Compound 1-66

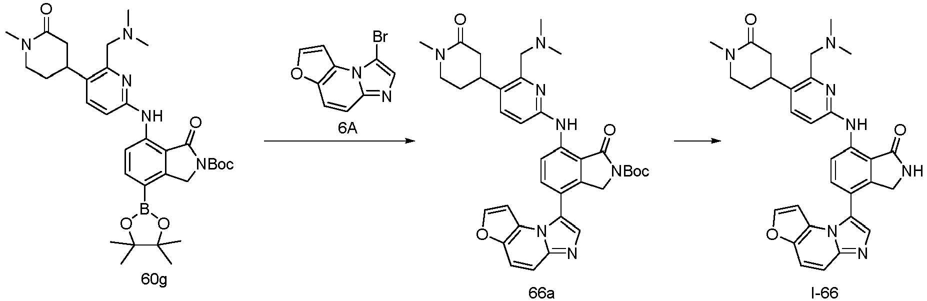

### Step A: Preparation of compound 66a

To a reaction flask were added compound 60g (300 mg), intermediate 6A (115 mg), chloro(2-dicyclohexylphosphino-2',4',6'-triisopropyl-1,1 '-biphenyl)[2-(2'-amino-1,1 '-biphenyl)]palladium (II) (38 mg), tripotassium phosphate (206 mg), 1,4-dioxane (7.0 mL), and water (1.0 mL) in sequence. The mixture was purged with nitrogen and stirred at 85 °C under microwave irradiation. After the reaction was completed, the reaction solution was concentrated and purified by silica gel column chromatography (dichloromethane/methanol = 20/1) to give compound 66a (127 mg).

MS (ESI, [M+H]⁺) m/z: 650.43.

### Step B: Preparation of compound 1-66

To a reaction flask were added compound 66a (127 mg), dichloromethane (2 mL), and a solution of hydrochloric acid in 1,4-dioxane (0.4 mL, 4 M) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and purified by silica gel column chromatography (dichloromethane/methanol = 10/1) to give compound I-66 (58 mg).

HRMS (ESI, [M+H]⁺) *m*/*z*: 550.2546.

¹H NMR (500 MHz, CDCl₃) δ 9.87 (s, 1H), 8.97 (d, J = 8.5 Hz, 1H), 7.66 (s, 1H), 7.60 (d, J = 8.6 Hz, 1H), 7.58 - 7.51 (m, 3H), 7.47 (d, J = 8.5 Hz, 1H), 6.87 (d, J = 8.5 Hz, 1H), 6.29 (d, J = 2.2 Hz, 1H), 6.11 (s, 1H), 4.24 (s, 2H), 3.65 (d, J = 12.3 Hz, 1H), 3.61 - 3.52 (m, 2H), 3.47 (td, J = 11.7, 4.8 Hz, 1H), 3.38 - 3.34 (m, 1H), 3.02 (s, 3H), 2.76 - 2.71 (m, 1H), 2.40 (dd, J = 17.3, 11.7 Hz, 1H), 2.28 (s, 6H), 2.07 - 1.94 (m, 2H).

### Example 67: Preparation of Compound I-67

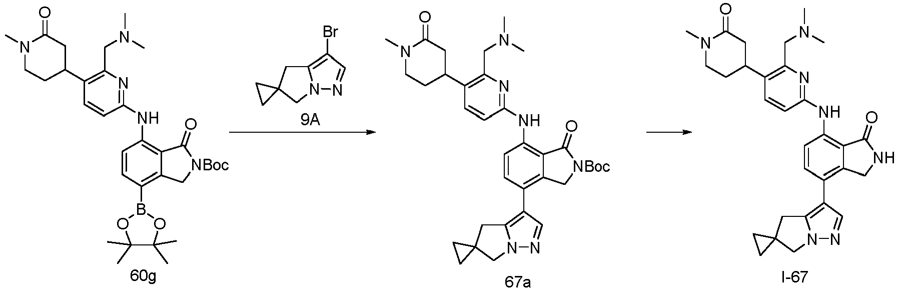

### Step A: Preparation of compound 67a

To a reaction flask were added compound 60g (250 mg), intermediate 9A (86 mg), chloro(2-dicyclohexylphosphino-2',4',6'-triisopropyl-1,1'-biphenyl)[2-(2'-amino-1,1'-biphenyl)]palladium (II) (32 mg), tripotassium phosphate (171 mg), 1,4-dioxane (7.0 mL), and water (1.0 mL) in sequence. The mixture was stirred at 85 °C under microwave irradiation under nitrogen atmosphere. After the reaction was completed, the reaction solution was concentrated and purified by silica gel column chromatography (dichloromethane/methanol = 20/1) to give compound 67a (72 mg). MS (ESI, [M+H]⁺) m/z: 626.51.

### Step B: Preparation of compound 1-67

To a reaction flask were added compound 67a (72 mg), methanesulfonic acid (0.10 mL), and dichloromethane (2 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and purified by silica gel column chromatography (dichloromethane/methanol = 5/1) to give compound I-67 (30 mg).

HRMS (ESI, [M+H]⁺) *m*/*z*: 526.2932.

¹H NMR (500 MHz, CDCl₃) δ 9.76 (s, 1H), 8.77 (d, J = 8.6 Hz, 1H), 7.64 (s, 1H), 7.43 (dd, J = 19.1, 8.5 Hz, 2H), 6.80 (d, J = 8.4 Hz, 1H), 6.42 (s, 1H), 4.53 (s, 2H), 4.14 (s, 2H), 3.63 (d, J = 12.2 Hz, 1H), 3.60 - 3.50 (m, 2H), 3.48 - 3.42 (m, 1H), 3.37 - 3.33 (m, 1H), 3.10 (s, 2H), 3.01 (s, 3H), 2.74 -2.69 (m, 1H), 2.39 (dd, J = 17.4, 11.6 Hz, 1H), 2.27 (s, 6H), 2.05 - 1.91 (m, 2H), 0.94 - 0.84 (m, 4H).

### Example 68 and Example 69: Preparation of Compound 1-68 and Compound 1-69

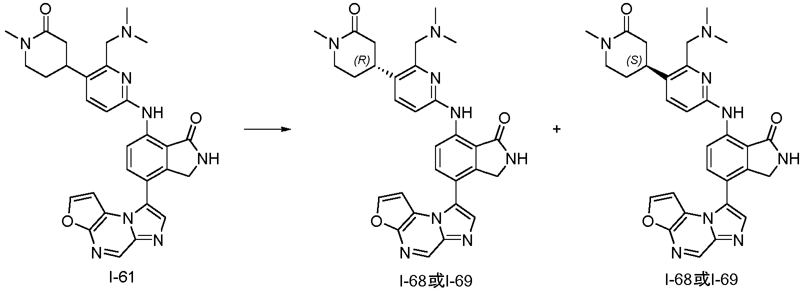

Compound I-61 (40 mg) was subjected to chiral preparation using YMC high-pressure preparative chromatograph on CHIRALART Amylose-SA (specification: 30 × 250 mm, 5 µm) column (mobile phase A: 0.1% ethanol:dichloromethane (1:1), mobile phase B: n-hexane, isocratic elution at a flow rate of 40 mL/min with 60% mobile phase B over 20 min) to give compound I-68 (prepeak: 18 mg) and compound I-69 (postpeak: 18 mg) in sequence.
I-68: HRMS (ESI, [M+H]⁺) *m*/*z:* 551.2515.
I-69: HRMS (ESI, [M+H]⁺) *m*/*z:* 551.2518.

### Example 70: Preparation of Compound 1-70

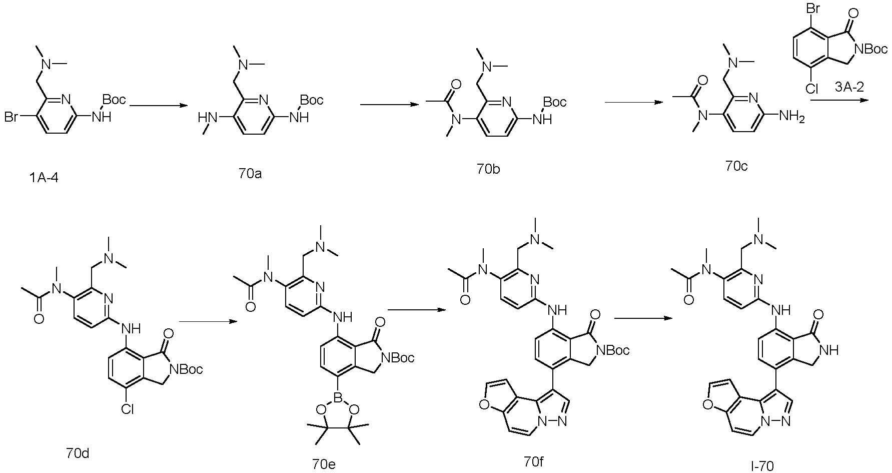

### Step A: Preparation of compound 70a

To a reaction flask were added intermediate 1A-4 (5.0 g), cuprous oxide (0.6 mL), and an aqueous methylamine solution (7.4 g) in sequence. After the addition, the mixture was purged with nitrogen and stirred at 80 °C. After the reaction was completed, the reaction solution was filtered, concentrated, and purified by silica gel column chromatography (dichloromethane/methanol = 30/1) to give compound 70a (4.0 g).

MS (ESI, [M+H]⁺) *m*/*z*: 281.0.

### Step B: Preparation of compound 70b

To a reaction flask were added compound 70a (4.0 g), dichloromethane (50.0 mL), triethylamine (19.8 mL), and acetic anhydride (2.2 g) in sequence. After the addition, the mixture was stirred at room temperature. After the reaction was completed, the reaction solution was extracted with a saturated aqueous ammonium chloride solution and dichloromethane. The organic phase was dried, filtered, concentrated, and purified by silica gel column chromatography (dichloromethane/methanol = 20/1) to give compound 70b (2.5 g).

MS (ESI, [M+H]⁺) *m*/*z*: 323.1.

### Step C: Preparation of compound 70c

To a reaction flask were added compound 70b (2.5 g), dichloromethane (10.0 mL), and a solution of hydrochloric acid in 1,4-dioxane (4 M, 19.0 mL) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, and concentrated to give compound 70c (1.2 g).

MS (ESI, [M+H]⁺) *m*/*z*: 223.4.

### Step D: Preparation of compound 70d

To a reaction flask were added intermediate 3A-2 (1.3 g), compound 70c (0.7 g), 1,4-dioxane (50.0 mL), 4,5-bis-diphenylphosphino-9,9-dimethylxanthene (0.4 g), cesium carbonate (2.9 g), and tris(dibenzylideneadone) (0.5 g) in sequence. After the addition, the mixture was purged with nitrogen and stirred at 110 °C. After the reaction was completed, the reaction solution was filtered, concentrated, and purified by silica gel column chromatography (dichloromethane/methanol = 30/1) to give compound 70d (0.7 g).

MS (ESI, [M+H]⁺) *m*/*z*: 488.3.

### Step E: Preparation of compound 70e

To a reaction flask were added compound 70d (0.7 g), potassium acetate (0.4 g), bis(pinacolato)diboron (0.5 g), chloro(2-dicyclohexylphosphino-2',4',6'-triisopropyl-1,1'-biphenyl)[2-(2'-amino-1,1'-biphenyl)]palladium (II) (0.2 g), and 1,4-dioxane (30.0 mL) in sequence. After the addition, the mixture was purged with nitrogen and stirred at 90 °C. After the reaction was completed, the reaction solution was filtered, concentrated, and purified by silica gel column chromatography (petroleum ether/ethyl acetate = 5/1) to give compound 70e (0.3 g).

MS (ESI, [M+H]⁺) *m*/*z*: 580.2.

### Step F: Preparation of compound 70f

To a reaction flask were added compound 70e (0.2 g), 2-dicyclohexylphosphine-2',4',6'-triisopropylbiphenyl (0.03 g), potassium phosphate (0.2 g), chloro(2-dicyclohexylphosphino-2',4',6'-trilsopropyl-1,1'-biphenyl)[2-(2'-amino-1,1'-biphenyl)]palladium (II) (0.1 g), intermediate 7A (0.07 g), water (3.0 mL), and 1,4-dioxane (12.0 mL) in sequence. After the addition, the mixture was stirred at 85 °C. After the reaction was completed, the reaction solution was filtered, concentrated, and purified by silica gel column chromatography (petroleum ether/ethyl acetate = 5/1) to give compound 70f (0.2 g).

MS (ESI, [M+H]⁺) *m*/*z*: 610.3.

### Step H: Preparation of compound 1-70

To a reaction flask were added compound 70f (0.2 g), methanesulfonic acid (0.3 mL), and dichloromethane (10 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and purified by silica gel column chromatography (dichloromethane/methanol = 5/1) to give compound I-70 (0.04 g).

HRMS (ESI, [M+H]⁺) *m*/*z*: 510.2255.

¹HNMR (500 MHz, CDCl₃) δ 10.02 (s, 1H), 8.94 (d, J = 8.5 Hz, 1H), 8.40 (d, J = 7.5 Hz, 1H), 8.01 (s, 1H), 7.67 (d, J = 8.5 Hz, 1H), 7.63 (d, J = 2.1 Hz, 1H), 7.35 (d, J = 8.5 Hz, 1H), 7.11 (dd, J = 7.6, 0.9 Hz, 1H), 6.87 (d, J = 8.5 Hz, 1H), 6.72 (dd, J = 2.1, 0.9 Hz, 1H), 6.37 (s, 1H), 4.41 (s, 2H), 3.49 (d, J = 2.3 Hz, 2H), 3.24 (s, 3H), 2.37 (s, 6H), 1.86 (s, 3H).

### Example 71: Preparation of Compound I-71a and Compound I-71b

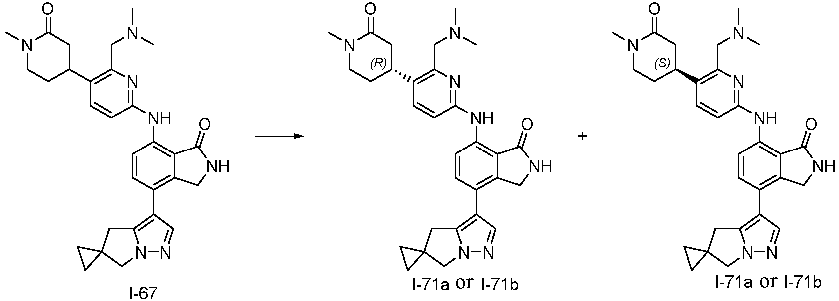

Compound I-67 (43 mg) was subjected to chiral resolution and preparation. Compound I-67 (43 mg) was subjected to chiral preparation using YMC high-pressure preparative chromatograph on CHIRALART Amylose-SA (specification: 30 × 250 mm, 5 µm) column (mobile phase A: 0.1% ethanol:dichloromethane (1:1), mobile phase B: n-hexane, isocratic elution at a flow rate of 40 mL/min with 60% mobile phase B over 20 min) to give compound I-71a (prepeak: 20 mg) and compound I-71b (postpeak: 22 mg) in sequence.
I-71a: HRMS (ESI, [M+H]⁺) *m*/*z*: 526.2935.
I-71b: HRMS (ESI, [M+H]⁺) *m*/*z*: 526.2926.

### Example 72 and Example 73: Preparation of Compound I-72 and Compound I-73

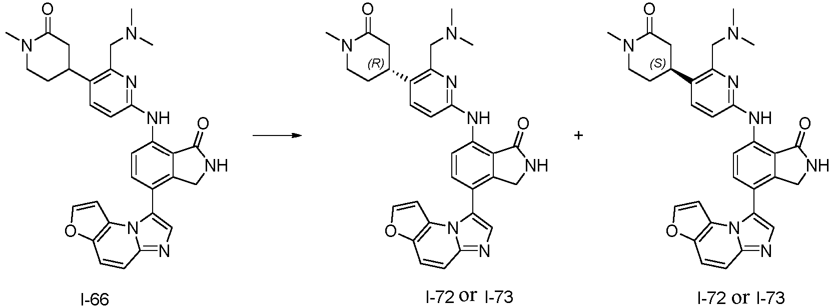

Compound I-66 (46 mg) was subjected to chiral resolution and preparation to give compound I-72 (23 mg) and compound I-73 (21 mg) in sequence. Compound I-66 (46 mg) was subjected to chiral preparation using YMC high-pressure preparative chromatograph on CHIRALART Amylose-SA (specification: 30 × 250 mm, 5 µm) column (mobile phase A: 0.1% ethanol:dichloromethane (1:1), mobile phase B: *n*-hexane, isocratic elution at a flow rate of 40 mL/min with 60% mobile phase B over 20 min) to give compound I-72 (prepeak: 23 mg) and compound I-73 (postpeak: 21 mg) in sequence.
I-72: HRMS (ESI, [M+H]⁺) *m*/*z:* 550.2558.
I-73: HRMS (ESI, [M+H]⁺) *m*/*z:* 550.2547.

### Example 74: Preparation of Compound I-74

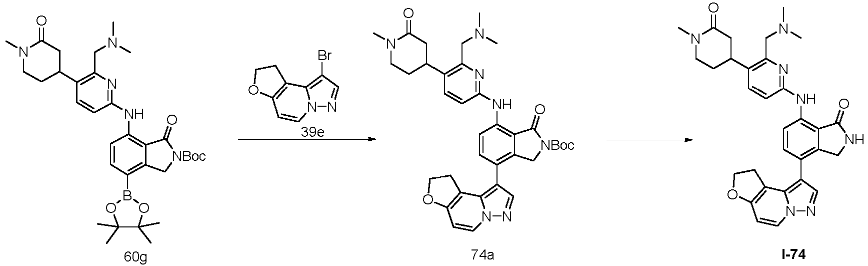

### Step A: Preparation of compound 74a

To a microwave reaction flask were added compound 60g (300 mg), compound 39e (150 mg), chloro(2-dicyclohexylphosphino-2',4',6'-triisopropyl-1,1 '-biphenyl)[2-(2'-amino-1,1 '-biphenyl)]palladium (II) (38 mg), tripotassium phosphate (308 mg), 1,4-dioxane (10.0 mL), and water (1.0 mL) in sequence. The mixture was stirred at 110 °C under nitrogen atmosphere. After the reaction was completed, the reaction solution was concentrated and purified by silica gel column chromatography (dichloromethane/methanol = 20/1) to give compound 74a (102 mg). MS (ESI, [M+H]⁺) m/z: 652.43.

### Step B: Preparation of compound I-74

To a reaction flask were added compound 74a (0.1 g), a solution of hydrochloric acid in 1,4-dioxane (4 M, 1 mL), and dichloromethane (10 mL) in sequence. After the addition, the mixture was stirred at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and purified by silica gel column chromatography (dichloromethane/methanol = 6/1) to give compound I-74 (0.046 g).

HRMS (ESI, [M+H]⁺) *m*/*z*: 552.2725.

¹H NMR (500 MHz, CDCl₃:CD₃OD=10:1) δ 8.80 (d, *J =* 8.6 Hz, 1H), 8.29 (s, 1H), 7.88 (s, 1H), 7.58 (d, *J =* 8.5 Hz, 1H), 7.42 (d, *J =* 8.5 Hz, 1H), 6.86 - 6.79 (m, 2H), 4.69 (t, *J =* 8.1 Hz, 2H), 4.42 (s, 2H), 3.68 (d, *J =* 12.3 Hz, 1H), 3.58 - 3.44 (m, 2H), 3.40 - 3.33 (m, 1H), 3.31 (td, *J =* 8.2, 1.7 Hz, 2H), 3.02 (s, 3H), 2.70 (dd, *J =* 17.5, 11.6 Hz, 1H), 2.39 (dd, *J =* 17.5, 11.6 Hz, 1H), 2.32 (s, 6H), 2.08 - 1.95 (m, 4H).

### Example 75: Preparation of Compound I-75

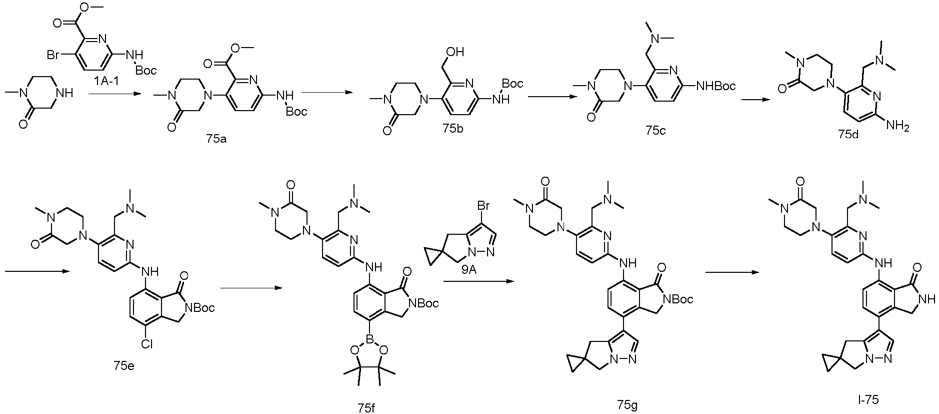

### Step A: Preparation of compound 75a

To a reaction flask were added 1-methylpiperazin-2-one (7.76 g), intermediate 1A-1 (15 g), tris(dibenzylideneacetone)dipalladium(0) (4.15 g), 4,5-bis-diphenylphosphino-9,9-dimethylxanthene (5.24 g), cesium carbonate (44.3 g), and 1,4-dioxane (300 mL) in sequence. After the addition, the mixture was heated and stirred at 110 °C under nitrogen atmosphere. After the reaction was completed, the reaction solution was filtered, concentrated, and purified by silica gel column chromatography (dichloromethane/methanol = 20/1) to give compound 75a (4.0 g).

MS (ESI, [M+H]⁺) m/z: 365.2.

### Step B: Preparation of compound 75b

To a reaction flask were added compound 75a (4.0 g), tetrahydrofuran (100 mL), and a solution of lithium borohydride in tetrahydrofuran (11 mL, 2 M) in sequence at 0 °C. After the addition, the mixture was stirred at room temperature. After the reaction was completed, the reaction was quenched with a saturated ammonium chloride solution, and the reaction solution was extracted with ethyl acetate. The organic phase was isolated, dried, filtered, and concentrated to give compound 75b (1.71 g).

MS (ESI, [M+H]⁺) *m*/*z:* 337.2.

### Step C: Preparation of compound 75c

To a reaction flask were added compound 75b (1.71 g), dichloromethane (30 mL), *N*,*N*-diisopropylethylamine (1.26 g), and methanesulfonic anhydride (2.20 g) in sequence at -10 °C. After the addition, the mixture was stirred at 0 °C. After the reaction was completed, the reaction solution was added dropwise to a solution of dimethylamine in tetrahydrofuran (2 M, 51.50 mL) at -10 °C. After the addition, the mixture was stirred at room temperature. After the reaction was completed, the reaction solution was concentrated and purified by silica gel column chromatography (dichloromethane:methanol = 20:1) to give compound 75c (1.30 g).

MS (ESI, [M+H]⁺) *m*/*z:* 364.29.

### Step D: Preparation of compound 75d

To a reaction flask were added compound 75c (1.00 g), dichloromethane (40 mL), and a solution of hydrochloric acid in 1,4-dioxane (10.32 mL, 4 M) in sequence. After the addition, the mixture was stirred at room temperature. After the reaction was completed, the reaction solution was concentrated, adjusted to alkalinity (about pH 10) with a saturated sodium bicarbonate solution, and extracted with dichloromethane. The organic phase was isolated, dried, filtered, and concentrated to give compound 70d (0.70 g).

MS (ESI, [M+H]⁺) *m*/*z*: 264.16.

### Step E: Preparation of compound 75e

To a reaction flask were added compound 75d (0.52 g), intermediate 3A-2 (0.68 g), tris(dibenzylideneacetone)dipalladium(0) (0.18 g), 4,5-bis-diphenylphosphino-9,9-dimethylxanthene (0.14 g), cesium carbonate (1.92 g) and 1,4-dioxane (50 mL) in sequence. The mixture was purged with nitrogen and was stirred at 110 °C. After the reaction was completed, the reaction solution was filtered, concentrated, and purified by silica gel column chromatography (dichloromethane/methanol = 20/1) to give compound 75e (0.90 g).

MS (ESI, [M+H]⁺) *m*/*z*: 529.28.

### Step F: Preparation of compound 75f

To a microwave reaction flask were added compound 75e (0.90 g), bis(pinacolato)diboron (0.78 g), chloro(2-dicyclohexylphosphino-2',4',6'-triisopropyl-1,1 '-biphenyl)[2-(2'-amino-1,1 '-biphenyl)]palladium (II) (0.27 g), potassium acetate (0.50 g), and 1,4-dioxane (15 mL) in sequence. The mixture was purged with nitrogen and stirred at 90 °C under microwave irradiation. After the reaction was completed, the reaction solution was filtered, concentrated, and purified by silica gel column chromatography (dichloromethane/methanol = 20/1) to give compound 75f (0.75 g).

MS (ESI, [M+H]⁺) m/z: 621.42.

### Step G: Preparation of compound 75g

To a microwave reaction flask were added compound 75f (300 mg), intermediate 9A (103 mg), chloro(2-dicyclohexylphosphino-2',4',6'-triisopropyl-1,1'-biphenyl)[2-(2'-atnino-1,1'-biphenyl)]palladium (II) (38 mg), 2-dicyclohexylphosphine-2',4',6'-triisopropylbiphenyl (23 mg), tripotassium phosphate (205 mg), 1,4-dioxane (7.0 mL), and water (1.0 mL) in sequence. The mixture was stirred at 85 °C under microwave irradiation under nitrogen atmosphere. After the reaction was completed, the reaction solution was concentrated and purified by silica gel column chromatography (dichloromethane/methanol = 20/1) to give compound 75g (94 mg).

MS (ESI, [M+H]⁺) m/z: 627.48.

### Step H: Preparation of compound 1-75

To a reaction flask were added compound 75g (94 mg), a solution of hydrochloric acid in 1,4-dioxane (4 M, 1.0 mL), and dichloromethane (4 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and subjected to column chromatography (dichloromethane/methanol = 5/1) to give compound I-75 (35 mg).

HRMS (ESI, [M+H]⁺) *m*/*z*: 527.2887.

¹H NMR (500 MHz, CDCl₃) δ 9.76 (s, 1H), 8.74 (d, J = 8.6 Hz, 1H), 7.63 (s, 1H), 7.42 (d, J = 8.6 Hz, 1H), 7.33 (d, J = 8.6 Hz, 1H), 6.79 (d, J = 8.6 Hz, 1H), 6.40 (s, 1H), 4.51 (s, 2H), 4.13 (s, 2H), 3.65 (d, J = 23.1 Hz, 4H), 3.37 (dt, J = 10.5, 4.9 Hz, 4H), 3.06 (d, J = 18.5 Hz, 5H), 2.37 (s, 6H), 0.88 (d, J = 7.6 Hz, 4H).

### Example 76: Preparation of Compound I-76

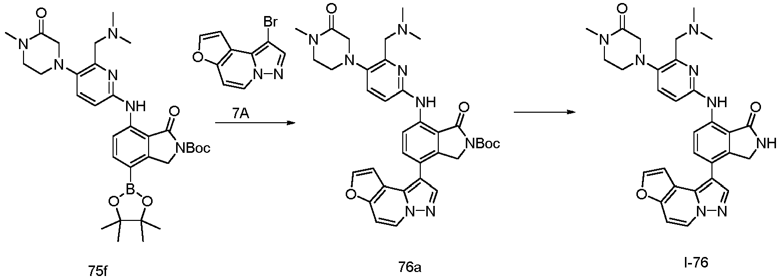

### Step A: Preparation of compound 76a

To a microwave reaction flask were added compound 75f (250 mg), intermediate 7A (96 mg), chloro(2-dicyclohexylphosphino-2',4',6'-triisopropyl-1,1'-biphenyl)[2-(2'-atnino-1,1'-biphenyl)]palladium (II) (32 mg), 2-dicyclohexylphosphine-2',4',6'-triisopropylbiphenyl (19 mg), tripotassium phosphate (171 mg), 1,4-dioxane (7.0 mL), and water (1.0 mL) in sequence. The mixture was stirred at 85 °C under microwave irradiation under nitrogen atmosphere. After the reaction was completed, the reaction solution was concentrated and purified by silica gel column chromatography (dichloromethane/methanol = 20/1) to give compound 76a (92 mg).

MS (ESI, [M+H]⁺) m/z: 651.39.

### Step B: Preparation of compound I-76

To a reaction flask were added compound 76a (92 mg), a solution of hydrochloric acid in 1,4-dioxane (4 M, 1.0 mL), and dichloromethane (4 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and purified by silica gel column chromatography (dichloromethane/methanol = 5/1) to give compound I-76 (30 mg).

HRMS (ESI, [M+H]⁺) *m*/*z*: 551.2519.

¹H NMR (500 MHz, CDCl₃) δ 9.78 (s, 1H), 8.87 (d, J = 8.5 Hz, 1H), 8.38 (d, J = 7.6 Hz, 1H), 7.99 (s, 1H), 7.62 (dd, J = 5.3, 3.2 Hz, 2H), 7.36 (d, J = 8.7 Hz, 1H), 7.09 (d, J = 7.6 Hz, 1H), 6.83 (d, J = 8.6 Hz, 1H), 6.72 (d, J = 1.5 Hz, 1H), 6.29 (s, 1H), 4.37 (s, 2H), 3.67 (d, J = 16.4 Hz, 4H), 3.39 (dt, J = 10.5, 4.9 Hz, 4H), 3.05 (s, 3H), 2.39 (s, 6H).

### Example 77: Preparation of Compound I-77

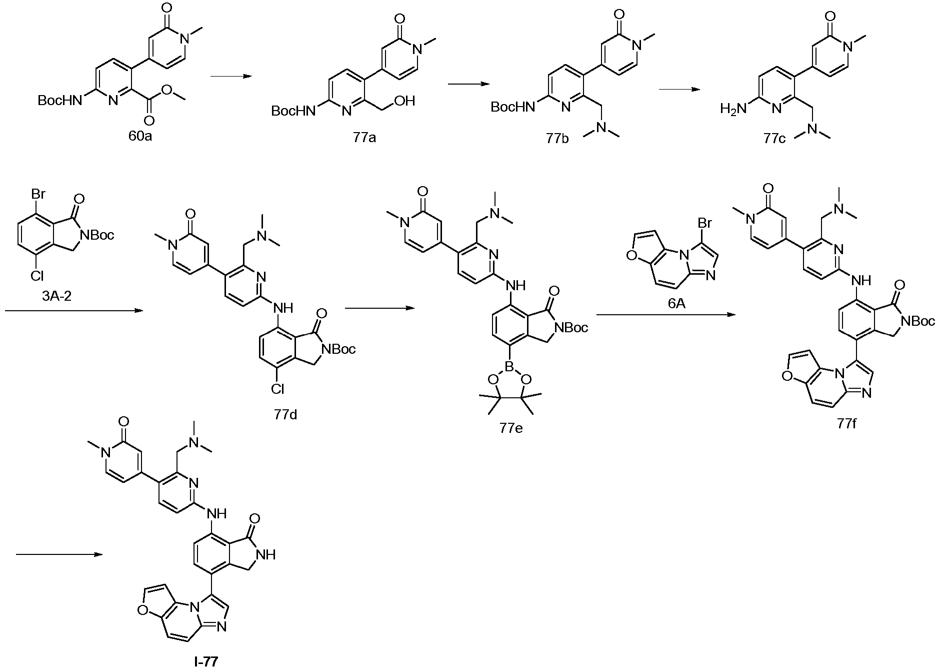

### Step A: Preparation of compound 77a

To a reaction flask were added compound 60a (2.00 g), tetrahydrofuran (25 mL), and a solution of lithium borohydride in tetrahydrofuran (6.96 mL, 2 M) in sequence at 0 °C. After the addition, the mixture was stirred at room temperature. After the reaction was completed, the reaction was quenched with a saturated ammonium chloride solution, and the reaction solution was extracted with ethyl acetate. The organic phase was isolated, dried, filtered, and concentrated to give compound 77a (1.21 g).

MS (ESI, [M+H]⁺) *m*/*z:* 332.10.

### Step B: Preparation of compound 77b

To a reaction flask were added compound 77a (1.21 g), dichloromethane (15 mL), *N*,*N*-diisopropylethylamine (0.94 g), and methanesulfonic anhydride (1.27 g) in sequence at -10 °C. After the addition, the mixture was stirred at 0 °C. After the reaction was completed, the reaction solution was added dropwise to a solution of dimethylamine in tetrahydrofuran (2 M, 18.25 mL) at -10 °C. After the addition, the mixture was stirred at room temperature. After the reaction was completed, the reaction solution was concentrated and purified by silica gel column chromatography (dichloromethane:methanol = 20:1) to give compound 77b (1.00 g).

MS (ESI, [M+H]⁺) *m*/*z*: 359.28.

### Step C: Preparation of compound 77c

To a reaction flask were added compound 77b (0.90 g), dichloromethane (10 mL), and a solution of hydrochloric acid in 1,4-dioxane (6.28 mL, 2 M) in sequence. After the addition, the mixture was stirred at room temperature. After the reaction was completed, the reaction solution was concentrated, adjusted to alkalinity (about pH 10) with a saturated sodium bicarbonate solution, and extracted with dichloromethane. The organic phase was isolated, dried, filtered, and concentrated to give compound 77c (0.67 g).

MS (ESI, [M+H]⁺) *m*/*z*: 259.15.

### Step D: Preparation of compound 77d

To a reaction flask were added compound 77c (0.67 g), intermediate 3A-2 (0.90 g), tris(dibenzylideneacetone)dipalladium(0) (0.24 g), 4,5-bis-diphenylphosphino-9,9-dimethylxanthene (0.18 g), cesium carbonate (2.54 g), and 1,4-dioxane (30 mL) under nitrogen atmosphere. The mixture was stirred at 110 °C. After the reaction was completed, the reaction solution was filtered, concentrated, and purified by silica gel column chromatography (dichloromethane/methanol = 20/1) to give compound 77d (0.97 g).

MS (ESI, [M+H]⁺) *m*/*z*: 524.13.

### Step E: Preparation of compound 77e

To a microwave reaction flask were added compound 77d (0.97 g), bis(pinacolato)diboron (0.85 g), chloro(2-dicyclohexylphosphino-2',4',6'-triisopropyl-1,1 '-biphenyl)[2-(2'-amino-1,1 '-biphenyl)]palladium (II) (0.29 g), potassium acetate (0.55 g), and 1,4-dioxane (15 mL) in sequence. The mixture was purged with nitrogen and stirred at 90 °C under microwave irradiation. After the reaction was completed, the reaction solution was filtered, concentrated, and purified by silica gel column chromatography (dichloromethane/methanol = 20/1) to give compound 77e (0.65 g).

MS (ESI, [M+H]⁺) m/z: 616.37.

### Step F: Preparation of compound 77f

To a reaction flask were added compound 77e (300 mg), intermediate 6A (117 mg), chloro(2-dicyclohexylphosphino-2',4',6'-triisopropyl-1,1 '-biphenyl)[2-(2'-amino-1,1 '-biphenyl)]palladium (II) (38 mg), tripotassium phosphate (207 mg), 1,4-dioxane (7.0 mL), and water (1.0 mL) in sequence. The mixture was stirred at 85 °C under microwave irradiation under nitrogen atmosphere. After the reaction was completed, the reaction solution was concentrated and purified by silica gel column chromatography (dichloromethane/methanol = 20/1) to give compound 77f (163 mg).

MS (ESI, [M+H]⁺) m/z: 646.38.

### Step G: Preparation of compound 1-77

To a reaction flask were added compound 77f (163 mg), methanesulfonic acid (0.16 mL), and dichloromethane (5 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and purified by silica gel column chromatography (dichloromethane/methanol = 5/1) to give compound I-77 (64 mg).

HRMS (ESI, [M+H]⁺) *m*/*z*: 546.2251.

¹H NMR (500 MHz, CDCl₃) δ 10.07 (s, 1H), 9.06 (d, J = 8.5 Hz, 1H), 7.72 - 7.61 (m, 2H), 7.58 - 7.50 (m, 4H), 7.31 (d, J = 7.0 Hz, 1H), 6.89 (d, J = 8.5 Hz, 1H), 6.72 (d, J = 1.9 Hz, 1H), 6.51 (dd, J = 7.0, 2.0 Hz, 1H), 6.38 (s, 1H), 6.29 (d, J = 2.3 Hz, 1H), 4.27 (s, 2H), 3.61 (s, 3H), 3.54 (s, 2H), 2.33 (s, 6H).

### Example 78: Preparation of Compound I-78

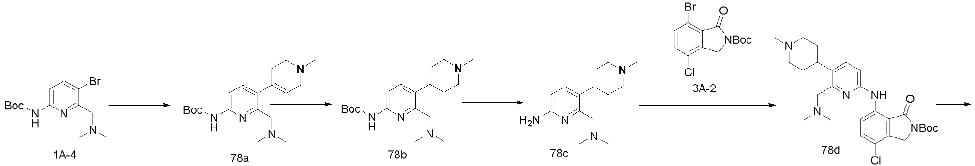

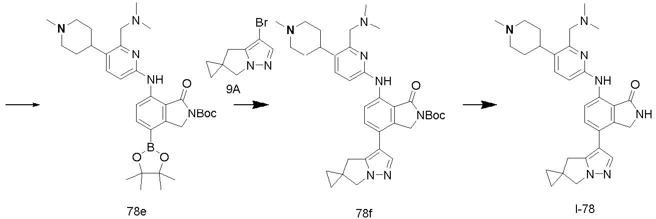

### Step A: Preparation of intermediate 78a

To a reaction flask were added intermediate 1A-4 (2.5 g), tripotassium phosphate (4.82 g), chloro(2-dicyclohexylphosphino-2',4',6'-triisopropyl-1,1'-biphenyl)[2-(2'-atnino-1,1'-biphenyl)]palladium (II) (0.6 g), 1-methyl-1,2,3,6-tetrahydropyridine-4-boronic acid pinacol ester (1.69 g), water (5 mL), and 1,4-dioxane (50 mL) in sequence. After the addition, the mixture was purged with nitrogen and stirred at 100 °C. After the reaction was completed, the reaction solution was filtered, concentrated, and purified by silica gel column chromatography (dichloromethane/methanol = 9/1) to give compound 78a (1.8 g).

MS (ESI, [M+H]⁺) *m*/*z*: 347.27.

### Step B: Preparation of intermediate 78b

To a reaction flask were added compound 78a (1.8 g), 10 wt% palladium hydroxide on carbon (1.8 g), and methanol (60 mL) in sequence. After the addition, the mixture was stirred at 35 °C under hydrogen atmosphere. After the reaction was completed, the reaction solution was filtered and concentrated to give compound 78b (1.37 g).

MS (ESI, [M+H]⁺) *m*/*z*: 349.29.

### Step C: Preparation of intermediate 78c

To a reaction flask were added compound 78b (1.37 g) and a solution of hydrochloric acid in 1,4-dioxane (4 mL, 4 M) in sequence. After the addition, the mixture was stirred at room temperature. After the reaction was completed, the reaction solution was adjusted to alkalinity (about pH 10) with a saturated sodium bicarbonate solution, extracted with dichloromethane, and concentrated to give compound 78c (1.5 g).

MS (ESI, [M+H]⁺) *m*/*z*: 249.24.

### Step D: Preparation of intermediate 78d

To a reaction flask were added compound 78c (1.29 g), 4,5-bis-diphenylphosphino-9,9-dimethylxanthene (0.3 g), tris(dibenzylideneacetone)dipalladium(0) (0.32 g), cesium carbonate (2.8 g), intermediate 3A-2 (1.2 g), and 1,4-dioxane (30 mL) in sequence. After the addition, the mixture was purged with nitrogen and stirred at 100 °C. After the reaction was completed, the reaction solution was filtered and concentrated to give a crude product, which was purified by silica gel column chromatography (dichloromethane/methanol = 20/1) to give compound 78d (1.4 g).

MS (ESI, [M+H]⁺) *m*/*z*: 514.33.

### Step E: Preparation of intermediate 78e

To a microwave reaction flask were added compound 78d (1.4 g), chloro(2-dicyclohexylphosphino-2',4',6'-triisopropyl-1,1'-biphenyl)[2-(2'-amino-1,1'-biphenyl)]palladium (II) (0.38 g), bis(pinacolato)diboron (1.2 g), potassium carbonate (1.1 g), and 1,4-dioxane (30 mL) in sequence. After the addition, the mixture was purged with nitrogen and reacted at 90 °C under microwave irradiation. After the reaction was completed, the reaction solution was filtered, concentrated, and purified by silica gel column chromatography (dichloromethane/methanol = 20/1) to give compound 78e (0.54 g).

MS (ESI, [M+H]⁺) *m*/*z*: 606.41.

### Step F: Preparation of compound 78f

To a microwave reaction flask were added compound 78e (0.2 g), intermediate 9A (55 mg), chloro(2-dicyclohexylphosphino-2',4',6'-triisopropyl-1,1 '-biphenyl)[2-(2'-amino-1,1 '-biphenyl)]palladium (II) (16 mg), tripotassium phosphate (120 mg), 1,4-dioxane (10.0 mL), and water (1.0 mL) in sequence. The mixture was stirred at 110 °C under nitrogen atmosphere. After the reaction was completed, the reaction solution was concentrated and purified by silica gel column chromatography (dichloromethane/methanol = 10/1) to give compound 78f (95 mg). MS (ESI, [M+H]⁺) m/z: 612.47.

### Step G: Preparation of compound I-78

To a reaction flask were added compound 78f (95 mg), a solution of hydrochloric acid in 1,4-dioxane (4 M, 1 mL), and dichloromethane (10 mL) in sequence. After the addition, the mixture was stirred at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and purified by silica gel column chromatography (dichloromethane/methanol = 6/1) to give compound I-78 (32 mg).

HRMS (ESI, [M+H]⁺) *m*/*z*: 512.3127.

¹H NMR (500 MHz, CDCl₃:CD₃OD=10:1) δ 8.69 (d, *J =* 8.7 Hz, 1H), 7.64 (s, 1H), 7.52 (d, *J =* 8.5 Hz, 1H), 7.44 (d, *J =* 8.8 Hz, 1H), 6.80 (d, *J =* 8.5 Hz, 1H), 4.50 (s, 2H), 4.13 (s, 2H), 3.60 (s, 2H), 3.08 (d, *J =* 13.5 Hz, 10H), 3.00 (d, *J =* 11.3 Hz, 3H), 2.12 (s, 3H), 1.28 (d, *J =* 23.5 Hz, 5H), 0.89 (d, *J =* 9.8 Hz, 5H).

### Example 79: Preparation of Compound I-79

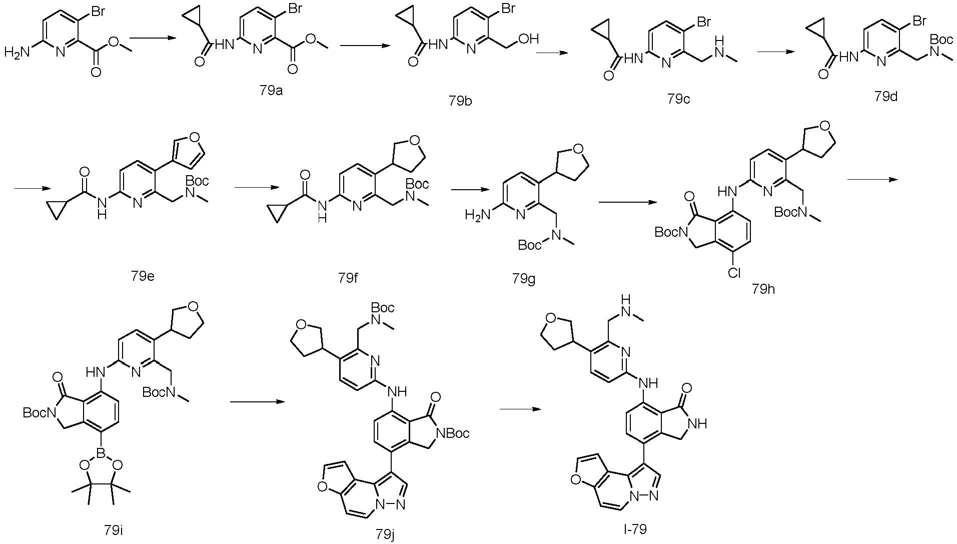

### Step A: Preparation of intermediate 79a

To a reaction flask were added the compounds methyl 6-amino-3-bromopicolinate (20 g), triethylamine (26.3 g), cyclopropanecarbonyl chloride (36.2 g), and dichloromethane (200 mL) in sequence. After the addition, the mixture was stirred at room temperature. After the reaction was completed, the reaction solution was extracted with a saturated aqueous sodium bicarbonate solution and dichloromethane. The organic phase was dried, filtered, and concentrated to give compound 79a (25.1 g).

MS (ESI, [M+H]⁺) *m*/*z*: 299.0.

### Step B: Preparation of compound 79b

To a reaction flask were added compound 79a (10.0 g), tetrahydrofuran (100.0 mL), and lithium borohydride (0.88 g) in sequence at 0 °C. After the addition, the mixture was purged with nitrogen and stirred at 0 °C. After the reaction was completed, water was added to the reaction solution to quench the reaction, and the resulting mixture was extracted with dichloromethane. The organic phase was isolated, dried, filtered, concentrated, and purified by silica gel column chromatography (petroleum ether/ethyl acetate = 5/1) to give compound 79b (7.0 g).

MS (ESI, [M+H]⁺) *m*/*z*: *271.0.*

### Step C: Preparation of compound 79c

To a reaction flask were added compound 79b (7.0 g), dichloromethane (50.0 mL), *N*,*N*-diisopropylethylatmine (8.34 mL), and methanesulfonic anhydride (11.24 g) in sequence at 0 °C. After the addition, the mixture was stirred at 0 °C. After the reaction was completed, the reaction solution was slowly added to an aqueous methylamine solution (7 M, 10 mL) at 0 °C. After the addition, the mixture was stirred at 0 °C. After the reaction was completed, the reaction solution was concentrated and purified by silica gel column chromatography (petroleum ether/ethyl acetate = 5/1) to give compound 79c (2.8 g).

MS (ESI, [M+H]⁺) *m*/*z*: 283.9.

### Step D: Preparation of compound 79d

To a reaction flask were added compound 79c (2.8 g), *N*,*N*-dimethylpyridin-4-amine (0.24 g), di-*tert*-butyl dicarbonate (2.58 g), and tetrahydrofuran (20 mL) in sequence. After the addition, the mixture was stirred at room temperature. After the reaction was completed, the reaction solution was extracted with 10% aqueous citric acid solution (80 mL, w/w) and dichloromethane. The organic phase was isolated, washed, dried, and concentrated to give compound 79d (3.1 g).

MS (ESI, [M+H]⁺) *m*/*z*: 384.3.

### Step E: Preparation of compound 79e

To a reaction flask were added compound 79d (2.0 g), 1,4-dioxane (20.0 mL), water (5.0 mL), potassium phosphate (3.31 g), chloro(2-dicyclohexylphosphino-2',4',6'-triisopropyl-1,1'-biphenyl)[2-(2'-amino-1,1'-biphenyl)] palladium (II) (0.40 g), and 3-furanboronic acid (0.70 g) in sequence. After the addition, the mixture was purged with nitrogen and stirred at 110 °C. After the reaction was completed, the reaction solution was filtered, concentrated, and purified by silica gel column chromatography (dichloromethane/methanol = 30/1) to give compound 79e (1.83 g).

MS (ESI, [M+H]⁺) *m*/*z*: 372.4.

### Step F: Preparation of compound 79f

To a reaction flask were added compound 79e (1.8 g), tetrahydrofuran (15.0 mL), methanol (15.0 mL), 10 wt% palladium hydroxide on carbon (3.4 g), acetic acid (0.6 mL), and ammonium formate (1.22 g) in sequence. After the addition, the mixture was purged with hydrogen and stirred at room temperature. After the reaction was completed, the reaction solution was filtered and concentrated. The residue was extracted with water and dichloromethane. The organic phase was isolated, dried, filtered, and concentrated to give compound 79f (1.75 g). MS (ESI, [M+H]⁺) *m*/*z*: 376.5.

### Step G: Preparation of compound 79g

To a reaction flask were added compound 79f (1.75 g), water (32.0 mL), methanol (4.0 mL), and sodium hydroxide (3.2 g) in sequence. After the addition, the mixture was stirred at 70 °C. After the reaction was completed, the reaction solution was diluted with water and extracted with dichloromethane. The organic phase was dried, filtered, and concentrated to give compound 79g (1.1 g).

MS (ESI, [M+H]⁺) *m*/*z*: 308.5.

### Step H: Preparation of compound 79h

To a reaction flask were added intermediate 3A-2 (0.54 g), compound 79g (0.7 g), 1,4-dioxane (10.0 mL), 4,5-bis-diphenylphosphino-9,9-dimethylxanthene (0.15 g), cesium carbonate (1.27 g), and tris(dibenzylideneadone) (0.18 g) in sequence. After the addition, the mixture was purged with nitrogen and stirred at 110 °C. After the reaction was completed, the reaction solution was filtered, concentrated, and purified by silica gel column chromatography (dichloromethane/methanol = 30/1) to give compound 79h (0.65 g).

MS (ESI, [M+H]⁺) *m*/*z*: 573.0.

### Step I: Preparation of compound 79i

To a reaction flask were added compound 79h (0.65 g), potassium acetate (0.26 g), bis(pinacolato)diboron (0.4 g), chloro(2-dicyclohexylphosphino-2',4',6'-triisopropyl-1,1'-biphenyl)[2-(2'-amino-1,1'-biphenyl)]palladium (II) (0.14 g), and 1,4-dioxane (20.0 mL) in sequence. After the addition, the mixture was purged with nitrogen and stirred at 90 °C. After the reaction was completed, the reaction solution was filtered, concentrated, and purified by silica gel column chromatography (dichloromethane/methanol = 30/1) to give compound 79i (521 mg).

MS (ESI, [M+H]⁺) *m*/*z*: 665.2.

### Step J: Preparation of compound 79j

To a reaction flask were added compound 79i (500 mg), potassium phosphate (534 mg), chloro(2-dicyclohexylphosphino-2',4',6'-triisopropyl-1,1 '-biphenyl)[2-(2'-amino-1,1 '-biphenyl)]palladium (II) (66 mg), intermediate 7A (199 mg), water (3.0 mL), and 1,4-dioxane (12.0 mL) in sequence. After the addition, the mixture was stirred at 110 °C. After the reaction was completed, the reaction solution was filtered, concentrated, and purified by silica gel column chromatography (dichloromethane/methanol = 30/1) to give compound 79j (231 mg).

MS (ESI, [M+H]⁺) *m*/*z*: 695.8.

### Step K: Preparation of compound 1-79

To a reaction flask were added compound 79j (230 mg), a solution of hydrochloric acid in 1,4-dioxane (4 M, 2 mL), and dichloromethane (3 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and purified by silica gel column chromatography (dichloromethane/methanol = 5/1) to give compound I-79 (90 mg).

HRMS (ESI, [M+H]⁺) *m*/*z*: 495.2142.

¹H NMR (500 MHz, DMSO) δ 9.93 (s, 1H), 8.75 (s, 1H), 8.63 (d, J = 5.2 Hz, 1H), 8.61 (d, J = 6.1 Hz, 1H), 8.32 (s, 1H), 8.07 (d, J = 2.1 Hz, 1H), 7.64 (d, J = 8.5 Hz, 1H), 7.61 (d, J = 8.5 Hz, 1H), 7.37 (d, J = 7.6 Hz, 1H), 6.91 (d, J = 8.5 Hz, 1H), 6.83 (d, J = 1.5 Hz, 1H), 4.43 (s, 2H), 3.98 (t, J = 7.7 Hz, 1H), 3.94 (td, J = 8.2, 4.9 Hz, 1H), 3.81 (d, J = 1.7 Hz, 2H), 3.80 (d, J = 8.2 Hz, 1H), 3.68 - 3.64 (m, 1H), 3.54 (dd, J = 8.0, 7.0 Hz, 1H), 2.37 (s, 3H), 2.30 - 2.26 (m, 1H), 1.89 - 1.83 (m, 1H).

### Example 80: Preparation of Compound 1-80

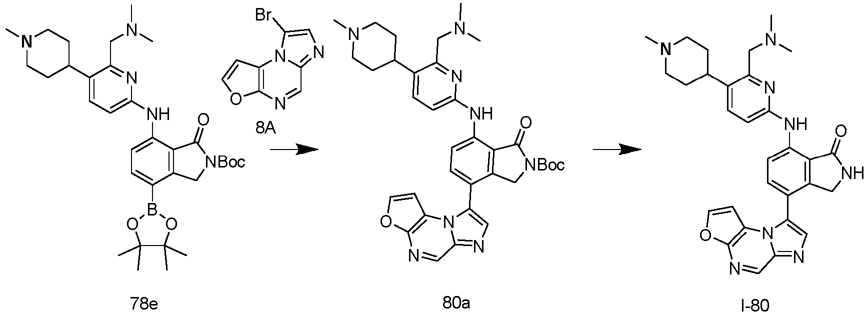

### Step A: Preparation of compound 80a

To a microwave reaction flask were added compound 78e (0.2 g), intermediate 8A (70 mg), chloro(2-dicyclohexylphosphino-2',4',6'-triisopropyl-1,1 '-biphenyl)[2-(2'-amino-1,1 '-biphenyl)]palladium (II) (16 mg), tripotassium phosphate (150 mg), 1,4-dioxane (10.0 mL), and water (1.0 mL) in sequence. The mixture was stirred at 110 °C under nitrogen atmosphere. After the reaction was completed, the reaction solution was concentrated and purified by silica gel column chromatography (dichloromethane/methanol = 10/1) to give compound 80a (90 mg). MS (ESI, [M+H]⁺) m/z: 637.45.

### Step B: Preparation of compound I-80

To a reaction flask were added compound 80a (90 mg), a solution of hydrochloric acid in 1,4-dioxane (4 M, 1 mL), and dichloromethane (10 mL) in sequence. After the addition, the mixture was stirred at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and purified by silica gel column chromatography (dichloromethane/methanol = 5/1) to give compound I-80 (35 mg).

HRMS (ESI, [M+H]⁺) *m*/*z*: 537.2718.

¹H NMR (500 MHz, CDCl₃:CD₃OD=10:1) δ 9.84 (s, 1H), 9.00 (d, *J =* 8.6 Hz, 1H), 8.83 (s, 1H), 7.86 (s, 1H), 7.64 - 7.59 (m, 2H), 7.57 (d, *J =* 8.4 Hz, 1H), 6.85 (d, *J =* 8.4 Hz, 1H), 6.47 (d, *J =* 2.5 Hz, 1H), 6.26 (s, 1H), 4.28 (s, 2H), 3.62 (s, 2H), 3.05 - 2.99 (m, 2H), 2.36 (s, 3H), 2.32 (s, 6H), 2.10 (dd, *J =* 8.1, 4.2 Hz, 2H), 1.79 (h, *J =* 3.7 Hz, 5H).

### Example 81: Preparation of Compound 1-81

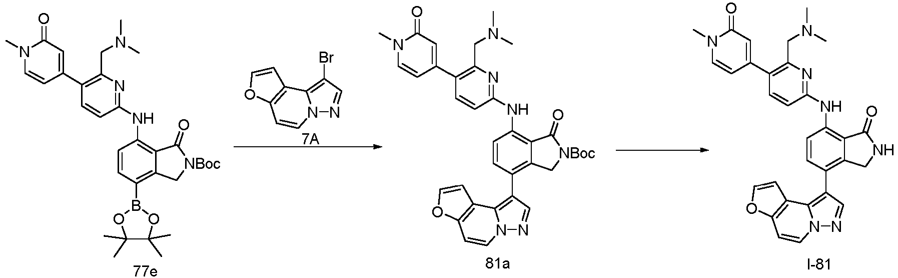

### Step A: Preparation of compound 81a

To a reaction flask were added compound 77e (200 mg), intermediate 7A (77 mg), chloro(2-dicyclohexylphosphino-2',4',6'-triisopropyl-1,1'-biphenyl)[2-(2'-amino-1,1'-biphenyl)]palladium (II) (26 mg), tripotassium phosphate (138 mg), 1,4-dioxane (7.0 mL), and water (1.0 mL) in sequence. The mixture was stirred at 85 °C under microwave irradiation under nitrogen atmosphere. After the reaction was completed, the reaction solution was concentrated and purified by silica gel column chromatography (dichloromethane/methanol = 20/1) to give compound 81a (152 mg). MS (ESI, [M+H]⁺) m/z: 646.60.

### Step B: Preparation of compound I-81

To a reaction flask were added compound 81a (152 mg), methanesulfonic acid (0.15 mL), and dichloromethane (5 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and purified by silica gel column chromatography (dichloromethane/methanol = 5/1) to give compound I-81 (68 mg).

HRMS (ESI, [M+H]⁺) *m*/*z:* 546.2261.

¹H NMR (500 MHz, CDCl₃) δ 9.99 (s, 1H), 8.99 (d, J = 8.5 Hz, 1H), 8.40 (d, J = 7.5 Hz, 1H), 8.01 (s, 1H), 7.70 - 7.61 (m, 2H), 7.51 (d, J = 8.4 Hz, 1H), 7.30 (d, J = 7.0 Hz, 1H), 7.10 (d, J = 7.6 Hz, 1H), 6.87 (d, J = 8.4 Hz, 1H), 6.77 - 6.70 (m, 2H), 6.52 (dd, J = 6.9, 2.0 Hz, 1H), 6.30 (s, 1H), 4.40 (s, 2H), 3.61 (s, 3H), 3.54 (s, 2H), 2.34 (s, 6H).

### Example 82: Preparation of Compound I-82

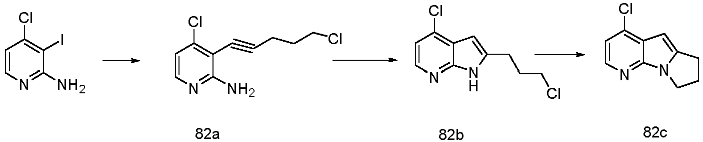

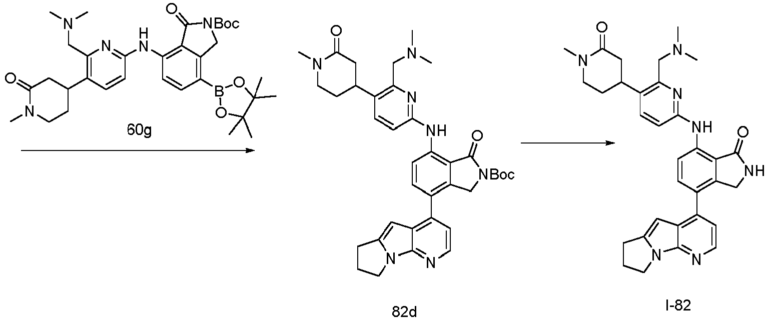

### Step A: Preparation of compound 82a

To a reaction flask were added 4-chloro-3-iodopyridin-2-ylamine (5.00 g), 5-chloropent-1-yne (2.22 g), copper(I) iodide (0.37 g), bis(triphenylphosphine)palladium(II) dichloride (0.69 g), and triethylamine (30 mL) in sequence. After the addition, the mixture was heated and stirred at 80 °C under nitrogen atmosphere. After the reaction was completed, the reaction solution was filtered, concentrated, and purified by silica gel column chromatography (petroleum ether/ethyl acetate = 5/1) to give compound 82a (3.0 g).

MS (ESI, [M+Na]⁺) m/z: 229.07.

### Step B: Preparation of compound 82b

To a reaction flask were added compound 82a (3.00 g), acetonitrile (30 mL), and trifluoroacetic acid (5.50 g) in sequence at 0 °C. After the addition, the mixture was stirred at 0 °C. After reacting for 2 h, the reaction solution was concentrated under reduced pressure to remove the solvent. Acetonitrile (30 mL) and palladium dichloride (0.35 g) were added to the residue in sequence. After the addition, the mixture was heated and stirred at 80 °C under nitrogen atmosphere. After the reaction was completed, the reaction solution was filtered, concentrated, and purified by silica gel column chromatography (petroleum ether/ethyl acetate = 5/1) to give compound 82b (1.50 g).

MS (ESI, [M+H]⁺) *m*/*z*: 229.05.

### Step C: Preparation of compound 82c

To a reaction flask were added compound 82b (3.00 g), tetrahydrofuran (30 mL), 60 wt% sodium hydride (1.05 g), and potassium iodide (1.36 g) in sequence at 0 °C. After the addition, the mixture was stirred at room temperature. After the reaction was completed, the reaction was quenched with a saturated ammonium chloride solution, and the reaction solution was extracted with dichloromethane. The organic phase was dried, filtered, concentrated, and purified by silica gel column chromatography (petroleum ether/ethyl acetate = 4/1) to give compound 82c (0.80 g). MS (ESI, [M+H]⁺) *m*/*z*: 193.27.

### Step D: Preparation of compound 82d

To a microwave reaction flask were added compound 60g (180 mg), compound 82c (56 mg), chloro(2-dicyclohexylphosphino-2',4',6'-triisopropyl-1,1'-biphenyl)[2-(2'-atnino-1,1'-biphenyl)]palladium (II) (23 mg), 2-dicyclohexylphosphine-2',4',6'-triisopropylbiphenyl (14 mg), tripotassium phosphate (123 mg), 1,4-dioxane (7.0 mL), and water (1.0 mL) in sequence. The mixture was stirred at 85 °C under microwave irradiation under nitrogen atmosphere. After the reaction was completed, the reaction solution was concentrated and purified by silica gel column chromatography (dichloromethane/methanol = 20/1) to give compound 82d (92 mg).

MS (ESI, [M+H]⁺) m/z: 650.20.

### Step E: Preparation of compound I-82

To a reaction flask were added compound 82d (92 mg), a solution of hydrochloric acid in 1,4-dioxane (4 M, 1.0 mL), and dichloromethane (4 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and purified by silica gel column chromatography (dichloromethane/methanol = 5/1) to give compound I-82 (30 mg).

HRMS (ESI, [M+H]⁺) *m*/*z*: 550.2931.

¹H NMR (500 MHz, MeOD) δ 8.77 (d, J = 8.6 Hz, 1H), 8.13 (d, J = 5.1 Hz, 1H), 7.68 (d, J = 8.6 Hz, 1H), 7.61 (d, J = 8.5 Hz, 1H), 7.10 (d, J = 5.1 Hz, 1H), 6.89 (d, J = 8.5 Hz, 1H), 6.11 (s, 1H), 4.40 (s, 2H), 4.21 (t, J = 7.0 Hz, 2H), 3.70 - 3.61 (m, 2H), 3.57 - 3.46 (m, 2H), 3.45 - 3.38 (m, 1H), 3.05 (t, J = 7.3 Hz, 2H), 2.99 (s, 3H), 2.69 - 2.60 (m, 3H), 2.46 - 2.37 (m, 1H), 2.32 (s, 6H), 2.09 - 1.97 (m, 2H).

### Example 83: Preparation of Compound 1-83

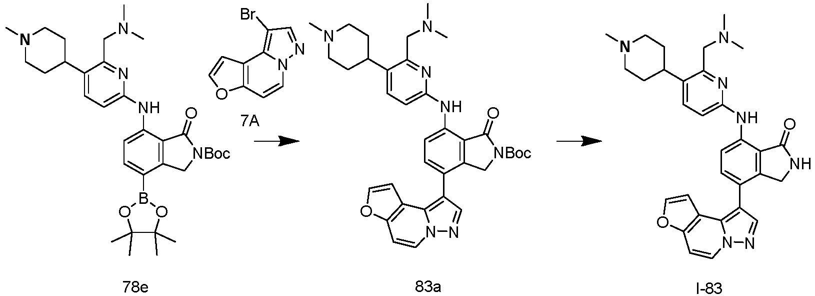

### Step A: Preparation of compound 83a

To a microwave reaction flask were added compound 78e (0.2 g), intermediate 7A (80mg), chloro(2-dicyclohexylphosphino-2',4',6'-triisopropyl-1,1 '-biphenyl)[2-(2'-amino-1,1 '-biphenyl)]palladium (II) (18 mg), tripotassium phosphate (160 mg), 1,4-dioxane (10.0 mL), and water (1.0 mL) in sequence. The mixture was stirred at 110 °C under nitrogen atmosphere. After the reaction was completed, the reaction solution was concentrated and purified by silica gel column chromatography (dichloromethane/methanol = 10/1) to give compound 83a (99 mg). MS (ESI, [M+H]⁺) m/z: 636.41.

### Step B: Preparation of compound I-83

To a reaction flask were added compound 83a (99 mg), a solution of hydrochloric acid in 1,4-dioxane (4 M, 1 mL), and dichloromethane (10 mL) in sequence. After the addition, the mixture was stirred at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and purified by silica gel column chromatography (dichloromethane/methanol = 4/1) to give compound I-83 (46 mg).

HRMS (ESI, [M+H]⁺) *m*/*z*: 536.2775.

¹H NMR (500 MHz, CDCl₃:CD₃OD=10:1) δ 8.80 (d, *J =* 8.5 Hz, 1H), 8.39 (d, *J* = 7.6 Hz, 1H), 8.00 (s, 1H), 7.69 - 7.55 (m, 3H), 7.30 (s, 2H), 6.87 (d, *J* = 8.5 Hz, 1H), 6.72 (s, 1H), 4.36 (s, 2H), 3.68 (s, 2H), 3.18 (s, 1H),2.48(s,3H), 2.29 (s, 6H), 1.95 (s, 1H), 1.85 (d, *J =* 12.9 Hz, 2H), 1.37 (s, 1H), 1.29 (s, 2H), 1.26 (s, 2H).

### Example 84: Preparation of Compound I-84

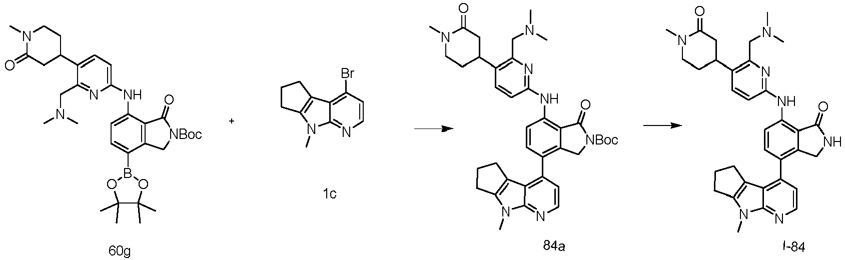

### Step A: Preparation of compound 84-a

To a reaction flask were added compound 60g (250 mg), potassium phosphate (257 mg), chloro(2-dicyclohexylphosphino-2',4',6'-triisopropyl-1,1 '-biphenyl)[2-(2'-amino-1,1 '-biphenyl)]palladium (II) (37 mg), compound 1c (101 mg), water (3.0 mL), and 1,4-dioxane (12.0 mL) in sequence. After the addition, the mixture was stirred at 110 °C. After the reaction was completed, the reaction solution was filtered, concentrated, and purified by silica gel column chromatography (dichloromethane/methanol = 30/1) to give compound 84a (201 mg).

MS (ESI, [M+H]⁺) *m*/*z:* 664.4.

### Step B: Preparation of compound I-84

To a reaction flask were added compound 84g (200 mg), a solution of hydrochloric acid in 1,4-dioxane (4 M, 2 mL), and dichloromethane (3 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and purified by silica gel column chromatography (dichloromethane/methanol = 5/1) to give compound I-84 (40 mg).

HRMS (ESI, [M+H]⁺) *m*/*z:* 564.3084.

¹H NMR (500 MHz, CDCl₃) δ 9.81 (s, 1H), 8.86 (d, J = 8.5 Hz, 1H), 8.22 (d, J = 4.9 Hz, 1H), 7.57 (d, J = 8.5 Hz, 1H), 7.43 (d, J = 8.5 Hz, 1H), 6.98 (d, J = 4.9 Hz, 1H), 6.83 (d, J = 8.5 Hz, 1H), 6.42 (s, 1H), 4.37 (s, 2H), 3.83 (s, 3H), 3.64 (d, J = 12.3 Hz, 1H), 3.59 - 3.52 (m, 2H), 3.44 (dd, J = 11.5, 4.7 Hz, 1H), 3.37 - 3.32 (m, 1H), 3.01 (s, 3H), 2.91 (t, J = 7.0 Hz, 2H), 2.72 (dd, J = 17.3, 3.5 Hz, 1H), 2.53 (t, J = 6.6 Hz, 2H), 2.43 - 2.35 (m, 2H), 2.27 (s, 6H), 2.06 - 1.99 (m, 1H), 1.96 (dd, J = 12.2, 4.9 Hz, 1H), 1.85 (s, 1H).

### Example 85: Preparation of Compound I-85

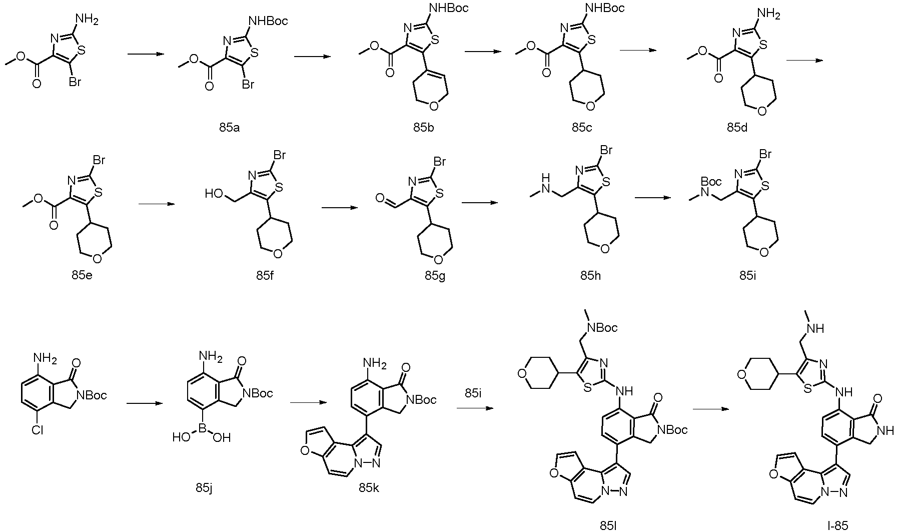

### Step A: Preparation of compound 85a

To a reaction flask were added the compounds methyl 2-amino-5-bromothiazole-4-carboxylate (25.0 g), tetrahydrofuran (500.0 mL), triethylamine (44.1 g), di-*tert*-butyl dicarbonate (36.4 g), and 4-dimethylaminopyridine (2.6 g) in sequence. After the addition, the mixture was stirred at room temperature. After the reaction was completed, the reaction solution was concentrated and purified by silica gel column chromatography (petroleum ether/ethyl acetate = 5/1) to give compound 85a (36.0 g).

MS (ESI, [M+H]⁺) *m*/*z*: 337.4.

### Step B: Preparation of compound 85b

To a reaction flask were added compound 85a (36.0 g), 3,6-dihydro-2*H*-pyran-4-boronic acid pinacol ester (33.6 g), [1,1'-bis(diphenylphosphino)ferrocene]palladium(II) dichloride (8.72 g), sodium carbonate (22.6 g), water (100.0 mL), and 1,4-dioxane (400.0 mL) in sequence. After the addition, the mixture was stirred at 90 °C. After the reaction was completed, the reaction solution was filtered, concentrated, and purified by silica gel column chromatography (petroleum ether/ethyl acetate = 5/1) to give compound 85b (15.0 g).

MS (ESI, [M+H]⁺) *m*/*z*: 341.3.

### Step C: Preparation of compound 85c

To a reaction flask were added compound 85b (15.0 g), 10 wt% palladium hydroxide on carbon (15.0 g), and methanol (50.0 mL) in sequence. After the addition, the mixture was stirred at 90 °C. After the reaction was completed, the reaction solution was filtered, concentrated, and purified by silica gel column chromatography (petroleum ether/ethyl acetate = 5/1) to give compound 85c (14.72 g).

MS (ESI, [M+H]⁺) *m*/*z*: 343.1.

### Step D: Preparation of compound 85d

To a reaction flask were added compound 85c (10.0 g), dichloromethane (10.0 mL), and trifluoroacetic acid (33.8 mL) in sequence. After the addition, the mixture was stirred at room temperature. After the reaction was completed, the reaction solution was adjusted to alkalinity (about pH 10) with a saturated sodium bicarbonate solution and extracted with dichloromethane. The organic phase was dried, filtered, concentrated, and purified by silica gel column chromatography (dichloromethane/methanol = 5/1) to give compound 85d (6.59 g).

MS (ESI, [M+H]⁺) *m*/*z*: 243.0.

### Step E: Preparation of compound 85e

To a reaction flask were added compound 85d (6.59 g), acetonitrile (50.0 mL), tert-butyl nitrite (2.0 g), and copper bromide (5.68 g) in sequence. After the addition, the mixture was stirred at 60 °C. After the reaction was completed, the reaction solution was adjusted to alkalinity (about pH 10) with a saturated sodium bicarbonate solution and extracted with ethyl acetate. The organic phase was dried, filtered, concentrated, and purified by silica gel column chromatography (dichloromethane/methanol = 2/1) to give compound 85e (3.7 g).

MS (ESI, [M+H]⁺) *m*/*z*: 306.1.

### Step F: Preparation of compound 85f

To a reaction flask were added compound 85e (3.7 g), tetrahydrofuran (50.0 mL), and lithium borohydride (0.6 g) in sequence at 0 °C. After the addition, the mixture was purged with nitrogen and stirred at 0 °C. After the reaction was completed, a saturated ammonium chloride solution was added to the reaction solution to quench the reaction, and the resulting mixture was extracted with dichloromethane. The organic phase was isolated, dried, filtered, concentrated, and purified by silica gel column chromatography (petroleum ether/ethyl acetate = 5/1) to give compound 85f (3.4 g).

MS (ESI, [M+H]⁺) *m*/*z*: 278.2.

### Step G: Preparation of compound 85g

To a reaction flask were added compound 85f (3.4 g), dichloromethane (100.0 mL), and Dess-Martin periodinane (6.4 g) in sequence at 0 °C. After the addition, the mixture was purged with nitrogen and stirred at 0 °C. After the reaction was completed, the reaction solution was adjusted to alkalinity (about pH 10) with a saturated sodium bicarbonate solution and extracted with ethyl acetate. The organic phase was dried, filtered, concentrated, and purified by silica gel column chromatography (petroleum ether/ethyl acetate = 1/1) to give compound 85g (1.3 g). MS (ESI, [M+H]⁺) *m*/*z*: 276.0.

### Step H: Preparation of compound 85h

To a reaction flask were added compound 85g (1.3 g), acetonitrile (100.0 mL), sodium triacetoxyborohydride (2.0 g), and methylamine hydrochloride (1.6 g) in sequence at 0 °C. After the addition, the mixture was purged with nitrogen and stirred at 0 °C. After the reaction was completed, the reaction solution was adjusted to alkalinity (about pH 10) with a saturated sodium bicarbonate solution and extracted with ethyl acetate. The organic phase was dried, filtered, concentrated, and purified by silica gel column chromatography (petroleum ether/ethyl acetate = 1/1) to give compound 85h (1.3 g).

MS (ESI, [M+H]⁺) *m*/*z*: 291.2.

### Step I: Preparation of compound 85i

To a reaction flask were added compound 85h (1.3 g), dichloromethane (100.0 mL), triethylamine (0.7 g), and di-*tert*-butyl dicarbonate (1.3 g) in sequence. After the addition, the mixture was stirred at room temperature. After the reaction was completed, the reaction solution was concentrated and purified by silica gel column chromatography (petroleum ether/ethyl acetate = 3/1) to give compound 85i (1.5 g).

MS (ESI, [M+H]⁺) *m*/*z*: 391.3.

### Step J: Preparation of compound 85j

To a reaction flask were added the compounds *tert*-butyl-7-amino-4-chloro-1-oxoisoindoline-2-carboxylate (3.1 g), ethanol (50.0 mL), ethylene glycol (2.0 g), 2-dicyclohexylphosphine-2',4',6'-triisopropylbiphenyl (1.1 g), tetrahydroxydiboron (3.0 g), chloro(2-dicyclohexylphosphino-2',4',6'-triisopropyl-1,1'-biphenyl)[2-(2'-amino-1,1'-biphenyl)]palladium (II) (0.8 g), and potassium phosphate (7.1 g) in sequence. After the addition, the mixture was stirred at 100 °C. After the reaction was completed, the reaction solution was concentrated and purified by silica gel column chromatography (petroleum ether/ethyl acetate = 2/1) to give compound 85j (1.7 g).

MS (ESI, [M+H]⁺) *m*/*z*: 293.2.

### Step K: Preparation of compound 85k

To a reaction flask were added compound 85j (1.7 g), chloro(2-dicyclohexylphosphino-2',4',6'-triisopropyl-1,1'-biphenyl)[2-(2'-amino-1,1'-biphenyl)]palladium (II) (0.1 g), potassium phosphate (0.9 g), intermediate 7A (0.07 g), water (4.0 mL), and 1,4-dioxane (16.0 mL) in sequence. After the addition, the mixture was stirred at 85 °C. After the reaction was completed, the reaction solution was concentrated and purified by silica gel column chromatography (petroleum ether/ethyl acetate = 1/1) to give compound 85k (0.3 g).

MS (ESI, [M+H]⁺) *m*/*z*: 405.2.

### Step L: Preparation of compound 851

To a reaction flask were added compound 85k (0.3 g), compound 85i (0.3 g), 1,4-dioxane (20.0 mL), 4,5-bis-diphenylphosphino-9,9-dimethylxanthene (0.1 g), cesium carbonate (0.9 g), and tris(dibenzylideneadone) (0.1 g) in sequence. After the addition, the mixture was stirred at 110 °C. After the reaction was completed, the reaction solution was filtered, concentrated, and purified by silica gel column chromatography (dichloromethane/methanol = 30/1) to give compound 851 (0.4 g).

MS (ESI, [M+H]⁺) *m*/*z*: 715.1.

### Step M: Preparation of compound I-85

To a reaction flask were added compound 851 (0.4 g), methanesulfonic acid (0.7 mL), and dichloromethane (10 mL) in sequence. After the addition, the mixture was reacted at room temperature. After the reaction was completed, the reaction solution was adjusted to alkalinity (about pH 9) with a saturated aqueous sodium bicarbonate solution and extracted with dichloromethane. The organic phase was dried, filtered, concentrated, and purified by silica gel column chromatography (dichloromethane/methanol = 5/1) to give compound I-85 (0.08 g).

HRMS (ESI, [M+H]⁺) *m*/*z*: 515.1871.

¹HNMR (500 MHz, CDCl₃) δ 8.40 (d, J = 7.6 Hz, 1H), 8.32 (d, J = 8.4 Hz, 1H), 8.01 (s, 1H), 7.70 - 7.62 (m, 2H), 7.14 (d, J = 7.5 Hz, 1H), 6.73 (d, J = 2.2 Hz, 1H), 4.40 (s, 2H), 4.06 (dd, J = 11.3, 4.2 Hz, 2H), 3.69 (d, J = 3.3 Hz, 2H), 3.54 (td, J = 11.8, 2.2 Hz, 2H), 3.16 - 3.05 (m, 1H), 2.45 (s, 3H), 1.91 - 1.82 (m, 2H), 1.82 - 1.71 (m, 2H).

### Experimental Example 1: Assay for In Vitro Inhibitory Activity Against HPK1 Kinase

A kinase buffer (Enzymatic buffer 5×) was diluted to 1×, and 10 mM MgCl₂, 1 mM DTT, and 0.005% Tween 20 were added. A 100 ng/µL HPK1 (Life technology) stock solution was diluted with the kinase buffer to obtain a 1.67×, 1.67 ng/µL working solution (final concentration: 1 ng/µL), and the working solution was seeded in a 384-well plate at 6 µL/well. Different compounds dissolved in DMSO were added to the wells using a nanoliter pipettor to obtain final concentrations of the compounds of 1000 nM to 0.244 nM (4-fold serial dilution, 7 concentrations in total), and blank control wells (without enzyme) and negative control wells (with enzyme, plus vehicle DMSO) were set; the wells were set in duplicate. After the enzyme and the compounds were incubated at room temperature for 1 h, a 5×, 5 mM ATP dilution (final concentration: 1 mM) in the kinase buffer was mixed with a 5x, 2.5 µM substrate (Cisbio, STK Substrate 1-biotin; final concentration: 500 nM) in equal volume, and the mixture was added to the plate at 4 µL/well. The plate was sealed with a film and then incubated at room temperature for 2 h. An assay antibody solution was prepared by mixing the antibody STK Antibody-cryptate (Cisbio, 5 µL/test) and a 4×, 500 nM Streptavidin-XL665 (Cisbio; final concentration: 125 nM) in equal volume and added to the plate at 10 µL/well. The plate was incubated at room temperature for 1 h. The signal values (excitation: 665 nm, emission: 620 nm) were measured using a PE Envision multimode microplate reader, and IC₅₀ was calculated by four-parameter fitting. The results are shown in Table 1 below:

**Table 1. Results for in vitro inhibitory activity of the compounds against enzyme**

| Compound No. | IC₅₀ (nM) for *in vitro* inhibitory activity against HPK1 kinase | Compound No. | IC₅₀ (nM) for *in vitro* inhibitory activity against HPK1 kinase |
|---|---|---|---|
| I-6 | 2.5 | I-49 | 6.1 |
| I-8 | 3.1 | I-50 | 29 |
| I-10 | 5.6 | I-51 | 5.8 |
| I-11 | 1.6 | I-52 | 36 |
| I-12 | 5.1 | I-53 | 2.7 |
| I-13 | 3.5 | I-54 | 6.6 |
| I-14 | 1.4 | I-55 | 15 |
| I-15 | 1.6 | I-57 | 17 |
| I-16 | 2.1 | I-58 | 5.6 |
| I-17 | 1.7 | I-59 | 2.3 |
| I-18 | 9.8 | I-60 | 2.8 |
| I-20 | 3.2 | I-61 | 3.7 |
| I-22 | 7.5 | I-64 | 2.9 |
| I-24 | 11 | I-65 | 21 |
| I-25 | 32 | I-66 | 2.5 |
| I-26 | 16 | I-67 | 4.6 |
| I-27 | 23 | I-68 | 7.7 |
| I-28 | 2 | I-69 | 3.7 |
| I-29 | 3.4 | I-70 | 7.5 |
| I-30 | 3.6 | I-71 | 3.6 |
| I-32 | 4.4 | I-72 | 3.7 |
| I-33 | 2.9 | I-73 | 2.9 |
| I-34 | 2.1 | I-74 | 3.4 |
| I-35 | 6.4 | I-75 | 3.4 |
| I-36 | 5.3 | I-76 | 1.8 |
| I-38 | 1.7 | I-77 | 2.9 |
| I-39 | 1.8 | I-78 | 2.3 |
| I-41 | 18 | I-79 | 2.3 |
| I-43 | 16 | I-80 | 2.8 |
| I-44 | 3.5 | I-81 | 2 |
| I-45 | 3.4 | I-82 | 3 |
| I-46 | 2.1 | I-83 | 2 |
| I-47 | 4.7 | I-84 | 2.6 |
| I-48 | 2.6 | I-85 | 3.8 |

According to the above results, the compounds of the present invention had an improved or excellent inhibitory effect on HPK1 kinase.

### Experimental Example 2: Assay for In Vitro Inhibitory Activity Against Cells

### 2.1 Assay for inhibitory activity against p-SLP76 phosphorylation of Jurkat cells

Jurkat cells in a good growth state were added to a centrifuge tube, centrifuged, and resuspended. The cell density was adjusted to 6.25 × 10⁶ cells/mL, and the cells were seeded in a 384-well small-volume white plate at 8 µL/well. Different compounds dissolved in DMSO were added to the wells using a nanoliter pipettor to obtain final concentrations of the compounds of 2500 nM to 10.29 nM, and a control was set; the wells were set in duplicate. After 1 h of cell culture, the stimulator CD3CD28 (manufacturer: stem cell, 4 µL) was added and the cells were incubated at 37 °C for 30 min. 3 µL of lysis buffer (manufacturer: BioAuxilium) was added to each well, and the plate was shaken at room temperature for 30 min. After the lysate was well mixed, 5 µL of pre-mixed antibody (manufacturer: BioAuxilium) in assay buffer was added and the plate was incubated overnight at room temperature. The signal values were measured on a PerkinElmer Envision multimode microplate reader (excitation: 320 nm, emission: 615 nm/665 nm), and IC₅₀ was calculated by four-parameter fitting. The results are shown in Table 2.

**Table 2. Results for in vitro inhibitory activity of the compounds against cells**

| Compound No. | IC₅₀ (nM) for inhibition of p-SLP76 phosphorylation of Jurkat cells | Compound No. | IC₅₀ (nM) for inhibition of p-SLP76 phosphorylation of Jurkat cells |
|---|---|---|---|
| I-6 | A | I-51 | A |
| I-8 | A | I-52 | A |
| I-10 | B | I-53 | A |
| I-16 | B | I-54 | A |
| I-17 | A | I-59 | A |
| I-22 | B | I-60 | A |
| I-24 | B | I-61 | B |
| I-28 | A | I-64 | A |
| I-29 | A | I-66 | A |
| I-30 | B | I-67 | A |
| I-32 | A | I-69 | A |
| I-33 | A | I-70 | A |
| I-34 | A | I-71 | A |
| I-35 | B | I-72 | A |
| I-36 | B | I-73 | A |
| I-38 | A | I-74 | A |
| I-39 | A | I-75 | B |
| I-44 | A | I-76 | A |
| I-45 | A | I-77 | A |
| I-46 | A | I-78 | B |
| I-47 | A | I-79 | A |
| I-48 | A | I-80 | A |
| I-49 | A | I-81 | A |
| | | I-85 | A |

A represents that IC₅₀ < 50 nM and B represents that 50 nM ≤ IC₅₀ < 100 nM.

According to the above results, the compounds of the present invention had improved or excellent inhibitory activity against p-SLP76 phosphorylation of Jurkat cells.

### Experimental Example 3: In Vitro Metabolic Stability in Liver Microsome

Liver microsome incubation samples were prepared by mixing a PBS buffer (pH 7.4), a liver microsome solution (0.5 mg/mL), a test compound, and an NADPH + MgCl₂ solution and incubated at 37 °C and 300 rpm for 1 h. Zero-hour samples were prepared by mixing a PBS buffer (pH 7.4), a liver microsome solution (0.5 mg/mL), and a test compound. An acetonitrile solution containing an internal standard was added to the samples, and supernatants were prepared by protein precipitation, diluted, and then assayed by LC/MS/MS.

The experimental results showed that the test compounds of the present invention exhibit stable *in vitro* metabolism (e.g., large residual content at 60 min).

### Experimental Example 4: In Vivo Pharmacokinetics

### 4.1 Pharmacokinetic assay in mice

ICR mice, weighing 21-23 g, were randomly divided into groups after 3-5 days of acclimatization, with 9 mice in each group, and then subjected to intragastrical administration with the solution of the compounds of the present application at a dose of 10 mg/kg (vehicle of intragastrical administration group: DMSO:HS15:0.5 mg/mL citrate-dextrose solution = 5:20:75).

Blood sampling time points for intravenous injection were 0.083 h (5 min), 0.167 h (10 min), 0.5 h (30 min), 1 h, 2 h, 6 h, 8 h, 10 h, and 24 h, and blood sampling time points for intragastrical administration were 0.25 h (15 min), 0.5 h (30 min), 1 h, 2 h, 4 h, 6 h, 8 h, 10 h, and 24 h. Plasma samples to be tested were prepared by collecting blood from the orbit.

30 µL of each of the plasma samples to be tested and a standard curve sample were taken, and an acetonitrile solution containing an internal standard was added. Supernatants were obtained by protein precipitation, diluted, and then assayed by LC/MS/MS.

The experimental results showed that the compounds had good *in vivo* pharmacokinetic properties.

## Claims

1. A compound of formula (**II**), a stereoisomer thereof, or a pharmaceutically acceptable salt thereof, 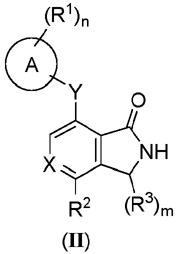 wherein,
X is selected from the group consisting of N and CH;
Y is selected from the group consisting of a bond, -O-, -S-, -NR^{a}-, -C(R^{a})₂-, -S(O)₂-, -S(O)₂NR^{a}-, -S(O)-, -S(O)NR^{a}-, -C(O)-, -C(O)O-, -C(O)NR^{a}-, -C(O)N(R^{a})O-, -OC(O)-, -OC(O)NR^{a}-, -N(R^{a})C(O)O-, -N(R^{a})C(O)-, and - N(R^{a})S(O)₂-;
ring A is selected from the group consisting of 3- to 10-membered heterocyclyl, C₅₋₁₀ aryl, and 5- to 10-membered heteroaryl;
R¹ are each independently selected from the group consisting of -NH₂, -NHR^{d}, -N(R^{d})₂, -OH, -OR^{d}, -CN, halogen, -COOR^{d}, -OCOR^{d}, -N(R^{d})C(O)(R^{d}), -CONH(R^{d}), -CON(R^{d})₂, -NHSO₂(R^{d}), -SO₂NH(R^{d}), -SO₂N(R^{d})₂, -PO(R^{d})₂, 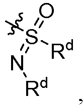 C₁₋₆ alkyl, C₅₋₁₀ aryl, 5- to 10-membered heteroaryl, and 3- to 10-membered heterocyclyl, wherein the C₁₋₆ alkyl, C₅₋₁₀ aryl, 5- to 10-membered heteroaryl, or 3- to 10-membered heterocyclyl is optionally substituted with one or more R^{b};
R^{d} are each independently selected from the group consisting of C₁₋₄ alkyl and C₃₋₇ cycloalkyl;
R² is selected from the group consisting of 5- to 6-membered monocyclic heteroaryl and a 9- to 14-membered saturated, partially saturated, or aromatic tricyclic ring, wherein the tricyclic ring contains 0-6 heteroatoms independently selected from the group consisting of N, O, and S, and R² is optionally substituted with one or more R^{c};
R³ are each independently selected from the group consisting of halogen, C₁₋₄ alkyl, and halogenated C₁₋₄ alkyl;
R^{a} are each independently selected from the group consisting of hydrogen and C₁₋₄ alkyl;
R^{b} are each independently selected from the group consisting of deuterium, -NH₂, -NH(C₁₋₄ alkyl), -N(C₁₋₄ alkyl)₂, -OH, -OC₁₋₄ alkyl, -CN, halogen, 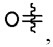 C₁₋₄ alkyl, and 3- to 6-membered heterocycloalkyl, wherein the -NH(C₁₋₄ alkyl), -N(C₁₋₄ alkyl)₂, -OC₁₋₄ alkyl, C₁₋₄ alkyl, and 3- to 6-membered heterocycloalkyl are optionally substituted
with one or more deuterium, halogen, or 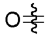 substituents;
R^{c} are each independently selected from the group consisting of -NH₂, -NH(C₁₋₄ alkyl), -N(C₁₋₄ alkyl)₂, -OH, -OC₁₋₄ alkyl, halogen, 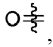 and C₁₋₆ alkyl optionally substituted with one or more deuterium;
n is selected from the group consisting of 1, 2, 3, and 4;
m is selected from the group consisting of 0, 1, and 2;
each X, Y, ring A, R¹, R^{d}, R², R³, R^{a}, R^{b}, or R^{c} is independently optionally substituted with one or more substituents.

2. The compound of formula (**II**), the stereoisomer thereof, or the pharmaceutically acceptable salt thereof according to claim 1,
wherein, Y is selected from the group consisting of a bond, -O-, -S-, and -NR^{a}-;
ring A is selected from the group consisting of C₅₋₁₀ aryl and 5- to 10-membered heteroaryl;
R¹ are each independently selected from the group consisting of -NH₂, -NHR^{d}, -N(R^{d})₂, -OH, -OR^{d}, -CN, halogen, -COOR^{d}, -OCOR^{d}, -N(R^{d})C(O)(R^{d}), -CONH(R^{d}), -CON(R^{d})₂, -NHSO₂(R^{d}), -SO₂NH(R^{d}), -SO₂N(R^{d})₂, -PO(R^{d})₂, 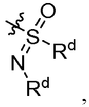 C₁₋₆ alkyl, and 3- to 10-membered heterocycloalkyl, wherein the C₁₋₆ alkyl and 3- to 10-membered heterocycloalkyl are optionally substituted with one or more R^{b};
R³ is selected from the group consisting of halogen, C₁₋₄ alkyl, and halogenated C₁₋₄ alkyl;
R^{a} is selected from the group consisting of hydrogen and C₁₋₄ alkyl.

3. The compound, the stereoisomer thereof, or the pharmaceutically acceptable salt thereof according to claim 1 or 2, being selected from a compound of formula (**I**), a stereoisomer thereof, or a pharmaceutically acceptable salt thereof, 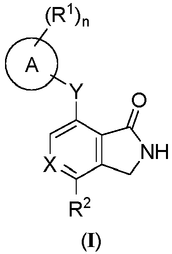
wherein, Y is selected from the group consisting of a bond, -O-, -S-, and -NR^{a}-;
ring A is selected from the group consisting of C₅₋₁₀ aryl and 5- to 10-membered heteroaryl;
R¹ are each independently selected from the group consisting of -NH₂, -NH(C₁₋₄ alkyl), -N(C₁₋₄ alkyl)₂, -OH, -OC₁₋₄ alkyl, -CN, halogen, C₁₋₆ alkyl, and 3- to 10-membered heterocycloalkyl, wherein the C₁₋₆ alkyl and 3- to 10-membered heterocycloalkyl are optionally substituted with one or more R^{b};
R^{b} are each independently selected from the group consisting of -NH₂, -NH(C₁₋₄ alkyl), -N(C₁₋₄ alkyl)₂, -OH, -OC₁₋₄ alkyl, -CN, halogen, 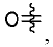 and 3- to 6-membered heterocycloalkyl, wherein the -NH(C₁₋₄ alkyl), -N(C₁₋₄ alkyl)₂, -OC₁₋₄ alkyl, and 3- to 6-membered heterocycloalkyl are optionally substituted with one or more deuterium, halogen, or 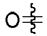 substituents.

4. The compound, the stereoisomer thereof, or the pharmaceutically acceptable salt thereof according to any one of claims 1-3, wherein X is CH; or, X is N.

5. The compound, the stereoisomer thereof, or the pharmaceutically acceptable salt thereof according to any one of claims 1-4, wherein R^{a} is hydrogen.

6. The compound, the stereoisomer thereof, or the pharmaceutically acceptable salt thereof according to any one of claims 1-5, wherein Y is selected from the group consisting of a bond, -O-, -S-, and -NR^{a}-; or, Y is selected from - NR^{a}-; or, Y is -NH-.

7. The compound, the stereoisomer thereof, or the pharmaceutically acceptable salt thereof according to any one of claims 1-6, wherein ring A is selected from the group consisting of C₅₋₈ aryl and 5- to 8-membered heteroaryl;
or, ring A is selected from the group consisting of C₅₋₆ aryl and 5- to 6-membered heteroaryl;
or, ring A is selected from 3- to 10-membered heterocycloalkyl;
or, ring A is selected from the group consisting of phenyl or 5- to 6-membered heteroaryl containing 1 or 2 heteroatoms selected from the group consisting of N, O, and S;
or, ring A is selected from the group consisting of phenyl or 6-membered heteroaryl containing 1 or 2 N atoms;
or, ring A is selected from the group consisting of phenyl, pyridinyl, pyrimidinyl, pyridazinyl, pyrazinyl, thiazolyl, imidazolyl, or oxazolyl;
or, ring A is selected from the group consisting of phenyl or pyridinyl;
or, ring A is pyridinyl.

8. The compound, the stereoisomer thereof, or the pharmaceutically acceptable salt thereof according to any one of claims 1-7, wherein R¹ are each independently selected from the group consisting of -NH₂, -NH(R^{d}), -N(R^{d})₂, halogen, -N(R^{d})C(O)(R^{d}), -NHSO₂(R^{d}), -SO₂NH(R^{d}), PO(R^{d})₂, 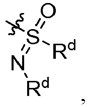 C₁₋₆ alkyl, 5- to 8-membered heteroaryl, and 3- to 10-membered heterocyclyl containing a heteroatom selected from the group consisting of N, O, S, and P, wherein the C₁₋₆ alkyl, 5- to 8-membered heteroaryl, or 3- to 10-membered heterocyclyl containing a heteroatom selected from the group consisting of N, O, S, and P is optionally substituted with one or more R^{b};
or, R¹ are each independently selected from the group consisting of -NH₂, -NH(R^{d}), -N(R^{d})₂, halogen, - N(R^{d})C(O)(R^{d}), -NHSO₂(R^{d}), -SO₂NH(R^{d}), PO(R^{d})₂, 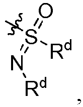 C₁₋₆alkyl, 3- to 10-membered heterocycloalkyl, 5- to 6-membered heteroaryl, and 5- to 10-membered heterocycloalkenyl, wherein the C₁₋₆ alkyl, 3- to 10-membered heterocycloalkyl, 5- to 6-membered heteroaryl, or 5- to 10-membered heterocycloalkenyl is optionally substituted with one or more R^{b};
or, R¹ are each independently selected from the group consisting of -NH₂, -NH(R^{d}), -N(R^{d})₂, F, Cl, Br, - N(R^{d})C(O)(R^{d}), -NHSO₂(R^{d}), -SO₂NH(R^{d}), -PO(R^{d})₂, 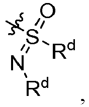 C₁₋₄ alkyl, 5- to 10-membered heterocycloalkyl, and 5- to 8-membered heterocycloalkenyl, wherein the C₁₋₄ alkyl, 5- to 10-membered heterocycloalkyl, or 5- to 8-membered heterocycloalkenyl is optionally substituted with one or more R^{b};
or, R¹ are each independently selected from the group consisting of -NH₂, -NH(R^{d}), -N(R^{d})₂, -N(R^{d})C(O)(R^{d}), - NHSO₂(R^{d}), -SO₂NH(R^{d}), -PO(R^{d})₂, 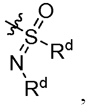 C₁₋₃ alkyl, 5- to 9-membered heterocycloalkyl, and 5- to 6-membered heterocycloalkenyl, wherein the C₁₋₃ alkyl, 5- to 9-membered heterocycloalkyl, or 5- to 6-membered heterocycloalkenyl is optionally substituted with one or more R^{b};
or, R¹ are each independently selected from the group consisting of -NH₂, -NHCH₃, -N(CH₃)₂, F, Cl, Br, methyl, ethyl, *n*-propyl, isopropyl, *n*-butyl, isobutyl, *tert*-butyl, tetrahydropyrrolyl, tetrahydropyranyl, tetrahydrofuranyl, tetrahydrothienyl, morpholinyl, piperidinyl, piperazinyl, tetrahydroimidazolyl, azoxycycloheptyl, azoxyspirononanyl, azoxyspiroheptyl, -N(CH₃)C(O)CH₃, -NHSO₂(CH₃), -SO₂NH(CH₃), -PO(CH₃)₂, 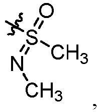 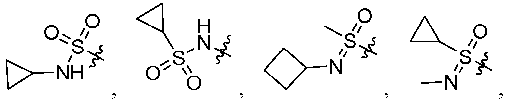 oxazolidinyl, phosphoxycyclohexyl, azoxycyclohexyl, phosphazacyclohexyl, dihydropyridinyl, furanyl, and thiazinyl, wherein the methyl, ethyl, *n*-propyl, isopropyl, *n-*butyl, isobutyl, *tert*-butyl, tetrahydropyrrolyl, tetrahydropyranyl, tetrahydrofuranyl, tetrahydrothienyl, morpholinyl, piperidinyl, piperazinyl, tetrahydroimidazolyl, azoxybicycloheptyl, azoxyspirononanyl, azoxyspiroheptyl, oxazolidinyl, phosphoxycyclohexyl, azoxycyclohexyl, phosphazacyclohexyl, dihydropyridinyl, furanyl, or thiazinyl is optionally substituted with one or more R^{b};
or, R¹ are each independently selected from the group consisting of -NH₂, F, methyl, ethyl, tetrahydropyrrolyl, tetrahydrofuranyl, tetrahydropyranyl, piperidinyl, morpholinyl, tetrahydroimidazolyl, -N(CH₃)C(O)CH₃, azoxycycloheptyl, azoxyspirononanyl, azoxyspiroheptyl, phosphoxycyclohexyl, azoxycyclohexyl, phosphazacyclohexyl, oxazolidinyl, -NHSO₂(CH₃), -SO₂NH(CH₃), -PO(CH₃)₂, 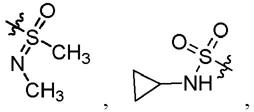 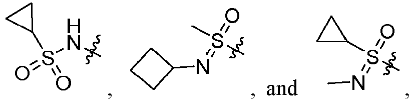 wherein the methyl, ethyl, tetrahydropyrrolyl, tetrahydrofuranyl, tetrahydropyranyl, piperidinyl, morpholinyl, tetrahydroimidazolyl, azoxybicycloheptyl, azoxyspirononanyl, azoxyspiroheptyl, phosphoxycyclohexyl, azoxycyclohexyl, phosphazacyclohexyl, or oxazolidinyl is optionally substituted with 1, 2, or 3 R^{b};
or, R¹ are each independently selected from the group consisting of -NH₂, F, methyl, ethyl, 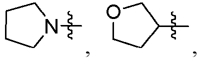 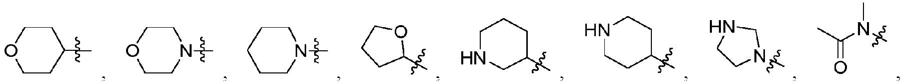 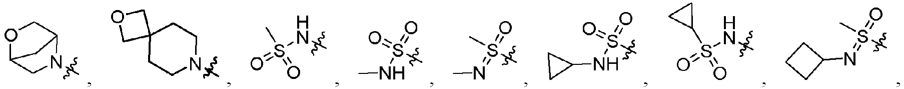 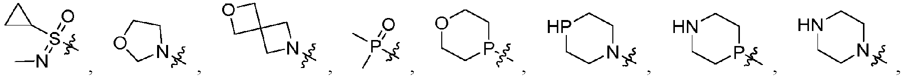 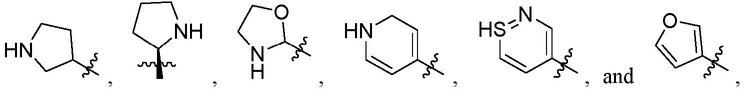 wherein the methyl, ethyl, 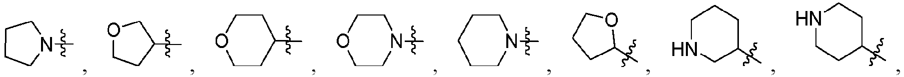 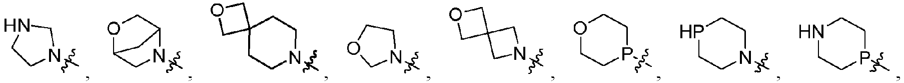 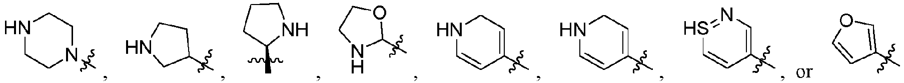 is optionally substituted with 1, 2, or 3 R^{b}.

9. The compound, the stereoisomer thereof, or the pharmaceutically acceptable salt thereof according to any one of claims 1-8, wherein n is selected from the group consisting of 1, 2, and 3; or, n is 2.

10. The compound, the stereoisomer thereof, or the pharmaceutically acceptable salt thereof according to claim 1 or 2, wherein m is selected from the group consisting of 0, 1, and 2; or, m is selected the group consisting of 0 and 1; or, m is 0.

11. The compound, the stereoisomer thereof, or the pharmaceutically acceptable salt thereof according to claim 1 or 2, wherein R³ is selected from the group consisting of halogen, C₁₋₃ alkyl, and halogenated C₁₋₃ alkyl;
or, R³ is selected from the group consisting of F, Cl, Br, and C₁₋₃ alkyl;
or, R³ is selected from the group consisting of F and methyl.

12. The compound, the stereoisomer thereof, or the pharmaceutically acceptable salt thereof according to any one of claims 1-11, wherein R^{b} are each independently selected from the group consisting of deuterium, -OH, C₁₋₄ alkyl, halogen, 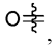 -N(C₁₋₄ alkyl)₂, and 3- to 6-membered heterocycloalkyl, wherein the -N(C₁₋₄ alkyl)₂ and 3- to 6-membered heterocycloalkyl are optionally substituted with one or more deuterium, halogen, or 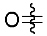 substituents;
or, R^{b} are each independently selected from the group consisting of deuterium, -OH, C₁₋₃ alkyl, F, Cl, Br, 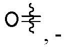 N(C₁₋₄ alkyl)₂, and 5-membered heterocycloalkyl, wherein the -N(C₁₋₄ alkyl)₂ and 5-membered heterocycloalkyl are optionally substituted with one or more deuterium, halogen, or 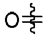 substituents;
or, R^{b} are each independently selected from the group consisting of deuterium, -OH, CH₃, F, 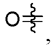 -N(CH₃)₂, - N(CH₃CH₃)₂, -N(CH₃CH₂CH₂)₂, -N(CH₃)CH₂CH₃, -N(CH₃)C(CH₃)₂, tetrahydropyrrolyl, tetrahydrofuranyl, and tetrahydrothienyl, wherein the -N(CH₃)₂, -N(CH₃CH₂)₂, -N(CH₃CH₂CH₂)₂, -N(CH₃)CH₂CH₃, -N(CH₃)C(CH₃)₂, tetrahydropyrrolyl, tetrahydrofuranyl, and tetrahydrothienyl are optionally substituted with one or more deuterium, halogen, or 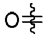 substituents;
or, R^{b} are each independently selected from the group consisting of deuterium, -OH, CH₃, F, 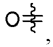 -N(CH₃)₂, - N(CH₃)CH₂CH₃, -N(CH₃)C(CH₃)₂, and tetrahydropyrrolyl, wherein the -N(CH₃)₂, -N(CH₃)CH₂CH₃, and tetrahydropyrrolyl are optionally substituted with one or more deuterium, halogen, or 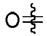 substituents;
or, R^{b} are each independently selected from the group consisting of deuterium, -OH, CH₃, F, 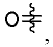 -N(CH₃)₂, - N(CD₃)₂,

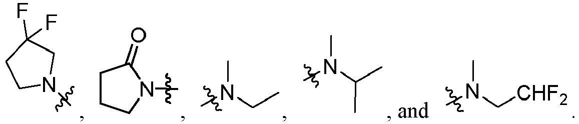

13. The compound, the stereoisomer thereof, or the pharmaceutically acceptable salt thereof according to any one of claims 1-12, wherein one, two, or three rings in the tricyclic rings are aromatic rings;
or, the monocyclic ring in the tricyclic ring to which the 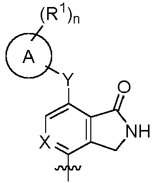 moiety of formula (**I**) or the 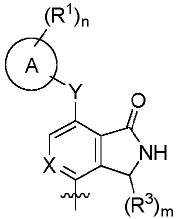 moiety of formula (**II**) is attached is an aromatic ring;
or, the monocyclic ring in the tricyclic ring to which the 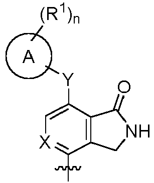 moiety of formula (**I**) or the 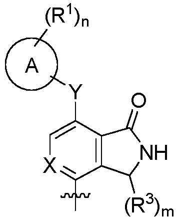 moiety of formula (**II**) is attached is an aromatic ring containing a N atom;
or, any two adjacent rings in the tricyclic ring optionally form a fused, bridged, or spiro ring;
or, the monocyclic ring in the tricyclic ring to which the 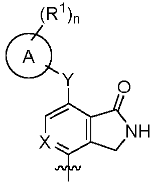 moiety of formula (**I**) or the 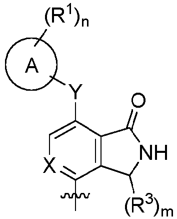 moiety of formula (**II**) is attached and the ring adjacent thereto form a fused ring.

14. The compound, the stereoisomer thereof, or the pharmaceutically acceptable salt thereof according to any one of claims 1-13, wherein R² is selected from the group consisting of 6-membered monocyclic heteroaryl and a 9- to 14-membered saturated, partially saturated, or aromatic tricyclic ring, wherein the tricyclic ring contains 0-6 heteroatoms independently selected from the group consisting of N, O, and S, and the R² is optionally substituted with one or more R^{c};
or, R² is selected from the group consisting of 6-membered monocyclic heteroaryl containing 1 or 2 N atoms and a 9- to 14-membered saturated, partially saturated, or aromatic tricyclic ring, wherein the tricyclic ring contains 1-6 heteroatoms independently selected from the group consisting of N, O, and S, and the R² is optionally substituted with one or more R^{c};
or, R² is selected from the group consisting of 6-membered monocyclic heteroaryl containing 1 or 2 N atoms and a 9- to 14-membered saturated, partially saturated, or aromatic tricyclic ring, wherein the tricyclic ring contains 1-6 heteroatoms independently selected from the group consisting of N and O, and the R² is optionally substituted with one or more R^{c};
or, R² is selected from the group consisting of 6-membered monocyclic heteroaryl containing 1 or 2 N atoms and a 9- to 14-membered partially saturated or aromatic tricyclic ring, wherein the tricyclic ring contains 1-6 heteroatoms independently selected from the group consisting of N and O, and the R² is optionally substituted with one or more R^{c};
or, R² is selected from the group consisting of 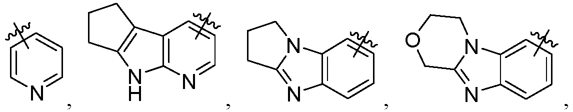 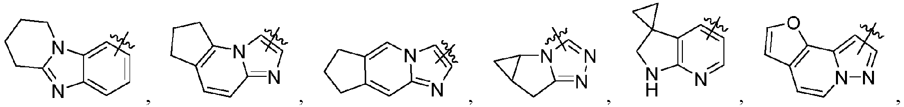 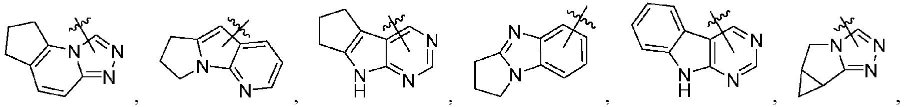 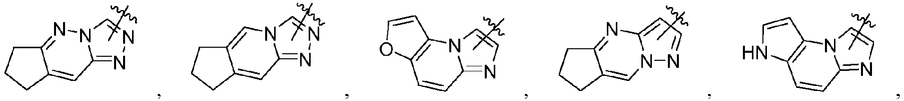 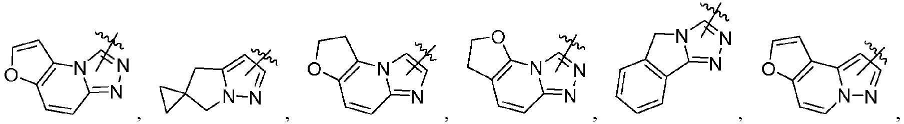 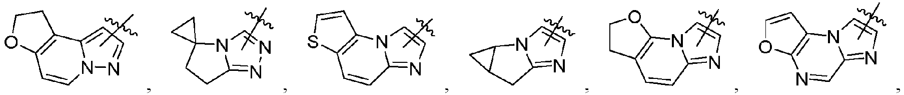 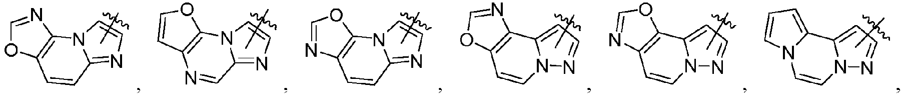 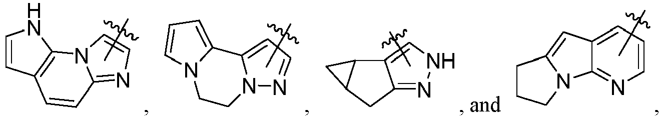 wherein the R² is optionally substituted with one or more R^{c};
or, R² is selected from the group consisting of 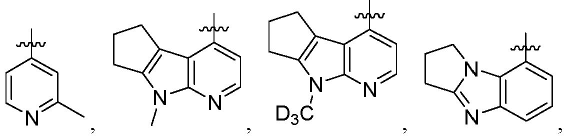 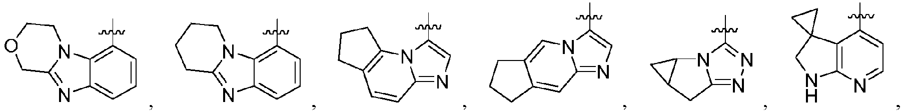 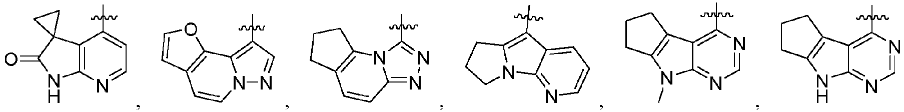 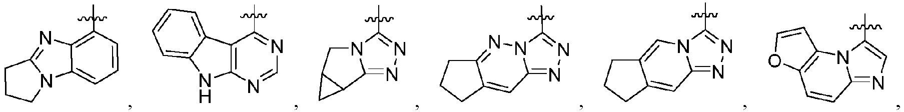 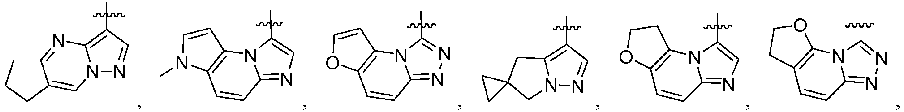 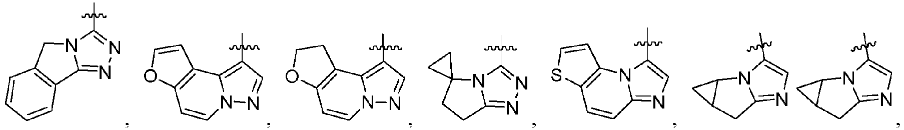 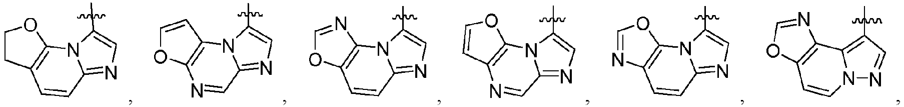 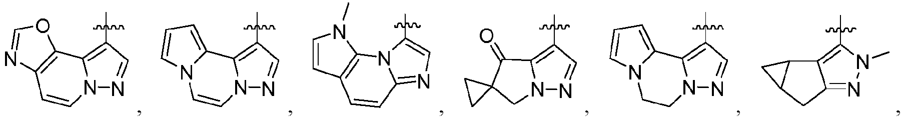 and

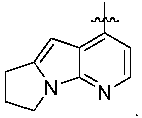

15. The compound, the stereoisomer thereof, or the pharmaceutically acceptable salt thereof according to any one of claims 1-14, wherein R² is selected from a 9- to 14-membered saturated, partially saturated, or aromatic tricyclic ring, wherein the tricyclic ring contains 0-6 heteroatoms independently selected from the group consisting of N, O, and S, and the R² is optionally substituted with one or more R^{c};
or, R² is selected from a 9- to 14-membered saturated, partially saturated, or aromatic tricyclic ring, wherein the tricyclic ring contains 1-6 heteroatoms independently selected from the group consisting of N, O, and S, and the R² is optionally substituted with one or more R^{c};
or, R² is selected from a 9- to 14-membered saturated, partially saturated, or aromatic tricyclic ring, wherein the tricyclic ring contains 1-6 heteroatoms independently selected from the group consisting of N and O, and the R² is optionally substituted with one or more R^{c};
or, R² is selected from a 9- to 14-membered partially saturated or aromatic tricyclic ring, wherein the tricyclic ring contains 1-6 heteroatoms independently selected from the group consisting of N and O, and the R² is optionally substituted with one or more R^{c}.

16. The compound, the stereoisomer thereof, or the pharmaceutically acceptable salt thereof according to any one
of claims 1-15, wherein R^{c} are each independently selected from the group consisting of 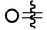 and C₁₋₆ alkyl optionally substituted with one or more deuterium;
or, R^{c} are each independently selected from the group consisting of 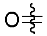 and C₁₋₄ alkyl optionally substituted with one or more deuterium;
or, R^{c} are each independently selected from the group consisting of 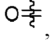 and methyl, ethyl, *n*-propyl, isopropyl, *n*-butyl, isobutyl, or *tert*-butyl optionally substituted with one or more deuterium;
or, R^{c} are each independently selected from the group consisting of 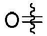 and methyl optionally substituted with one or more deuterium;
or, R^{c} are each independently selected from the group consisting of 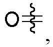 methyl, and -CD₃.

17. The compound, the stereoisomer thereof, or the pharmaceutically acceptable salt thereof according to any one of claims 1-16, wherein R^{d} are each independently selected from the group consisting of C₁₋₄ alkyl and C₃₋₆ cycloalkyl;
or, R^{d} are each independently selected from the group consisting of C₁₋₂ alkyl and C₃₋₄ cycloalkyl;
or, R^{d} are each independently selected from C₁₋₂ alkyl;
or, R^{d} are each independently selected from C₃₋₄ cycloalkyl.

18. The compound, the stereoisomer thereof, or the pharmaceutically acceptable salt thereof according to any one of claims 1-3, being selected from the group consisting of the following compounds of formula (Ia), formula (Ib), formula (Ic), and formula (Id), stereoisomers thereof, or pharmaceutically acceptable salts thereof:

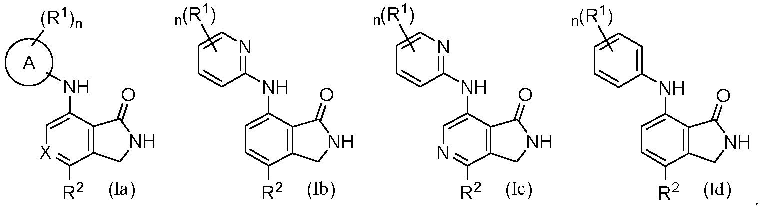

19. The compound, the stereoisomer thereof, or the pharmaceutically acceptable salt thereof according to any one of claims 1-18, being selected from the group consisting of the following compounds, stereoisomers thereof, or pharmaceutically acceptable salts thereof:

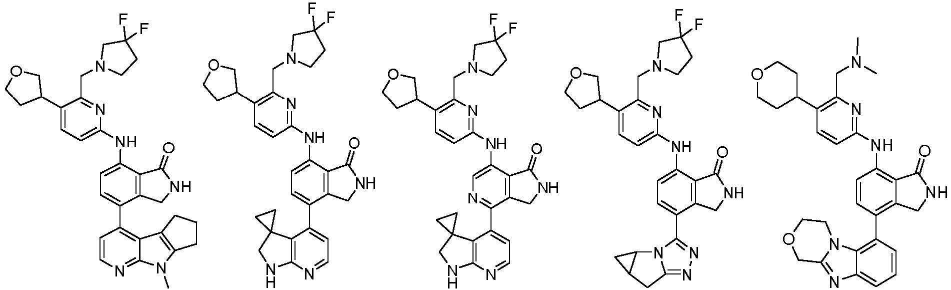

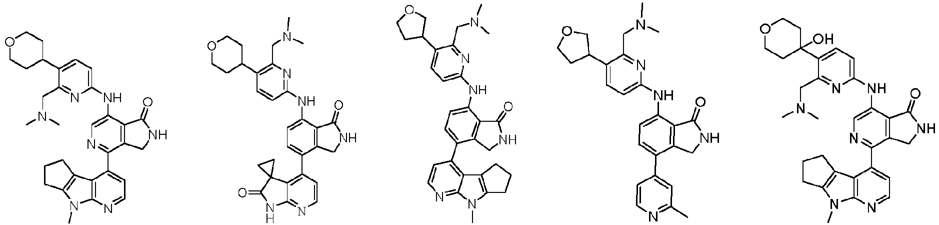

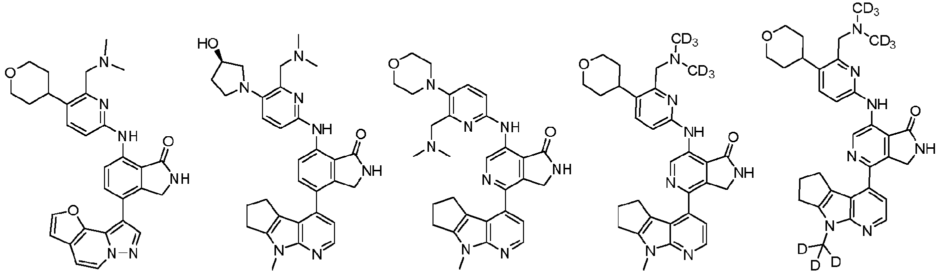

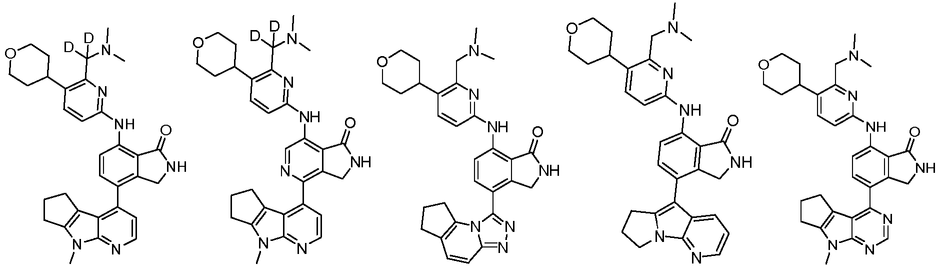

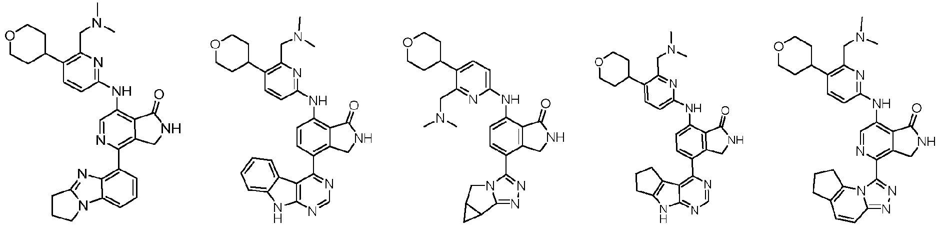

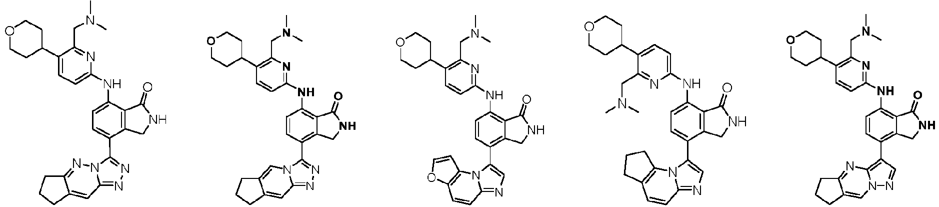

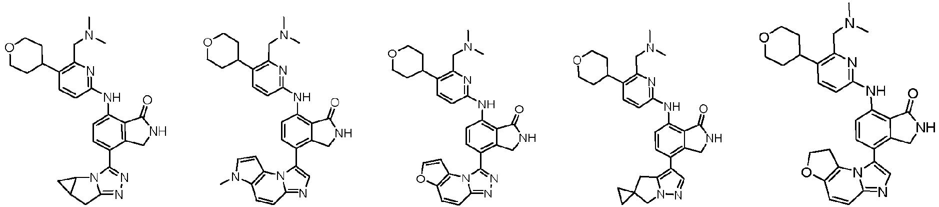

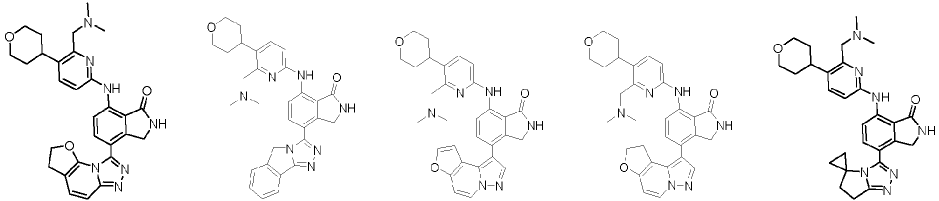

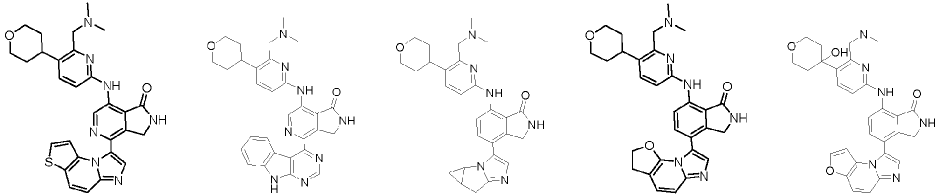

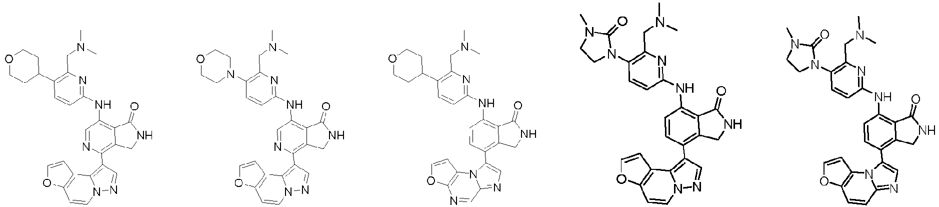

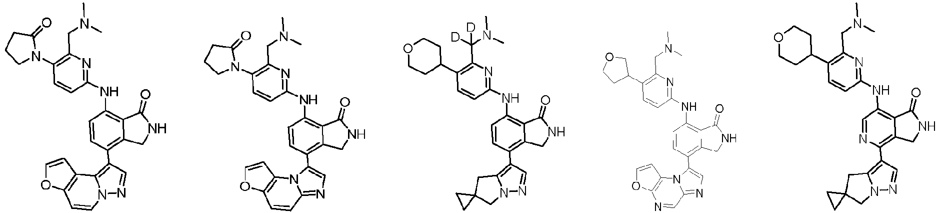

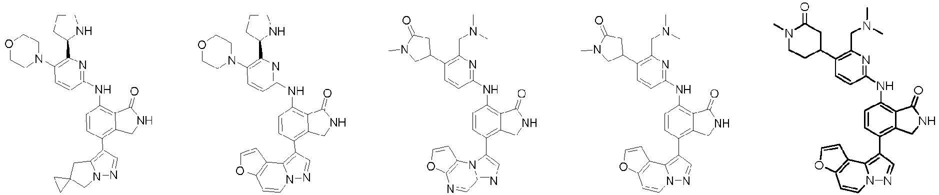

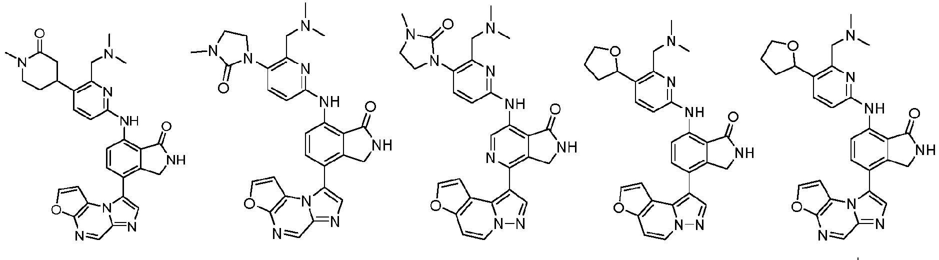

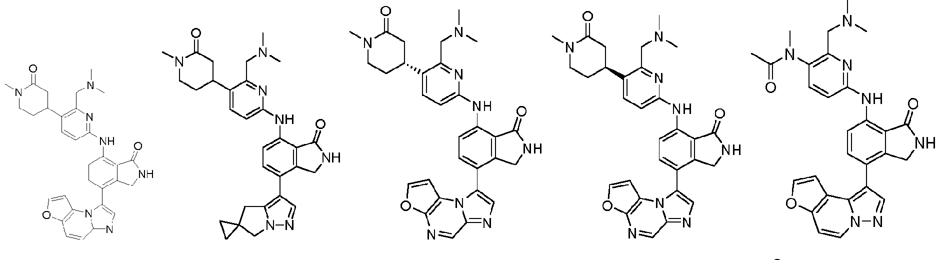

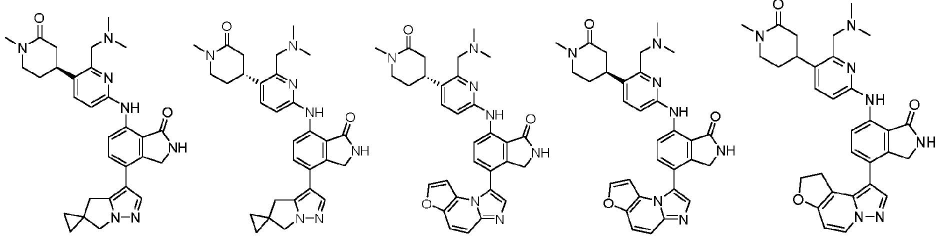

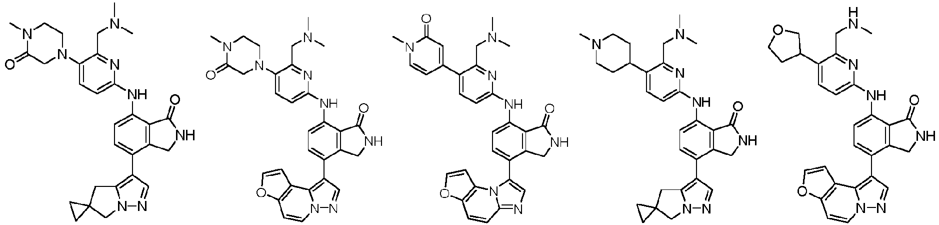

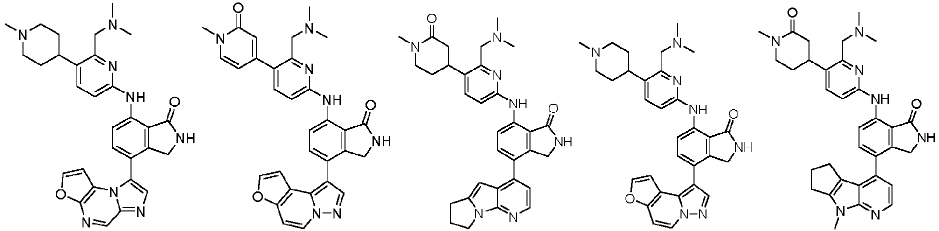

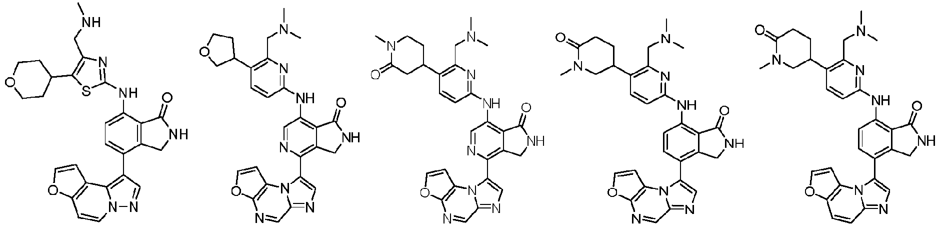

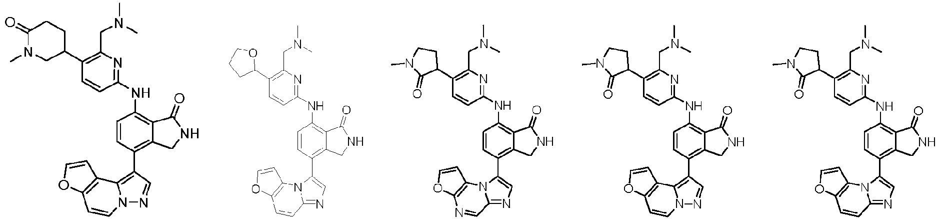

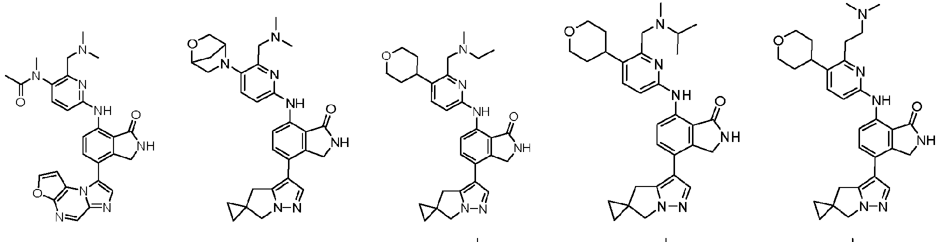

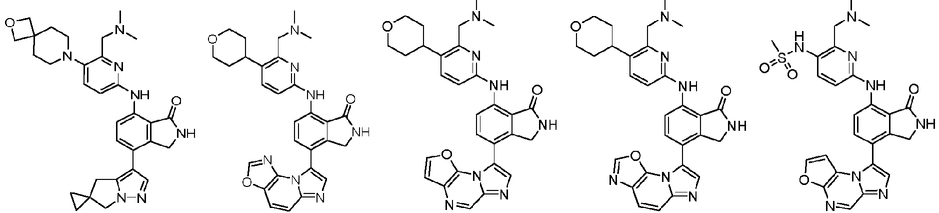

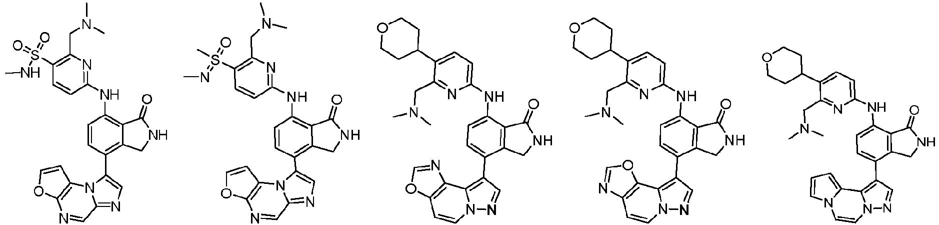

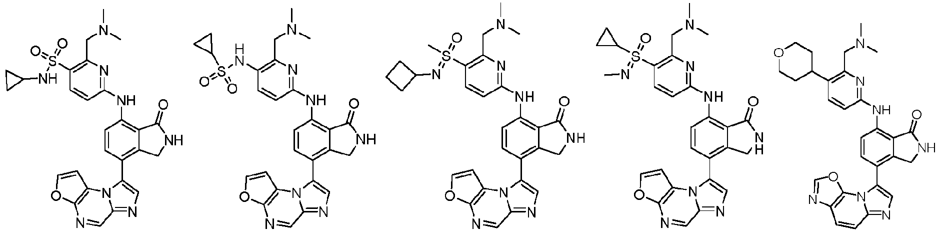

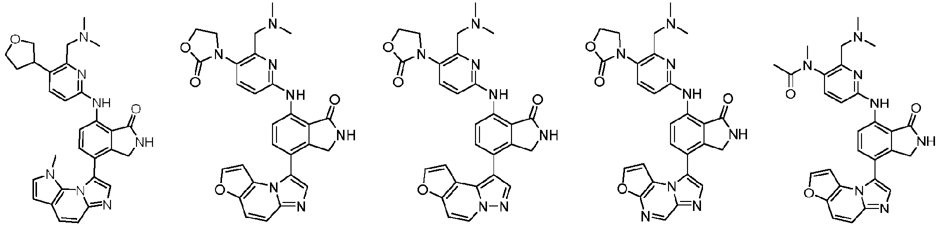

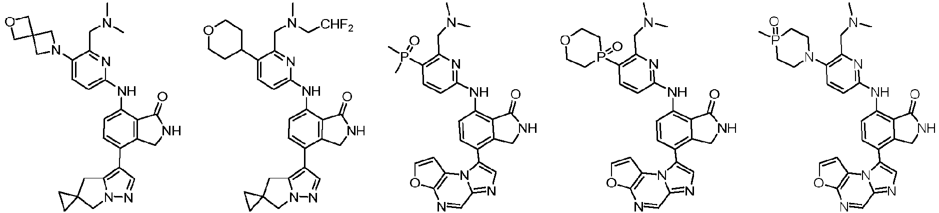

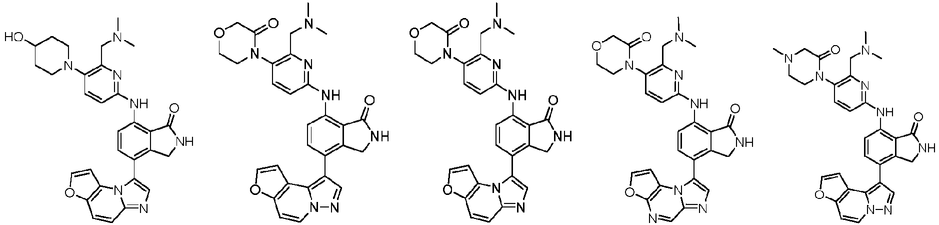

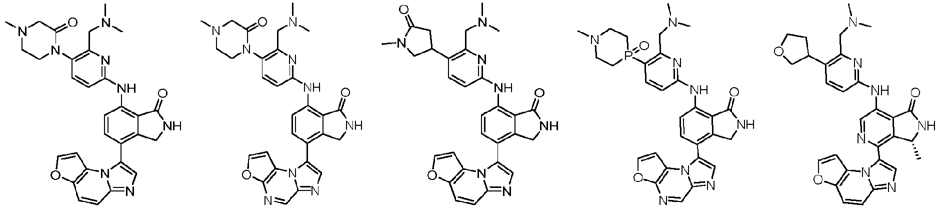

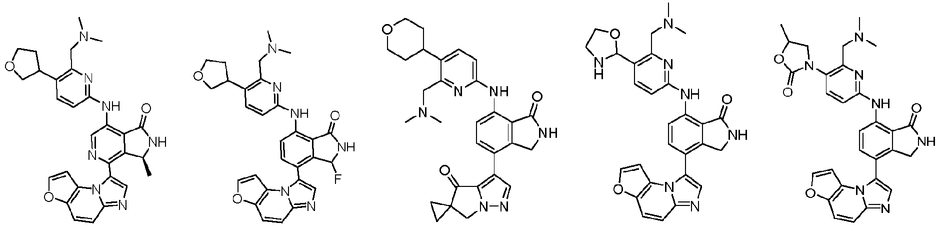

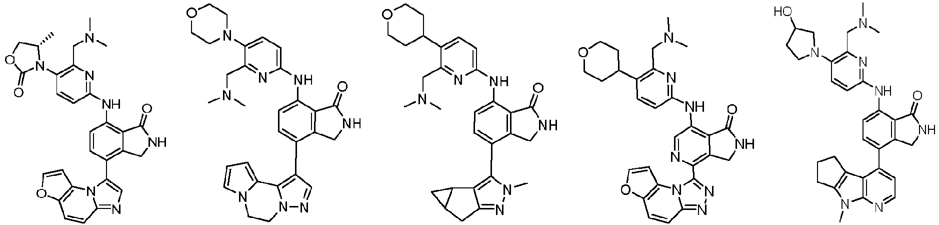

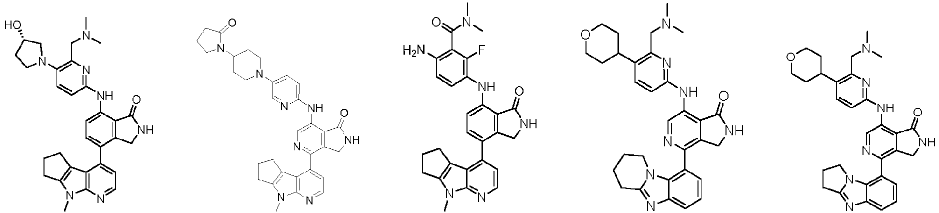

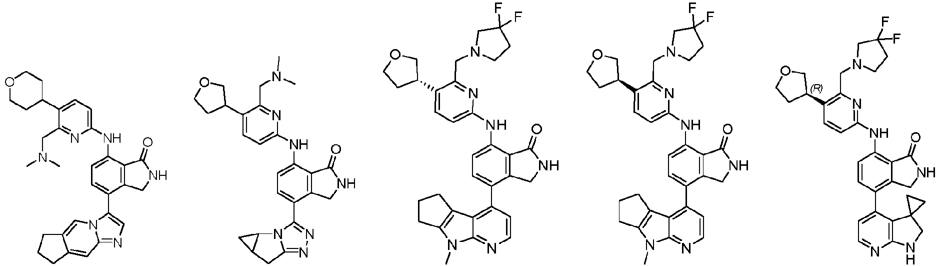

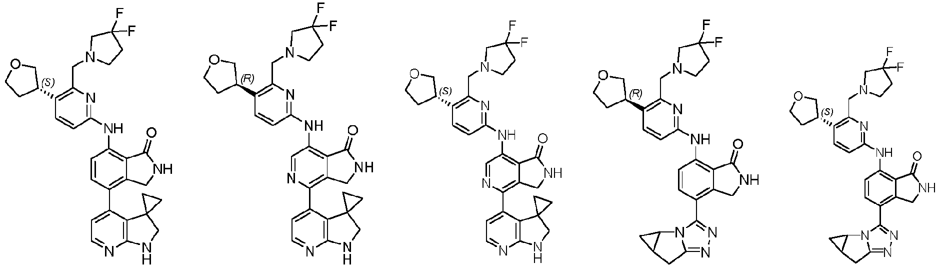

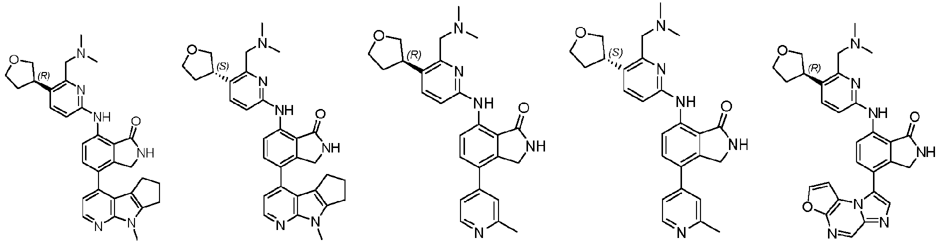

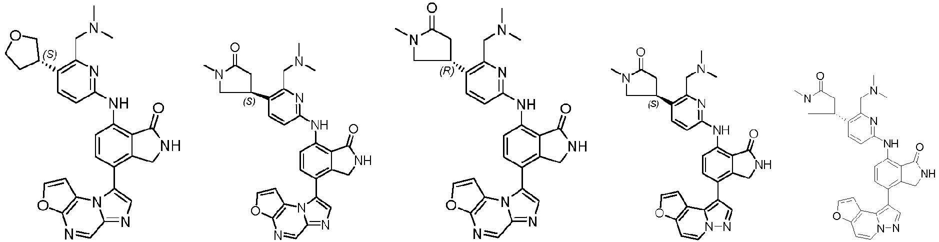

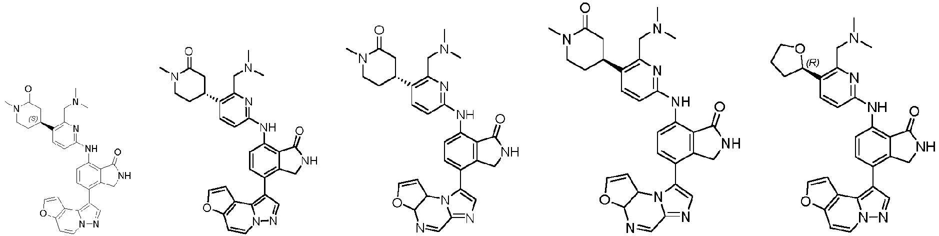

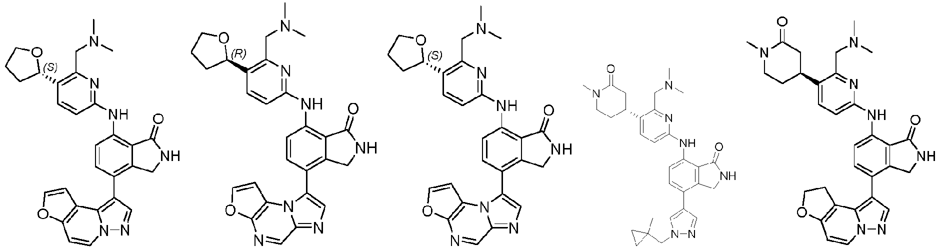

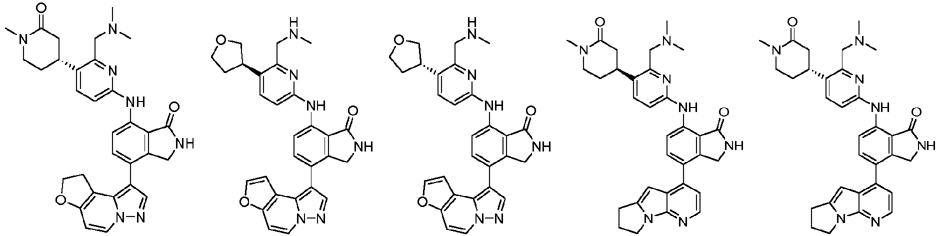

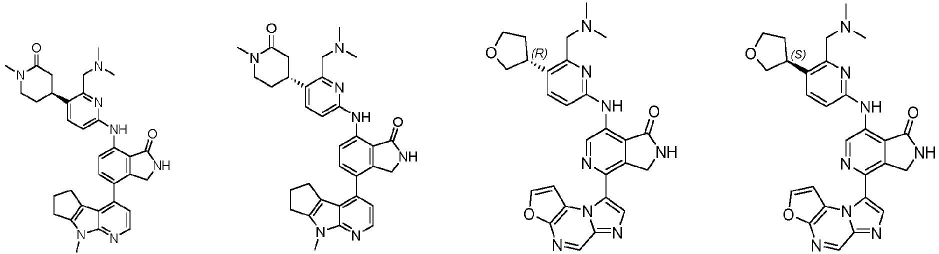

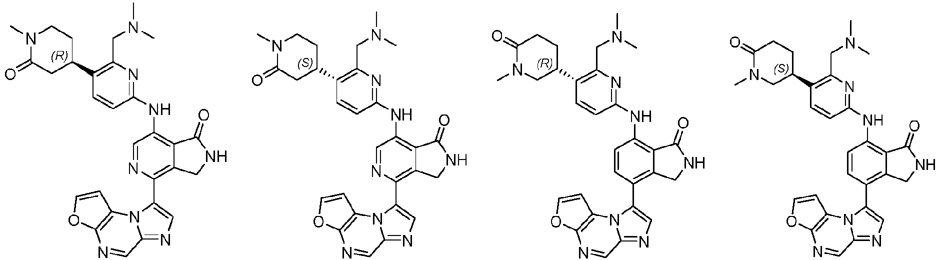

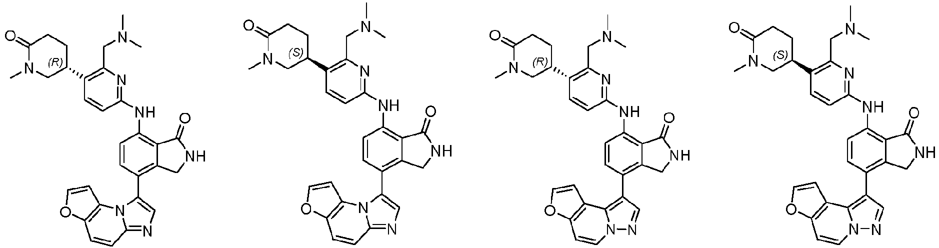

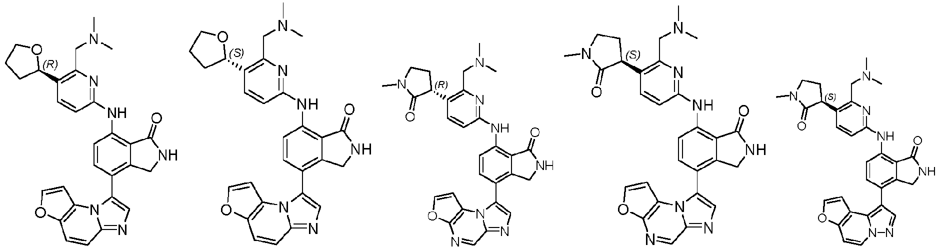

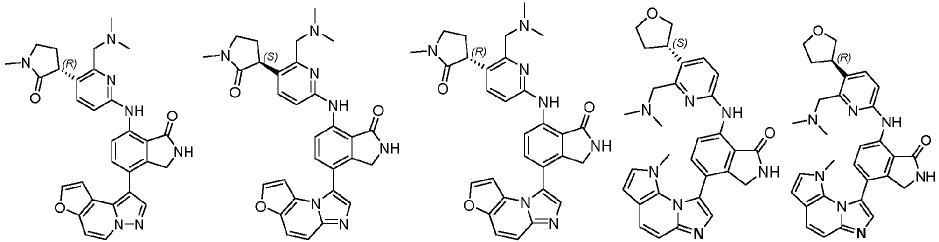

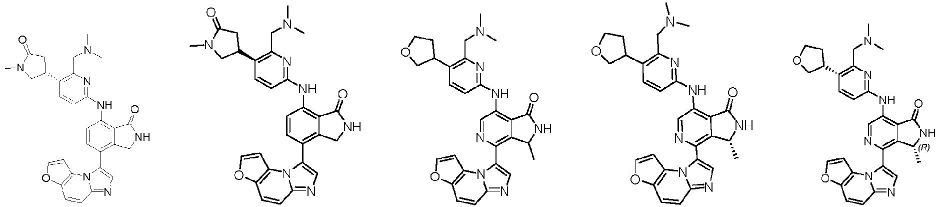

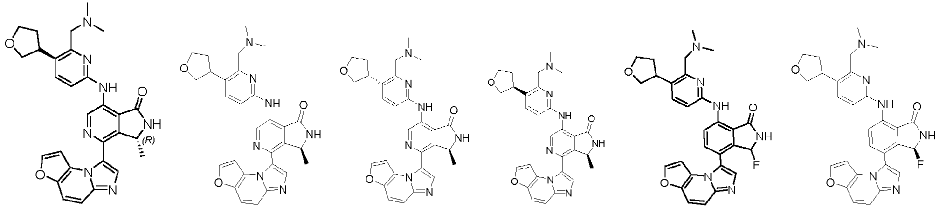

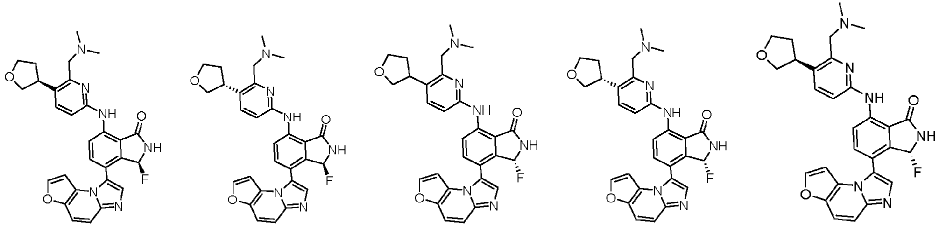

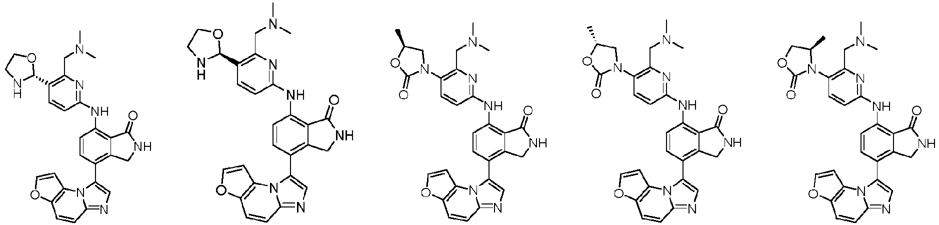

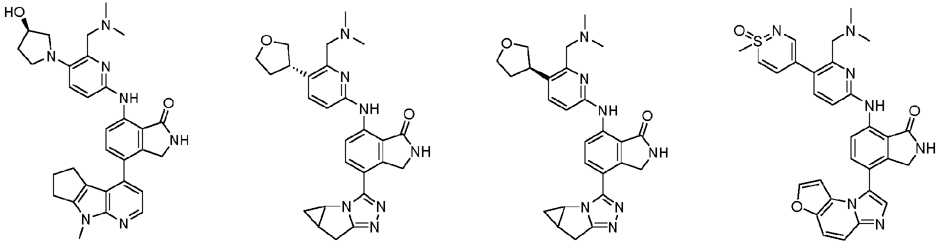

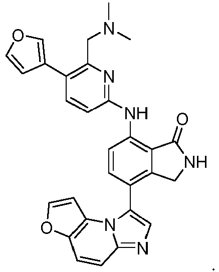

20. Use of the compound, the stereoisomer thereof, or the pharmaceutically acceptable salt thereof according to any one of claims 1-19 for preparing a medicament for treating a disease, wherein optionally, the disease is selected from cancer; optionally, the cancer is selected from the group consisting of a solid tumor, leukemia, and lymphoma.
